# EUROPEAN PATENT SPECIFICATION

(11) **EP 1 924 572 B1**
(45) Date of publication and mention of the grant of the patent: **30.12.2009**
(21) Application number: 06813938.5
(22) Date of filing: 28.08.2006
(51) Int. Cl.: C07D 333/40, C07D 409/04

(54) **REGIOSELECTIVE PROCESS FOR PREPARING BENZIMIDAZOLE THIOPHENES**
REGIOSELEKTIVES VERFAHREN ZUR ZUBEREITUNG VON BENZIMIDAZOL-THIOPHENEN
PROCEDE REGIOSELECTIF DE PREPARATION DE BENZIMIDAZOLE THIOPHENES

(30) Priority: 06.09.2005 US 714301 P
(43) Date of publication of application: 28.05.2008
(73) Proprietor: SmithKline Beecham Corporation, Philadelphia, PA 19101 (US)
(72) Inventor: HORNBERGER, Keith, Research Triangle Park, NC 27709 (US); CHEUNG, Mui, Collegeville, PA 19426 (US); POBANZ, Mark, Andrew, Westfield, IN 46074 (US); EMMITTE, Kyle, Allen, Research Triangle Park, NC 27709 (US); KUNTZ, Kevin, Wayne, Research Triangle Park, NC 27709 (US); BADIANG, Jennifer, Gabriel, Research Triangle Park, NC 27709 (US)
(74) Representative: Learoyd, Stephanie Anne
(86) International application number: PCT/US2006/033793
(87) International publication number: WO 2007/030366

(56) References cited:
- WO-A-01/66525
- WO-A-2004/014899
- WO-A-2005/037827
- WO-A2-2004/041777
- US-A1- 2003 171 360
- TARASOV ET AL: "Reaction of 1-(ortho-Aminophenyl)-1,2,3-triazole-5-thi ols with Cyclizing Reagents" RUSSIAN JOURNAL OF ORGANIC CHEMISTRY, vol. 40, no. 6, 2004, pages 870-873, XP002415193
- CORRAL ET AL: "Reactions of Methyl 3-Hydroxythiophene-2-carboxylate. Part 4. Synthesis of Methyl 5-Azolyl-3-hydroxythiophene-2-carboxylates " JOURNAL OF HETEROCYCLIC CHEMISTRY, HETEROCORPORATION. PROVO, US, vol. 24, September 1987 (1987-09), pages 1301-1303, XP002320501 ISSN: 0022-152X

## Description

### BACKGROUND OF THE INVENTION

The present invention relates to a novel process for preparing benzimidazole thiophene compounds. Benzimidazole thiophene compounds which may be prepared using the processes of the present invention are described in PCT Application WO 2004/014899, filed 4 August 2003, to GlaxoSmithKline, together with pharmaceutical formulations containing the same, therapeutic uses thereof and other processes for their preparation.

### BRIEF SUMMARY OF THE INVENTION

The present invention provides processes for preparing a compound of formula (I): 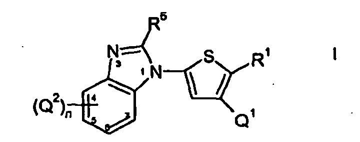 wherein:
- R¹: is selected from the group consisting of H, alkyl, alkenyl, alkynyl, -C(O)R⁷, -CO₂R⁷, -C(O)NR⁷R⁸, -C(O)N(R⁷)OR⁸, -C(O)N(R⁷)-R²-OR⁸, -C(O)N(R⁷)-Ph, -C(O)N(R⁷)-R²-Ph, -C(O)N(R⁷)C(O)R⁸, -C(O)N(R⁷)CO₂R⁸, -C(O)N(R⁷)C(O)NR⁷R⁸, -C(O)N(R⁷)S(O)₂R⁸, -R²-OR⁷, -R²-O-C(O)R⁷, -C(S)R⁷, -C(S)NR⁷R⁸, -C(S)N(R⁷)-Ph, -C(S)N(R⁷)-R²-Ph, -R²-SR⁷, -C(=NR⁷)NR⁷R⁸, -C(=NR⁷)N(R⁸)-Ph, -C(=NR⁷)N(R⁸)-R²-Ph, -R²-NR⁷R⁸, -CN, -OR⁷, -NR⁷R⁸, -N(R⁷)-Ph, -N(R⁷)-R²-Ph, -N(R⁷)-SO₂R⁸ and Het;
- Ph: is phenyl optionally substituted from 1 to 3 times with a substituent selected from the group consisting of halo, alkyl, -OH, -R²-OH, -O-alkyl, -R²-O-alkyl, -NH₂, -N(H)alkyl, -N(alkyl)₂, -CN and -N₃;
- Het: is a 5-7 membered heterocycle having 1, 2, 3 or 4 heteroatoms selected from N, O and S, or a 5-6 membered heteroaryl having 1, 2, 3 or 4 heteroatoms selected from N, O and S, each optionally substituted from 1 to 2 times with a substituent selected from the group consisting of halo, alkyl, oxo, -OH, -R²-OH, -O-alkyl, -R²-O-alkyl, -NH₂, -N(H)alkyl, -N(alkyl)₂, -CN and -N₃;
- Q¹: is a group of formula: -(R²)ₐ-(Y¹)_{b}-(R²)_{c}-R³
- a, b and c: are the same or different and are each independently 0 or 1 and at least one of a or b is 1;
- n: is 0, 1, 2 or 3;
- each Q²: is the same or different and is independently a group of formula: -(R²)ₐₐ-(Y²)_{bb}-(R²)_{cc}-R⁴
or two adjacent Q² groups are selected from the group consisting of alkyl, alkenyl, -OR⁷, -S(O)_{f}R⁷ and -NR⁷R⁸ and together with the carbon atoms to which they are bound, they form a C₅₋₆cycloalkyl, C₅₋₆cycloalkenyl, phenyl, 5-7 membered heterocycle having 1 or 2 heteroatoms selected from N, O and S, or 5-6 membered heteroaryl having 1 or 2 heteroatoms selected from N, O and S;
- aa, bb and cc: are the same or different and are each independently 0 or 1;
- each Y¹ and Y²: is the same or different and is independently selected from the group consisting of -O-, -S(O)_{f}-, -N(R⁷)-, -C(O)-, -OC(O)-, -CO₂-, -C(O)N(R⁷)-, -C(O)N(R⁷)S(O)₂-, -OC(O)N(R⁷)-, -OS(O)₂-, -S(O)₂N(R⁷)-, -S(O)₂N(R⁷)C(O)-, -N(R⁷)S(O)₂-, -N(R⁷)C(O)-, -N(R⁷)CO₂- and -N(R⁷)C(O)N(R⁷)-;
- each R²: is the same or different and is independently selected from the group consisting of alkylene, alkenylene and alkynylene;
- each R³ and R⁴: is the same or different and is each independently selected from the group consisting of H, halo, alkyl, alkenyl, alkynyl, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii): 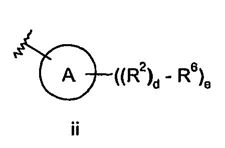 wherein:
Ring A is selected from the group consisting of C₅₋₁₀cycloalkyl, C₅₋₁₀cycloalkenyl, aryl, 5-10 membered heterocycle having 1, 2 or 3 heteroatoms selected from N, O and S and 5-10 membered heteroaryl having 1, 2 or 3 heteroatoms selected from N, O and S;
each d is 0 or 1;
each e is 0, 1, 2, 3 or 4;
each R⁶ is the same or different and is independently selected from the group consisting of H, halo, alkyl, alkenyl, alkynyl, cycloalkyl, cycloalkenyl, Ph, Het, -CH(OH)-R²-OH, -C(O)R⁷, -CO₂R⁷, -CO₂-R₂-Ph, -CO₂-R²-Het, -C(O)NR⁷R⁸, -C(O)N(R⁷)C(O)R⁷, -C(O)N(R⁷)CO₂R⁷, -C(O)N(R⁷)C(O)NR⁷R⁸, -C(O)N(R⁷)S(O)₂R⁷, -C(S)R⁷, -C(S)NR⁷R⁸, -C(=NR⁷)R⁸, -C(=NR⁷)NR⁷R⁸, -CR⁷=N-OR⁸, =O, -OR⁷, -OC(O)R⁷, -OC(O)Ph, -OC(O)Het, -OC(O)NR⁷R⁸, -O-R²-S(O)₂R⁷, -S(O)_{f}R⁷, -S(O)₂NR⁷R⁸, -S(O)₂Ph, -S(O)₂Het, -NR⁷R⁸, -N(R⁷)C(O)R⁸, -N(R⁷)CO₂R⁸, -N(R⁷)-R²-CO₂R⁸, -N(R⁷)C(O)NR⁷R⁸, -N(R⁷)-R²-C(O)NR⁷R⁸, -N(R⁷)C(O)Ph, -N(R⁷)C(O)Het, -N(R⁷)Ph, -N(R⁷)Het, -N(R⁷)C(O)NR⁷-R²-NR⁷R⁸, -N(R⁷)C(O)N(R⁷)Ph, -N(R⁷)C(O)N(R⁷)Het, -N(R⁷)C(O)N(R⁷)-R²-Het, -N(R⁷)S(O)₂R⁸, -N(R⁷)-R²-S(O)²R⁸, -NO₂, -CN and -N₃; wherein when Q¹ is defined where b is 1 and c is 0, R³ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN or -N₃;
wherein when Q² is defined where bb is 1 and cc is 0, R⁴ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂-NR⁷R⁸, -NR⁷R⁸, -N(R⁷)C(O)R⁸, -N(R⁷)S(O)₂R⁸, -NO₂, -CN or -N₃;
- R⁵: is selected H.
- f: is 0, 1 or 2; and
- each R⁷ and each R⁸: are the same or different and are each independently selected from the group consisting of H, alkyl, alkenyl, alkynyl, cycloalkyl and cycloalkenyl;
or a pharmaceutically acceptable salt or solvate thereof.

According to the first aspect the present invention provides a process for preparing compounds of formula (I) above or a pharmaceutically acceptable salt or solvate thereof, comprising the the steps of:
a) cyclizing a compound of formula (VIII): 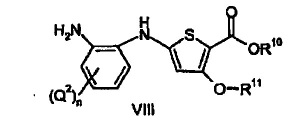 wherein
   R¹⁰ is selected from alkyl, alkenyl, alkynyl, cycloalkyl, cycloalkenyl and suitable carboxylic acid protecting groups; and
   R¹¹ is H or a triisopropylsilyl protecting group;
   and optionally removing the triisopropylsilyl protecting group to prepare a compound of formula (I-A): 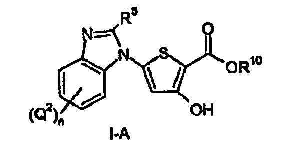
b) optionally converting the compound of formula (I-A) to a pharmaceutically acceptable salt or solvate thereof; and
c) optionally converting the compound of formula (I-A) or a pharmaceutically acceptable salt or solvate thereof to a different compound of formula (I) or a pharmaceutically acceptable salt or solvate thereof.
   According to a second aspect the present invention provides a process for preparing a compound of formula (VIII): 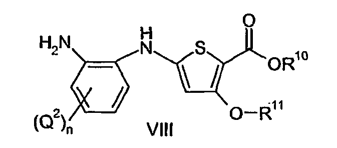 wherein all variables are as defined above,
   comprising reducing a compound of formula (VII): 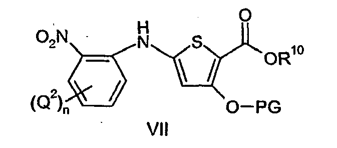 wherein PG is a triisopropylsilyl protecting group.
   According to a third aspect, the present invention provides a process for preparing a compound of formula (VII): 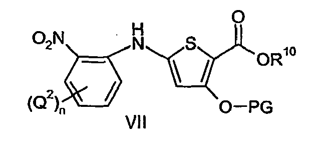 wherein all variables are as defined above, comprising reacting a compound of formula (V) with a compound of formula (VI):

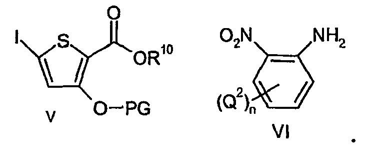

According to a fourth aspect, the present invention provides a process for preparing a compound of formula (I) above wherein n is 1, 2 or 3 and all other variables are as defined above; or a pharmaceutical acceptable salt or solvate thereof. The process comprises cyclizing a compound of formula (XXIII): 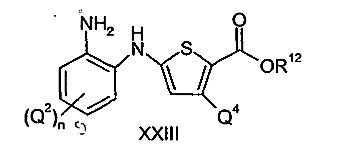 wherein:
Q⁴ is a group of formula - O - (R²)_{c} - R^{3a}, -O - Si(alkyl)₃, or -O - (R²)_{c} - Si(alkyl)₃;
   wherein c is 1; and
   R^{3a} is a group of formula (iii): 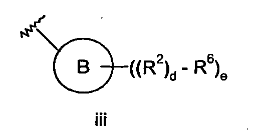 wherein Ring B is phenyl or 5-6 membered heteroaryl containing 1, 2 or 3 heteroatoms selected from N, O and S; and
R¹² is methyl;
   to prepare a compound of formula (I-G):

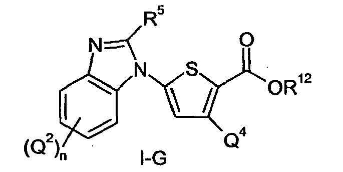

b) optionally converting the compound of formula (I-G) to a pharmaceutically acceptable salt or solvate thereof; and
c) optionally converting the compound of formula (I-G) or a pharmaceutically acceptable salt or solvate thereof to a different compound of formula (I) or a pharmaceutically acceptable salt or solvate thereof.

According to a fifth aspect, the present invention provides a compound of formula (XXIII): 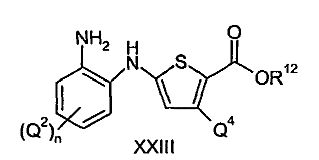 wherein all variables are as defined above.

According to a sixth aspect, the present invention provides a process for preparing a compound of formula (XXIII) above. The process comprises reducing a compound of formula (XXII):

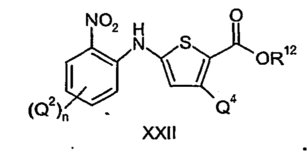

According to a seventh aspect, the present invention provides a compound of formula (XXII): 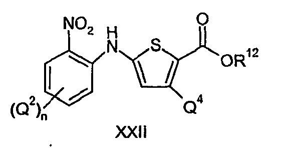 wherein all variables are as defined above.

According to an eighth aspect, the present invention provides a process for preparing a compound of formula (XXII) above. The process comprises coupling a compound of formula (XX) with a compound of formula (XXI): 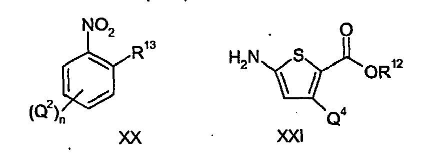 wherein R¹³ is halide or is O-triflate.

According to a ninth aspect, the present invention provides a process for preparing a compound of formula (I) above wherein n is 1, 2 or 3 and all other variables are as defined above; or a pharmaceutically acceptable salt or solvate thereof. The process comprises cyclizing a compound of formula (XXIII-A): 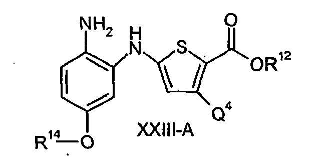 wherein R¹⁴ is a suitable phenol protecting group.

According to a tenth aspect, the present invention provides a process for preparing a compound of formula (I) above, wherein n is 1, 2 or 3 and all other variables are as defined above; or a pharmaceutically acceptable salt or solvate thereof, The process comprises cyclizing a compound of formula (XXIII-B): 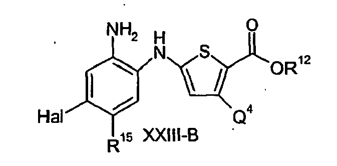 wherein Hal is Cl, Br or I; and
R¹⁵ is H or -O-R¹⁴_{.}

In another aspect, the present invention provides a process for preparing a compound of formula (I) wherein n is 1, 2 or 3 and all other variables are as defined above; or a pharmaceutically acceptable salt or solvate thereof. The process comprises cyclizing a compound of formula (XXXII):

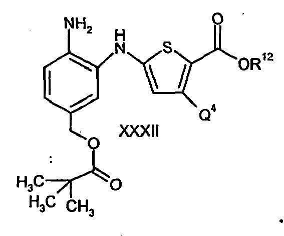

### DETAILED DESCRIPTION OF THE INVENTION

As used herein, "a compound of formula (I)" means a compound of formula (I) or a pharmaceutically acceptable salt or solvate thereof. Similarly, with respect to isolatable intermediates such as for example, compounds of formula (III) and (VIII) the phrase "a compound of formula (*number*)" means a compound having that formula and pharmaceutically acceptable salts and solvates thereof.

As used herein, the terms "alkyl" (and "alkylene") refer to straight or branched hydrocarbon chains containing from 1 to 8 carbon atoms. Examples of "alkyl" as used herein include, but are not limited to, methyl, ethyl, n-propyl, n-butyl, n-pentyl, isobutyl, isopropyl, and tert-butyl. Examples of "alkylene" as used herein include, but are not limited to, methylene, ethylene, propylene, butylene, and isobutylene. "Alkyl" also includes substituted alkyl. The alkyl groups may be optionally substituted one or more times with a halogen. Thus, the term "alkyl" includes trifluoromethyl and trifluoroethyl, among other halogenated alkyls.

As used herein, the term "alkenyl" refers to straight or branched hydrocarbon chains containing from 2 to 8 carbon atoms (unless a different number of atoms is specified) and at least one and up to three carbon-carbon double bonds. Examples of "alkenyl" as used herein include, but are not limited to ethenyl and propenyl. "Alkenyl" also includes substituted alkenyl. The alkenyl groups may optionally be substituted one or more times with a halogen.

As used herein, the term "alkynyl" refers to straight or branched hydrocarbon chains containing from 2 to 8 carbon atoms (unless a different number of atoms is specified) and at least one and up to three carbon-carbon triple bonds. Examples of "alkynyl" as used herein include, but are not limited to ethynyl and propynyl. "Alkynyl" also includes substituted alkynyl. The alkynyl groups may optionally be substituted one or more times with a halogen. As used herein, the term "cycloalkyl" refers to a non-aromatic monocyclic carbocyclic ring having from 3 to 8 carbon atoms (unless a different number of atoms is specified) and no carbon-carbon double bonds. "Cycloalkyl" includes by way of example cyclopropyl, cyclobutyl, cyclopentyl, cyclohexyl, cycloheptyl and cyclooctyl. "Cycloalkyl" also includes substituted cycloalkyl. The cycloalkyl may optionally be substituted on any available carbon with one or more substituents selected from the group consisting of halo, C₁₋₃alkyl (including haloalkyl, e.g., perfluoroalkyl), -OH, -O-C₁₋₃alkyl, -NH₂, -NH(C₁₋₃alkyl) -N(C₁₋₃alkyl)₂, -CN and -N₃. Preferred cycloalkyl groups include C₃₋₆cycloalkyl and substituted C₃₋₆cycloalkyl.

As used herein, the term "cycloalkenyl" refers to a non-aromatic monocyclic carbocyclic ring having from 3 to 8 carbon atoms (unless a different number of atoms is specified) and up to 3 carbon-carbon double bonds. "Cycloalkenyl" includes by way of example cyclobutenyl, cyclopentenyl and cyclohexenyl. "Cycloalkenyl" also includes substituted cycloalkenyl. The cycloalkenyl may optionally be substituted on any available carbon with one or more substituents selected from the group consisting of halo, C₁₋₃alkyl (including haloalkyl, e.g., perfluoroalkyl), -OH, -O-C₁₋₃alkyl, -NH₂, -NH(C₁₋₃alkyl); -N(C₁₋₃alkyl)₂, -CN and -N₃.

The term "halo" or "halogen" refers to fluorine, chlorine, bromine and iodine.

The term "oxo" as used herein refers to the group =O attached directly to a carbon atom of a hydrocarbon ring (i.e., cycloalkenyl, aryl, heterocycle or heteroaryl ring) as well as -N-oxides, sulfones and sulfoxides wherein the N or S are atoms of a heterocyclic or heteroaryl ring.

The term "aryl" refers to monocyclic carbocyclic groups and fused bicyclic carbocyclic groups having from 6 to 13 carbon atoms (unless a different number of atoms is specified) and having at least one aromatic ring. Examples of particular aryl groups include but are not limited to phenyl and naphthyl. One particular aryl group according to the invention is phenyl.

The terms "heterocycle" and "heterocyclic" refer to monocyclic saturated or unsaturated non-aromatic groups and fused bicyclic saturated or unsaturated non-aromatic groups, having the specified number of members and containing 1, 2, 3 or 4 heteroatoms selected from N, O and S (unless a different number of heteroatoms is specified). Examples of particular heterocyclic groups include but are not limited to tetrahydrofuran, dihydropyran, tetrahydropyran, pyran, tetrahydropyran, thietane, 1,4-dioxane, 1,3-dioxane, 1,3-dioxalane, piperidine, piperazine, tetrahydropyrimidine, pyrrolidine, morpholine, thiomorpholine, thiazolidine, oxazolidine, tetrahydrothiopyran, tetrahydrothiophene, and the like.

The term "heteroaryl" refers to aromatic monocyclic groups and fused bicyclic groups wherein at least one ring is aromatic, having the specified number of members and containing 1, 2, 3, or 4 heteroatoms selected from N, O and S (unless a different number of heteroatoms is specified). Examples of particular heteroaryl groups include but are not limited to furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, and indazole. The term "members" (and variants thereof e.g., "membered") in the context of heterocyclic and heteroaryl groups refers to the total atoms, carbon and heteroatoms N, O and/or S, which form the ring. Thus, an example of a 6-membered heterocyclic ring is piperidine and an example of a 6-membered heteroaryl ring is pyridine.

As used herein, the term "optionally" means that the subsequently described event(s) may or may not occur, and includes both event(s) that occur and events that do not occur.

The present invention provides a new process for preparing compounds of formula (I): 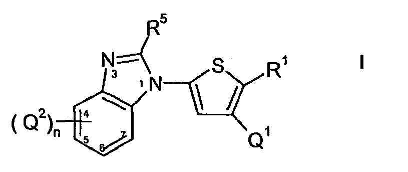 wherein:
- R¹: is selected from the group consisting of H, alkyl, alkenyl, alkynyl, -C(O)R⁷, -CO₂R⁷, -C(O)NR⁷R⁸, -C(O)N(R⁷)OR⁸, -C(O)N(R⁷)-R²-OR⁸, -C(O)N(R⁷)-Ph, -C(O)N(R⁷)-R²-Ph, -C(O)N(R⁷)C(O)R⁸, -C(O)N(R⁷)CO₂R⁸, -C(O)N(R⁷)C(O)NR⁷R⁸, -C(O)N(R⁷)S(O)₂R⁸, -R²-OR⁷, -R²-O-C(O)R⁷, -C(S)R⁷, -C(S)NR⁷R⁸, -C(S)N(R⁷)-Ph, -C(S)N(R⁷)-R²-Ph, -R²-SR⁷, -C(=NR⁷)NR⁷R⁸, -C(=NR⁷)N(R⁸)-Ph, -C(=NR⁷)N(R⁸)-R²-Ph, -R²-NR⁷R⁸, -CN, -OR⁷, -NR⁷R⁸, N(R⁷)-Ph, -N(R⁷)-R²-Ph, -N(R⁷)-SO₂R⁸ and Het;
- Ph: is phenyl optionally substituted from 1 to 3 times with a substituent selected from the group consisting of halo, alkyl, -OH, -R²-OH, -O-alkyl, -R²-O-alkyl, -NH₂, -N(H)alkyl, -N(alkyl)₂, -CN and -N₃;
- Het i: s a 5-7 membered heterocycle having 1, 2, 3 or 4 heteroatoms selected from N, O and S, or a 5-6 membered heteroaryl having 1, 2, 3 or 4 heteroatoms selected from N, O and S, each optionally substituted from 1 to 2 times with a substituent selected from the group consisting of halo, alkyl, oxo, -OH, -R²-OH, -O-alkyl, -R²-O-alkyl, -NH₂, -N(H)alkyl, -N(alkyl)₂, -CN and -N₃;
- Q¹: is a group of formula: -(R²)ₐ-(Y¹)_{b}-(R²)_{c}-R³
- a, b and c: are the same or different and are each independently 0 or 1 and at least one of a or b is 1;
- n: is 0, 1, 2 or 3;
- each Q²: is the same or different and is independently a group of formula: -(R²)ₐₐ-(Y²)_{bb}-(R²)_{cc}-R⁴
or two adjacent Q² groups are selected from the group consisting of alkyl, alkenyl, -OR⁷, -S(O)_{f}R⁷ and -NR⁷R⁸ and together with the carbon atoms to which they are bound, they form a C₅₋₆cycloalkyl, C₅₋₆cycloalkenyl, phenyl, 5-7 membered heterocycle having 1 or 2 heteroatoms selected from N, O and S, or 5-6 membered heteroaryl having 1 or 2 heteroatoms selected from N, O and S;
- aa, bb and cc: are the same or different and are each independently 0 or 1;
- each Y¹ and Y²: is the same or different and is independently selected from the group consisting of -O-, -S(O)_{f}-, -N(R⁷)-, -C(O)-, -OC(O)-, -CO₂-, -C(O)N(R⁷)-, -C(O)N(R⁷)S(O)₂-, -OC(O)N(R⁷)-, -OS(O)₂-, -S(O)₂N(R⁷)-, -S(O)₂N(R⁷)C(O)-, -N(R⁷)S(O)₂-, -N(R⁷)C(O)-, -N(R⁷)CO₂- and -N(R⁷)C(O)N(R⁷)-;
- each R²: is the same or different and is independently selected from the group consisting of alkylene, alkenylene and alkynylene;
- each R³ and R⁴: is the same or different and is each independently selected from the group consisting of H, halo, alkyl, alkenyl, alkynyl, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii): 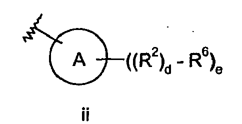 wherein:
Ring A is selected from the group consisting of C₅₋₁₀cycloalkyl, C₅₋₁₀cycyloalkenyl, aryl, 5-10 membered heterocycle having 1, 2 or 3 heteroatoms selected from N, O and S and 5-10 membered heteroaryl having 1, 2 or 3 heteroatoms selected from N, O and S;
each d is 0 or 1;
each e is 0, 1, 2, 3 or 4;
each R⁶ is the same or different and is independently selected from the group consisting of H, halo, alkyl, alkenyl, alkynyl, cycloalkyl, cycloalkenyl, Ph, Het, -CH(OH)-R²-OH, -C(O)R⁷, -CO₂R⁷, -CO₂-R₂-Ph, -CO₂-R²-Het, -C(O)NR⁷R⁸, -C(O)N(R⁷)C(O)R⁷, -C(O)N(R⁷)CO₂R⁷, -C(O)N(R⁷)C(O)NR⁷R⁸, -C(O)N(R⁷)S(O)₂R⁷, -C(S)R⁷, -C(S)NR⁷R⁸, -C(=NR⁷)R⁸, -C(=NR⁷)NR⁷R⁸, -CR⁷=N-OR⁸, =O, -OR⁷, -OC(O)R⁷, -OC(O)Ph, -OC(O)Het, -OC(O)NR⁷R⁸, -O-R²-S(O)₂R⁷, -S(O)_{f}R⁷, -S(O)₂NR⁷R⁸, -S(O)₂Ph, -S(O)₂Het, -NR⁷R⁸, -N(R⁷)C(O)R⁸, -N(R⁷)CO₂R⁸, -N(R⁷)-R²-CO₂R⁸, -N(R⁷)C(O)NR⁷R⁸, -N(R⁷)-R²-C(O)NR⁷R⁸, -N(R⁷)C(O)Ph, -N(R⁷)C(O)Het, -N(R⁷)Ph, -N(R⁷)Het, -N(R⁷)C(O)NR⁷-R²-NR⁷R⁸, -N(R⁷)C(O)N(R⁷)Ph, -N(R⁷)C(O)N(R⁷)Het, -N(R⁷)C(O)N(R⁷)-R²-Het, -N(R⁷)S(O)₂R⁸, -N(R⁷)-R²-S(O)₂R⁸, -NO₂, -CN and -N₃;
- R⁵: is H,
- f: is 0, 1 or 2; and
- each R⁷ and each R⁸: are the same or different and are each independently selected from the group consisting of H, alkyl, alkenyl, alkynyl, cycloalkyl and cycloalkenyl;
and pharmaceutically acceptable salts and solvates thereof.

In one embodiment, the compounds of formula (I) are defined wherein R¹ is selected from the group consisting of alkyl, alkenyl, alkynyl, -C(O)R⁷, -CO₂R⁷, -C(O)NR⁷R⁸, -C(O)N(R⁷)-R²-OR⁸, -R²-OR⁷, -C(S)NR⁷R⁸, -C(=NR⁷)NR⁷R⁸, -CN, and Het, or any subset thereof. In one embodiment, the compounds of formula (I) are defined wherein R¹ is selected from the group consisting of -C(O)R⁷, -CO₂R⁷, -C(S)NR⁷R⁸, Het, and -C(O)NR⁷R⁸, or any subset thereof. In one embodiment, the compounds of formula (I) are defined wherein R¹ is selected from the group consisting of -C(O)R⁷, -CO₂R⁷ and -C(O)NR⁷R⁸, or any subset thereof. In one particular embodiment, R¹ is selected from the group consisting of -CO₂R⁷ and -C(O)NR⁷R⁸, or any subset thereof. In one embodiment, R¹ is -CO₂R⁷. In one embodiment, R¹ is -C(O)NR⁷R⁸.

Specific examples of groups defining R¹ include but are not limited to -C(O)H, -C(O)CH₃, -CO₂H, -CO₂CH₃, -C(O)NH₂, -C(O)NH(alkyl), -C(O)N(alkyl)(alkyl), -C(O)NH(Et-OH), -C(O)NH(benzyl), -C(O)NH(phenyl), -CH₂OH, -C(S)NH₂, -CN, and -tetrazole, or any subset thereof. In one particular embodiment, R¹ is selected from the group consisting of -CO₂H and -C(O)NH₂.

Q¹ is defined as a group of formula: -(R²)ₐ-(Y¹)_{b}-(R²)_{c}-R³.

In the foregoing formula, a, b and c are the same or different and are each independently 0 or 1.

In one embodiment, Q¹ is defined wherein a is 0. In the embodiment wherein a is 1 and thus the (R²)ₐ group is present, R² is typically alkylene or alkenylene, more particularly alkylene. In one particular embodiment, Q¹ is defined where a is 1 and (R²)ₐ is C₁₋₃alkylene.

In one embodiment, Q¹ in the compounds of formula (I) is defined where b is 1; thus Y¹ is present. In one such embodiment, Y¹ is selected from -O-, -S(O)_{f}-, -N(R⁷)-, -C(O)-, -OC(O)-, -CO₂-, -C(O)N(R⁷)-, -C(O)N(R⁷)S(O)₂-, -OC(O)N(R⁷)-, -OS(O)₂-, -S(O)₂N(R⁷)-, -S(O)₂N(R⁷)C(O)-, -N(R⁷)S(O)₂-, -N(R⁷)C(O)-, -N(R⁷)CO₂- and -N(R⁷)C(O)N(R⁷)-. In one particular embodiment, Y¹ is selected from -O-, -N(R⁷)-, -C(O)-, -OC(O)-, -C(O)N(R⁷)-, -OS(O)₂-, -S(O)₂N(R⁷)-, -N(R⁷)S(O)₂-, and -N(R⁷)C(O)-, or any subset thereof. In another particular embodiment, Y¹ is selected from -O-, -N(R⁷)-, -C(O)-, -OS(O)₂-, -N(R⁷)S(O)₂-, and -N(R⁷)C(O)-, or any subset therof. In one particular embodiment, b is 1 and Y¹ is -O-, -N(R⁷)-, -C(O)- or -OS(O)₂-, or any subset thereof. In one particular embodiment, b is 1 and Y¹ is -O-. In another particular embodiment, b is 1 and Y¹ is -N(R⁷)- (and in one embodiment R⁷ is H or alkyl, more particularly H). In another particular embodiment, b is 1 and Y¹ is -C(O)-. In another particular embodiment, b is 1 and Y¹ is -OS(O)₂-.

The variable c in the formula Q¹ can be 0 or 1. In one embodiment, c is 1. In one such embodiment (R²)_{c} is alkylene or alkenylene, more particularly alkylene. In one particular embodiment, Q¹ is defined where c is 1 and (R²)_{c} is C₁₋₃alkylene.

In one embodiment of the invention, the compounds of formula (I) are defined to include a substitution at the position indicated by Q¹; thus, when a, b and c are all 0, then R³ is not H. In one particular embodiment the compounds of the present invention are defined wherein, at least one of a or b is 1. In one particular embodiment, Q¹ is defined wherein both b and c are 1. In one particular embodiment, Q¹ is defined wherein a is 0 and both b and c are 1.

Consistent with the definition of b, Y¹ and c, the group R³ may be selected from the group consisting of H, halo, alkyl, alkenyl, alkynyl, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii):

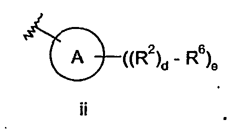

In one particular embodiment, R³ is a group of formula (ii). 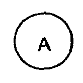 in formula (ii) is referred to herein as "Ring A." Ring A is selected from C₅₋₁₀cycloalkyl, C₅₋₁₀cycloalkenyl, aryl, 5-10 membered heterocycle having 1, 2 or 3 heteroatoms selected from N, O and S and 5-10 membered heteroaryl having 1, 2 or 3 heteroatoms selected from N, O and S. In Q¹, Ring A may be bound to R², Y¹ (when c is 0) or the thiophene ring (when a, b and c are 0) through any suitable carbon or heteroatom. In one embodiment, Q¹ is defined wherein R³ is a group of formula (ii) and Ring A is selected from C₅₋₁₀cycloalkyl, C₅₋₁₀cycloalkenyl, aryl, 5-10 membered heterocycle having 1, 2 or 3 heteroatoms selected from N, O and S and 5-10 membered heteroaryl having 1, 2 or 3 heteroatoms selected from N, O and S. In one embodiment, Q¹ is defined wherein R³ is a group of formula (ii) and Ring A is selected from aryl, 5-10 membered heterocycle having 1, 2 or 3 heteroatoms selected from N, O and S and 5-10 membered heteroaryl having 1, 2 or 3 heteroatoms selected from N, O and S. In one particular embodiment, Q¹ is defined wherein R³ is a group of formula (ii) and Ring A is selected from aryl and 5-10 membered heteroaryl having 1, 2 or 3 heteroatoms selected from N, O and S.

In one embodiment, Q¹ is defined wherein R³ is a group of formula (ii) and Ring A is selected from the group consisting of cycloalkyl, tetrahydropyran, tetrahydrofuran, morpholine, piperidine, phenyl, naphthyl, thiophene, furan, thiazole, pyrrole, pyrrolidine, pyrrolidinone, imidazole, benzofuran, benzimidazole, pyridyl, 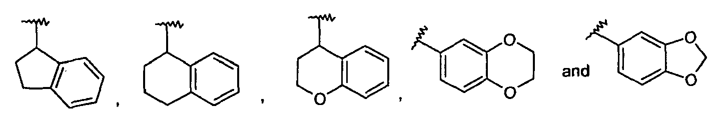 or any subset thereof. In one particular embodiment, Ring A is selected from phenyl, thiophene, thiazole and pyridyl, or any subet thereof. In one particular embodiment, Ring A is phenyl. In one particular embodiment Ring A is pyridyl.

Particular, more specific, examples of groups defining Q¹ in the compounds of formula (I) are selected from the group consisting of:

-OH

and 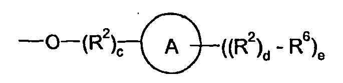 or any subset thereof.

One particular group defining Q¹ is

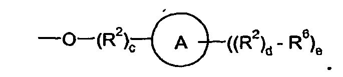

In one particular embodiment, Q¹ is

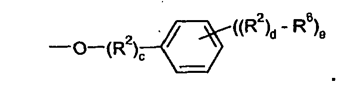

In one particular embodiment, Q¹ is

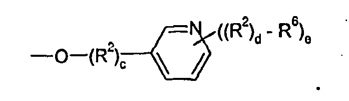

In one particular embodiment, Q¹ is

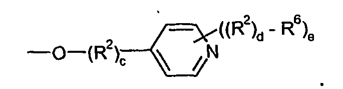

In one particular embodiment, Q¹ is

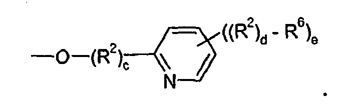

In one embodiment the compounds of formula (I) are defined wherein R³ is a group of formula (ii) and d is 0 or 1. In a particular embodiment, wherein R³ is a group of formula (ii) and d is 1, R² is C₁₋₃alkylene. In one embodiment, d is 0.

In one embodiment, wherein the compounds of formula (I) are defined wherein R³ is a group of formula (ii), e is 0, 1, 2 or 3. In one particular embodiment, e is 0 or 1. In one embodiment, e is 1. In one embodiment, e is 2.

In one embodiment, wherein the compounds of formula (I) are defined wherein R³ is a group of formula (ii), each R⁶ is the same or different and is independently selected from the group consisting of H, halo, alkyl, alkenyl, alkynyl, cycloalkyl, Ph, Het, -CH(OH)-R²-OH, -C(O)R⁷, -C(O)NR⁷R⁸, =O, -OR⁷, -S(O)_{f}R⁷, -S(O)₂NR⁷R⁸, -SO₂Ph, -NR⁷R⁸, -N(R⁷)C(O)R⁸, -N(R⁷)CO₂R⁸, -N(R⁷)S(O)₂R⁸, -NO₂, -CN and -N₃, or any subset thereof. In one particular embodiment, R³ is a group of formula (ii) and each R⁶ is the same or different and is independently selected from the group consisting of halo, -OR⁷, -NR⁷R⁸, -N(R⁷)C(O)R⁸, -N(R⁷)CO₂R⁸, -N(R⁷)S(O)₂R⁸, -NO₂ and -N₃ or any subset thereof. In one particular embodiment, R³ is a group of formula (ii) and each R⁶ is the same or different and is independently selected from the group consisting of halo, -OR⁷, -NR⁷R⁸ and -NO₂, or any subset thereof. In one embodiment, R³ is a group of formula (ii) and each R⁶ is the same or different and is independently selected from the group consisting of halo, -OR⁷ and -NO₂, or any subset thereof.

More specifically, in one embodiment wherein R³ is a group of formula (ii), each R⁶ is the same or different and is independently selected from the group consisting of F, Cl, Br, I, methyl, trifluoromethyl, O-methyl, O-difluoromethyl, O-trifluoromethyl, O-ethyl, O-propyl, O-isopropyl, O-cyclopropyl -NH₂, -NH(alkyl), -N(alkyl)alkyl, -NH(cyclopropyl), -NHSO₂-methyl, -NO₂, and -N₃, or any subset thereof.

In one embodiment, Q¹ is defined such that when b is 1 and c is 0, R³ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN or -N₃.

In one embodiment, n is 1 or 2, or any subset thereof.

Each Q² is the same or different and is independently a group of formula -(R²)ₐₐ-(Y²)_{bb}-(R²)_{cc}-R⁴. Q² may be bound at any one or more of the C-4, C-5, C-6 and C-7 positions of the benzimidazole ring (as numbered in formula (I)). In one embodiment, n is 1 and Q² is at C-5. In one embodiment, n is 1 and Q² is at C-6. In one embodiment, n is 2 and a Q² group is bound at each of the C-5 and C-6 positions of the benzimidazole.

In the foregoing formula, aa, bb and cc are the same or different and are each independently 0 or 1.

In one embodiment, aa is 0; thus the group (R²)ₐₐ is not present. In the embodiment wherein aa is 1, (R²)ₐₐ is typically alkylene or alkenylene, more particularly alkylene. In one particular embodiment, Q² is defined where aa is 1 and (R²)ₐₐ is G₁₋₃alkylene.

In one embodiment, the compounds of formula (I) are defined wherein bb is 0. In another embodiment, Q² in the compounds of formula (I) is defined where bb is 1; thus Y² is present. In one such embodiment, Y² is selected from -O-, -S(O)_{f}-, -N(R⁷)-, -C(O)-, -OC(O)-, -CO₂-, -C(O)N(R⁷)-, -C(O)N(R⁷)S(O)₂-, -OC(O)N(R⁷)-, -OS(O)₂-, -S(O)₂N(R⁷)-, -S(O)₂N(R⁷)C(O)-, -N(R⁷)S(O)₂-, -N(R⁷)C(O)-, -N(R⁷)CO₂- and -N(R⁷)C(O)N(R⁷)-. In one particular embodiment, bb is 1 and Y² is selected from -O-, -S(O)_{f}-, -N(R⁷)-, -C(O)-, -OC(O)-, -CO₂-, -C(O)N(R⁷)-, -OS(O)₂-, -S(O)₂N(R⁷)-, -N(R⁷)S(O)₂-, -N(R⁷)C(O)-, -N(R⁷)CO₂- and -N(R⁷)C(O)N(R⁷)-, or any subset thereof. In another particular embodiment, bb is 1 and Y² is selected from -O-, -S(O)_{f}-, -N(R⁷)-, -C(O)-, -CO₂-, -C(O)N(R⁷)-, -S(O)₂N(R⁷)-, -N(R⁷)S(O)₂-, -N(R⁷)C(O)-, -N(R⁷)CO₂-, and -N(R⁷)C(O)N(R⁷)-, or any subset thereof. In one particular embodiment, bb is 1 and Y² is selected from -O-, -S(O)_{f}-, -N(R⁷)-, -CO-, -C(O)N(R⁷)-, -S(O)₂N(R⁷)-, -N(R⁷)S(O)₂-, and -N(R⁷)C(O)-, or any subset thereof. In one particular embodiment, Q² is defined wherein bb is 1 and Y² is -O-. In one particular embodiment, Q² is defined wherein bb is 1 and Y² is -S(O)_{f}-, more particularly wherein f is 2. In another particular embodiment, bb is 1 and Y² is -N(R⁷)- and R⁷ is H or alkyl, more particularly H. In another particular embodiment, bb is 1 and Y² is -CO-. In another particular embodiment, bb is 1 and Y² is -C(O)N(R⁷)-. In another particular embodiment, bb is 1 and Y² is -N(R⁷)C(O)-. In another particular embodiment, bb is 1 and Y² is -SO₂N(R⁷)-.

The variable cc in the formula Q² can be 0 or 1. In one embodiment, cc is 1. In one such embodiment (R²)_{cc} is alkylene or alkenylene, more particularly alkylene. In one particular embodiment, Q² is defined where cc is 1 and (R²)_{cc} is C₁₋₃alkylene.

Consistent with the definition of bb, Y² and cc, the group R⁴ may be selected from the group consisting of H, halo, alkyl, alkenyl, alkynyl, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii):

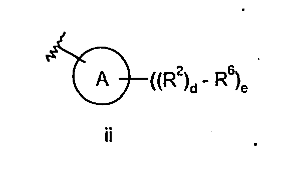

In one embodiment, R⁴ in the definition of Q² is selected from the group consisting H, halo, alkyl, alkenyl, alkynyl, -C(O)NR⁷R⁸, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii), or any subset thereof. In one particular embodiment, R⁴ is selected from the group consisting of H, halo, alkyl, -OR⁷, -S(O)_{f}R⁷, -S(O)₂NR⁷R⁸, -NR⁷R⁸, and a group of formula (ii), or any subset thereof. In one embodiment, R⁴ is selected from H, halo, alkyl, -OR⁷, -NR⁷R⁸, and a group of formula (ii), or any subset thereof.

In one particular embodiment, R⁴ is a group of formula (ii). In the embodiment, wherein R⁴ is a group of formula (ii), Ring A is selected from C₅₋₁₀cycloalkyl, C₅₋₁₀cycloalkenyl, aryl, 5-10 membered heterocycle having 1, 2 or 3 heteroatoms selected from N, O and S and 5-10 membered heteroaryl having 1, 2 or 3 heteroatoms selected from N, O and S. In one embodiment, wherein R⁴ is a group of formula (ii), Ring A is selected from C₅₋₆cycloalkyl, C₅₋₆cycloalkenyl, aryl, 5-10 membered heterocycle having 1, 2 or 3 heteroatoms selected from N, O and S and 5-10 membered heteroaryl having 1, 2 or 3 heteroatoms selected from N, O and S. In Q², Ring A may be bound to the R², Y² (when cc is 0) or the benzimidazole (when aa, bb and cc are 0) through any suitable carbon or heteroatom. In one embodiment, Q² is defined wherein R⁴ is a group of formula (ii) and Ring A is selected from aryl, 5-10 membered heterocycle having 1, 2 or 3 heteroatoms selected from N, O and S and 5-10 membered heteroaryl having 1, 2 or 3 heteroatoms selected from N, O and S. In one particular embodiment, Q² is defined wherein R⁴ is a group of formula (ii) and Ring A is selected from aryl and 5-10 membered heterocycle having 1, 2 or 3 heteroatoms selected from N, O and S.

In one embodiment, Q² is defined wherein R⁴ is a group of formula (ii) and Ring A is selected from the group consisting of cycloalkyl, oxetane, oxazole, pyrazole, thiazole, morpholine, piperidine, piperazine, phenyl, naphthyl, thiophene, furan, pyrrolidine, pyrrolidinone, imidazole, triazole, tetrazole, pyrimidine, imidazolidinone, benzofuran, benzodioxolane, benzimidazole, indazole, hexahydroazepine, hexahydrodiazepine, and pyridyl, or any subset thereof. In one particular embodiment, Q² is defined wherein R⁴ is a group of formula (ii) and Ring A is selected from morpholine, piperidine, piperazine, phenyl, pyrrolidinone, imidazolidinone, pyridyl, pyrazole, pyrimidine, hexahydroazapine, hexahydrodiazepine, and pyrrolidine, or any subset thereof.

More specifically, in one embodiment, each R⁴ is the same or different and is independently selected from the group consisting of H, F, CI, Br, I, methyl, trifluoromethyl, ethyl, propyl, isopropyl, cyclopropyl, iso-butyl, t-butyl, ethenyl, propenyl, acetylene, O-methyl, O-trifluoromethyl, O-ethyl, O-propyl, O-isopropyl, O-cyclopropyl, -SO₂-methyl, -SO₂NH₂, -NH₂, -NH(alkyl), -N(alkyl)₂, -NH(cyclopropyl), -NHC(O)-methyl, -NHC(O)NH₂, -NHSO₂-methyl, morpholine, piperidine, piperazine, phenyl, pyrrolidinone, imidazolidinone, pyridyl, pyrazole, pyrimidine, hexahydroazapine, hexahydrodiazepine and pyrrolidine, or any subset thereof.

Particular, more specific, examples of groups defining Q² in the compounds of formula (I) are selected from the group consisting of:
halo, alkyl (e.g., -CF₃), -OH, -O-alkyl , 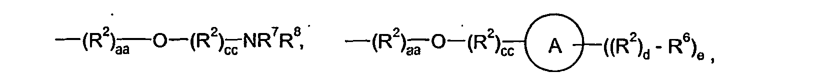 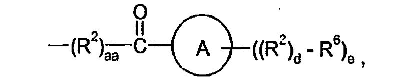 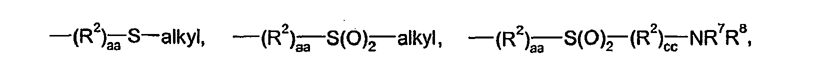 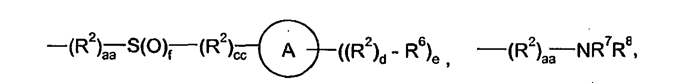 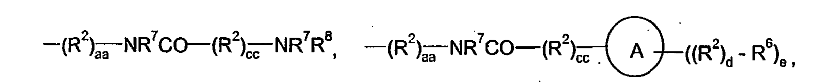 and

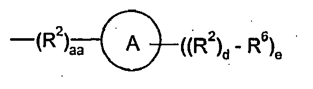

In one embodiment, Q² is -O-alkyl. In one particular embodiment, Q² is halo. In one particular embodiment, Q² is

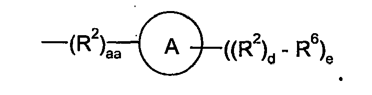

In one embodiment the compounds of formula (I) are defined wherein R⁴ is a group of formula (ii) and d is 0 or 1. In a particular embodiment, wherein R⁴ is a group of formula (ii) and d is 1, R² is C₁₋₃alkylene. In one embodiment, d is 0.

In one embodiment, wherein the compounds of formula (I) are defined wherein R⁴ is a group of formula (ii), e is 0, 1, 2 or 3. In one particular embodiment, e is 0 or 1. In one embodiment, e is 0. In one embodiment, e is 1. In one embodiment, e is 2.

In one embodiment, wherein the compounds of formula (I) are defined wherein R⁴ is a group of formula (ii), each R⁶ is the same or different and is independently selected from the group consisting of H, halo, alkyl, alkenyl, alkynyl, Het, -C(O)R⁷, -CO₂R⁷, -C(O)NR⁷R⁸, =O -OR⁷, -S(O)_{f}R⁷, -S(O)₂NR⁷R⁸, -NR⁷R⁸ and -N(R⁷)S(O)₂R⁸, or any subset thereof. In one particular embodiment, each R⁶ is the same or different and is independently selected from the group consisting of H, halo, alkyl, =O, -OR⁷, -S(O)_{f}R⁷, -S(O)₂NR⁷R⁸ and -NR⁷R⁸, or any subset thereof.

More specifically, in one embodiment, each R⁶ is the same or different and is independently selected from the group consisting of H, methyl, ethyl, propyl, isopropyl, iso-butyl, t-butyl, ethenyl, propenyl, cyclopropyl, pyrimidyl, -C(O)-alkyl, -CO₂-alkyl, -C(O)NH₂, acetylene, oxo, O-methyl, O-ethyl, O-propyl, O-isopropyl, O-cyclopropyl, -SO₂-methyl, -SO₂NH₂, -NH₂, -NH(alkyl), -N(alkyl)alkyl, -NH(cyclopropyl) and -NHSO₂-methyl, or any subset thereof.

In another embodiment of the present invention, two adjacent Q² groups are selected from the group consisting of alkyl, alkenyl, -OR⁷, -S(O)_{f}R⁷ and -NR⁷R⁸ and together with the carbon atoms to which they are bound, they form a C₅₋₆cycloalkyl, C₅₋₆cycloalkenyl, phenyl, 5-7 membered heterocycle having 1 or 2 heteroatoms selected from N, O and S, or 5-6 membered heteroaryl having 1 or 2 heteroatoms selected from N, O and S. By "two adjacent Q² groups" is meant that two Q² groups are bound to adjacent carbon atoms (e.g., C-5 and C-6). For example, in one embodiment two adjacent Q² groups are -OR⁷ and together with the atoms to which they are bound, they form a heterocyclic group such as:

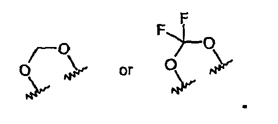

In another embodiment, two adjacent Q² groups are alkyl and together with the atoms to which they are bound, they form a cycloalkyl group such as:

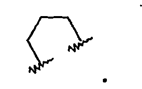

In another embodiment two adjacent Q² groups are defined as -OR⁷ and -NR⁷R⁸ respectively and together with the atoms to which they are bound, they form a heterocyclic group such as:

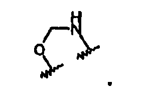

From these examples, additional embodiments can be readily ascertained by those skilled in the art. Preferably the compounds of formula (I) are defined wherein when n is 2, two adjacent Q² groups together with the atoms to which they are bound do not form a C₅₋₆cycloalkyl, C₅₋₆cycloalkenyl, phenyl, 5-7 membered heterocycle having 1 or 2 heteroatoms selected from N, O and S, or 5-6 membered heteroaryl having 1 or 2 heteroatoms selected from N, O and S.

In one embodiment, Q² is defined such that when bb is 1 and cc is 0, R⁴ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸ -NO₂, -CN or-N₃;

The processes of the present invention are useful for preparing compounds of formula (Ia): 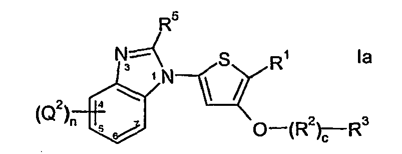 wherein all variables are as defined above, and pharmaceutically acceptable salts and solvates thereof.

In one embodiment, the processes of the present invention are useful for preparing compounds of formula (Ib): 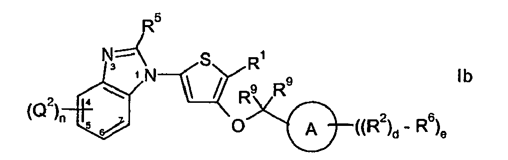 wherein each R⁹ is the same or different and is selected from H, halo and alkyl; and all other variables are as defined above, and pharmaceutically acceptable salts and solvates thereof.

In one embodiment, the processes of the present invention are useful for preparing compounds of formula (Ic): 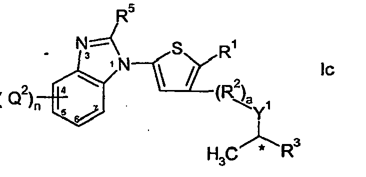 wherein:
indicates a chiral carbon and all other variables are as defined above.

In one particular embodiment, the stereochemistry of the chirl carbon is R.

In a particularly preferred embodiment, the processes of the present invention are useful for preparing compounds of formula (Id): 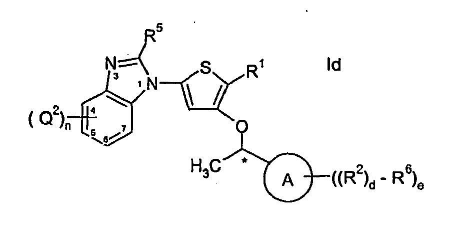 wherein:
indicates a chiral carbon and all other variables are as defined above.

In one particular embodiment, the stereochemistry of the chirl carbon is R.

It is to be understood that the present invention includes all combinations and subsets of the particular groups defined hereinabove.

Specific compounds of formula (I) which can be synthesized using the process of the present invention are described in the Example section that follows.

The pharmaceutically acceptable salts of the compounds of formula (I) include conventional salts formed from pharmaceutically acceptable inorganic or organic acids or bases as well as quaternary ammonium salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, palmoic, malonic, hydroxymaleic, phenylacetic, glutamic, benzoic, salicylic, fumaric, toluenesulfonic, methanesulfonic (mesylate), naphthalene-2-sulfonic, benzenesulfonic hydroxynaphthoic, hydroiodic, malic, steroic, tannic and the like.

Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts.

The term "solvate" as used herein refers to a complex of variable stoichiometry formed by a solute (a compound of formula (I)) and a solvent. Solvents, by way of example, include water, methanol, ethanol, or acetic acid.

Processes for preparing pharmaceutically acceptable salts and solvates of the compounds of formula (I) are conventional in the art. *See,* e.g., Burger's Medicinal Chemistry And Drug Discovery 5th Edition, Vol 1: Principles And Practice.

As will be apparent to those skilled in the art, in the processes described below for the preparation of compounds of formula (I), certain intermediates, may be in the form of pharmaceutically acceptable salts or solvates of the compound. Those terms as applied to any intermediate employed in the process of preparing compounds of formula (I) have the same meanings as noted above with respect to compounds of formula (I). Processes for preparing pharmaceutically acceptable salts and solvates of such intermediates are known in the art and are analogous to the process for preparing pharmaceutically acceptable salts and solvates of the compounds of formula (I).

Pharmaceutical compositions and therapeutic uses for the compounds of formula (I) are described in PCT Publication No. WO04/014899, published 19 February 2004 to GlaxoSmithKline.

Advantageously the process of the present invention permits the regioselective synthesis of a compound of formula (I). Prior synthetic processes frequently yielded mixtures of regioisomers which required further separation. The processes of the instant invention produce the desired regioisomer via a more direct and efficient route, utilizing less resource and leading to better yield. A more efficient process presents obvious advantages for large-scale production of commercial size batches.

In one embodiment, compounds of formula (I) may be conveniently prepared by the regioselective synthesis process outlined in Scheme 1 below. 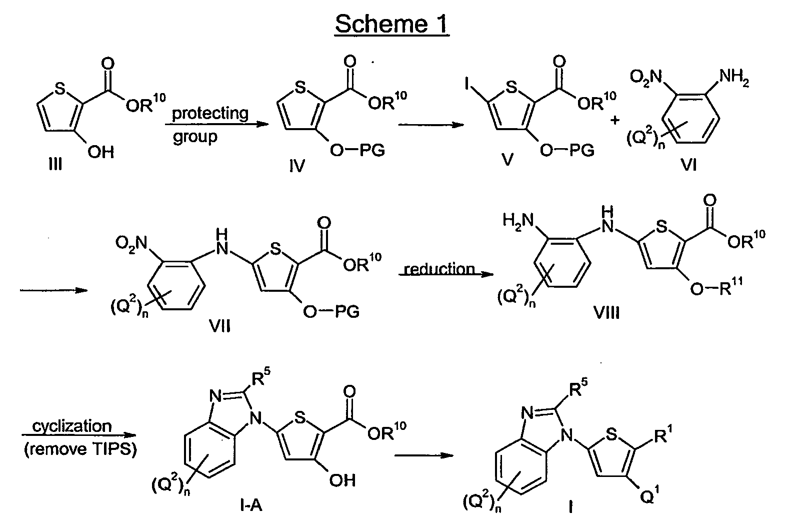 wherein:
R¹⁰ is selected from alkyl, alkenyl, alkynyl, cycloalkyl, cycloalkenyl and suitable carboxylic acid protecting groups;
PG is a triisopropylsilyl (TIPS) protecting group;
R¹¹ is H or a triisopropylsilyl protecting group;
   and all other variables are as defined above.

A compound of formula (I-A) may be converted into a pharmaceutically acceptable salt or solvate thereof or may be converted to a different compound of formula (I) or a pharmaceutically acceptable salt or solvate thereof using techniques described hereinbelow and those conventional in the art.

Generally, the process for preparing the compounds of formula (I) (all formulas and all variables having been defined above) comprises the steps of:
a) cyclizing a compound of formula (VIII) and optionally removing the triisopropylsilyl protecting group to prepare a compound of formula (I-A);
b) optionally converting the compound of formula (I-A) to a pharmaceutically acceptable salt or solvate thereof; and
c) optionally converting the compound of formula (I-A) or a pharmaceutically acceptable salt or solvate thereof to a different compound of formula (I) or a pharmaceutically acceptable salt or solvate thereof.

More specifically, compounds of formula (I) can be prepared by cyclizing a compound of formula (VIII) with a suitable cyclizing agent and optionally removing the triisopropylsilyl protecting group to prepare a compound of formula (I-A). 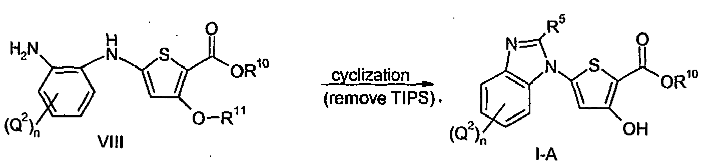 wherein all variables are as defined above.

Suitable cyclizing agents will be apparent to those skilled in the art of organic synthesis and include, for example triethyl orthoformate or trimethyl orthoformate, optionally in the presence of an acid catalyst, such as for example, pyridinium *p-*toluenesulfonate. In one embodiment, the cyclizing agent is triethyl orthoformate. Conveniently, the reaction of a compound of formula (VIII) with the cyclization agent may be carried out neat, at a temperature of from about 25°C to about 100°C. In one embodiment the reaction is carried out at a temperature of about 90°C-95°C.

In the embodiment wherein R¹¹ of the compound (VIII) is a triisopropylsilyl protecting group, the cyclization step is followed by removing the triisopropylsilyl protecting group, to prepare a compound of formula (I-A) (the removal of the triisopropylsilyl protecting group is not required when R¹¹ is H). The triisopropylsilyl group may be removed using conventional techniques for the removal of this protecting group. *See,* Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991.

The compound of formula (VIII) may be prepared by reducing a compound of formula (VII). 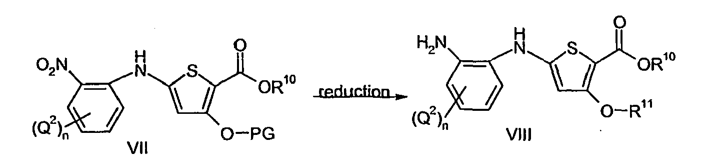 wherein all variables are as defined above.

The step of reducing a compound of formula (VII) may be carried out using conventional reduction techniques suitable for such compounds. In particular, the reduction may be effected using conditions such as palladium on carbon under a hydrogen atmosphere. The reaction may be carried out in an inert solvent at elevated pressure. Suitable inert solvents include but are not limited to ethanol, methanol and ethyl acetate. The reduction of a compound of formula (VII) yields both a compound of formula (VIII) wherein R¹¹ is H and a compound of formula (VIII) wherein R¹¹ is TIPS in differing amounts. If desired, the TIPS protecting group of the compound of formula (VIII) may be removed at this stage. Removal of the TIPS group may be accomplished as described above and eliminates the need for removal of the protecting group following cyclization of the compound of formula (VIII).

A compound of formula (VII) may be prepared by reacting a compound of formula (V) with a compound of formula (VI). 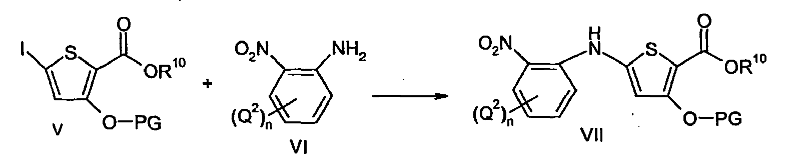 wherein all variables are as defined above.

The reaction of a compound of formula (V) with a compound of formula (VI) may be carried out by using coupling techniques conventional in the art of organic synthesis. Examples of suitable coupling reactions include but are not limited to palladium catalyzed cross-coupling conditions. Palladiums catalyzed cross-coupling conditions include but are not limited to reacting the compound of formula (V) with the compound of formula (VI) in the presence of a palladium source, a phosphine ligand, and a base in a suitable inert solvent. Examples of suitable palladium sources include but are not limited to tris(dibenzylideneacetone)-dipalladium (0), palladium (II) acetate, or tetrakis(triphenylphosphine)palladium (0). Examples of suitable phosphine ligands include but are not limited to 2-(di-t-butylphosphino)biphenyl and 2,2'-bis(diphenylphosphino)-1,1'-binaphthyl. Examples of suitable bases include but are not limited to cesium carbonate, potassium carbonate, potassium phosphate. Examples of suitable inert solvents include but are not limited to toluene, tetrahydrofuran, *N,N*-dimethylformamide and 1,4-dioxane. The reaction may be carried out at a temperature of between about 50°C and about 100°C. *See,* Yang, B.H.; Buchwald, S.L. Journal of Organometallic Chemistry 1999, 576, 125-146.

The compound of formula (V) may be prepared by either of two methods. According to the first method, the compound of formula (V) may be prepared by reacting a compound of formula (IV) with an iodine source in the presence of lithium diisopropyl amide at reduced temperature. 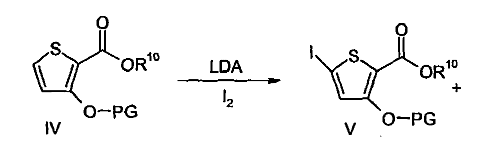 wherein all variables are as described above.

The reaction is typically carried out in a non-protic solvent, such as for example tetrahydrofuran or diethyl ether. The iodine source may be solid iodine or iodine dissolved in a non-protic solvent such as tetrahydrofuran or diethyl ether. The reaction is typically carried out at reduced temperature of less than about -50°C. In one embodiment, the reaction is carried out at a temperature of from about -60 to about -90°C.

According to the second method of preparing a compound of formula (V), a compound of formula (IV) is reacted with an iodine source in the presence of magnesium chloride diisopropylamide. 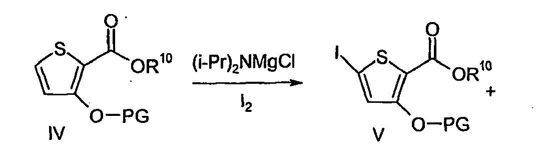 wherein all variables are as described above.

The reaction is typically carried out in a non-protic solvent, such as for example tetrahydrofuran or diethyl ether. The iodine source may be solid iodine or iodine dissolved in a non-protic solvent such as tetrahydrofuran or diethyl ether. Conventiently, the reaction may be carried out at ambient temperature.

The compound of formula (IV) may be prepared using conventional techniques for installing the triisopropylsilyl protecting group, such as for example, reacting a compound of formula (III) with triisopropylsilyl chloride and imidazole. The reaction is typically carried out in a suitable sovlent. Examples of suitable solvents include, but are not limited to dichloromethane, tetrahydrofuran, diethyl ether and *N,N*-dimethylformamide. Conveniently, the reaction may be carried out at ambient temperature. The compounds of formula (III) are commercially available or can be prepared using conventional knowledge in the art. *See,* Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991.

A compound of formula (VI) above may be prepared by several methods. In one embodiment, the compound of formula (VI) is prepared by reacting a compound of formula (XII) with ammonia. 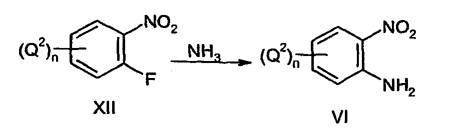 wherein all variables are as defined above.

This reaction may be carried out using conventional techniques. *See,* Silvestri, R., et al., Bioorg. Med Chem. 8:2305-2309 (2000); Hankovszky, H.O., et al., Can. J. Chem. 67:1392-1400 (1989); Nasielski-Hinkens, R.; et al., Heterocycles 26:2433-2442 (1987); Chu, K.Y., et al., J. Chem. Soc., Perkin Trans. 1 10:1194-1198 (1978). This reaction is typically carried out with an excess of ammonia and may be optionally heated to a temperature of from about 50°C to about 100°C. Examples of suitable solvents for this reaction include but are not limited to, water, methanol, ethanol, isopropanol, tetrahydrofuran, dioxane, and 1,2-dimethoxyethane. The compounds of formula (XII) are commercially available or may be prepared using conventional techniques and reagents.

In another embodiment, the compound of formula (VI) may be prepared by reacting a protected compound of formula (XIII) under nitration conditions to prepare a protected compound of formula (VI) (i.e., VI-A) and then removing the protecting group from the compound of formula (VI-A). 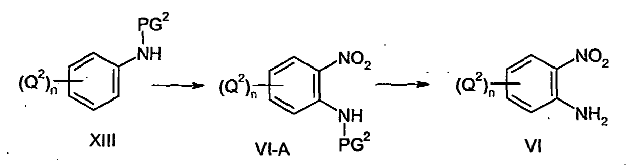 wherein PG² is a protecting group and all other variables are as defined above.

The protection of anilines is a common transformation well known to one skilled in the art. *See,* Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991. Suitable protecting groups for this application include but are not limited to acetyl, trifluoroacetyl, benzyloxycarbonyl, allyloxycarbonyl, 2-(trimethylsilyl)-ethoxycarbonyl, phenylsulfonyl, and p-toluenesulfonyl. Reagents and conditions vary according to the nature of the particular protecting group. Some typical reagents include but are not limited to acetic anhydride, trifluoroacetic anhydride, benzyl chloroformate, allyl chloroformate, 4-nitrophenyl 2-(trimethylsilyl)ethyl carbonate, phenylsulfonyl chloride, and p-toluensulfonyl chloride. In certain cases the addition of some base is required. Examples of suitable bases include but are not limited to potassium carbonate, sodium carbonate, trialkylamines, pyridine, and potassium t-butoxide. Suitable solvents for these conversions include but are not limited to dichloromethane, chloroform, tetrahydrofuran, acetic acid, methanol, ethanol, water, toluene, and diethyl ether.

The nitration of anilines is also well documented in the literature and the foregoing reaction may be carried out using these conventional techniques. *See,* Wissner, A., et. al., J. Med Chem. 46:49-63 (2003); Duggan, S. A., et. al., J. Org. Chem. 66: 4419-4426 (2001); Clews, J., et. al., Tetrahedron 56: 8735-8746 (2000); and Kagechika, H., J. Med Chem. 31: 2182-2192 (1988). The nitration may be carried out with a variety of nitrating reagents including but not limited to 70% aqueous nitric acid, red fuming nitric acid, ammonium nitrate with trifluoroacetic anhydride, and potassium nitrate with trifluoromethanesulfonic acid. The reaction is typically conducted at room temperature, but may be optionally heated to a temperature of from about 40 to about 100 °C in certain cases. Suitable solvents include but are not limited to acetic acid, sulfuric acid, acetic anhydride, dichloromethane, and chloroform.

The nitration results in a compound of formula (VI-A), (i.e., a protected compound of formula (VI)). The cleavage of the aniline protecting group, to result in a compound of formula (VI) can be accomplished through many different conventional methods. *See,* Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991.

The compounds of formula (XIII) may be prepared by installing a protecting group on the corresponding aniline. Such anilines are commercially available or may be prepared using conventional techniques.

The present invention may optionally include the further step of converting the compound of formula (I-A) to a pharmaceutically acceptable salt or solvate thereof and/or to a different compound of formula (I). Processes for preparing pharmaceutically acceptable salts and solvates of the compounds such as compounds of formula (I) are conventional in the art. *See,* e.g., Burger's Medicinal Chemistry And Drug Discovery 5th Edition, Vol 1: Principles And Practice.

As will be apparent to those skilled in the art, a compound of formula (I) may be converted to another compound of formula (I) using techniques well known in the art. For example, a compound of formula (I-A) may optionally be converted to a compound of formula (I-B) according to the process outlined in Scheme 1-A. 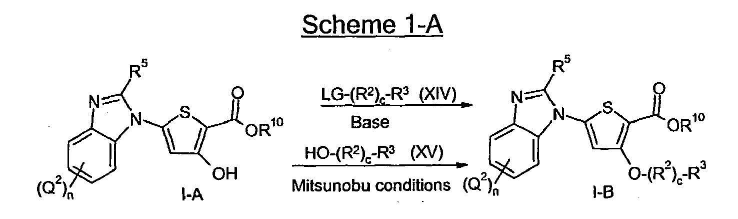 wherein
LG is a suitable leaving group; and
all other variables are as defined above.

In general the process for preparing a compound of formula (I-B) comprises the steps of:
a) reacting the compound of formula (I-A) with a base and a compound of formula (XIV) to prepare a compound of the formula (I-B); or
b) reacting the compound of formula (I-A) with a compound of formula (XV) under Mitsunobu conditions to prepare a compound of formula (I-B).

The compounds of formula (XIV) are commercially available or can be prepared using conventional knowledge in the art. The reaction may be carried out in an inert solvent, conveniently at room temperature, in the presence of a suitable base. The compound of formula (I-A) and the compound of formula (XIV) may be present in equimolar amounts; however, a slight excess of the compound of formula (XIV) may be employed if desired. Examples of suitable bases for this reaction include but are not limited to, potassium carbonate, sodium carbonate, cesium carbonate, sodium hydride, and potassium hydride. Examples of suitable inert solvents for this reaction include but are not limited to, *N,N*-dimethylformamide, tetrahydrofuran, dioxane, and 1,2-dimethoxyethane.

The compounds of formula (XV) are also commercially available or can be prepared using conventional knowledge in the art. The reaction is carried out in an inert solvent under standard Mitsunobu conditions. *See,* Hughes, D.L, Org. React. 42:335-656 (1992); and Mitsunobu, O., Synthesis 1-28 (1981). Typically the compound of formula (I-A), the compound of formula (XV), a triarylphosphine, and a dialkyl azodicarboxylate are reacted together at room temperature. Examples of suitable triarylphosphines include but are not limited to, triphenylphosphine, tri-*p-*tolylphosphine, and trimesitylphosphine. Examples of suitable dialkyl azodicarboxylates include but are not limited to, diethyl azodicarboxylate, diisopropyl azodicarboxylate, and di-*tert*-butyl azodicarboxylate. Examples of suitable inert solvents for this reaction include but are not limited to, tetrahydrofuran, dioxane, 1,2-dimethoxyethane, dichloromethane, and toluene.

A compound of formula (I-A) may also be converted to a compound of formula (I-C) according to the following Scheme 1-B. 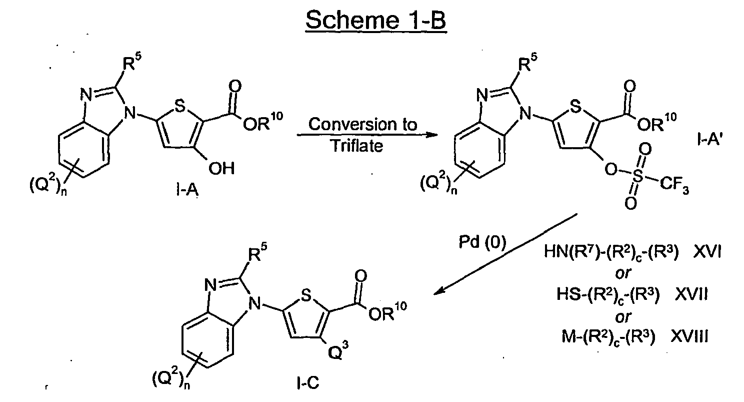 wherein:
- M: is -B(OH)₂, -B(OR¹⁶)₂, -Sn(R¹⁶)₂, Zn-halo, Zn-R¹⁶, Mg-halo, Cu-halo, Cu-R ¹⁶;
- R¹⁶: is alkyl or cycloalkyl;
- Q²: is a group of formula: -(R²)ₐ-(Y³)ⱼ(R²)_{c}-R³
- j: is 0 or 1;
- Y³: is selected from the group consisting of -S-, -N(R⁷)-, -N(R⁷)S(O)₂-, -N(R⁷)C(O)-, -N(R⁷)CO₂- and -N(R⁷)C(O)N(R⁷)-;
and all other variables are as defined above.

Generally, the process for preparing a compound of formula (I-C) comprises the steps of:
a) reacting a compound of formula (I-A) with a suitable triflating reagent to prepare a compound of formula (I-A'); and
b) coupling the compound of formula (I-A') with a compound selected from the group consisting of a compound of formula (XVI), (XVII), and (XVIII) using a palladium (0) catalyst to prepare a compound of the formula (I-C).

More specifically, a compound of formula (I-C) can be prepared by reacting a compound of formula (I-A') with a compound selected from the group consisting of a compound of formula (XVI), (XVII), and (XVIII) using a palladium (0) catalyst. This reaction may be carried out in an inert solvent, in the presence of palladium (0). The reaction may optionally be heated to a temperature of from about 50°C to about 150°C. Typically, the reaction is carried out by reacting an equimolar amount of a compound of formula (I-A') with an equimolar amount of the compound selected from the group consisting of compounds of formula (XVI), (XVII), and (XVIII). The palladium (0) catalyst is typically present in 1-10 mole percent compared to the compound of formula (I-A'). Examples of suitable palladium catalysts include but are not limited to, tetrakis(triphenylphosphine)palladium (0) and tris(dibenzylideneacetone)dipalladium (0). It is also possible to generate the palladium (0) catalyst *in situ* using palladium (II) sources. Examples of suitable palladium (II) sources include but are not limited to, palladium (II) acetate, palladium (II) chloride, palladium (II) trifluoroacetate, dichlorobis(triphenyl-phosphine)palladium (II), and bis(diphenylphosphinoferrocene)palladium (II) dichloride. Suitable solvents for this reaction include but are not limited to *N,N*-dimethylformamide, tetrahydrofuran, dioxane, toluene, benzene, 1,2-dimethoxyethane, and 1-methyl-2-pyrrolidinone. Bases and phosphines may be included as additives in the reaction if desired. Examples of suitable bases include but are not limited to cesium carbonate, sodium carbonate, and trialkylamines. Examples of suitable phosphine additives include but are not limited to triphenylphosphine, tributylphosphine, diphenylphosphinoethane, and 2,2'-bis(diphenylphosphino)-1,1'-binaphthyl. Compounds of the formula (XVI), (XVII), and (XVIII) may be obtained from commercial sources or prepared either as discreet compounds or generated *in situ* using conventional knowledge in the art. *See,* Luker, T.J., et al., Tetrahedron Lett 41:7731-7735 (2000); Yin, J., et al., Org. Lett 2:1101-1104 (2000); Wolfe, J.P., et al., Can. J. Chem. 78:957-962 (2000); Littke, A.F., et al., J. Am. Chem. Soc. 122:4020-4028 (2000); Hundertmark, T., et al., org. Lett. 2:1729-1731 (2000); Buchwald, S.L., Acc. Chem. Res. 31:805-818 (1998); Suzuki, A., J. Organomet Chem. 576:147-168 (1999); Negishi, E., J. Organomet. Chem. 576:179-194 (1999); Stanforth, S.P., Tetrahedron 54:263-303 (1998); Littke, A.F., Angew. Chem., Int. Ed 37:3387-3388 (1999); and Thorand, S., et al., J. Org. Chem. 63:8551-8553 (1998).

A compound of formula (I-A') can be prepared from a compound of formula (I-A) using a suitable triflating reagent. This reaction is typically carried out in an inert solvent using a base and a reagent designed for conversion of alcohols into triflates (i.e., a triflating reagent). Examples of suitable bases include but are not limited to sodium carbonate, trialkylamines, pyridine, sodium hydride, and lithium bis(trimethylsilyl) amide. The reaction is preferably run at a temperature of from about 0°C to about 25°C. Suitable triflating reagents for this reaction include but are not limited to, trifluoromethanesulfonic anhydride, trifluoromethanesulfonyl chloride, and *N-*phenyltrifluoromethanesulfonimide. Suitable inert solvents for this reaction include but are not limited to tetrahydrofuran, dichloromethane, toluene, chloroform, diethyl ether, and dioxane.

As a further example of methods for converting a compound of formula (I) to another compound of formula (I), a compound of formula (I-A), (I-B), or (I-C) (collectively referred to as a compound of formula "(I-D)" may be converted to a different compound of formula (I) 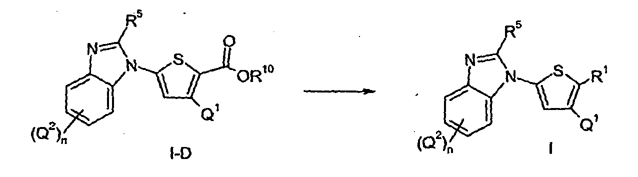 wherein:
R¹ is other than -CO₂R¹⁰;
   and all other variables are as defined above.

Several methods, using conventional techniques can be employed to convert a compound of formula (I-D) to a different compound of formula (I), depending upon the particular compound of formula (I) that is desired. A compound of formula (I-D) may be converted to a compound of formula (I-E) using hydrolysis. Additionally, either the compound of formula (I-D) or the compound of formula (I-E) may be converted to a compound of formula (I-F) using conventional amide bond coupling reactions with an amine of formula HNR⁷R⁸. 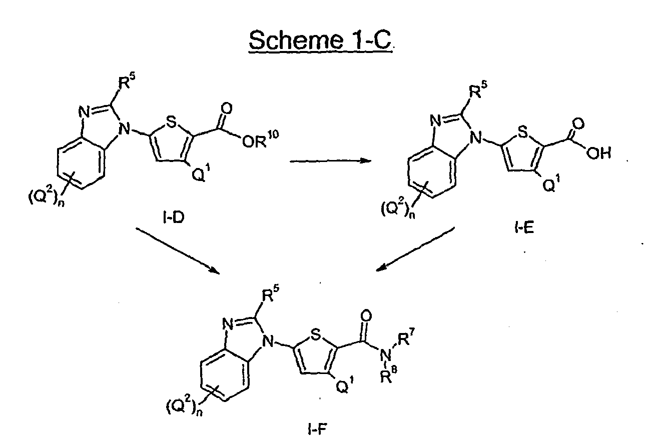 wherein all variables are as defined above.

Generally, one process for converting a compound of formula (I-D) to a compound of formula (I-F) comprises the steps of:
a) hydrolyzing the compound of formula (I-D) to prepare a compound of formula (I-E); and
b) reacting the compound of formula (I-E) with ammonia to prepare a compound of formula (I).

The hydrolysis of a compound of formula (I-D) to prepare the compound of formula (I-E) may be carried out using conventional hydrolysis techniques well known to those skilled in the art.

The compound of formula (I-E) may be reacted with ammonia using conventional amide bond coupling conditions to prepare the compound of formula (I). This reaction can be carried out in an inert solvent using a variety of commercially available coupling reagents. Suitable coupling reagents include but are not limited to *N,N*-dicydohexylcarbodiimide, 1-(3-dimethylaminopropyl)-3-ethylcarbodiimide hydrochloride, 1,1'-carbonyldiimidazole, and benzotriazol-1-yloxytris(dimethylamino)phosphonium hexafluorophosphate. Other suitable coupling reagents will be readily apparent to those skilled in the art. The carboxylic acid optionally may be converted into the corresponding acid chloride and subsequently treated with ammonia. Suitable reagents for the reaction of such acid chlorides include but are not limited to oxalyl chloride, thionyl chloride, and 1-chloro-*N,N*2-trimethyl-1-propenylamine. Base may be optionally added to the coupling reaction. The reaction may optionally require heating to a temperature of from about 40°C to about 100°C. Suitable basses include but are not limited to trialkylamines, pyridine, and 4-. (dimethylamino)pyridine. Examples of suitable solvents for this reaction include but are not limited to dichloromethane, chloroform, benzene, toluene, *N,N*-dimethylformamide and dichloroethane.

Alternatively, a compound of formula (I-F) can be obtained directly from a compound of formula (I-D) by heating the reaction in a sealed vessel with an excess of ammonia at temperature of from about 50°C to about 120°C. Suitable solvents for this reaction include but are not limited to methanol, ethanol, isopropanol, tetrahydrofuran, and dioxane.

As will be apparent to those of skill in the art, the foregoing transformation for converting the ester to the amide may, if desired, be carried out before the transformation described in Scheme 1-B. No particular order of steps is required for these transformations and they may be carried out in any order deemed convenient for the preparation of the particular compound desired. Additional transformations for converting a particular compound of formula (I) into a different compound of formula (I) are described in PCT Publication No. WO04/014899.

In another embodiment, the present invention provides a further regioselective synthesis process for preparing compounds of formula (I). The process is outlined in Scheme 2 below. 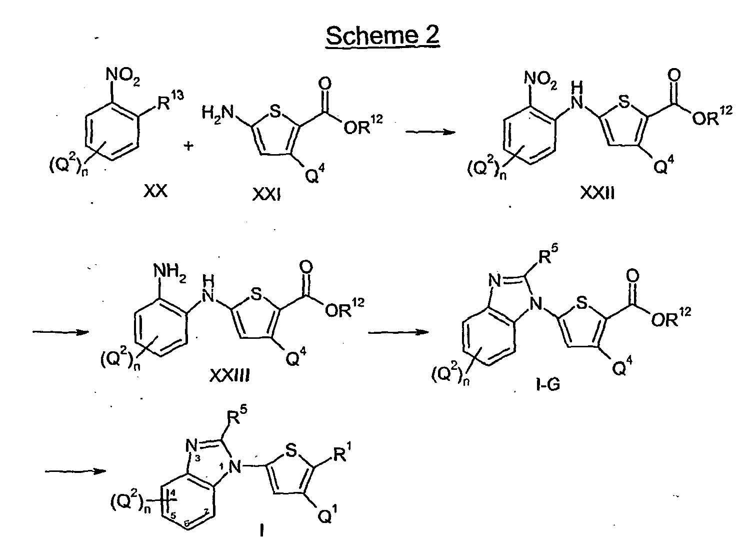 wherein:
- n: is 1, 2 or 3;
- Q⁴: is a group of formula - O - (R²)_{c} - R^{3a}, -O - Si(alkyl)₃, or -O- (R²)_{c}-Si(alkyl)₃;
wherein c is 1;
R^{3a} is a group of formula (iii): 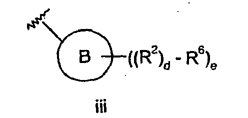 wherein Ring B is phenyl or 5-6 membered heteroaryl containing 1, 2 or 3 heteroatoms selected from N, O and S;
- R¹²: is methyl;
- R¹³: is halide or is O-triflate (OTf) (i.e., O-S(O)₂CF₃); and
all other variables are as defined above;

Generally, the process for preparing the compounds of formula (I) comprises the steps of:
a) coupling a compound of formula (XX) to a compound of formula (XXI) to prepare a compound of formula (XXII);
b) reducing the compound of formula (XXII) to prepare a compound of formula (XXIII);
c) cyclizing the compound of formula (XXIII) to prepare a compound of formula (I-G);
d) optionally converting the compound of formula (I-G) to a pharmaceutically acceptable salt or solvate thereof; and
e) optionally converting the compound of formula (I-G) or a pharmaceutically acceptable salt or solvate thereof to a different compound of formula (I) or a pharmaceutically acceptable salt or solvate thereof.

More specifically, compounds of formula (I) can be prepared by cyclizing a compound of formula (XXIII) with a suitable cyclizing agent to prepare a compound of formula (I-G). 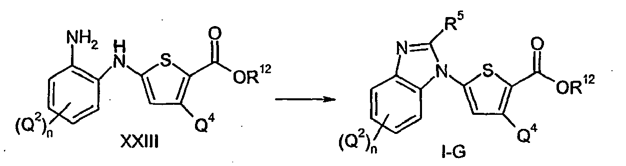 wherein all variables are as defined above.

Suitable cyclizing agents will be apparent to those skilled in the art of organic synthesis and include, for example triethyl orthoformate or-trimethyl orthoformate, optionally in the presence of an acid catalyst, for example *p-*toluenesulfonic acid or pyridinium *p*-toluenesulfonate. In one embodiment, the cyclizing agent is triethyl orthoformate and the catalyst is pyridinium *p-*toluenesulfonate. Conveniently, the reaction of a compound of formula (XXIII) with the cyclization agent may be carried out neat, at a temperature of from about 25°C to about 100°C. In one embodiment the reaction is carried out at about 25°C.

The compound of formula (XXIII) may be prepared by reduction of a compound of formula (XXII). 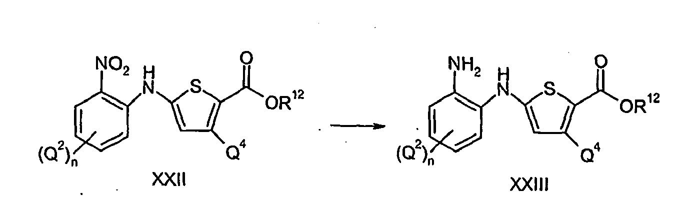 wherein all variables are as defined above.

The step of reducing a compound of formula (XXII) may be carried out using conventional reduction techniques suitable for such compounds. Suitable reduction conditions will be apparent to those skilled in the art of organic synthesis and may include, for example, palladium on carbon under a hydrogen atmosphere, sulfided platinum on carbon under a hydrogen atmosphere, or iron powder in acetic acid. In one embodiment, the reduction may be effected using conditions such as sulfided platinum on carbon under a hydrogen atmosphere. The reaction may be carried out in an inert solvent at either atmospheric or elevated pressure. Suitable inert solvents include but are not limited to ethanol, methanol, and ethyl acetate.

In another embodiment, the process of preparing a compound of formula (I-G) may be conveniently carried out by performing a one-pot reduction-cyclization procedure on a compound of formula (XXII). This transformation may be accomplished by reacting a compound of formula (XXII) under the conditions described above for the conversion of (XXII) to (XXIII) in the presence of a cyclizing agent and optional acid catalyst as described in the transformation of (XXIII) to (I-G). In one embodiment, the one-pot reduction-cyclization of (XXII) to provide (I-G) may be effected using conditions such as sulfided platinum on carbon under a hydrogen atmosphere in the presence of triethyl orthoformate and pyridinium *p-*toluenesulfonate. In this embodiment, triethyl orthoformate may be used as a solvent or a co-solvent with another suitable inert solvent, such as ethyl acetate.

The compound of formula (XXII) may be prepared by a Buchwald coupling of a compound of formula (XX) and (XXI). 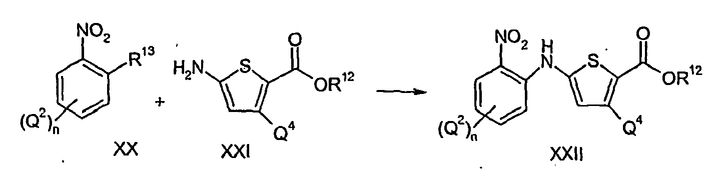 wherein all variables are as defined above.

The step of coupling a compound of formula (XX) to a compound of formula (XXI) may be carried out using coupling techniques conventional in the art of organic synthesis. Examples of suitable coupling reactions include but are not limited to palladium catalyzed cross-coupling conditions. Palladium catalyzed cross-coupling conditions include but are not limited to reacting the compound of formula (XX) with the compound of formula (XXI) in the presence of a palladium source, optionally a phosphine ligand, and a base in a suitable inert solvent. Examples of suitable palladium sources include but are not limited to tris(dibenzylideneacetone)-dipalladium (0) or acetato(2'-di-*t* butylphosphino-1,1'-biphenyl-2-yl)palladium (II). Examples of suitable phosphine ligands include but are not limited to 9,9-dimethyl-4,5-bis(diphenylphosphino)-xanthene. Examples of suitable bases include but are not limited to cesium carbonate, sodium methoxide, and triethylamine. Examples of suitable inert solvents include but are not limited to toluene or 1,4-dioxane. The reaction may be carried out at a temperature of between about room temperature and about 100°C. In one embodiment, the temperature is about 60°C. For a review of palladium-catalyzed cross-couplings of haloarenes and amines, see: Yang, B.H.; Buchwald, S.L. Journal of Organometallic Chemistry 1999, 576, 125-146. See also: Yin, J.; Zhao, M.M.; Huffman, M.A.; McNamara, J.M. Journal of Organic Chemistry 2002, 4, 3481-3484.

The compound of formula (XX) may be prepared from 3-halo-4-nitrophenol or 3-triflate-4-nitrophenol using techniques known in the art. 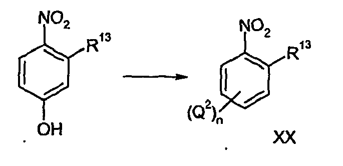 wherein all variables are as defined above.

For example, a methoxymethyl (MOM) ether may be installed by treatment with methoxymethyl chloride (MOMCI) and diisopropylethylamine (DIEA). A *para*-methoxy benzyl ether may be installed by treatment with *para-*methoxybenzyl chloride (PMBCI) and diisopropylethylamine (DIEA), optionally in the presence of a catalyst such as tetrabutylammonium iodide.

The reaction is typically carried out in a suitable sovlent. Examples of suitable solvents include, but are not limited to dichloromethane, tetrahydrofuran, diethyl ether, or *N,N*-dimethylformamide. Conveniently, the reaction may be carried out at ambient temperature. The protection of phenols as methoxymethyl ethers is a common transformation well known to one skilled in the art. *See,* Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991.

Alternatively, the compound of formula (XX) may be prepared from 4-fluoro-3-bromonitrobenzene using techniques known in the art. 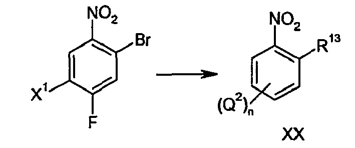 wherein X¹ is H or Br and all other variables are as defined above.

For example, a *para*-methoxybenzyl ether may be installed by treatment with *para*-methoxybenzyl alcohol in the presence of a mixture of a metal hydroxide salt dissolved in water, a suitable inert organic solvent such as dichloromethane, and a phase-transfer catalyst such as tetrabutylammonium hydrogen sulfate. See, for example: Marriott, J.H.; Moreno Barber, A.M.; Hardcastle, I.R.; Rowlands, M.G.; Grimshaw, R.M.; Neidle, S.; Jarman, M. Journal of the Chemical Society, Perkin Transactions I, 2000, 4265-4.278.

A compound of formula (XXI) may be prepared by reducing a compound of formula (XXIV). 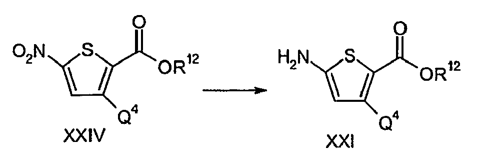 wherein all variables are as defined above.

Appropriate conditions for the reduction reaction will be apparent to those skilled in the art and include, for example, hydrogenation using an acid, such as acetic acid, and iron. The reaction may be carried out a elevated temperatures, such as about 50°C. Depending upon the particular definition of the group Q⁴, it may be advantageous or desireable to install a protecting group at that site prior to proceeding with the reduction reaction. Suitable protecting groups, methods for their installation and removal and circumstance wherein they would be desireable will be apparent to those skilled in the art of organic synthesis.

The compound of formula (XXIV) may be prepared by coupling a compound of formula (XXV) with an alkyl or benzyl halide, which may be a protecting group, under alkylation conditions or a benzyl alcohol under Mitsunobu reaction conditions. Conditions used are analogous to those described in Scheme 1-A for the conversion of a compound of formula (I-A) to a compound of formula (I-B). 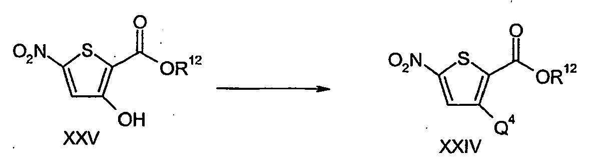 wherein all variables are as defined above.

In one particular embodiment of this process, the compounds of formula (I) are, prepared according to Scheme 2-A below. 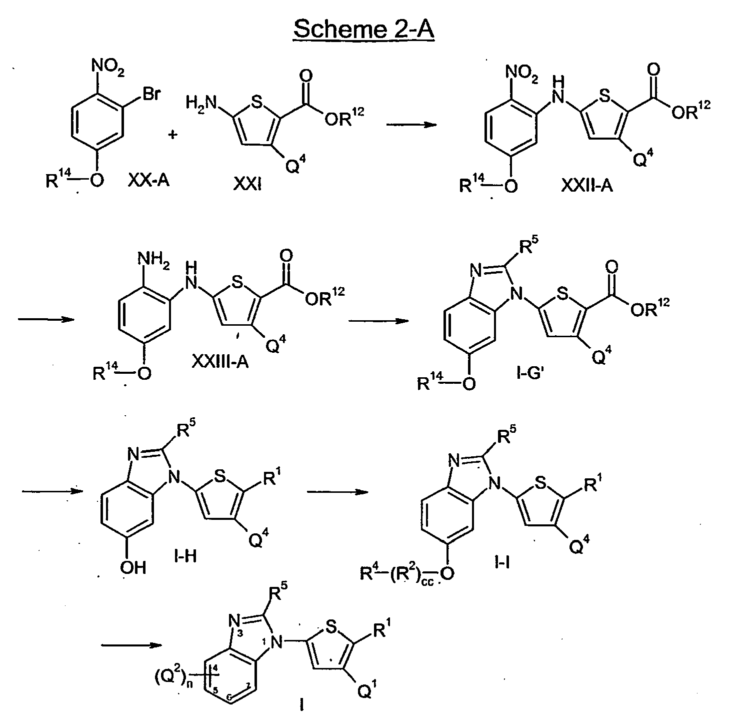 wherein:
- Q⁴: is a group of formula - O - (R²)_{c} - R^{3a}, -O- Si(alkyl)₃, or -O-(R²)_{c}- Si(alkyl)₃;
wherein c is 1;
R^{3a} is a group of formula (iii): 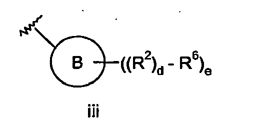 wherein Ring B is phenyl or 5-6 membered heteroaryl containing 1, 2 or 3 heteroatoms selected from N, O and S;
- R¹²: is methyl;
- R¹⁴: is a suitable phenol protecting group, such as substituted alkyl ethers, e.g., methoxymethyl; substituted benzyl ethers, e.g., para-methoxy benzyl; silyl ethers and alkyl silyl ethers; and
all other variables are as defined above;

Generally, the process for preparing the compounds of formula (I) according to Scheme 2-A comprises the steps of:
a) coupling a compound of formula (XX-A) to a compound of formula (XXI) to prepare a compound of formula (XXII-A);
b) reducing the compound of formula (XXII-A) to prepare a compound of formula (XXIII-A);
c) cyclizing the compound of formula (XXIII-A) to prepare a compound of formula (I-G');
d) optionally converting the compound of formula (I-G') to a pharmaceutically acceptable salt or solvate thereof; and
e) optionally converting the compound of formula (I-G') or a pharmaceutically acceptable salt or solvate thereof to a different compound of formula (I) or a pharmaceutically acceptable salt or solvate thereof.

The foregoing steps may be carried out using the procedures described above in Scheme 2.

Appropriate conditions for the conversion of the compound of formula (I-G') to a compound of formula (I-H) will be apparent to those skilled in the art and include, for example, treatment with trifluoroacetic acid in an inert solvent such as methylene chloride. Conveniently, the reaction may be carried out at room temperature.

If desired, the compound of formula (I-H) may then be converted to a different compound of formula (I) (e.g. a compound of formula (I-I)) using procedures and transformations analogous to those described above in Schemes 1-A, 1-B and 1-C.

Additionally, the compound of formula (I-H) may be converted to a compound of formula (I-J), which may then be converted to a compound of formula (I-K) according to the following Scheme 2-B. 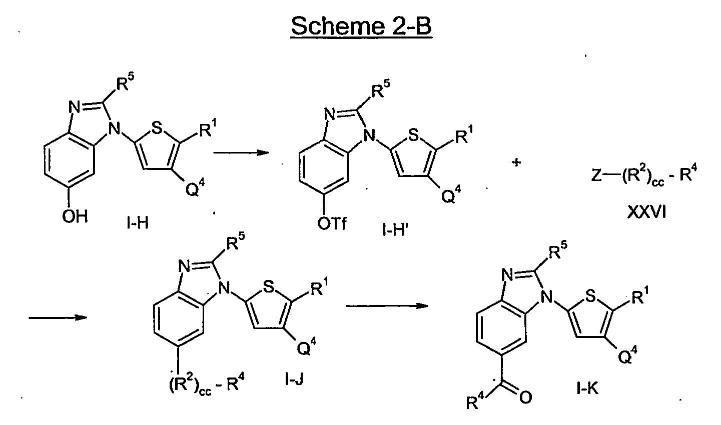 wherein:
OTf is O-triflate (i.e., O-S(O)₂CF₃);
Z is boronic acid, boronic ester, trifluoroborate salt, stannane, or zinc halide; and
all other variables are as defined above.

The conversion of the compound of formula (I-H) to the corresponding triflate of formula (I-H') can be carried out using methods analogous to those described above for the conversion of the compound of formula (I-A) to the corresponding triflate of formula (I-A'). The triflate of formula (I-H') is then reacted with the compound of formula (XXVI) under palladium-catalyzed cross-coupling conditions to prepare the corresponding compound of formula (I-J).

The step of coupling a compound of formula (XXVI) to a compound of formula (I-H') to provide a compound of formula (I-J) may be carried out using palladium-catalyzed Suzuki, Stille, or Negishi palladium-catalyzed cross-coupling techniques conventional in the art of organic synthesis. For a review of the Suzuki cross-coupling reaction, see: Miyaura, N.; Suzuki, A. Chemical Reviews 1995, 95, 2457-2483. In the embodiment of the Suzuki cross-coupling reaction, Z is a boronic acid, boronic ester, or trifluoroborate salt.

The Suzuki coupling may be carried out using a suitable catalyst such as dichloro[1,1'-bis(diphenylphosphino)ferrocene] palladium(II) dichloromethane adduct, a base such as aqueous sodium carbonate or triethylamine, and a suitable inert solvent such as *N,N*-dimethylacetamide or *n-*propanol, optionally in the presence of microwave irradiation, at temperatures from about 50°C to about 150°C. For a review of the Stille cross-coupling reaction, see: Mitchell, T.N. Synthesis 1992, 803-815. In the embodiment of the Stille cross-coupling reaction, Z is a stannane. The Stille coupling may be carried out using tetrakis(triphenylphoshine)palladium (0) as the catalyst, in the presence of promoters such as cesium fluoride and copper (I) iodide, in a suitable inert solvent such as *N,N*-dimethylformamide at a temperature of about 45 °C. For a review of the Negishi cross-coupling reaction, see: Negishi, E.; Zingzhong, T.Z.; Qian, M.; Hu, Q.; Huang, Z. Metal Catalyzed Cross-Coupling Reactions (2nd Edition), 2004, 2, 815-889. In the embodiment of the Negishi cross-coupling reaction, Z is a zinc halide: The Negishi coupling may be carried out using dichlora[1,1'-bis(diphenylphosphino)-ferrocene] palladium(II) dichloromethane adduct as the catalyst, in the presence of a promoter such as copper (I) iodide, in a suitable inert solvent such as *N,N*-dimethylacetamide at a temperature of about 80 °C.

In the case of a compound of formula (I-J) where R² is vinyl or a 1,1-disubstituted alkene and cc is 1, such a compound may be transformed into a ketone of formula (I-K) by conventional methods known to one skilled in the art of organic chemistry. Suitable methods include but are not limited to sequential dihydroxylation with an oxidant such as AD-Mix or osmium tetroxide, and cleavage of the resultant diol with an oxidant such as sodium periodate.

The additional transformations described above and those described in PCT Publication No. WO04/014899 are similarly useful for converting a compound of formula (I-G), (I-G'), (I-H), (I-I), (I-J) or (I-K) into a different compound of formula (I).

In another particular embodiment of Scheme 2, compounds of formula (I) wherein n is 1 or 2 and Q² at C-5 is Cl, Br or I, may be synthesized according to the following Scheme 2-C. 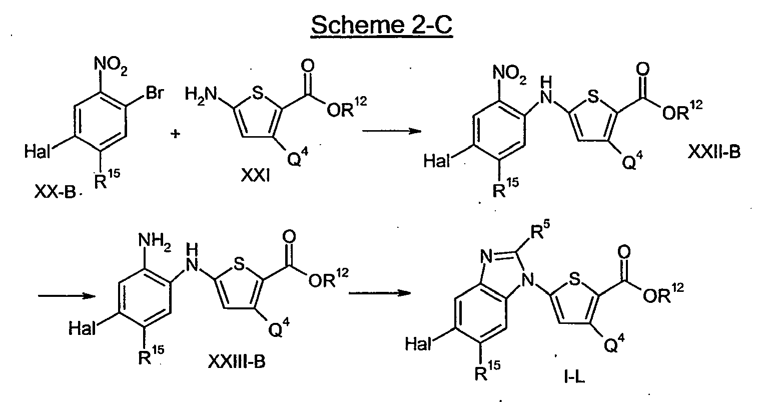 wherein Hal is Cl, Br or I (preferably Br);
R¹⁵ is H or -O-R¹⁴; and
all variables are as defined above.

Generally, the process for preparing the compounds of formula (I-L) (all formulas and all variables having been defined above) comprises the steps of:
a) coupling a compound of formula (XX-B) to a compound of formula (XXI) to prepare a compound of formula (XXII-B);
b) reducing the compound of formula (XXII-B) to prepare a compound of formula (XXIII-B);
c) cyclizing the compound of formula (XXIII-B) to prepare a compound of formula (I-L);
d) optionally converting the compound of formula (I-L) to a pharmaceutically acceptable salt or solvate thereof; and
e) optionally converting the compound of formula (I-L) or a pharmaceutically acceptable salt or solvate thereof to a different compound of formula (I) or a pharmaceutically acceptable salt or solvate thereof.

The foregoing steps may be carried out using the procedures described above in Scheme 2. The compounds of formula (XX-B) are commercially available or may be prepared using conventional techniques.

The compounds of formula (I-L) may be employed in numerous transformations, including those described herein and those described in PCT Publication No. WO04/014899, for the preparation of various other compounds of formula (I). In particular, a compound of formula (I-L) may be converted to a compound of formula (I-M) in a manner analogous to that described above for the conversion of a compound of formula (I-D) to a compound of formula (I-F). 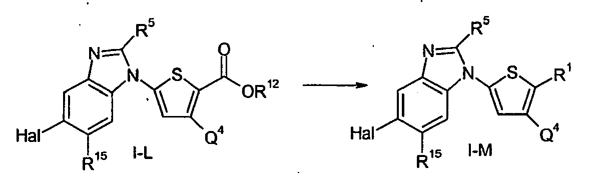 wherein all variables are as defined above. In one preferred embodiment, Hal is Br.

The 5-halo analogs of formula (I-M) may be converted to the corresponding compounds of formula (I-N).

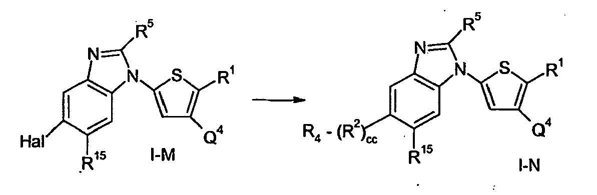

According to this process, the compound of formula (I-M) is reacted with a compound of formula (XXVI):

Z-(R²)_{cc}- R⁴ XXVI

wherein:
Z is a boronic acid, boronic ester, trifluoroborate salt, stannane, or zinc halide; and
   all other variables are as defined above.

The step of coupling a compound of formula (XXVI) to a compound of formula (I-M) to provide a compound of formula (I-N) may be carried out using palladium-catalyzed Suzuki, Stille, or Negishi palladium-catalyzed cross-coupling techniques conventional in the art of organic synthesis. The procedures used in this transformation may be carried out in a manner analogous to those described in Scheme 2-B above for the coupling of a compound of formula (I-H') and a compound of formula (XXVI) to provide a compound of formula (I-J).

Conveniently, the compounds of formula (I-M) may be synthesized using the processes described above or the non-regioselective synthesis methods which are described in PCT Publication No. WO04/014899.

The transformations described above and those described in PCT Publication No. WO04/014899 are similarly useful for converting a compound of formula (I-M) or (I-N) into a different compound of formula (I).

In another particular embodiment of this process, the present invention provides a further regioselective synthesis process for preparing compounds of formula (I) wherein n is 1 and Q² is bound at the C-6 position of the benzimidazole. The process is outlined in Scheme 2-D below. 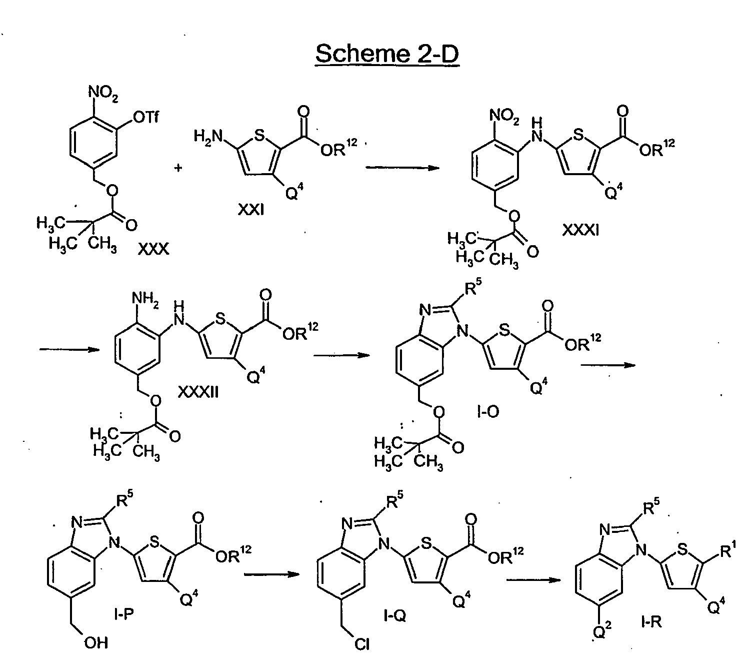 wherein:
- Q⁴: is a group of formula -O-(R²)_{c}- R^{3a}, -O- Si(alkyl)₃, or -O- (R²)_{c}-Si(alkyl)₃;
wherein c is 1;
R^{3a} is a group of formula (iii): 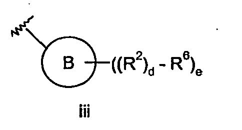 wherein Ring B is phenyl or 5-6 membered heteroaryl containing 1, 2 or 3 heteroatoms selected from N, O and S;
- R¹²: is methyl;
- OTf: is O-triflate; and
all other variables are as defined above.

Generally, the process for preparing the compounds of formula (I) (all formulas and all variables having been defined above) comprises the steps of:
a) coupling a compound of formula (XXX) to a compound of formula (XXI) to prepare a compound of formula (XXXI);
b) reducing the compound of formula (XXXI) to prepare a compound of formula (XXXII);
c) cyclizing the compound of formula (XXXII) to prepare a compound of formula (I-O);
d) optionally converting the compound of formula (I-O) to a pharmaceutically acceptable salt or solvate thereof; and
e) optionally converting the compound of formula (I-O) or a pharmaceutically acceptable salt or solvate thereof to a different compound of formula (I) or a pharmaceutically acceptable salt or solvate thereof.

More specifically, compounds of formula (I-O) can be prepared by cyclizing a compound of formula (XXXII) with a suitable cyclizing agent. The cycilzation step can be carried out in the same manner as described above for the cyclization of a compound of formula (XXIII) to a compound of formula (I-G).

The compound of formula (I-P) may be prepared from the compound of formula (I-O) using conventional techniques for the deprotection of pivaloate ester to the corresponding alcohol. As will be apparent to those skilled in the art, the compound of formula (I-P) may be converted to another compound of formual (I), e.g., a compound of formula (I-R)..

The deprotection of pivaloate esters to the corresponding alcohols is a common transformation well known to one skilled in the art. *See,* Kocienski, P.J. Protecting Groups, Georg Thieme Verlag, Stuttgart, 1994; and Greene, T.W., Wuts, P. G. M. Protecting Groups in Organic Synthesis (2nd Edition), J., Wiley and Sons, 1991.

If desired, the compound of formula (I-P) may be converted to a different compound of formula (I) (e.g. a compound of formula.(I-Q)) using a variety of procedures. For example, the compound of formula (I-P) may be converted into a compound of formula (I-Q) methods well known to one skilled in the art for the conversion of an alcohol to an alkyl chloride. Such methods include but are not limited to treatment of the alcohol with N-chlorosuccinimide and triphenylphosphine in an inert solvent.

If desired, the compound of formula (I-Q) may be converted to another compound of formula (I), (e.g., a compound of formula (I-R) by displacing the benzylic chloride of (I-Q) with a nucleophile. Examples of suitable nucleophiles include but are not limited to amines, N-containing heterocycles and heteroaryls, alcohols, and thiols. As will be apparent to those skilled in the art, the thiols may be oxidezed to the corresponding sufoxides or sulfones.

The transformations described above and those described in PCT Publication No. WO04/014899 are similarly useful for converting a compound of formulas (I-O), (I-P), (I-Q) and (I-R) into a different compound of formula (I). For example, compounds of formula (I) wherein Q² is a group of the formula -R²- Y² - (R²)_{cc} - R₄ (i.e., aa and bb are each 1) can be prepared using procedures analogous to those described in Schemes 1-A, 1-B and 1-C.

The compound of formula (XXXII) may be prepared by reduction of a compound of formula (XXXI) in the same manner as described above for the reduction of the compound of formula (XXII) to a compound of formula (XXIII).

The compound of formula (XXXI) may be prepared by a Buchwald coupling of a compound of formula (XXX) and (XXI). 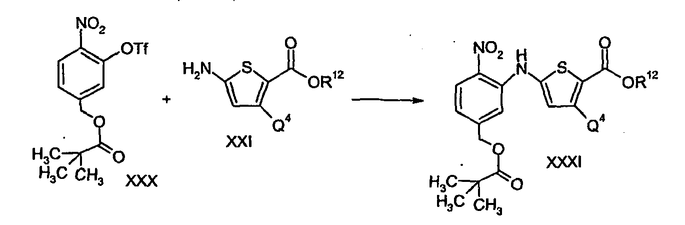 wherein all variables are as defined above.

This coupling reaction is carried out in a manner similar to that described above for the Buchwald coupling of a compound of formula (XX) and a compound of formula (XXI) according to Scheme 2.

The compound of formula (XXX) may be prepared by converting (3-hydroxy-4-nitrophenyl)methyl 2,2-dimethylpropanoate to the corresponding triflate using conventional methods such as those described hereinabove.

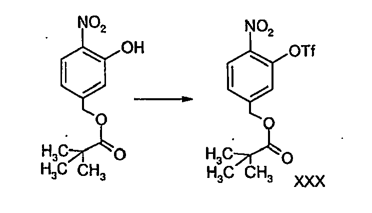

(3-hydroxy-4-nitrophenyl)methyl 2,2-dimethylpropanoate may be prepared according to Scheme 2-E below.

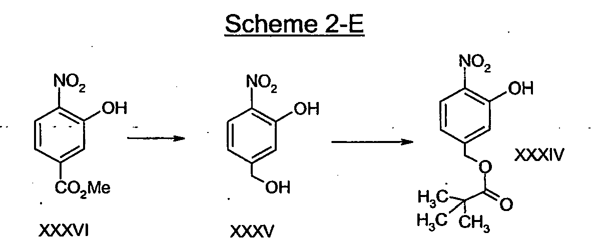

Commercially available compound of formula (XXXVI) may be reduced to a compound of formula (XXXV) by methods well-known to those skilled in the art of organic synthesis. Such methods may include but are not limited to treatment with lithium aluminum hydride, diisobutylaluminum hydride, or borane-pyridine complex in a suitable inert solvent such as diethyl ether, tetrahydrofuran, or dichloroethane.

The protection of alcohols as their pivaloate esters, as in the conversion of a compound of formula (XXXV) to (3-hydroxy-4-nitrophenyl)methyl 2,2-dimethylpropanoate, is a common transformation well known to one skilled in the art. See, for example: Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991. Also see: Yamada, S.; Sugaki, T.; Matsuzaki, K. Journal of Organic Chemistry 1996, 61, 5932-5938..

The transformations described above and those described in PCT Publication No. WO04/014899 are similarly useful for converting a particular compound of formula (I) into a different compound of formula (I).

The following examples are intended for illustration only and are not intended to limit the scope of the invention in any way, the invention being defined by the claims which follow.

Reagents are commercially available or are prepared according to procedures in the literature.

As used herein, the symbols and conventions used in these processes, schemes and examples are consistent with those used in the contemporary scientific literature, for example, the Journal of the American Chemical Society or the Journal of Biological Chemistry. Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| i. v. (intravenous); | Hz (Hertz); |
| MHz (megahertz); | mol (moles); |
| mmol (millimoles); | rt (room temperature); |
| min (minutes); | h (hours); |
| mp (melting point); | TLC (thin layer chromatography); |
| Tᵣ (retention time); | RP (reverse phase); |
| MeOH (methanol); | i-PrOH (isopropanol); |
| TEA (triethylamine); | TFA (trifluoroacetic acid); |
| TFAA (trifluoroacetic anhydride); | THF (tetrahydrofuran); |
| DMSO (dimethylsulfoxide); | AcOEt (EtOAc); |
| DME (1,2-dimethoxyethane); | DCM (CH2CI2); |
| DCE (dichloroethane); | DMF (N,N-dimethylformamide); |
| DMPU (N,N'-dimethylpropyleneurea); | CDI (1,1-carbonyldiimidazole); |
| HOAc (acetic acid); | |
| HOSu (N-hydroxysuccinimide); | HOBT (1-hydroxybenzotriazole); |
| mCPBA (meta-chloroperbenzoic acid); | |
| BOC (tert-butyloxycarbonyl); | FMOC (9-fluorenylmethoxycarbonyl); |
| DCC (dicyclohexylcarbodiimide); | CBZ (benzyloxycarbonyl); |
| Ac (acetyl); | atm (atmosphere); |
| TMSE (2-(trimethylsilyl)ethyl); | TMS (trimethylsilyl); |
| TIPS (triisopropylsilyl); | TBS (t-butyldimethylsilyl); |
| DMAP (4-dimethylaminopyridine); | BSA (bovine serum albumin) |
| ATP (adenosine triphosphate); | HRP (horseradish peroxidase); |
| DMEM (Dulbecco's modified Eagle medium); | |
| HPLC (high pressure liquid chromatography); | |
| BOP (bis(2-oxo-3-oxazolidinyl)phosphinic chloride); | |
| TBAF (tetra-n-butylammonium fluoride); | |
| HBTU (O-Benzotriazole-1-yl-N,N,N',N'-tetramethyluronium hexafluorophosphate); | |
| HEPES (4-(2-hydroxyethyl)-1-piperazine ethane sulfonic acid); | |
| DPPA (diphenylphosphoryl azide); | |
| fHNO₃ (fumed HNO₃); | |
| EDC (ethylcarbodiimide hydrochloride); and | |
| EDTA (ethylenediaminetetraacetic acid). | |

All references to ether are to diethyl ether; brine refers to a saturated aqueous solution of NaCl. Unless otherwise indicated, all temperatures are expressed in °C (degrees Centigrade). All reactions are conducted under an inert atmosphere at room temperature unless otherwise noted.

¹H NMR spectra were recorded on a Varian VXR-300, a Varian Unity-300, a Varian Unity-400 instrument, or a General Electric QE-300. Chemical shifts are expressed in parts per million (ppm, δ units). Coupling constants are in units of hertz (Hz). Splitting patterns describe apparent multiplicities and are designated as s (singlet), d (doublet), t (triplet), q (quartet), m (multiplet), br (broad).

Low-resolution mass spectra (MS) were recorded on a JOEL JMS-AX505HA, JOEL SX-102, or a SCIEX-APIiii spectrometer; high resolution MS were obtained using a JOEL SX-102A spectrometer. All mass spectra were taken under electrospray ionization (ESI), atmospheric pressure chemical ionization (APCI), electron impact (EI) or by fast atom bombardment (FAB) methods. Infrared (IR) spectra were obtained on a Nicolet 510 FT-IR spectrometer using a 1-mm NaCl cell. All reactions were monitored by thin-layer chromatography on 0.25 mm E. Merck silica gel plates (60F-254), visualized with UV light, 5% ethanolic phosphomolybdic acid or p-anisaldehyde solution or mass spectrometry (electrospray or AP). Flash column chromatography was performed on silica gel (230-400 mesh, Merck) or using automated silica gel chromatography (Isco, Inc. Sq 16x or 100sg Combiflash).

### Intermediate Example 1: methyl 5-amino-3-({(1R)-1-[2-(trifluoromethyl)phenyl]ethyl}oxy)-2-thiophenecarboxylate

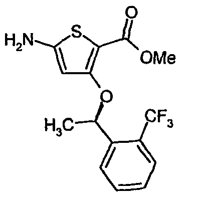

### Step A - Methyl 5-nitro-3-({(1R)-1-[2-(trifluoromethyl)phenyl]ethyl}oxy)-2-thiophenecarboxylate

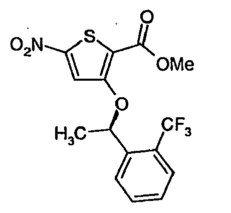

A slurry of polymer-supported triphenylphosphine (62.36 g, 2.21 mmol/g, 137.8 mmol) in dichloromethane (1.0 L) was stirred at room temperature for 10 minutes. The mixture was cooled to 0°C. Methyl 3-hydroxy-5-nitro-2-thiophenecarboxylate (20.00 g, 98.44 mmol), which may be prepared in a manner analogous to the literature procedure (Barker, J.M.; Huddleston, P.R.; Wood, M.L.; Burkitt, S.A. Journal of Chemical Research (Miniprint) 2001, 1001-1022) was added, followed by (1*S*)-1-[2-(trifluoromethyl)phenyl]ethanol (26.20 g, 137.8 mmol) and di-*tert-*butyl azodicarboxylate (31.73 g, 137.8 mmol). The reaction mixture was stirred at room temperature for 21.25 h and then was filtered through a fritted funnel and concentrated. The residue was treated with 4 N HCl in 1,4-dioxane (300 mL) and stirred at room temperature for 3 h. The mixture was then quenched by addition of 3 N sodium hydroxide (300 mL) and saturated aqueous sodium bicarbonate (200 mL). The mixture was extracted with dichloromethane (3 x 250 mL). The combined organic fractions were dried over magnesium sulfate, filtered, and concentrated onto silica gel. Purification by column chromatography (0 to 25% ethyl acetate:hexanes) provided 36.08 g (98%) of the title compound as yellow oil. ¹H NMR (300 MHz, CDCl₃): δ 7.82 (d, 1H, *J*= 7.8 Hz), 7.68 (d, 1H, *J*= 7.8 Hz), 7.59 (t, 1H, *J*= 7.4 Hz), 7.46 (s, 1H), 7.42 (t, 1H, *J*= 7.6 Hz), 5.77 (q, 1H, *J*= 6.1 Hz), 3.94 (s, 3H), 1.74 (d, 3H, *J*= 6.1 Hz).

### Step B - Methyl 5-amino-3-({(1R)-1-[2-(trifluoromethyl)phenyl]ethyl}oxy)-2-thiophenecarboxylate (Title Compound)

To a flask equipped with a temperature probe, an overhead mechanical stirrer, a reflux condenser, and an addition funnel was added iron powder (26.84 g, 480.6 mmol) and acetic acid (130 mL). The iron/acetic acid slurry was stirred mechanically and heated to an internal temperature of 50 °C. To the addition funnel was added a solution of methyl 5-nitro-3-({(1*R*)-1-[2-(trifluoromethyl)phenyl]ethyl}oxy)-2-thiophenecarboxylate (36.08 g, 96.13 mmol) in acetic acid (160 mL). The solution in the addition funnel was then added dropwise to the iron/acetic acid slurry at a rate such that the internal temperature was maintained at <60 °C (2.5 h total addition time). The reaction mixture was cooled to room temperature, diluted with dichloromethane (500 mL), and then quenched by addition of 6 N sodium hydroxide (750 mL) and saturated aqueous sodium bicarbonate (200 mL). The entire mixture was then filtered through a pad of Celite to remove insoluble material, rinsing the Celite with additional dichloromethane (250 mL). The aqueous and organic fractions were separated. The aqueous fraction was extracted with ethyl acetate (2 x 400 mL). The organic fractions were combined, dried over magnesium sulfate, filtered, and concentrated to afford 30.66 g (92%) of the title compound as an orange solid. ¹H NMR (300 MHz, CDCl₃): δ 7.89 (d, 1H, *J*= 7.7 Hz), 7.62 (d, 1H, *J*= 7.7 Hz), 7.56 (t, 1H, *J*= 7.7 Hz), 7.36 (t, 1H, *J*= 7.7 Hz), 5.72 (s, 1H), 5.65 (q, 1H, *J*= 6.3 Hz), 4.26 (br s, 2H), 3.80 (s, 3H), 1.66 (d, 3H, *J*= 6.3 Hz); MS (APCI): 368.00 [M+Na]⁺.

### Intermediate Example 2: Methyl 5-amino-3-{[(1R)-1-(2-chlorophenyl)ethyl]-oxy}-2-thiophenecarboxylate

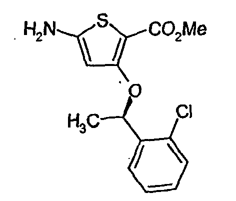

### Step A - Methyl 3-{[(1R)-1-(2-chlorophenyl)ethyl]oxy}-5-nitro-2-thiophenecarboxylate

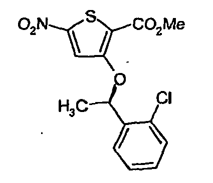

The title compound was prepared from methyl 3-hydroxy-5-nitro-2-thiophenecarboxylate and (1S)-1-(2-chlorophenyl)ethanol by a procedure analogous to Intermediate **Example 1**, Step A. ¹H NMR (400 MHz, DMSO-*d₆*): δ 7.96 (s, 1H), 7.65 (dd, 1H, *J* = 1.7, 7.8 Hz), 7.47 (dd, 1H, *J* = 1.5, 7.7 Hz), 7.40 (dt, 1H, *J*= 1.3, 7.5 Hz), 7.34 (dt, 1H, *J*= 1.9, 7.5 Hz), 5.98 (q, 1H, *J* = 6.0 Hz), 3.85 (s, 3H), 1.59 (d, 3H, *J*= 6.2 Hz).

### Step B - Methyl 5-amino-3-{[(1R)-1-(2-chlorophenyl)ethyl]oxy}-2-thiophenecarboxylate (Title Compound)

The title compound was prepared from methyl 3-{[(1*R*)-1-(2-chlorophenyl)ethyl]oxy}-5-nitro-2-thiophenecarboxylate by a procedure analogous to Intermediate **Example 1**, Step B. ¹H NMR (400 MHz, DMSO-*d₆*): δ 7.54 (dd, 1H, *J*= 1.8, 7.9 Hz), 7.45 (dd, 1H, *J*= 1.4, 7.7 Hz), 7.37 (dt, 1H, *J*= 1.4, 7.7 Hz), 7.31 (dt, 1H, *J*= 1.8, 7.6 Hz), 6.76 (br s, 2H), 5.57 (q, 1H, *J*= 6.2 Hz), 5.49 (s, 1H), 3.63 (s, 3H), 1.51 (d, 3H, *J*= 6.4 Hz); MS (ESI): 334.03 [M+Na]⁺.

### Example 1: Methyl 3-{[(1R)-1-(2-chlorophenyl)ethyl]oxy}-5-(6-hydroxy-1H-benzimidazol-1-yl)-2-thiophenecarboxylate

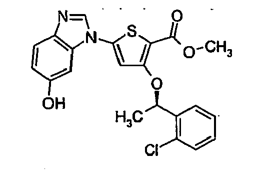

### Step A - 2-Bromo-4-({[4-(methyloxy)phenyl]methyl}oxy)-1-nitrobenzene

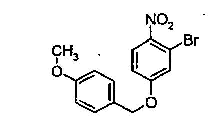

2-bromo-4-fluoro-1-nitrobenzene (20.0 g, 90.9 mmol) and 4-methoxybenzyl alcohol (22.7 mL, 182 mmol) were dissolved in dichloromethane (400 mL) with stirring. 1 N sodium hydroxide solution (400 mL) was added followed by tetrabutylammonium hydrogensulfate (3.09 g, 9.10 mmol). The reaction was stirred for 8 h and poured into a separatory funnel. The layers were separated and the aqueous was extracted once with dichloromethane and once with diethyl ether. The combined organic layers were dried over magnesium sulfate, filtered, and concentrated *in vacuo.* Purification by flash chromatography afforded 28.01 g (91 %) of the title compound. ¹H NMR (400 MHz, CDCl₃): δ 8.03 (d, 1H, *J*= 9.2 Hz), 7.50 (d, 1H, *J*= 2.6 Hz), 7.39-7.34 (m, 2H), 7.17 (dd, 1H, *J*= 2.7, 9.0 Hz), 6.95-6.91 (m, 2H), 5.14 (s, 2H), 3.73 (s, 3H).

### Step B - Methyl 3-{[(1R)-1-(2-chlorophenyl)ethyl]oxy}-5-{[5-({[4-(methyloxy)phenyl]methyl}oxy)-2-nitrophenyl]amino}-2-thiophenecarboxylate

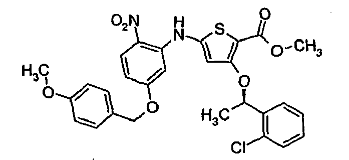

2-bromo-4-({[4-(methyloxy)phenyl]methyl}oxy)-1-nitrobenzene (20.19 g, 59.7 mmol) and methyl 5-amino-3-{[(1R)-1-(2-chlorophenyl)ethyl]oxy}-2-thiophenecarboxylate (18.60 g, 59.7 mmol) were dissolved in 1,4-dioxane (500 mL) with stirring in a flask equipped with a mechanical stirrer, reflux condenser, and thermometer. The solution was degassed for 75 min by bubbling nitrogen through the stirring solution. 9,9-Dimethyl-4,5-bis(diphenylphosphino)xanthene (1.52 g, 2.63 mmol), cesium carbonate (97.26 g, 299 mmol), and tris(dibenzylideneacetone) dipalladium(0) (1.09 g, 1.19 mmol) were added. The reaction was heated to 60 ºC and stirred for 16 hours. The reaction was cooled to room temperature and filtered through Celite. The solid was washed with 20% methanol in dichloromethane. The filtrate was concentrated onto approximately 200 g of silica gel. The solid was placed in a fritted funnel and washed with 10% ethyl acetate in dichloromethane. The filtrate was concentrated in vacuo. Purification by flash chromatography provided 27.18 g (80%) of the title compound. ¹H NMR (400 MHz, CDCl₃): δ 9.87 (s, 1H), 8.10 (d, 1H, *J* = 9.5 Hz), 7.63 (m, 1H), 7.39-7.29 (m, 4H), 7.23 (m, 1H), 6.96-6.90 (m, 2H), 6.80 (d, 1H, *J*= 2.6 Hz), 6.75 (s, 1H), 6.69 (dd, 1H, *J*= 2.6, 9.3 Hz), 5.75 (q, 1H, *J*= 6.3 Hz), 5.03 (AB, 2H, J_{AB} = 13.2 Hz, J_{AB} = 11.3 Hz), 3.74 (s, 3H), 3.74 (s, 3H), 1.55 (d, 3H, *J*= 6.4 Hz).

### Step C - Methyl 5-{[2-amino-5-({[4-(methyloxy)phenyl]methyl}oxy)-phenyl]amino}-3-{[(1R)-1-(2-chlorophenyl)ethyl]oxy}-2-thiophenecarboxylate

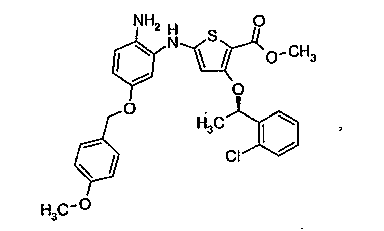

Methyl 3-{[(1*R*)-1-(2-chlorophenyl)ethyl]oxy}-5-{[5-({[4-(methyloxy)phenyl]-methyl}oxy)-2-nitrophenyl]amino}-2-thiophenecarboxylate (27.18 g, 47.8 mmol) was dissolved in ethyl acetate (400 mL) with stirring. Sulfided platinum (5% weight on carbon, 2.80 g) was added, and the reaction was placed under 1 atm of hydrogen using a balloon apparatus. After 36 h an additional amount of catalyst (2.80 g) was added and stirring was continued under 1 atm of hydrogen. After 16 h more, the reaction was filtered through a Celite pad washing with ethyl acetate. The filtrate was concentrated to afford the title compound, which was immediately carried into the next step. ¹H NMR (400 MHz, CDCl₃): δ 8.6 (br s, 1H); 7.56 (dd, 1H, *J*= 1.7, 7.8 Hz), 7.41-7.09 (m, 5H), 6.93-6.88 (m, 2H), 6.71 (d, 1H, *J*= 2.8 Hz), 6.65 (d, 1H, *J*= 8.6 Hz), 6.57 (dd, 1H, *J*= 2.7, 8.6 Hz), 5.87 (s, 1H), 5.62 (q, 1H, *J*= 6.4 Hz), 4.82 (s, 2H), 4.46 (br s, 2H), 3.73 (s, 3H), 3.64 (s, 3H), 1.52 (d, 3H, *J*= 6.2 Hz).

### Step D - Methyl 3-{[(1R)-1-(2-chlorophenyl)ethyl]oxy}-5-[6-({[4-(methyloxy)phenyl]methyl}oxy)-1H-benzimidazol-1-yl]-2-thiophenecarboxylate

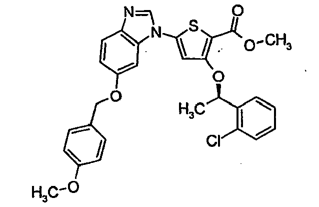

Methyl 5-{[2-amino-5-({[4-(methyloxy)phenyl]methyl}oxy)phenyl]amino}-3-{[(1*R*)-1-(2-chlorophenyl)-ethyl]oxy}-2-thiophenecarboxylate (from **Example 1,** Step C) was dissolved in trimethyl orthoformate (100 mL) and diethyl ether (100 mL) with stirring. Pyridinium *p*-toluenesulfonate (0.601 g, 2.39 mmol) was added in a single portion. The reaction was stirred for 2.5 h and quenched by the addition of triethylamine (approximately 3 mL). The mixture was concentrated and purified by flash chromatography to afford 25.45 g (97% over 2 steps) of the title compound. ¹H NMR (400 MHz, CDCl₃): δ 8.47 (s, 1H), 7.71 (dd, 1H, *J*= 1.6, 8.2 Hz), 7.63 (d, 1H, *J*= 9.0 Hz), 7.43-7.08 (m, 7H), 7.00 (dd, 1H, *J*= 2.4, 8.8 Hz), 6.96-6.90 (m, 2H), 5.97 (q, 1H, *J*= 6.4 Hz), 5.03 (AB, 2H, J_{AB} = 17.1 Hz, J_{AB} = 11.3 Hz), 3.80 (s, 3H), 3.73 (s, 3H), 1.60 (d, 3H, *J*= 6.4 Hz).

### Step E - Methyl 3-{[(1R)-1-(2-chlorophenyl)ethyl]oxy}-5-(6-hydroxy-1H-benzimidazol-1-yl)-2-thiophenecarboxylate (Title Compound)

Methyl 3-{[(1*R*)-1-(2-chlorophenyl)ethyl]oxy}-5-[6-({[4-(methyloxy)phenyl]-methyl}oxy)-1*H*-benzimidazol-1-yl]-2-thiophenecarboxylate (25.45 g, 46.4 mmol) was dissolved in dichloromethane (120 mL) and cooled to 0 ºC with stirring. Trifluoroacetic acid (40.0 mL, 519 mmol) was added dropwise *via* addition funnel. The reaction was stirred for 1 h and sodium hydroxide (20.0 g, 500 mmol) in water (120 mL) was added dropwise *via* addition funnel. The pH of the mixture was then adjusted to neutral with saturated sodium bicarbonate solution. The reaction was poured into a separatory funnel, and the layers were separated. The aqueous layer was washed with ethyl acetate. The combined organic layers were dried over magnesium sulfate, filtered, and concentrated *in vacuo.* Purification by flash chromatography afforded 14.86 g (75%) of the title compound. ¹H NMR (400 MHz, CDCl₃): δ 9.62 (s, 1H), 8.45 (s, 1H), 7.74 (dd, 1H, *J*= 1.7, 7.7 Hz), 7.53 (d, 1H, *J*= 8.8 Hz), 7.46-7.38 (m, 3H), 7.32 (m, 1H), 7.08 (d, 1H, *J*= 2.2 Hz), 6.79 (dd, 1H, *J* = 2.2, 8.6 Hz), 5.94 (q, 1H, *J*= 6.2 Hz), 3.79 (s, 3H), 1.60 (d, 3H, *J*= 6.2 Hz).

### Example 2: Methyl 5-(6-hydroxy-1H-benzimidazol-1-yl)-3-({(1R)-1-[2-(trifluoromethyl)phenyl]ethyl}oxy)-2-thiophenecarboxylate

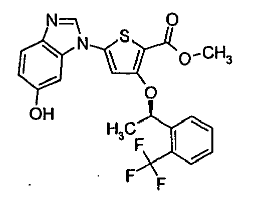

### Step A - Methyl 5-{[5-({[4-(methyloxy)phenyl]methyl}oxy)-2-nitrophenyl]amino}-3-({(1R)-1-[2-[(trifluoromethyl)phenyl]ethyl}oxy)-2-thiophenecarboxylate

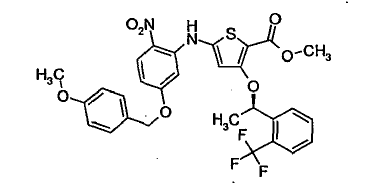

The title compound was prepared from methyl 5-amino-3-({(1*R*)-1-[2-(trifluoromethyl)phenyl]ethyl}oxy)-2-thiophene carboxylate and 2-bromo-4-({[4-(methyloxy)phenyl]methyl}oxy)-1-nitrobenzene by a procedure analogous to **Example 1**, Step B. MS (ESI): 603 [M+H]⁺.

### Step B - Methyl 5-{[2-amino-5-({[4-(methyloxy)phenyl]methyl}oxy) phenyl]amino}-3-({(1R)-1-[2-(trifluoromethyl)phenyl]ethyl}oxy)-2-thiophenecarboxylate

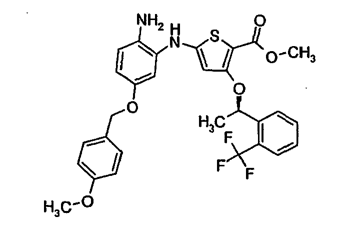

The title compound was prepared from methyl 5-{[5-({[4-(methyloxy)phenyl]methyl}oxy)-2-nitrophenyl]amino}-3-({(1*R*)-1-[2-(trifluoromethyl)phenyl]ethyl}oxy)-2-thiophenecarboxylate by a procedure analogous to **Example 1**, Step C. MS (ESI): 573 [M+H]⁺.

### Step C - Methyl 5-(6-hydroxy-1H-benzimidazol-1-yl)-3-({(1R)-1-[2-(trifluoromethyl)phenyl]ethyl}oxy)-2-thiophenecarboxylate (Title Compound)

Methyl 5-{[2-amino-5-({[4-(methyloxy)phenyl]methyl}oxy) phenyl]amino}-3-({(1*R*)-1-[2-(trifluoromethyl)phenyl]ethyl}oxy)-2-thiophenecarboxylate (11g, 19.19 mmol) was dissolved in 100 mL of trimethyl orthoformate with stirring. Pyridinium *p*-toluenesulfonate (0.502 g, 1.91 mmol) was added in a single portion. The reaction was stirred for 2.5 h. The mixture was concentrated and the crude methyl 5-[6-({[4-(methyloxy)phenyl]methyl}oxy)-1H-benzimidazol-1-yl]-3-({(1*R*)-1-[2-(trifluoromethyl)phenyl]ethyl}oxy)-2-thiophenecarboxylate was dissolved in chloroform (75 mL) and cooled to 0 ºC with stirring. Trifluoroacetic acid (50.0 mL, 649 mmol) was added. The reaction was stirred for 1 h and allowed to come to room temperature. The mixture was concentrated while cooling to remove most of the trifluoroacetic acid. The mixture was dissolved in chloroform (200 mL). The reaction was poured into a separatory funnel, and the layers were separated. The pH of the mixture was then adjusted to neutral with saturated sodium bicarbonate solution. The aqueous layer was washed with chloroform. The combined organic layers were dried over magnesium sulfate, filtered, and concentrated *in vacuo.* Purification by flash chromatography afforded 8.16 g (92% over 2 steps) of the title compound. ¹H NMR (400 MHz, CDCl₃): δ 7.88 (d, 1H, *J*= 7.87 Hz), 7.83 (s, 1H), 7.66-7.55 (m, 3H), 7.40 (t, 1H, *J*= 7.7 Hz), 6.92 (d, 1H, *J*= 2.2 Hz), 6.85 (dd, 1H, *J*= 2.3, 8.7 Hz), 5.78 (q, 1H, *J*= 6.23 Hz), 5.47 (s, 1H), 3.91 (s, 3H), 1.75 (d, 3H, *J*= 6.23 Hz); MS (ESI): 463 [M+H]⁺.

### Example 3: Methyl 5-[6-chloromethyl-1H-benzimidazol-1-yl]-3-({(1R)-1-[2-(trifluoromethyl)phenyl]ethyl}oxy)thiophene-2-carboxylate

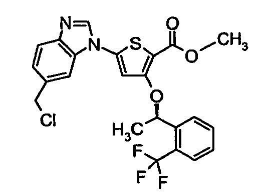

### Step A - 5-(Hydroxymethyl)-2-nitrophenol

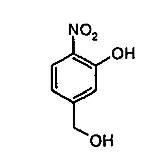

To a mixture of 3-hydroxy-4-nitrobenzoic acid (5.0 g, 27.3 mmol) in 1,2-dichloroethane (100 mL) was added trimethyl borate (4.9 mL, 43.7 mmol), followed by boron trifluoride diethyl etherate (5.5 mL, 43.7 mmol). Borane-pyridine complex (4.1 mL, 41.0 mmol) was then slowly added dropwise. The reaction was stirred 4 hrs at room temperature, then cooled to 0 °C and quenched with methanol (10 mL). The mixture was concentrated under vacuum and the residue taken up in toluene (200 mL), then extracted with aqueous 1 N sodium hydroxide (3 x 100 mL). The combined aqueous layers were adjusted to pH 1.0 by addition of 12 N HCl, then extracted with ethyl acetate (3 x 250 mL). The combined organic layers were washed with water, brine, dried over magnesium sulfate and concentrated under vacuum to give 4.55 g (98%) of the title compound as a light yellow solid. ¹H NMR (400 MHz, DMSO-*d₆*): δ 10.87 (s, 1H), 7.85 (d, 1H, *J*= 8.6 Hz), 7.08 (s, 1H), 6.88 (dd, 1H, *J*= 1.19, 8.51 Hz), 5.43 (s, 1H), 3.33 (s, 2H).

### Step B - 3-Hydroxy-4-nitrobenzyl pivalate

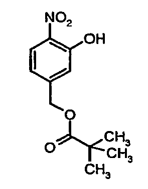

A mixture of 5-(hydroxymethyl)-2-nitrophenol (11.35 g, 67.15 mmol) and 3-(2,2-dimethylpropanoyl)-1,3-thiazolidine-2-thione (15.0 g, 73.89 mmol), which may be prepared in a manner analogous to the literature procedure (Yamada, S. Tetrahedron Letters 1992, 33, 2171-2174), was stirred in toluene (670 mL) at 100 °C for 40 h, then cooled to room temperature. The reaction was concentrated under vacuum to a volume of approximately 200 mL and the resulting slurry was filtered through filter paper, washing the solid with cold toluene. The filtrate was then concentrated under vacuum and purified by silica gel chromatography (0 to 20% ethyl acetate:hexanes) to give 11.09 g (65%) of the title compound as a clear yellow oil. ¹H NMR (400 MHz, DMSO-*d₆*): δ 11.05 (s, 1H), 7.87 (d, 1H, *J*= 8.42 Hz), 7.06 (s, 1H), 6.90 (dd, 1H, *J=* 1.46, 8.42 Hz), 5.09 (s, 2H), 1.18 (s, 9H).

### Step C - 4-Nitro-3-{[(trifluoromethyl)sulfonyl]oxy}benzyl pivalate

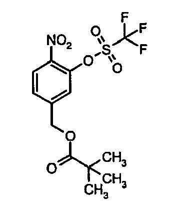

To a stirred, cooled (0 °C) solution of 3-hydroxy-4-nitrobenzyl pivalate (11.11 g, 43.9 mmol) and *N*-phenyltrifluoromethanesulfonimide (16.51 g, 46.2 mmol) in dichloromethane (220 mL) was slowly added *N*,*N*-diisopropylethylamine (15.5 mL, 88.9 mmol). The reaction was stirred for 45 min at 0 °C, then 45 min at room temperature. The reaction was then concentrated under vacuum and purified by silica gel chromatography (5 to 20% ethyl acetate:hexanes) to give 16.87 g (99%) of the title compound as an off white solid. ¹H NMR (400 MHz, DMSO-*d₆*): δ 8.36 (d, 1H, *J*= 8.42 Hz), 7.69-7.75 (m, 2H), 5.27 (s, 2H), 1.19 (s, 9H).

### Step D - Methyl 5-[(5-{[(2,2-dimethylpropanoyl)oxy]methyl}-2-nitrophenyl)amino]-3-({(1R)-1-[2-(trifluoromethyl)phenyl]ethyl}oxy)thiophene-2-carboxylate

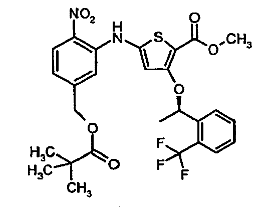

A mixture of 4-nitro-3-{[(trifluoromethyl)sulfonyl]oxy}benzyl pivalate (1.0 g, 2.60 mmol), methyl 5-amino-3-({(1R)-1-[2-(trifluoromethyl)phenyl]ethyl}-oxy)thiophene-2-carboxylate (1.34 g, 3.88 mmol), tetrakis(triphenylphosphine)palladium (0) (150 mg, 0.13 mmol), triphenylphosphine (68 mg, 0.26 mmol) and potassium carbonate (900 mg, 6.5 mmol) were stirred in toluene (5.2 mL) at 100 °C for 2 h, then cooled to room temperature and filtered through Celite, washing with ethyl acetate and dichloromethane. The filtrate was concentrated under vacuum and purified by silica gel chromatography (5 to 25% ethyl acetate:hexanes) to give 1.26 g (84%) of the title compound as a red oil. ¹H NMR (400 MHz, DMSO-d₆): δ 9.75 (s, 1H), 8.09 (d, 1H, *J*= 8.6 Hz), 7.89 (d, 1H, *J* = 7.87 Hz), 7.69-7.78 (m, 2H), 7.52 (t, 1H, *J* = 7.59 Hz), 7.34 (s, 1H), 7.01 (dd, 1H, *J* = 1.46, 8.60 Hz), 6.62 (s, 1H), 5.70-5.75 (m, 1H), 5.07 (s, 2H), 3.74 (s, 3H), 1.58 (d, 3H, *J* = 6.22 Hz), 1.13 (s, 9H).

### Step E - Methyl 5-[(2-amino-5-{[(2,2-dimethylpropanoyl)oxy]methyl} phenyl)amino]-3-({(1R)-1-[2-(trifluoromethyl)phenyl]ethyl}oxy)thiophene-2-carboxylate

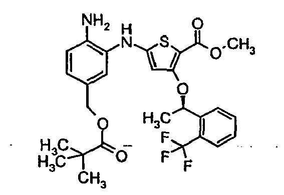

A mixture of methyl 5-[(5-{[(2,2-dimethylpropanoyl)oxy]methyl}-2-nitrophenyl)amino]-3-({(1R)-1-[2-(trifluoromethyl)phenyl]ethyl}oxy)thiophene-2-carboxylate (2.42 g, 4.17 mmol) and platinum (sulfided, 5 wt% on carbon) (811 mg, 0.21 mmol) in ethyl acetate (30 mL) was added to a high-pressure reaction flask. The reaction was purged with vacuum and nitrogen gas, then hydrogen gas was applied at 50 psi for 1 h. The reaction mixture was filtered through Celite, washing with ethyl acetate. The filtrate was concentrated under vacuum to give 2.27 g (99%) of the title compound as a tan solid. ¹H NMR (400 MHz, DMSO-*d₆*): δ 8.62 (s, 1H), 7.84 (d, 1H, *J*= 7.87 Hz), 7.72 (dd, 2H, *J*= 7.60, 13.09 Hz), 7.50 (t, 1H, *J*= 7.60 Hz), 7.01 (d, 1H, *J*= 1.46 Hz), 6.88 (dd, 1H, *J*= 1.74, 8.15), 6.68 (d, 1H, *J*= 8.24 Hz), 5.83 (s, 1H), 5.59-5.65 (m, 1H), 4.97 (s, 2H), 4.85 (s, 2H), 3.64 (s, 3H), 1.55 (d, 3H, *J*= 6.23 Hz), 1.11 (s, 9H); MS (ESI): 550 [M+H]⁺.

### Step F - Methyl 5-(6-{[(2,2-dimethylpropanoyl)oxy]methyl}-1H-benzimidazol-1-yl)-3-({(1R)-1-[2-(trifluoromethyl)phenyl]ethyl}oxy)thiophene-2-carboxylate

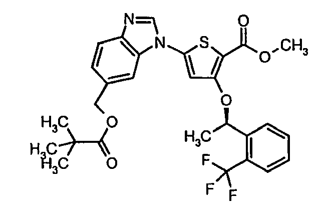

To a mixture of methyl 5-[(2-amino-5-{[(2,2-dimethylpropanoyl)oxy] methyl}phenyl)amino]-3-({(1R)-1-[2-(trifluoromethyl)phenyl]ethyl}-oxy)thiophene-2-carboxylate (2.27 g, 4.13 mmol) in triethyl orthoformate (10 mL, 60.2 mmol) and dichloromethane (3 mL) was added pyridinium p-toluenesulfonate (100 mg, 0.4 mmol). The reaction was stirred at 40°C for 1 h, then cooled to room temperature. The entire reaction mixture was loaded onto silica gel and purified by silica gel chromatography (0 to 50% ethyl acetate:hexanes) to give 2.0 g (86%) of the title compound as a light tan solid. ¹H NMR (400 MHz, DMSO-*d₆*): δ 8.65 (s, 1H), 7.99 (d, 1H, *J*= 7.87 Hz), 7.75-7.80 (m, 2H), 7.72 (d, 1H, *J*= 7.87 Hz), 7.63 (s, 1H), 7.53 (t, 1H, *J*= 7.60 Hz), 7.40 (s, 1H), 7.35 (d, 1H, *J* = 8.42 Hz), 5.96 (q, 1H, *J=* 6.10 Hz), 5.21 (s, 2H), 3.83 (s, 3H), 1.65 (d, 3H, *J*= 6.23 Hz), 1.16 (s, 9H); MS (ESI): 561 [M+H]⁺.

### Step G - Methyl 5-[6-(hydroxymethyl)-1H-benzimidazol-1-yl]-3-({(1R)-1-[2-(trifluoromethyl)phenyl]ethyl}oxy)thiophene-2-carboxylate

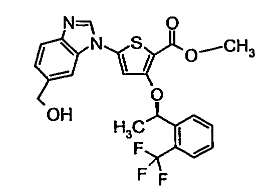

To a stirred solution of methyl 5-(6-{[(2,2-dimethylpropanoyl)oxy]methyl}-1H-benzimidazol-1-yl)-3-({(1R)-1-[2-(trifluoromethyl)phenyl]ethyl}oxy)thiophene-2-carboxylate (5.21 g, 9.30 mmol) in methanol (24 mL) was added 0.5M sodium hydroxide in methanol (24.0 mL, 12 mmol). The reaction was stirred at room temperature for 72 h, then quenched with acetic acid (2 mL). The mixture was diluted with dichloromethane (350 mL) and half saturated aqueous brine solution (150 mL). The aqueous layer was extracted with dichloromethane (250 mL). The combined organics were dried over magnesium sulfate and concentrated under vacuum to give 4.40 g (99%) of the title compound as a light yellow solid. ¹H NMR (400 MHz, DMSO-*d₆*): δ 8.58 (s, 1H), 7.99 (d, 1H, *J*= 7.87 Hz), 7.69-7.81 (m, 3H), 7.51-7.58 (m, 2H), 7.38 (s, 1H), 7.30 (d, 1H, *J* = 8.42), 5.96 (q, 1H, *J*= 6.10 Hz), 5.30 (t, 1H, *J*= 5.77 Hz) 4.62 (d, 2H, *J*= 5.86 Hz), 3.83 (s, 3H), 1.65 (d, 3H, *J*= 6.23 Hz); MS (ESI): 477 [M+H]⁺.

### Step H - Methyl 5-[6-(chloromethyl)-1H-benzimidazol-1-yl]-3-({(1R)-1-[2-(trifluoromethyl)phenyl]ethyl}oxy)thiophene-2-carboxylate (Title Compound)

To a stirred solution of methyl 5-[6-(hydroxymethyl)-1H-benzimidazol-1-yl]-3-({(1R)-1-[2-(trifluoromethyl)phenyl]ethyl}oxy)thiophene-2-carboxylate (1.47 g, 3.08 mmol) and triphenylphosphine (1.05 g, 4.01 mmol) in dichloromethane (30 mL) was added N-chlorosuccinimide (0.53 g, 4.01 mmol). The reaction was then heated to reflux and stirred for 20 minutes, then cooled to room temperature. The reaction was diluted with dichloromethane (400 mL) and half saturated aqueous brine solution (150 mL). The aqueous layer was then extracted with dichloromethane. The combined organic layers were dried over sodium sulfate, concentrated under vacuum and purified by silica gel chromatography (10 to 60% ethyl acetate:hexanes) to give 1.4 g (92%) of the title compound as a white solid. ¹H NMR (400 MHz, DMSO-*d₆*): δ 8.65 (s, 1H), 7.99 (d, 1H, *J*= 7.87 Hz), 7.72-7.81 (m, 3H), 7.69 (s, 1H), 7.54 (t, 1H, *J*= 7.69 Hz), 7.43 (d, 1H, *J*= 8.42), 7.38 (s, 1H), 5.97 (q, 1H, *J*= 6.10 Hz), 4.91 (s, 2H), 3.84 (s, 3H), 1.66 (d, 3H, *J*= 6.23 Hz); MS (ESI): 494 [M+H]⁺.

### Example 4: Methyl 5-[6-(chloromethyl)-1H-benzimidazol-1-yl]-3-{[(1R)-1-(2-chlorophenyl)ethyl]oxy}(thiophene-2-carboxylate

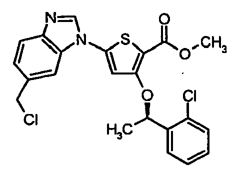

### Step A - Methyl 3-{[(1R)-1-(2-chlorophenyl)ethyl]oxy}-5-[(5-{[(2,2-dimethylpropanoyl)oxy]methyl}-2-nitrophenyl)amino]thiophene-2-carboxylate

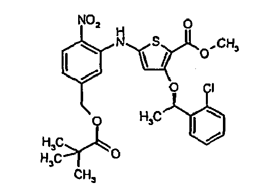

To a mixture of methyl 5-amino-3-{[(1R)-1-(2-chlorophenyl)ethyl]oxy}-thiophene-2-carboxylate (9.09 g, 29.2 mmol) and 4-nitro-3-{[(trifluoromethyl)sulfonyl]oxy} benzyl pivalate (10.7 g, 27.8 mmol) in toluene (200 mL) was added tris(dibenzylideneacetone)dipalladium (0) (0.51 g, 0.56 mmol), 9,9-dimethyl-4,5-bis(diphenylphosphino)xanthene (0.71 g, 1.22 mmol) and cesium carbonate (45.2 g, 139 mmol). The reaction was stirred at 60 °C for 3 h, then cooled to room temperature and filtered, washing the solids with ethyl acetate and dichloromethane. The filtrate was concentrated under vacuum and purified by silica gel chromatography (0 to 50% ethyl acetate:hexanes) to give 7.8 g (51 %) of the title compound as a red foam. MS (ESI): 547 [M+H]⁺.

### Step B - Methyl 5-[(2-amino-5-{[(2,2-dimethylpropanoyl)oxy]methyl} phenyl)amino]-3-{[(1R)-1-(2-chlorophenyl)-ethyl]oxy}thiophene-2-carboxylate

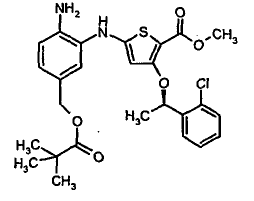

The title compound was prepared from methyl 3-{[(1R)-1-(2-chlorophenyl)ethyl]oxy}-5-[(5-{[(2,2-dimethylpropanoyl)oxy]methyl}-2-nitrophenyl)amino]thiophene-2-carboxylate by a procedure analogous to **Example 3**, Step E. ¹H NMR (400 MHz, DMSO-*d₆*): δ 8.63 (s, 1H), 7.57 (dd, 1H, *J*= 1.65, 7.69 Hz), 7.44 (d, 1H, *J*= 7.87 Hz), 7.37-7.41 (m, 1H), 7.29-7.34 (m, 1H), 7.05 (d, 1H, *J*= 1.83 Hz), 6.88 (dd, 1H, *J*=1.83, 8.06 Hz), 6.68 (d, 1H, *J=* 8.06 Hz), 5.84 (s, 1H), 5.62 (q, 1H, *J*= 6.29 Hz), 5.01 (br s, 2H), 4.87 (s, 2H), 3.63 (s, 3H), 1.53 (d, 3H, *J=* 6.23 Hz), 1.12 (s, 9H).

### Step C - Methyl 3-{[(1R)-1-(2-chlorophenyl)ethyl]oxy}-5-(6-{[2,2-dimethylpropanoyl)oxy]methyl}-1H-benzimidazol-1-yl)thiophene-2-carboxylate

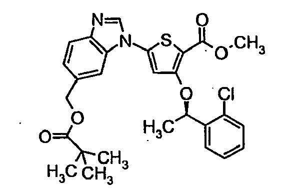

The title compound was prepared from methyl 5-[(2-amino-5-{[(2,2-dimethylpropanoyl)oxy]methyl}phenyl)amino]-3-{[(1R)-1-(2-chlorophenyl)-ethyl]oxy}thiophene-2-carboxylate by a procedure analogous to **Example 3**, Step F. ¹H NMR (400 MHz, DMSO-*d₆*): δ 8.69 (s, 1H), 7.78 (d, 1H, *J*=8.23 Hz), 7.75 (dd, 1H, *J*=1.46, 7.68 Hz), 7.66 (s,1H), 7.41-7.47 (m, 3H), 7.32-7.37 (m, 2H), 5.96 (q, 1H, *J* = 6.28 Hz), 5.23 (s, 2H), 3.82 (s, 3H), 1.63 (d, 3H, *J*= 6.22 Hz), 1.17 (s, 9H).

### Step D - Methyl 3-{[(1R)-1-(2-chlorophenyl)ethyl]oxy}-5-[6-(hydroxymethyl)-1H-benzimidazol-1-yl]thiophene-2-carboxylate

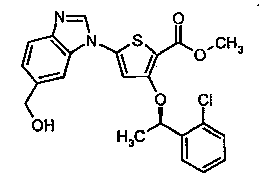

The title compound was prepared from methyl 3-{[(1R)-1-(2-chlorophenyl)ethyl]oxy}-5-(6-{[(2,2-dimethylpropanoyl)oxy]methyl}-1H-benzimidazol-1-yl)thiophene-2-carboxylate by a procedure analogous to **Example 3**, Step G. ¹H NMR (400 MHz, DMSO-d₆): δ 8.61 (s, 1H), 7.67-7.74 (m, 2H), 7.61 (s, 1H), 7.39-7.46 (m, 3H), 7.27-7.34 (m, 2H); 5.94 (q, 1H, *J* = 6.29 Hz), 5.29 (t, 1H, *J* = 5.77 Hz), 4.61 (d, 2H, *J* = 5.68 Hz), 3.80 (s, 3H), 1.61 (d, 3H, *J* = 6.23 Hz).

### Step E - Methyl 5-[6-(chloromethyl)-1H-benzimidazol-1-yl]-3-{[(1R)-1-(2-chlorophenyl)ethyl]oxy}thiophene-2-carboxylate (Title Compound)

The title compound was prepared from methyl 3-{[(1R)-1-(2-chlorophenyl)ethyl]oxy}-5-[6-(hydroxymethyl)-1H-benzimidazol-1-yl]thiophene-2-carboxylate by a procedure analogous to **Example 3**, Step H. ¹H NMR (400 MHz, DMSO-*d₆*): δ 8.69 (s, 1H), 7.78 (d, 1H, *J*= 8.24 Hz), 7.72-7.75 (m, 2H) 7.42-7.49 (m, 4H), 7.35 (dt, 1H, *J*= 1.37, 7.65 Hz), 5.97 (q, 1H, *J*= 6.29 Hz), 4.93 (s, 2H), 3.83 (s, 3H) 1.64 (d, 3H, *J*= 6.23 Hz); MS (ESI): 461 [M+H]⁺.

### Example 5: 5-(5-Bromo-1H-benzimidazol-1-yl)-3-({(1R)-1-[2-(trifluoromethyl)phenyl]ethyl}oxy)thiophene-2-carboxamide

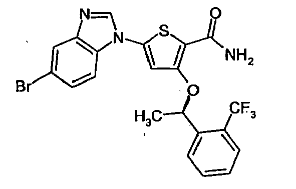

### Step A - methyl 5-[(4-bromo-2-nitrophenyl)amino]-3-({(1R)-1-[2-(trifluoromethyl)phenyl]ethyl}oxy)thiophene-2-carboxylate

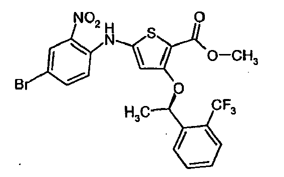

To a stirred mixture of methyl 5-amino-3-({(1*R*)-1-[2-(trifluoromethyl)phenyl]ethyl}oxy)-2-thiophenecarboxylate (10.0 g, 29.0 mmol), 2,5-dibromonitrobenzene (8.13 g, 29.0 mmol), tris(dibenzylideneacetone)dipalladium (0) (0.53 g, 0.58 mmol), and 4,5-bis(diphenylphosphino)-9,9 dimethylxanthene (0.73 g, 1.3 mmol) in toluene (100 mL) under nitrogen was added cesium carbonate (47.0 g, 144 mmol). The reaction mixture was stirred at 55 °C for 1.75 h after which time the mixture was cooled slightly and concentrated to dryness *in vacuo.* The solids were slurried in dichloromethane and filtered. The filtrate was concentrated onto silica gel. Purification by column chromatography (3 to 40% ethyl acetate:hexanes) provided 9.69 g (61 %) of the title compound as a red foam. ¹H NMR (400 MHz, CDCl₃): δ 9.46 (s, 1H), 8.31 (d, 1H, *J*= 2.4 Hz), 7.88 (s, 1H, *J*= 8.0 Hz), 7.61 (m, 2H), 7.47 (m, 2H), 7.10 (d, 1H, *J*= 8.8 Hz), 6.42 (s, 1H), 5.70 (m, 1H), 3.86 (s, 3H), 1.71 (d, 3H, *J*= 6.4 Hz).

### Step B - Methyl 5-[(2-amino-4-bromophenyl)amino]-3-({(1R)-1-[2-(trifluoromethyl)phenyl]ethyl}oxy)thiophene-2-carboxylate

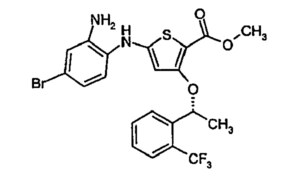

Methyl 5-[(4-bromo-2-nitrophenyl)amino]-3-({(1*R*)-1-[2-(trifluoromethyl)phenyl]-ethyl}oxy)thiophene-2-carboxylate (8.54 g, 15.7 mmol) and sulfided platinum (5 wt% on carbon, 3.05 g, 0.785 mmol) were stirred in ethyl acetate (250 mL) under 55 psi of hydrogen for 1.25 h after which time the mixture was filtered and concentrated to give the title compound as a yellow foam (8.66 g crude product). MS (ESI): 516.0 [M]⁺.

### Step C - Methyl 5-(5-bromo-1H-benzimidazol-1-yl)-3-({(1R)-1-[2-(trifluoromethyl)-phenyl]ethyl}oxy)thiophene-2-carboxylate

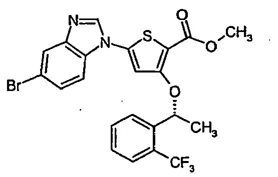

Methyl 5-[(2-amino-4-bromophenyl)amino]-3-({(1*R*)-1-[2-(trifluoromethyl)phenyl]-ethyl}oxy)thiophene-2-carboxylate (8.66 g crude product) and a catalytic amount of pyridinium p-toluenesulfonate (∼20 mg) were stirred in triethyl orthoformate:dichloromethane (30 mL:10 mL) at room temperature for 45 min after which time the reaction mixture was purified by column chromatography (0-30-50% ethyl acetate:hexanes). The resulting foam was triturated in ether to give 6.04 g (73%, 2 steps) of the title compound as a gray solid. ¹H NMR (400 MHz, CDCl₃): δ 7.90 (m, 3H), 7.61 (m, 2H), 7.41 (m, 2H), 7.28 (d, 1H, *J*= 8.4 Hz), 6.72 (s, 1H), 5.78 (m, 1H), 3.90 (s, 3H), 1.75 (d, 3H, *J*= 6.4 Hz).

### Step D - 5-(5-bromo-1H-benzimidazol-1-yl)-3-({(1R)-1-[2-(trifluoromethyl)phenyl]-ethyl}oxy)thiophene-2-carboxamide (Title Compound)

The title compound was prepared from methyl 5-(5-bromo-1*H-*benzimidazol-1-yl)-3-({(1*R*)-1-[2-(trifluoromethyl)phenyl]ethyl}oxy)thiophene-2-carboxylate (3.98 g, 7.58 mmol) and 100 mL 7 N ammonia in methanol heating in a sealed vessel at 95 °C for 72 h. The mixture was cooled to room temperature and filtered to give a yellow solid. The remaining filtrate was purified by column chromatography (0 to 20% methanol:dichloromethane), and the resulting solid was combined with the above yellow solid to give 3.21 g (83%) of the title compound. ¹H NMR (400 MHz, CDCl₃): δ 7.94 (d, 1H, *J*= 1.6Hz), 7.89 (s, 1H), 7.66 (m, 3H), 7.43 (m, 2H), 7.28 (m, 1H), 7.17 (s, 1H), 6.61 (s, 1H), 5.83 (s, 1H), 5.78 (m, 1H), 1.79 (d, 3H, *J*= 6.4 Hz). MS (ESI): 511.0 [M+H]⁺.

### Example 6: 5-(5-Bromo-1H-benzimidazol-1-yl)-3-{[(1R)-1-(2-chlorophenyl)ethyl]oxy}thiophene-2-carboxamide

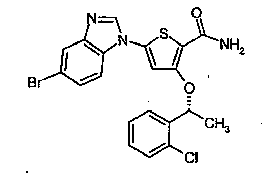

### Step A - Methyl 5-[(4-bromo-2-nitrophenyl)amino]-3-{[(1R)-1-(2-chlorophenyl)ethyl]oxy}thiophene-2-carboxylate

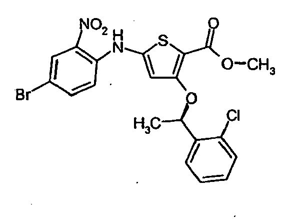

The title compound was prepared from methyl 5-amino-3-{[(1*R*)-1-(2-chlorophenyl)ethyl]oxy}-2-thiophenecarboxylate by a procedure analogous to **Example 5**, Step A. MS (ESI): 534.0 [M+Na]⁺.

### Step B - Methyl 5-[(2-amino-4-bromophenyl)amino]-3-{[(1R)-1-(2-chlorophenyl)ethyl]oxy}thiophene-2-carboxylate

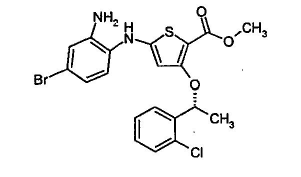

The title compound was prepared from methyl 5-[(4-bromo-2-nitrophenyl)amino]-3-{[(1*R*)-1-(2-chlorophenyl)ethyl]oxy}thiophene-2-carboxylate by a procedure analogous to **Example 5**, Step B. MS (ESI): 482.0 [M+H]⁺.

### Step C - Methyl 5-(5-bromo-1H-benzimidazol-1-yl)-3-{[(1R)-1-(2-chlorophenyl)ethyl]oxy}thiophene-2-carboxylate

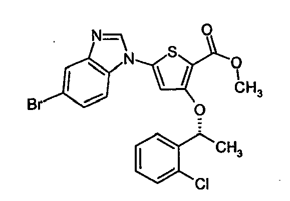

The title compound was prepared from methyl 5-[(2-amino-4-bromophenyl)amino]-3-{[(1*R*)-1-(2-chlorophenyl)ethyl]oxy}thiophene-2-carboxylate by a procedure analogous to **Example 5**, Step C. ¹H NMR (300 MHz, CDCl₃): δ 8.02 (s, 1H), 7.70 (dd, 1H, *J*= 1.8, 7.6 Hz), 7.51-7.28 (m, 6H), 6.74(s, 1H), 5.86 (q, 1H, *J*= 6.3 Hz), 3.97 (s, 3H), 1.79 (d, 3H, *J*= 6.5 Hz); MS (APCI): 492.79 [M+H]⁺.

### Step D - 5-(5-Bromo-1H-benzimidazol-1-yl)-3-{[(1R)-1-(2-chlorophenyl)ethyl]-oxy}thiophene-2-carboxamide (Title Compound)

The title compound was prepared from methyl 5-(5-bromo-1*H*-benzimidazol-1-yl)-3-{[(1*R*)-1-(2-chlorophenyl)ethyl]oxy}thiophene-2-carboxylate by a procedure analogous to **Example 5**, Step D. ¹H NMR (400 MHz, CDCl₃): δ 8.00 (s, 1H), 7.97 (d, *J*=1.3Hz, 1H), 7.46-7.41 (m, 3H), 7.35-7.26 (m, 3H), 7.17 (br s, 1H), 6.60 (s, 1H), 5.85 (q, *J*=6.41 Hz, 1H), 5.75 (br s, 1H), 1.76 (d, *J*=6.41 Hz, 3H). MS (APCI): 478.0 [M+H]⁺.

### Example 7: 5-[5-6-Fluoropyridin-3-yl)-1H-benzimidazol-1-yl]-3-({(1R)-1-[2-(trifluoromethyl)phenyl]ethyl}oxy)thiophene-2-carboxamide

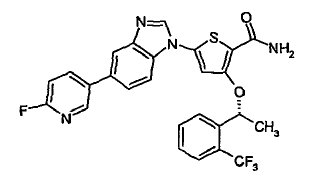

6-Fluoropyridin-3-ylboronic acid (0.45 g, 3.20 mmol), 5-(5-bromo-1*H-*benzimidazol-1-yl)-3-{[(1*R*)-1-(2-(trifluoromethyl)phenyl)ethyl]oxy}thiophene-2-carboxamide (1.27 g, 2.66 mmol), 1,1'-bisdiphenylphosphinoferrocene dichloropalladium (II) (0.24 g, 0.29 mmol), and sodium carbonate (1 N in water, 7.8 mL) were combined in *N,N*-dimethylacetamide (10 mL). The reaction mixture was stirred under nitrogen while heating at 100 °C for 10 min after which time the mixture was cooled slightly, and the solvent removed *in vacuo.* The resulting solid was dissolved in dichloromethane and purified by column chromatography (0 to 10% methanol:dichloromethane) to afford 1.04 g (79%) as a tan solid. ¹H NMR (400 MHz, CDCl₃): δ 8.46 (s, 1H), 8.00 (m, 3H), 7.69 (m, 3H), 7.51 (m, 3H), 7.20(s, 1H), 7.03 (m, 1H), 6.69 (s, 1H), 5.87 (m, 1H), 5.78 (br s, 1H), 1.82 (d, 3H, *J*= 6.0 Hz); MS (ESI): 527.2 [M+H]⁺.

### Example 8: 3-{[(1R)-1-(2-Chlorophenyl)ethyl]oxy}-5-[5-(6-fluoropyridin-3-yl)-1H-benzimidazol-1-yl]thiophene-2-carboxamide

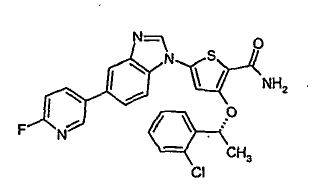

The title compound was prepared from 5-(5-bromo-1*H*-benzimidazol-1-yl)-3-{[(1*R*)-1-(2-chlorophenyl)ethyl]oxy}thiophene-2-carboxamide by a procedure analogous to **Example 7**. ¹H NMR (400 MHz, CDCl₃): δ 8.47 (s, 1H), 8.02 (m, 2H), 7.49 (m, 4H), 7.34 (m, 2H), 7.21 (br s, 1H), 7.03 (m, 1H), 6.67 (s, 1H), 5.89 (m, 1H), 5.76 (br s, 1H), 1.79 (d, 3H, *J* = 5.6 Hz); MS (ESI): 493.1 [M+H]⁺.

### Example 9: Methyl 5-[5-(6-chloropyridin-3-yl)-1H-benzimidazol-1-yl]-3-({(1R)-1-[2-trifluoromethyl)phenyl]ethyl}oxy)thiophene-2-carboxylate

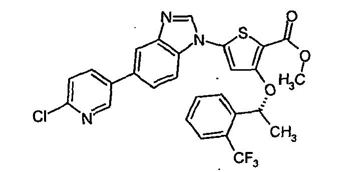

The title compound was prepared from methyl 5-(5-bromo-1*H*-benzimidazol-1-yl)-3-({(1*R*)-1-[2-(trifluoromethyl)phenyl]ethyl}oxy)thiophene-2-carboxylate by a procedure analogous to **Example 7**. ¹H NMR (400 MHz, CDCl₃): δ 8.64 (d, 1H, *J*= 2.8 Hz), 8.00 (d, 2H, *J*= 14.8 Hz), 7.88 (m, 2H), 7.58 (m, 4H), 7.34 (m, 2H), 6.78 (s, 1H), 5.81 (m, 1H), 3.92 (s, 3H), 1.77 (d, 3H, *J*= 6.4 Hz); MS (ESI): 558.1 [M+H]⁺.

### Example 10: Methyl 5-[5-(2-chloropyridin-4-yl)-1H-benzimidazol-1-yl]-3 ({(1R)-1-[2-(trifluoromethyl)phenyl]ethyl}oxy)thiophene-2-carboxylate

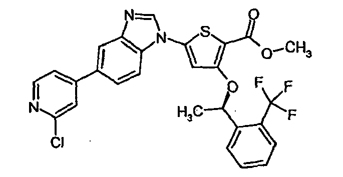

2-Chloropyridine-4-boronic acid (0.206 g, 0.86 mmol), methyl 5-(5-bromo-1*H-*benzimidazol-1-yl)-3-({(1*R*)-1-[2-(trifluoromethyl)phenyl]ethyl}oxy)thiophene-2-carboxylate (0.226 g, 0.43 mmol), and sodium carbonate (1 N in water, 1.7 mL) were combined in *N,N*-dimethylacetamide (6 mL). 1,1'-bisdiphenylphosphino-ferrocene dichloropalladium (II) (0.070 g, 0.086 mmol) was added and the reaction mixture was stirred under nitrogen while heating at 80 °C for 3 h. The mixture was then cooled and partitioned between 5:1 dichloromethane:methanol and saturated aqueous sodium bicarbonate. The organic layer was washed with twice with brine, dried over sodium sulfate, and concentrated onto Celite. Purification by column chromatography (10 to 100% 1/9/90 ammonium hydroxide/methanol/dichloromethane: dichloromethane) provided 0.105 g (44%) of the title compound as an off-white solid. ¹H NMR (400 MHz, CDCl₃): δ 8.43 (d, 1H, *J*= 5.3 Hz), 8.04 (d, 2H, *J*= 13.9 Hz), 7.90 (d, 1H, *J*= 7.9 Hz), 7.66 (d, 1H, *J*= 7.9 Hz), 7.58 (m, 4H), 7.47 (dd, 1H, *J*= 1.1, 5.1 Hz), 7.41 (t, 1H, *J*= 7.6 Hz), 6.78 (s, 1H), 5.81 (q, 1H, *J*= 6.2 Hz), 3.92 (s, 3H), 1.77 (d, 3H, *J*= 6.2 Hz); MS (ESI): 558.1 [M+H]⁺.

### Example 11: 5-[5-(2-Chloropyridin-4-yl)-1H-benzimidazol-1-yl]-3-({(1R)-1-[2-(trifluoromethyl)phenyl]ethyl}oxy)thiophene-2-carboxamide

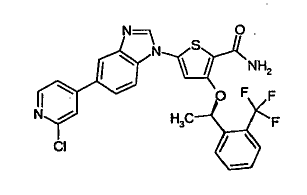

The title compound was prepared from methyl 5-[5-(2-chloropyridin-4-yl)-1*H-*benzimidazol-1-yl]-3-({(1*R*)-1-[2-(trifluoromethyl)phenyl]ethyl}oxy)thiophene-2-carboxylate by a procedure analogous to **Example 5**, Step D. ¹H NMR (400 MHz, CDCl₃): δ 8.50 (d, 1H, *J*= 5.2 Hz), 8.10 (d, 2H, *J*= 13.9 Hz), 7.66 (m, 8H), 7.26 (s, 1H), 6.74 (s, 1H), 5.92 (m, 2H), 1.87 (d, 3H, *J*= 6.2 Hz).

### Example 12: Methyl 3-{[(1R)-1-(2-chlorophenyl)ethyl]oxy}-5-[5-(2-chloropyridin-4-yl)-1H-benzimidazol-1-yl]thiophene-2-carboxylate

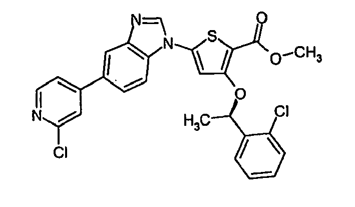

The title compound was prepared from methyl 5-(5-bromo-1*H*-benzimidazol-1-yl)-3-{[(1*R*)-1-(2-chlorophenyl)ethyl]oxylthiophene-2-carboxylate by a procedure analogous to **Example 10**. ¹H NMR (400 MHz, CDCl₃): δ 8.51 (d, 1H, *J*= 5.2 Hz), 8.18 (d, 2H, *J*= 11.2 Hz), 7.63 (m, 1H), 7.40 (m, 2H), 6.81 (s, 1H), 5.89 (q, 1H, *J*= 6.3 Hz), 3.98 (s, 3H), 1.81 (d, 3H, *J*= 6.3 Hz).

### Example 13: 3-{[(1R)-1-(2-Chlorophenyl)ethyl]oxy}-5-[5-(2-chloropyridin-4-yl)-1H-benzimidazol-1-yl]thiophene-2-carboxamide

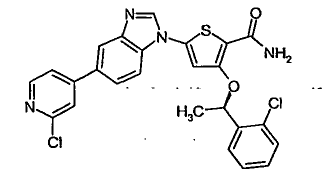

The title compound was prepared from methyl 3-{[(1*R*)-1-(2-chlorophenyl)ethyl]oxy}-5-[5-(2-chloropyridin-4-yl)-1*H*-benzimidazol-1-yl]thiophene-2-carboxylate by a procedure analogous to **Example 5**, Step D. ¹H NMR (400 MHz, CDCl₃): δ 8.45 (d, 1H, *J*= 5.1 Hz), 8.12 (d, 2H, *J*= 17.6 Hz), 7.60 (d, 2H, *J*= 8.2 Hz), 7.53 (d, 1H, *J*= 8.6 Hz), 7.45 (m, 3H), 7.33 (m, 2H), 7.19 (br s, 1H), 6.67 (s, 1H), 5.88 (q, 1H, *J*= 6.4 Hz), 5.76 (br s, 1H), 1.78 (d, 3H, *J*= 6.2 Hz).

### Example 14: 5-[5-(2-Fluoropyridin-4-yl)-1H-benzimidazol-1-yl]-3-({(R)-1-[2-(trifluoromethyl)phenyl]ethyl}oxy)thiophene-2-carboxamide

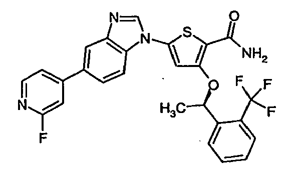

2-Fluoropyridine-4-boronic acid (0.21 g, 1.5 mmol), 5-(5-bromo-1*H-*benzimidazol-1-yl)-3-({(1*R*)-1-[2-(trifluoromethyl)phenyl]ethyl}oxy)thiophene-2-carboxamide (0.505 g, 0.99 mmol), and sodium carbonate (1 N in water, 3.4 mL) were combined in *N,N*-dimethylacetamide (10 mL). 1,1'-bisdiphenylphosphino-ferrocene dichloropalladium (II) (0.078 g, 0.1 mmol) was added and the reaction mixture was stirred under nitrogen while heating at 80 °C for 2 h. The mixture was then cooled and partitioned between ethyl acetate and water. The aqueous layer was extracted twice with ethyl acetate. The combined organics were dried over magnesium sulfate, concentrated onto silica gel and purified by column chromatography (0 to 70% ethyl acetate:hexanes) to afford 0.343 g (67%) of the title compound as a light tan solid. ¹H NMR (400 MHz, CDCl₃): δ 8.29 (d, 1H, *J* = 5.3 Hz), 8.12 (s, 2H), 7.68 (m, 4H), 7.51 (m, 3H), 7.21 (s, 1H), 7.18 (s, 1H), 6.71 (s, 1H), 5.85 (m, 2H), 1.82 (d, 3H, *J*= 5.9 Hz); MS (ESI): 527.2 [M+H]⁺.

### Example 15: 3-{[(1R)-(2-Chlorophenyl)ethyl]oxy}-5-[5-(2-fluoropyridin-4-yl)-1H-benzimidazol-1-yl]thiophene-2-carboxamide

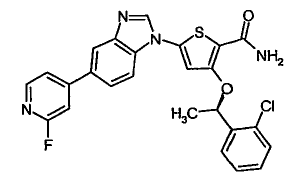

2-Fluoropyridine-4-boronic acid (0.440 g, 3.15 mmol), 5-(5-bromo-1*H-*benzimidazol-1-yl)-3-{[(1*R*)-1-(2-chlorophenyl)ethyl]oxy}thiophene-2-carboxamide (1.0 g, 2.10 mmol), and sodium carbonate (1 N in water, 7.4 mL) were combined in *N,N*-dimethylacetamide (25 mL). 1,1'-bisdiphenylphosphino-ferrocene dichloropalladium (II) (0.171 g, 0.21 mmol) was added and the reaction mixture was stirred under nitrogen while heating at 80 °C for 2 h. The mixture was then cooled and partitioned between dichloromethane and water. The aqueous layer was extracted with dichloromethane (5x). The combined organics were dried over magnesium sulfate, concentrated onto silica gel and purified by column chromatography (10 to 100% ethyl acetate:hexanes) to afford 0.735 g (71%) of the title compound as a tan solid. ¹H NMR (400 MHz, CDCl₃): δ 8.33 (d, 1H, *J*= 5.3 Hz), 8.11 (d, 2H, *J*= 16.0 Hz), 7.50 (m, 7H), 7.26 (s, 1H), 7.23 (br s, 1H), 6.71 (s, 1H), 5.94 (q, 1H, *J*= 6.3 Hz), 5.82 (br s, 1H), 1.84 (d, 3H, *J*= 6.5 Hz); MS (ESI): 493.1 [M+H]⁺.

### Analog 1: 5-[5-(4,4,5,5-Tetramethyl-1,3,2-dioxaborolan-2-yl)-1H-benzimidazol-1-yl]-3-({(1R)-1-[2-(trifluoromethyl)phenyl]ethyl}oxy)ythiophene-2-carboxamide

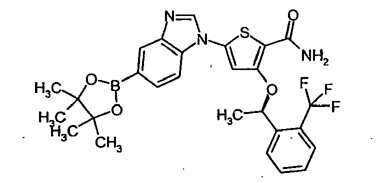

5-(5-bromo-1*H*-benzimidazol-1-yl)-3-({(1*R*)-1-[2-(trifluoromethyl)phenyl]ethyl}-oxy)thiophene-2-carboxamide (0.114 g, 0.223 mmol), bis(pinacolato)diboron (0.068 g, 0.268 mmol), potassium acetate (0.055 g, 0.669 mmol), and dichlorobis(triphenylphosphine)palladium(II) (0.016 g, 0.022 mmol) were combined in *N,N*-dimethylformamide (1.5 mL). The reaction mixture was heated in a Personal Chemistry microwave at 150 °C for 20 minutes after which time the mixture was diluted with ethyl acetate and concentrated onto silica gel. Purification by column chromatography (10-100% ethyl acetate:hexanes) provided 0.062 g (50%) of the title compound as an off-white solid. MS (ESI): 558.2 [M+H]⁺.

### Analog 2: 3-{[(1R)-1-(2-Chlorophenyl)ethyl]oxy}-5-[5-(4,4,5,5-tetramethyl-1,3,2-dioxaborolan-2-yl)-1H-benzimidazol-1-yl]thiophene-2-carboxamide

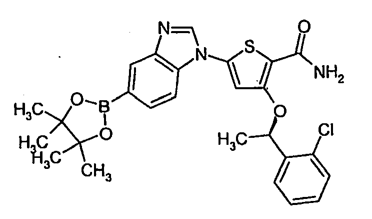

5-(5-bromo-1*H*-benzimidazol-1-yl)-3-{[(1*R*)-1-(2-chlorophenyl)ethyl]oxy}-thiophene-2-carboxamide (0.521 g, 1.09 mmol), bis(pinacolato)diboron (0.330 g, 1.30 mmol), potassium acetate (0.270 g, 3.30 mmol), and dichlorobis(triphenylphosphine)palladium(II) (0.077 g, 0.110 mmol) were combined in *N,N*-dimethylformamide (7.0 mL). The reaction mixture was divided equally between two 5 mL microwave vessels and both heated in a Personal Chemistry microwave at 150 °C for 20 minutes after which time the mixtures were diluted with ethyl acetate, combined, and concentrated onto silica gel. Purification by column chromatography (10 to 100% ethyl acetate:hexanes) provided 0.062 g (50%) of the title compound as an off-white solid. MS (ESI): 524.2 [M+H]⁺.

### Example 16: 5-[5-(2-Chloropyrimidin-4-yl)-1H-benzimidazol-1-yl]-3-({(1R)-1-[2-(trifluoromethyl)phenyl]ethyl}oxy)thiophene-2-carboxamide

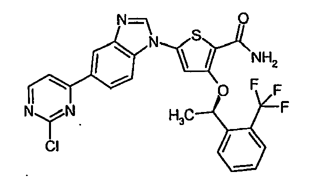

5-[5-(4,4,5,5-tetramethyl-1,3,2-dioxaborolan-2-yl)-1*H*-benzimidazol-1-yl]-3-({(1*R*)-1-[2-(trifluoromethyl)phenyl]ethyl}oxy)thiophene-2-carboxamide (0.193 g, 0.350 mmol) and 2,4-dichloropyrimidine (0.089 g, 0.600 mmol) were dissolved in *N,N*-dimethylacetamide (2.0 mL). Sodium carbonate (1 N in water, 0.7 mL) was added followed by 1,1'-bisdiphenylphosphinoferrocene dichloropalladium (II) (0.029 g, 0.035 mmol). The reaction mixture was stirred under nitrogen while heating at 80 °C for 15 h. The mixture was then cooled and partitioned between ethyl acetate and water. The aqueous layer was extracted with ethyl acetate. The combined organics were dried over magnesium sulfate, concentrated onto silica gel and purified by column chromatography (10 to 100% ethyl acetate:hexanes) to afford 0.100 g (53%) of the title compound as a white solid. MS (ESI): 544.1 [M+H]⁺.

### Example 17: 3-{[1R)-1-(2-Chlorophenyl)ethyl]oxy}-5-[5-(2-chloropyrimidin-4-yl)-1H-benzimidazol-1-yl]thiophene-2-carboxamide

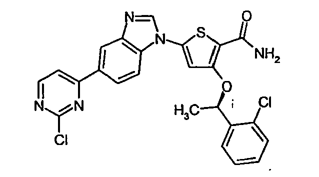

The title compound was prepared from 3-{[(1*R*)-1-(2-chlorophenyl)ethyl]oxy}-5-[5-(4,4,5,5-tetramethyl-1,3,2-dioxaborolan-2-yl)-1*H*-benzimidazol-1-yl]thiophene-2-carboxamide by a procedure analogous to **Example 16**. MS (ESI): 510.1 [M+H]⁺.

### Example 18: Methyl 5-(5-bromo-1H-benzimidazol-1-yl)-3-[(phenylmethyl)oxy]-2-thiophenecarboxylate

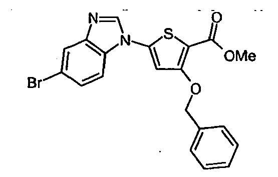

### Step A - Methyl 5-nitro-3-[(phenylmethyl)oxy]-2-thiophenecarboxylate

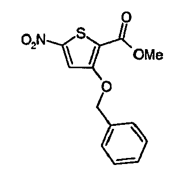

To a solution of methyl 3-hydroxy-5-nitro-2-thiophenecarboxylate (26.4 g, 130 mmol) in *N,N*-dimethylformamide (300 mL) was added potassium carbonate (20.0 g, 145 mmol), followed by benzyl bromide (22.3 g, 130 mmol), and the reaction mixture was stirred at room temperature for 18 h. The solution was filtered to remove the solids, and the filtrate was poured slowly into 1 N HCl (600 mL). A yellow solid precipitated, and this solid was collected by vacuum filtration and was washed with water (3 x 300 mL) providing 37.0 g (97%) of the title compound. ¹H NMR (400 MHz, DMSO-*d₆*): δ 8.23 (s, 1H), 7.48-7.28 (m, 5H), 5.37 (s, 2H), 3.79 (s, 3H).

### Step B - Methyl 5-amino-3-[(phenylmethyl)oxy]-2-thiophenecarboxylate

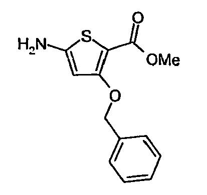

To a flask equipped with a temperature probe, an overhead mechanical stirrer, a reflux condenser, and an addition funnel was added iron powder (36.3 g, 650 mmol) and acetic acid (230 mL). The iron/acetic acid slurry was stirred mechanically and heated to an internal temperature of 50 °C. To the addition funnel was added a solution of methyl 5-nitro-3-[(phenylmethyl)oxy]-2-thiophenecarboxylate (37.0 g, 126 mmol) in acetic acid (300 mL). The solution in the addition funnel was then added dropwise to the iron/acetic acid slurry at a rate such that the internal temperature was maintained at <60 °C (2.5 h total addition time). The reaction mixture was cooled to room temperature, and the entire mixture was then filtered through filter paper to remove insoluble material, rinsing with dichloromethane (500 mL). The solution was concentrated to about 200 mL, rediluted with ethyl acetate (500 mL) and then quenched by addition of 6 N sodium hydroxide (250 mL) and saturated aqueous sodium bicarbonate (200 mL). The aqueous and organic fractions were separated. The aqueous fraction was extracted with ethyl acetate (2 x 400 mL). The organic fractions were combined, dried over magnesium sulfate, filtered, and concentrated to afford 27.0 g (82%) of the title compound as a tan solid. ¹H NMR (400 MHz, DMSO-*d₆*): δ 7.42-7.26 (m, 5H), 6.78 (s, 2H), 5.76 (s, 1H), 5.10 (s, 2H), 3.56 (s, 3H); MS (ESI): 286 [M+Na]⁺.

### Step C - Methyl -5-[(4-bromo-2-nitrophenyl)amino]-3-[(phenylmethyl)oxy]-2 thiophenecarboxylate

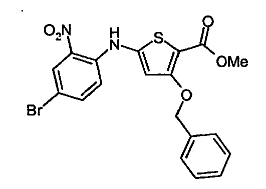

The title compound was prepared from methyl 5-amino-3-[(phenylmethyl)oxy]-2-thiophenecarboxylate and 1,4-dibromo-2-nitrobenzene by a procedure analogous to **Example 1**, Step B. ¹H NMR (400 MHz, CDCl₃): δ 9.54 (s, 1H), 8.33 (s, 1H), 7.53-7.20 (m, 7H), 6.56 (s, 1H), 5.23 (s, 2H), 3.85 (s, 3H).

### Step D - Methyl 5-(5-bromo-1H-benzimidazol-1-yl)-3-[(phenylmethyl)oxy]-2-thiophenecarboxylate (Title Compound)

The title compound was prepared from methyl 5-[(4-bromo-2-nitrophenyl)amino]-3-[(phenylmethyl)oxy]-2-thiophenecarboxylate by a procedure analogous to **Example 1**, Steps C and D. ¹H NMR (400 MHz, CDCl₃): δ 8.05 (d, 1H), 8.01-7.99 (m, 1H), 7.49-7.35 (m, 6H), 7.26 (s, 1H), 6.88 (s, 1H), 5.33 (s, 2H), 3.91 (s, 3H); MS (ESI): 443 & 445 [M+1 & M+3]⁺.

### Intermediate Example 3: 1,1-Dimethylethyl 4-[(3-bromo-4-nitrophenyl)oxy]-1-piperidinecarboxylate

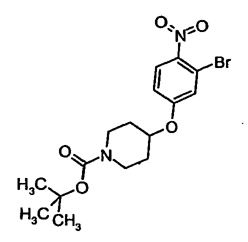

### Step A - 3-Bromo-4-nitrophenol

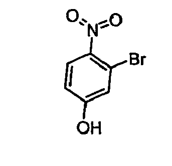

Sulfuric acid (98%, 20 mL, 375 mmol) was added dropwise to a solution of sodium nitrate (27 g, 318 mmol) in water (60 mL) keeping the temperature between 20 and 30 °C. A solution of 3-bromophenol (24 g, 140 mmoL) dissolved in ethanol (50 mL) was then added dropwise, keeping the temperature close to room temperature. The mixture was stirred overnight at room temperature, then poured into ice water (600 mL) and extracted with dichloromethane (3 x 200 mL). The combined organic fractions were dried over magnesium sulfate, filtered, and concentrated onto silica gel. Purification by column chromatography (isocratic, dichloromethane) provided 11.0 g (36%) of the title compound as brown solid. ¹H NMR (300 MHz, DMSO-*d₆*): δ 11.2 (br s, 1H), 8.79 (d, 1H, *J*= 9.0 Hz), 7.17 (d, 1H, *J*= 2.5 Hz), 6.91 (dd, 1H, *J*= 2.5, 9.0 Hz).

### Step B -1,1-Dimethylethyl 4-[(3-bromo-4-nitrophenyl)oxy]-1-piperidinecarboxylate (Title Compound)

3-Bromo-4-nitrophenol (11.6 g, 53.2 mmol), 1,1-dimethylethyl 4-hydroxy-1-piperidinecarboxylate (10.7 g, 53.2 mmol) and triphenylphosphine (24 g, 91 mmol) were combined in diethyl ether (400 mL) at room temperature. Diisopropyl azodicarboxylate (17.2 mL, 87.8 mmol) was added over 10 min. The solution was stirred at 25°C for 18 h. The reaction mixture was extracted with 1 N sodium hydroxide (2 x 50 mL), and brine (100 mL). The organic fractions were combined, dried over magnesium sulfate, filtered, and concentrated onto silica gel. Purification by column chromatography (0 to 40% ethyl acetate:hexanes) provided 16.0 g (75%) of the title compound as yellow solid. ¹H NMR (300 MHz, DMSO-*d₆*): δ 8.04 (d, 1H, *J*= 9.2 Hz), 7.48 (d, 1H, *J*= 2.6 Hz), 7.18 (dd, 1H, *J*= 2.6, 9.2 Hz), 4.79 (m, 1H), 3.6 (m, 2H), 3.15 (m, 2H), 1.89 (m, 2H), 1.52 (s, 2H), 1.38 (s, 9H).

### Example 19: 1,1-Dimethylethyl 4-[(1-{4-hydroxy-5-[(methyloxy)carbonyl]-2-thienyl}-1H-benzimidazol-6-yl)oxyl-1-piperidinecarboxylate

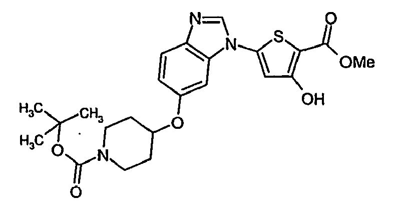

### Step A - Methyl 5-nitro-3-{[2-(trimethylsilyl)ethyl]oxy}-2-thiophenecarboxylate

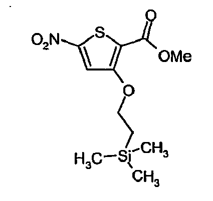

To a slurry of polymer-supported triphenylphosphine (179.0 g, 2.2 mmol/g, 393.8 mmol) in dichloromethane (1 L) was added 2-(trimethylsilyl)ethanol (56.0 mL, 393.8 mmol) and methyl 3-hydroxy-5-nitro-2-thiophenecarboxylate (20.0 g, 98.4 mmol), which may be prepared in a manner analogous to the literature procedure (Barker, J.M.; Huddleston, P.R.; Wood, M.L.; Burkitt, S.A. Journal of Chemical Research (Miniprint) 2001, 1001-1022). The mixture was cooled to 0 °C and diethylazodicarboxylate (62.0 mL, 393.8 mmol) was added dropwise *via* addition funnel. The reaction mixture was stirred at room temperature for 5 h and then was filtered through a fritted funnel and concentrated onto silica gel. Purification by column chromatography provided 29.7 g (99%) of the title compound. ¹H NMR (400 MHz, DMSO-*d*₆): δ 8.17 (s, 1H), 4.30 (t, 2H, *J*= 8.2 Hz), 3.76 (s, 3H), 1.07 (t, 2H, *J*= 8.4 Hz), 0.04 (s, 9 H).

### Step B - Methyl 5 amino-3-{[2-(trimethylsilyl)ethyl]oxy}-2 thiophenecarboxylate

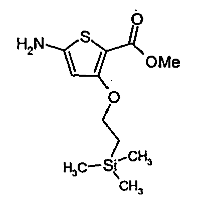

To a flask equipped with a temperature probe, an overhead mechanical stirrer, a reflux condenser, and an addition funnel was added iron powder (21.8 g, 391 mmol) and acetic acid (150 mL). The iron/acetic acid slurry was stirred mechanically and heated to an internal temperature of 50 °C. To the addition funnel was added a solution of methyl 5-nitro-3-{[2-(trimethylsilyl)ethyl]oxy}-2-thiophenecarboxylate (29.7 g, 97.9 mmol) in acetic acid (150 mL). The solution in the addition funnel was then added dropwise to the iron/acetic acid slurry at a rate such that the internal temperature was maintained at <60 °C (2.5 h total addition time). The reaction mixture was cooled to room temperature, and most of the acetic acid was removed *in vacuo.* The remaining material was diluted with ethyl acetate, and then quenched by addition of 5 N sodium hydroxide and saturated aqueous sodium bicarbonate. The entire mixture was then filtered through a pad of Celite to remove insoluble material, rinsing the Celite with additional ethyl acetate. The aqueous and organic fractions were separated. The aqueous fraction was extracted with ethyl acetate. The organic fractions were combined, dried over magnesium sulfate, filtered, and concentrated to afford 26.4 g (98%) of the title compound. ¹H NMR (400 MHz, DMSO-*d*₆): δ 6.74 (s, 2H), 5.72 (s, 1H), 4.06 (t, 2H, *J*= 8.1 Hz), 3.53 (s, 3H), 1.02 (t, *J*= 7.9 Hz, 2H), 0.03 (s, 9H).

### Step C - 1,1-Dimethylethyl 4-({3-[(5-[(methyloxy)carbonyl]-4-{[2-(trimethylsilyl)ethyl]oxy}-2-thienyl)amino]-4-nitrophenyl}oxy)-1-piperidinecarboxylate

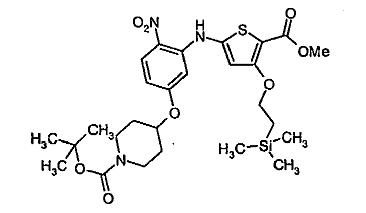

To a stirred mixture of methyl 5-amino-3-{[2-(trimethylsilyl)ethyl]oxy}-2-thiophenecarboxylate (17.6 g, 64.5 mmol) and 1,1-dimethylethyl 4-[(3-bromo-4-nitrophenyl)oxy]-1-piperidinecarboxylate (25.9 g, 64.5 mmol) in degassed 1,4-dioxane (350 mL) under nitrogen was added tris(dibenzylideneacetone)-dipalladium (0) (1.18 g, 1.29 mmol), 4,5-bis(diphenylphosphino)-9,9 dimethylxanthene (1.64 g, 2.84 mmol), and cesium carbonate (105 g, 323 mmol). The reaction mixture was stirred at 60 °C for 18 h after which time the mixture was cooled to room temperature, diluted with diethyl ether and filtered through Celite, washing the solids with diethyl ether and 20% methanol:dichloromethane. The filtrate was concentrated onto silica gel. Purification by column afforded 27.95 g (73%) of the title compound. ¹H NMR (400 MHz, DMSO-*d*₆): δ 9.95 (s, 1H), 8.12 (d, 1H, *J*= 9.4 Hz); 6.99 (s, 1H), 6.94 (d, 1H, *J*= 2.5 Hz), 6.69 (dd, 1H, *J*= 2.6, 9.6 Hz), 4.68 (m, 1H), 4.17 (t, 2H, *J*= 8.3 Hz), 3.67 (s, 3H), 3.61 (m, 2H), 3.16 (m, 2H), 1.90 (m, 2H), 1.54 (m, 2H), 1.38 (s, 9H), 1.07 (t, 2H, *J*= 8.1 Hz), 0.04 (s, 9H).

### Step D - 1,1-Dimethylethyl 4-({4-amino-3-[(5-[(methyloxy)carbonyl]-4-{[2-(trimethylsilyl)ethyl)oxy}-2-thienyl)amino]pheny}oxy)-1-piperidinecarboxylate

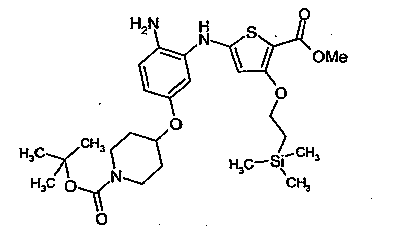

1,1-dimethylethyl 4-({3-[(5-[(methyloxy)carbonyl]-4-{[2-(trimethylsilyl)ethyl]oxy}-2-thienyl)amino]-4-nitrophenyl}oxy)-1-piperidinecarboxylate (27.95 g, 47.07 mmol) and sulfided platinum (5 wt% on carbon, 2.75 g, 14.12 mmol) were stirred in ethyl acetate (200 mL) under 1 atm of hydrogen for 65 h after which time the mixture was filtered and concentrated to give the title compound (26.5 g crude product). ¹H NMR (400 MHz, DMSO-*d*₆): δ 8.64 (s, 1H), 6.80 (s, 1H), 6.66 (d, 1H, *J*= 8.6 Hz), 6.56 (d, 1H, *J*= 8.6 Hz), 6.02 (s, 1H), 4.53 (s, 2H), 4.26 (m, 1H), 4.09 (t, 2H, *J*= 7.7 Hz), 3.60 (m, 2H), 3.56 (s, 3H), 3.10 (m, 2H), 1.80 (m, 2H), 1.44 (m, 2H), 1.36 (s, 9H), 1.03 (t, 2H, *J*= 7.9 Hz), 0.02 (s, 9H).

### Step E - 1,1-Dimethylethyl 4-{[1-(5-[(methyloxy)carbonyl]-4-{[2-(trimethylsilyl)ethyl]oxy}-2-thienyl)-1H-benzimidazol-6-yl]oxy}-1-piperidinecarboxylate

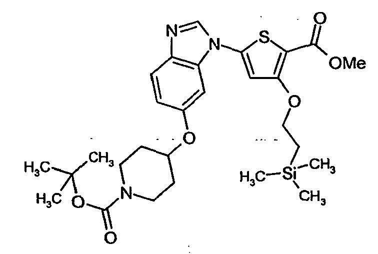

1,1-dimethylethyl 4-({4-amino-3-[(5-[(methyloxy)carbonyl]-4-{[2-(trimethylsilyl)ethyl]oxy}-2-thienyl)amino]phenyl}oxy)-1-piperidinecarboxylate 26.5 g crude product) and a catalytic amount of pyridinium p-toluenesulfonate (591 mg, 2.35 mmol) was stirred in trimethylorthoformate/diethyl ether (113 mL: 50 mL) at room temperature for 2 h after which time the mixture was adsorbed onto silica gel and purified by column chromatography to give the title compound 27.0 g (99% over 2 steps). ¹H NMR (400 MHz, DMSO-*d*₆): δ 8.54 (s, 1H), 7.65 (d, 1H, *J*= 8.9 Hz), 7.53 (s, 1H), 7.29 (d, 1H, *J*= 2.2 Hz), 7.01 (dd, 1H, *J*= 2.2, 8.9 Hz), 4.62 (m, 1H), 4.31 (t, 2H, *J*= 8.2 Hz), 3.73 (s, 3H), 3.62 (m, 2H), 3.18 (m, 2H), 1.89 (m, 2H), 1.53 (m, 2H), 1.37 (s, 9H), 1.11 (t, 2H, *J*= 8.4 Hz), 0.05 (s, 9H); MS (ESI): 574 [M+H]⁺.

### Step F-1,1-Dimethylethyl 4-[(1-{4-hydroxy-5-[(methyloxy)carbonyl]-2-thienyl}-1H-benzimidazol-6-yl)oxy]-1-piperidinecarboxylate (Title Compound)

To a solution of 1,1-dimethylethyl 4-{[1-(5-[(methyloxy)carbonyl]-4-{[2-(trimethylsilyl)ethyl]oxy}-2-thienyl)-1*H-*benzimidazol-6-yl]oxy}-1-piperidinecarboxylate (27 g, 47 mmol) in tetrahydrofuran (50 mL) was added 1.0 M tetrabutylammonium fluoride in tetrahydrofuran (52 mL, 52 mmol). The reaction mixture was stirred for 3 h and quenched with saturated aqueous ammonium chloride solution and extracted with ethyl acetate. The combined organics were dried over magnesium sulfate, filtered, and concentrated onto silica gel. Purification by column chromatography afforded 16.5 g (74%) of the title compound. ¹H NMR (400 MHz, DMSO-*d*₆): δ 8.51 (s, 1H), 7.66 (d, 1H, *J*= 8.8 Hz), 7.27 (d, 1H, *J*= 2:3 Hz), 7.13 (s, 1H), 7.02 (dd, 1H, *J*= 2.3, 8.9), 4.63 (m, 1H), 3.77 (s, 3H) 3.61 (m, 2H), 3.21 (m, 2H), 1.89 (m, 2H), 1.55 (m, 2H), 1.38 (s, 9H); MS (ESI): 474 [M+H]⁺.

### Example 20: 3-{[(1R)-1-(2-Chlorophenyl)ethyl]oxy}-5-[6-(4-piperidinyloxy)-1H-benzimidazol-1-yl]-2-thiophenecarboxamide

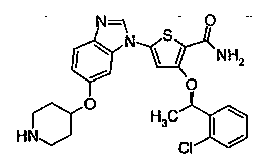

### Step A - 1,1-Dimethylethyl 4-[(1-{4-{[(1R)-1-(2-chlorophenyl)ethyl]oxy}-5-(methyloxy)carbonyl]-2-thienyl}-1H-benzimidazol-6-yl)oxy]-1-piperidinecarboxylate

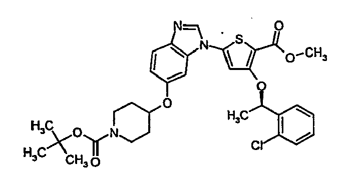

Methyl 3-{[(1*R*)-1-(2-chlorophenyl)ethyl]oxy}-5-(6-hydroxy-1*H-*benzimidazol-1-yl)-2-thiophenecarboxylate (2.00 g, 4.66 mmol), triphenylphosphine (4.89 g, 18.6 mmol), and *t*-butyl 4-hydroxy-1-piperidinecarboxylate (1.88 g, 9.34 mmol) were dissolved in dichloromethane (50 mL) with stirring and cooled to -10 ºC. Diisopropyl azodicarboxylate (1.84 mL, 9.35 mmol) was added dropwise *via* syringe. The reaction was stirred for 5 minutes and allowed to warm to room temperature. The reaction was stirred for 4 h and adsorbed onto silica gel. Purification by flash chromatography afforded the title compound along with small amounts of impurity. ¹H NMR (400 MHz, DMSO-*d*₆): δ 8.47 (s, 1H), 7.70 (dd, 1H, *J*= 1.6, 7.7 Hz), 7.64 (d, 1H, *J*= 8.8 Hz), 7.44-7.37 (m, 2H), 7.34 (s, 1H), 7.31 (m, 1H), 7.15 (d, 1H, *J*= 2.4 Hz), 7.01 (dd, 1H, *J*= 2.2, 8.8 Hz), 5.97 (q, 1H, *J*= 6.2 Hz), 4.59 (m, 1H), 3.80 (s, 3H), 3.65-3.56 (m, 2H), 3.27-3.14 (m, 2H), 1.92-1.81 (m, 2H), 1.60 (d, 3H, *J*= 6.2 Hz), 1.60-1.47 (m, 2H), 1.38 (s, 9H); MS (ESI): 612 [M+H]⁺.

### Step B - Methyl 3-{[(1R)-1-(2-chlorophenyl)ethyl]oxy}-5-[6-(4-piperidinyloxy)-1H-benzimidazol-1-yl]-2-thiophenecarboxylate

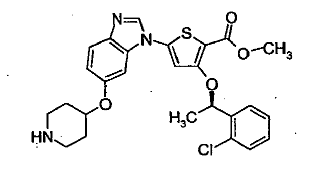

1,1-Dimethylethyl 4-[(1-{4-{[(1*R*-1-(2-chlorophenyl)ethyl]oxy}-5-[(methyloxy)-carbonyl]-2-thienyl}-1*H-*benzimidazol-6-yl)oxy]-1-piperidinecarboxylate from Step A was dissolved in dichloromethane (60 mL) and cooled to 0 °C. Trifluoroacetic acid (15.0 mL, 195 mmol) was added dropwise *via* addition funnel. The reaction was stirred for 1.5 h, and 2 N sodium hydroxide solution (88 mL) was added dropwise *via* addition funnel. Saturated aqueous sodium bicarbonate solution was used to adjust the pH to ∼8. The mixture was poured into a separatory funnel, and the layers were separated. The aqueous layer was washed with dichloromethane (3x) and ethyl acetate (1 x). The combined organic layers were dried over magnesium sulfate, filtered, and concentrated *in vacuo.* Purification by flash chromatography provided 1.95 g (82% over 2 steps) of the title compound. ¹H NMR (400 MHz, DMSO-*d₆*): δ 8.46 (s, 1H), 7.71 (dd, 1H, *J*= 1.7, 7.7 Hz), 7.62 (d, 1H, *J*= 8.8 Hz), 7.46-7.38 (m, 2H), 7.35 (s, 1H), 7.32 (m, 1H), 7.09 (d, 1H, *J*= 2.2 Hz), 6.97 (dd, 1H, *J*= 2.2, 8.8 Hz), 5.97 (q, 1H, *J*= 6.2 Hz), 4.42 (m, 1H), 3.80 (s, 3H), 2.96-2.88 (m, 2H), 2.58-2.49 (m, 2H), 1.93-1.84 (m, 2H), 1.60 (d, 3H, *J*= 6.2 Hz), 1.51-1.39 (m, 2H); MS (ESI): 512 [M+1]⁺.

### Step C - 3-{[(1R)-1-(2-Chlorophenyl)ethyl]oxy}-5-[6-(4-piperidinyloxy)-1H-benzimidazol-1-yl]-2-thiophenecarboxamide (Title Compound)

Methyl 3-{[(1*R*)-1-(2-chlorophenyl)ethyl]oxy}-5-[6-(4-piperidinyloxy)-1*H-*benzimidazol-1-yl]-2-thiophenecarboxylate (0.150 g, 0.293 mmol) was dissolved in 7 N ammonia in methanol (12.0 mL, 84.0 mmol) in a sealed tube and heated to 80ºC for 48 h. The solution was concentrated down and recharged with fresh 7 N ammonia in methanol (12.0 mL, 84.0 mmol) and heated to 110 ºC for 72 h. The reaction was cooled to room temperature and concentrated *in vacuo.* Purification by flash chromatography afforded 0.126 g (87%) of the title compound. ¹H NMR (400 MHz, DMSO-*d₆*): δ 8.38 (s, 1H), 7.79 (br s, 1H), 7.66 (dd, 1H, *J*= 1.6, 7.7 Hz, 1H), 7.61 (d, 1H, *J*= 8.8 Hz), 7.45 (dd, 1H, *J*= 1.3, 7.8 Hz), 7.40 (m, 1H), 7.84 (m, 1H), 7.11 (s, 1H), 7.11 (br s, 1H),7.01 (d, 1H, *J*= 2.2 Hz), 6.96 (dd, 1H, *J*= 2.3, 8.7 Hz), 5.98 (q, 1H, *J*= 6.2 Hz), 4.41 (m, 1H), 2.98-2.89 (m, 2H), 2.62-2.53 (m, 2H), 1.94-1.84 (m, 2H), 1.70 (d, 3H, *J*= 6.2 Hz), 1.54-1.40 (m, 2H); MS (ESI): 497 [M+H]⁺.

### Example 21: 3-{[(1R)-1-(2-Chlorophenyl)ethyl]oxy}-5-{6-[(1-methyl-4-piperidinyl)oxy]-1H-benzimidazol-1-yl}-2-thiophenecarboxamide

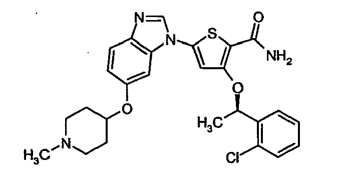

### Step A - Methyl 3-{[(1R)-1-(2-chlorophenyl)ethyl]oxy}-5-{6-[(1-methyl-4-piperidinyl)oxy]-1H-benzimidazol-1-yl}-2-thiophenecarboxylate

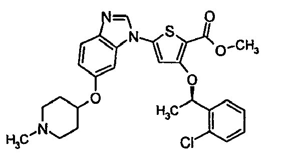

Methyl 3-{[(1*R*)-1-(2-chlorophenyl)ethyl]oxy}-5-[6-(4-piperidinyloxy)-1*H-*benzimidazol-1-yl]-2-thiophenecarboxylate (0.260 g, 0.508 mmol) was dissolved in dichloromethane (4 mL) and methanol (2 mL). Acetic acid (0.035 mL, 0.61 mmol) and formaldeldehyde (0.076 mL, 37% in water, 1.0 mmol) were added *via* syringe. Sodium triacetoxyborohydride (0.161 g, 0.760 mmol) was added in a single portion. The reaction was stirred for 2 h and poured into dichloromethane and half-saturated aqueous sodium bicarbonate. The layers were separated, and the aqueous layer was washed with dichloromethane and ethyl acetate. The combined organic layers were dried over magnesium sulfate, filtered, and concentrated *in vacuo.* Purification by flash chromatography afforded 0.215 g (80%) of the title compound. ¹H NMR (400 MHz, DMSO-*d₆*): δ 8.48 (s, 1H), 7.73 (dd, 1H, *J*= 1.8, 7.7 Hz), 7.63 (d, 1H, *J*= 8.8 Hz), 7.47-7.40 (m, 2H), 7.38 (s, 1H), 7.34 (m, 1H), 7.12 (d, 1H*, J*= 2.2 Hz), 6.99 (dd, 1H, *J*= 2.2, 8.8 Hz), 5.98 (q, 1H, *J*= 6.2 Hz), 4.41 (m, 1H), 3.80 (s, 3H), 2.65-2.54 (m, 2H), 2.24-2.13 (m, 2H), 2.17 (s, 3H), 1.97-1.87 (m, 2H), 1.72-1.61 (m, 2H), 1.62 (d, 3H, *J*= 6.2 Hz); MS (ESI): 526 [M+H]⁺.

### Step B - 3-{[(1R)-1-(2-Chlorophenyl)ethyl]oxy}-5-{6[(1-methyl-4-piperidinyl)oxy]-1H-benzimidazol-1-yl}-2-thiophenecarboxamide (Title Compound)

Methyl 3-{[(1*R*)-1-(2-chlorophenyl)ethyl]oxy}-5-{6-[(1-methyl-4-piperidinyl)oxy]-1*H-*benzimidazol-1-yl}-2-thiophenecarboxylate (0.214 g, 0.407 mmol) was dissolved in 7 N ammonia in methanol (12.0 mL, 84.0 mmol) in a sealed tube and heated to 80 ºC for 2.5 days. The reaction was cooled to room temperature and concentrated *in vacuo.* Purification by flash chromatography afforded 0.208 g (100%) of the title compound. ¹H NMR (400 MHz, DMSO-*d₆):* δ 8.39 (s, 1H), 7.80 (br s, 1H), 7.67 (dd, 1H, *J=* 1.5, 7.6 Hz), 7.62 (d, 1H, *J*= 8.6 Hz), 7.45 (m, 1H), 7.41 (m, 1H), 7.34 (m, 1H), 7.13 (s, 1H), 7.11 (br s, 1H), 7.02 (d, 1H, *J*= 2.0 Hz), 6.97 (dd, 1H, *J*= 2.2, 8.8 Hz), 5.99 (q, 1H, *J*= 6.2 Hz), 4.37 (m, 1H), 2.63-2.53 (m, 2H), 2.22-2.14 (m, 2H), 2.17 (s, 3H), 1.95-1.86 (m, 2H), 1.71 (d, 3H, *J*= 6.2 Hz), 1.70-1.59 (m, 2H); MS (ESI): 511 [M+H]⁺.

### Example 22: 3-{[(1R)-1-(2-Chlorophenyl)ethyl]oxy}-5-{6-[(1-ethyl-4-piperidinyl)oxy]-1H-benzimidazol-1-yl}-2-thiophenecarboxamide

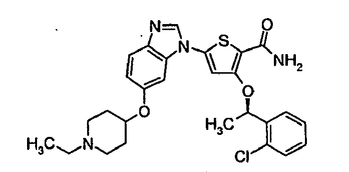

### Step A - Methyl 3-{[(1R)-1-(2-chlorophenyl)ethyl]oxy}-5-{6-[(1-ethyl-4-piperidinyl)oxy]-1H-benzimidazol-1-yl}-2-thiophenecarboxylate

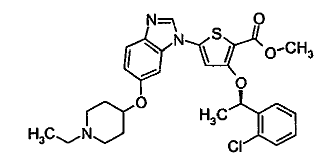

Methyl 3-{[(1*R*)-1-(2-chlorophenyl)ethyl]oxy}-5-[6-(4-piperidinyloxy)-1*H-*benzimidazol-1-yl]-2-thiophenecarboxylate (0.200 g, 0.391 mmol) was dissolved in tetrahydrofuran (10 mL). Sodium carbonate (0.166 g, 1.57 mmol) and ethyl bromide (0.29 mL, 3.9 mmol) was added, and the reaction was heated to reflux overnight. The reaction was cooled to room temperature and poured into half-saturated aqueous sodium bicarbonate. The mixture was extracted with dichloromethane (2x) and ethyl acetate (1x). The combined organic layers were dried over magnesium sulfate, filtered, and concentrated *in vacuo.* Purification by flash chromatography provided 0.164 g (78%) of the title compound. ¹H NMR (400 MHz, DMSO-*d₆*): δ 8.49 (s, 1H), 7.73 (dd, 1H, *J* = 1.8, 7.8 Hz), 7.64 (d, 1H, *J*= 8.4 Hz), 7.48-7.31 (m, 4H), 7.12 (m, 1H), 6.99 (m, 1H), 5.98 (q, 1H, *J*= 6.2 Hz), 4.41 (m, 1H), 3.81 (s, 3H), 2.72-2.62 (m, 2H), 2.37-2.28 (m, 2H), 2.24-2.12 (m, 2H), 1.99-1.87 (m, 2H), 1.70-1.58 (m, 2H), 1.62 (d, 3H, *J*= 6.2 Hz), 0.99 (t, 3H, *J*= 6.8 Hz); MS (ESI): 540 [M+H]⁺.

### Step B - 3{[(1R)-1-(2-Chlorophenyl)ethyl]oxy}-5-{6-[(1-ethyl-4-piperidinyl)oxy]-1H-benzimidazol-1-yl}-2-thiophenecarboxamide (Title Compound)

The title compound was prepared from methyl 3-{[(1*R*)-1-(2-chlorophenyl)ethyl]oxy}-5-{6-[(1-ethyl-4-piperidinyl)oxy]-1*H-*benzimidazol-1-yl}-2-thiophenecarboxylate by a procedure analogous to **Example 21, Step B.** ¹H NMR (400 MHz, DMSO-*d₆*): δ 8.38 (s, 1H), 7.79 (br s, 1H), 7.66 (dd, 1H, *J* = 1.8, 7.8 Hz), 7.61 (d, *J*= 8.8 Hz, 1H), 7.45 (dd, 1H, *J*= 1.3, 7.9 Hz, 1H), 7.40 (m, 1H), 7.33 (m, 1H), 7.12 (s, 1H), 7.10 (br s, 1H), 7.01 (d, 1H, *J*= 2.0 Hz), 6.95 (dd, 1H, *J*= 2.4, 8.8 Hz), 5.96 (q, 1H, *J*= 6.2 Hz), 4.37 (m, 1H), 2.71-2.58 (m, 2H), 2.37-2.26 (m, 2H), 2.24-2.12 (m, 2H), 1.96-1.85 (m, 2H), 1.70 (d, 3H, *J*= 6.2 Hz), 1.68-1.56 (m, 2H), 0.98 (t, 3H, *J*= 7.0 Hz); MS (ESI): 525 [M+H]⁺.

### Example 23: 3-{[(1R)-1-(2-Chlorophenyl)ethyl]oxy}-5-(6-{[(3R)-3-piperidinylmethyl]oxy}-1H-benzimidazol-1-yl)-2-thiophenecarboxamide

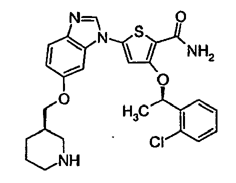

### Step A - 1, 1-Dimethylethyl (3R)-3-({[(4-methylphenyl)sulfonyl]oxy}methyl}-1-piperidinecarboxylate

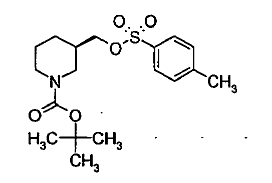

1,1-Dimethylethyl (3*R*)-3-(hydroxymethyl)-1-piperidinecarboxylate (Wirz, B.; Walther, W. Tetrahedron: Asymmetry 1992, 3, 1049-1054.) (2.67 g, 12.5 mmol) was dissolved in dichloromethane (100 mL). Triethylamine (2.60 mL, 18.7 mmol) and 4-*N,N*-dimethylaminopyridine (0.153 g, 1.25 mmol) were added followed by *p*-toluenesulfonyl chloride (3.57 g, 18.7 mmol). The reaction was stirred for 2.5 days and adsorbed onto silica gel. Purification by flash chromatography afforded 4.41 g (95%) of the title compound. ¹H NMR (400 MHz, DMSO-*d₆*): δ 7.78-7.73 (m, 2H), 7.49-7.43 (m, 2H), 3.90-3.81 (m, 2H), 3.72-3.54 (br s, 2H), 2.81-2.69 (m, 2H), 2.39 (s, 3H), 1.74-1.64 (m, 2H), 1.47 (m, 1H), 1.33 (s, 9H), 1.30-1.03 (m, 2H).

### Step B-1,1-Dimethylethyl (3R)-3-{[1-{4-{[(1R)-1-(2-chlorophenyl)ethyl]oxy}-5-[(methyloxy)carbonyl]-2-thienyl}-1H-benzimidazol-6-yl)oxy]methyl}-1-piperidinecarboxylate

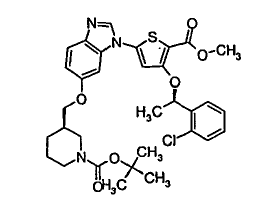

Methyl 3-{[(1*R*)-1-(2-chlorophenyl)ethyl]oxy}-5-(6-hydroxy-1*H-*benzimidazol-1-yl)-2-thiophenecarboxylate (0.400 g, 0.933 mmol), 1,1-dimethylethyl (3*R*)*-3-*({[(4-methylphenyl)sulfonyl]oxy}methyl)-1-piperidinecarboxylate (0.517 g, 1.40 mmol) and cesium carbonate (0.456 g, 1.40 mmol) were stirred in *N,N-*dimethylformamide (10 mL) at 60 ºC overnight. The reaction was cooled to room temperature and poured into ethyl acetate and water. The layers were separated, and the organic layer was washed with brine. The combined aqueous layers were extracted with ethyl acetate, and the combined organic layers were dried over magnesium sulfate, filtered, and concentrated in *vacuo.* Purification by flash chromatography gave 0.574 g (98%) of the title compound. ¹H NMR (400 MHz, DMSO-*d₆*): δ 8.46 (s, 1H), 7.71 (dd, 1H, *J*= 1.5, 7.9 Hz), 7.63 (d, 1H, *J*= 8.8 Hz), 7.44 (m, 1H), 7.39 (m, 1H), 7.37 (m, 1H), 7.32 (m, 1H), 7.08 (d, 1H, *J*= 2.2 Hz), 6.95 (dd, 1H, *J*= 2.5, 8.7 Hz), 5.97 (q, 1H, *J*= 6.2 Hz), 3.90 (m, 1H), 3.87-5.57 (br m, 2H), 3.83 (m, 1H), 3.80 (s, 3H), 2.96-2.74 (br m, 2H), 1.94-1.74 (m, 2H), 1.62 (m, 1H), 1.60 (d, 3H, *J* = 6.2 Hz), 1.44-1.23 (br m, 11H); MS (ESI): 626 [M+H]⁺.

### Step C - 1,1-Dimethylethyl (3R)-3-({[1-(5-(aminocarbonyl)-4-{[(1R)-1-(2-chlorophenyl)ethy]oxy}-2-thienyl)-1H-benzimidazol-6-yl]oxy}methyl)-1-piperidinecarboxylate

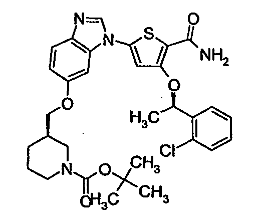

The title compound was prepared from 1,1-dimethylethyl (3*R*)-3-{[(1-{4-{[(1*R*)-1-(2-chlorophenyl)ethyl]oxy}-5-[(methyloxy)carbonyl]-2-thienyl}-1*H-*benzimidazol-6-yl)oxy]methyl}-1-piperidinecarboxylate by a procedure analogous to **Example 21**, Step B. ¹H NMR (400 MHz, DMSO-*d₆*): δ 8.37 (s, 1H), 7.79 (br s, 1H), 7.66 (dd, 1H, *J*= 1.7, 7.7), 7.61 (d, 1H, *J*= 8.8 Hz), 7.46 (m, 1H), 7.40 (m, 1H), 7.33 (m, 1H), 7.12 (s, 1H), 7.10 (br s, 1H), 6.99 (d, 1H, *J*= 2.2 Hz), 6.93 (dd, 1H, *J*= 2.2, 8.8 Hz), 5.97 (q, 1H, *J*= 6.2 Hz), 3.87 (m, 1H), 3.83-3.68 (br m, 2H), 3.81 (m, 1H), 2.93-2.77 (br m, 2H), 1.95-1.76 (br m, 2H), 1.69 (d, 3H, *J*= 6.2 Hz), 1.61 (m, 1H), 1.42-1.22 (br m, 11H); MS (ESI): 611 [M+H]⁺.

### Step D - 3-{[(1R)-1-(2-Chlorophenyl)ethyl]oxy}-5-(6-{[(3R)-3-piperidinylmethyl]-oxy}-1H-benzimidazol-1-yl)-2-thiophenecarboxamide (Title Compound)

The title compound was prepared from 1,1-dimethylethyl (3*R*)-3-({[1-(5-(aminocarbonyl)-4-{[(1*R*)-1-(2-chlorophenyl)ethyl]oxy}-2-thienyl)-1*H-*benzimidazol-6-yl]oxy}methyl)-1-piperidinecarboxylate by a procedure analogous to **Example 20**, Step B. ¹HNMR (400 MHz, DMSO-*d₆*): δ 8.39 (s, 1H), 7.80 (br s, 1H), 7.66 (dd, 1H, *J*= 1.7, 7.7 Hz), 7.63 (d, 1H, *J*= 8.8 Hz), 7.45 (dd, 1H, *J*= 1.3, 7.9 Hz), 7.40 (m, 1H), 7.33 (m, 1H), 7.15 (s, 1H), 7.10 (br s, 1H), 7.00 (d, 1H, *J*= 2.2 Hz), 6.94 (dd, 1H, *J* = 2.2, 8.8 Hz), 5.97 (q, 1H, *J*= 6.2 Hz), 3.93 (m, 1H), 3.84 (m, 1H), 3.27 (m, 1H), 3.13 (m, 1H), 2.75-2.60 (br m, 2H), 2.09 (m, 1H), 1.83 (m, 1H), 1.75 (m, 1H), 1.70 (d, 3H, *J*= 6.2 Hz), 1.55 (m, 1H), 1.31 (m, 1H); MS (ESI): 511 [M+H]⁺.

### Intermediate Example 4: 1,1-Dimethylethyl (2R)-2-({[(4-methylphenyl)sulfonyl]oxy}methyl)-4-morpholinecarboxylate

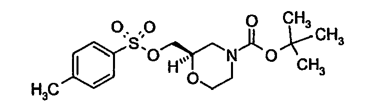

### Step A - (2R)-1[(2-hydroxyethyl)amino]-3-(phenylmethyl)oxy]-2-propanol

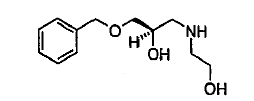

Benzyl (*R*)-(-)-glycidyl ether (3.00 g, 18.3 mmol) was dissolved in *n*-propanol (30 mL) with stirring. Ethanolamine (4.4 mL, 73 mmol) was added *via* syringe, and the reaction was heated to reflux for 16 h. The reaction was cooled to room temperature and poured into ethyl acetate and half-saturated aqueous sodium bicarbonate. The layers were separated, and the organic layer was washed with brine. The combined aqueous layers were extracted with dichloromethane (2x) and ethyl acetate (1x). The combined organic layers were dried over magnesium sulfate, filtered, and concentrated. Purification by flash chromatography provided the title compound. ¹H NMR (400 MHz, DMSO-*d₆*): δ 7.34-7.21 (m, 5H), 4.69 (br s, 1H), 4.44 (s, 2H), 4.42 (m, 1H), 3.66 (m, 1H), 3.44-3.27 (m, 4H), 2.59-2.40 (m, 5H).

### Step B - N-(2-hydroxyethyl)-N-{(2R)-2-hydroxy-3-[(phenylmethyl)oxy]propyl}-4-methylbenzenesulfonamide

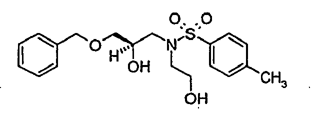

(2*R*)-1-[(2-Hydroxyethyl)amino]-3-[(phenylmethyl)oxy]-2-propanol from Step A was dissolved in dichloromethane (120 mL) and cooled to 0 ºC with stirring. Triethylamine (5.10 mL, 36.6 mmol) was added followed by *p*-toluenesulfonyl chloride (3.84 g, 20.1 mmol). The reaction was allowed to warm to room temperature and stirred for a total of 5 h. The reaction was concentrated onto silica gel and purified by flash chromatography to afford 4.85 g (70% over 2 steps) of the title compound. ¹H NMR (400 MHz, DMSO-*d₆*): δ 7.69-7.62 (m, 2H), 7.41-7.24 (m, 7H), 5.11 (d, 1H, *J*= 5.1 Hz), 4.82 (t, 1H, *J*= 5.5 Hz), 4.46 (s, 2H), 3.84 (m, 1H), 3.55-3.46 (m, 2H), 3.34 (d, 2H, *J*= 5.5 Hz), 3.29-3.20 (m, 2H), 3.05 (m, 1H), 2.93 (dd, 1H, *J*= 7.9, 14.3), 2.37 (s, 3H).

### Step C - (2R)-4-[(4-Methylphenyl)sulfonyl]-2-{[(phenylmethyl)oxy]methyl}-morpholine

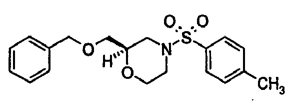

Sodium hydride (1.28 g, 60% dispersion in mineral oil, 32.0 mmol) was washed with hexanes (2x), and tetrahydrofuran (80 mL) was added. The mixture was cooled to 0 ºC with stirring. *N-*(2-Hydroxyethyl)-*N*-{(2*R*)-2-hydroxy-3-[(phenylmethyl)oxy]propyl}-4-methylbenzenesulfonamide (4.85 g, 12.8 mmol) was dissolved in tetrahydrofuran (15 mL) and added to the reaction flask slowly *via* syringe. The flask previously containing the diol was rinsed with additional tetrahydrofuran (15 mL), and that solution was added to the reaction *via* syringe. The reaction was warmed to room temperature and stirred for 1 h. The reaction was cooled to 0 ºC), and 1-(*p-*toluenesulfonyl)imidazole (2.85 g, 12.8 mmol) was added in a single portion. The reaction was warmed to room temperature and stirred overnight. The reaction was cooled to 0 ºC and quenched by the addition of half-saturated aqueous ammonium chloride solution. The mixture was poured into a separatory funnel with ethyl acetate. The layers were separated, and the organic layer was washed with brine. The combined aqueous layers were extracted with ethyl acetate. The combined organic layers were dried over magnesium sulfate, filtered, and concentrated *in vacuo.* Purification by flash chromatography afforded 3.07 g (66%) of the title compound. ¹H NMR (300 MHz, DMSO-*d₆*): δ 7.69-7.61 (m, 2H), 7.55-7.47 (m, 2H), 7.43-7.26 (m, 5H), 4.48 (s, 2H), 3.88 (m, 1H), 3.68 (m, 2H), 3.61-3.38 (m, 4H), 2.45 (s, 3H), 2.26 (m, 1H), 2.08 (dd, 1H, *J*= 10.4, 11.4 Hz).

### Step D - {(2R)-4-[(4-Methylphenyl)sulfonyl]-2-morpholinyl}methanol

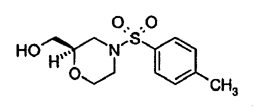

(2*R*)-4[(4-Methylphenyl)sulfonyl]-2-{[(phenylmethyl)oxy]methyl}-morpholine (3.07 g, 8.49 mmol) was dissolved in ethyl acetate (100 mL) with stirring. Palladium (10 wt% on carbon, 0.452 g) was added and the reaction was placed under 1 atmosphere of hydrogen using a balloon apparatus. The reaction was stirred under these conditions for 6 days, and filtered through a Celite pad washing with ethyl acetate. The filtrate was concentrated, and purification by flash chromatography gave 1.64 g (71 %) of the title compound. ¹H NMR (400 MHz, DMSO-*d₆*): δ 7.59 (d, 2H, *J*= 8.4 Hz), 7.44 (d, 2H, *J*= 7.9 Hz), 4.74 (t, 1H, *J*= 5.7 Hz), 3.81 (m, 1H), 3.53-3.32 (m, 5H), 3.26 (m, 1H), 2.39 (s, 3H), 2.18 (m, 1H), 1.95 (m, 1H).

### Step E - 1,1-dimethylethyl (2R)-2-(hydroxymethyl)-4-morpholinecarboxylate

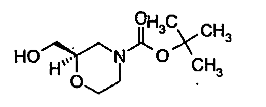

Sodium metal (2.78 g, 121 mmol) was placed in a flask with naphthalene (17.0 g, 133 mmol) in tetrahydrofuran (140 mL) and sonicated for 2.5 h to give a dark green solution. In a separate flask ((2R)-4-[(4-methylphenyl)sulfonyl]-2-morpholinyl)methanol (1.64 g, 6.04 mmol) was dissolved in tetrahydrofuran (40 mL) and cooled to 0 ºC. 20 mL of the sodium naphthalenide solution was added slowly *via* syringe, and the reaction was checked by TLC. An additional 5 mL of the sodium naphthalenide solution was added slowly *via* syringe. The reaction was stirred for 5 min and quenched by the addition of 2 N aqueous hydrochloric acid solution (∼150 mL). The mixture was poured into a separatory funnel and extracted with diethyl ether (2x). The diethyl ether layers were discarded. The aqueous layer was basified with solid sodium hydroxide and extracted with dichloromethane, 4:1 dichloromethane:isopropanol, and 4:1 ethyl acetate:isopropanol. These combined organic layers were dried over magnesium sulfate, filtered, and concentrated *in vacuo.* Very little material was obtained and was thus discarded. To the aqueous layer was added 100 mL of dioxane and di-tert butyl dicarbonate (1.32 g, 6.05 mmol). The addition of three large scoops of solid sodium bicarbonate and additional di-tert-butyl dicarbonate (1.32 g, 6.05 mmol) was followed by one large scoop of solid sodium hydroxide. The mixture was stirred overnight, and the majority of the dioxane was removed *in vacuo.* The aqueous layer was extracted with dichloromethane (2x) and ethyl acetate (1x). The combined organic layers were dried over magnesium sulfate, filtered, and concentrated *in vacuo.* Purification by flash chromatography provided 1.07 g (82%) of the title compound. ¹H NMR (400 MHz, CDCl₃): δ 3.96-3.75 (m, 3H), 3.66 (dd, 1H, *J*= 3.4, 11.4 Hz), 3.60-3.43 (m, 3H), 2.92 (m, 1H), 2.74 (m, 1H), 1.45 (s, 9H).

### Step F-1,1-dimethylethyl (2R)-2-({[(4-methylphenyl)sulfonyl]oxy}methyl)-4-morpholinecarboxylate (Title Compound)

The title compound was prepared from 1,1-dimethylethyl (2*R*)-2-(hydroxymethyl)-4-morpholinecarboxylate by a procedure analogous to Example 23, Step A. ¹H NMR (400 MHz, DMSO-*d₆*): δ 7.78-7.73 (m, 2H), 7.49-7.43 (m, 2H), 4.05 (dd, 1H, *J*= 3.5, 10.8 Hz), 3.97 (dd, 1H, *J*= 6.0, 10.8 Hz), 3.76-3.57 (m, 3H), 3.48 (m, 1H), 3.30 (m, 1H), 2.77 (br s, 1H), 2.56 (br s, 1H), 2.39 (s, 3H), 1.35 (s, 9H).

### Example 24: 3-{[(1R)-1-(2-Chlorophenyl)ethyl]oxy}-5-(6-{[(2R)-2-morpholinylmethyl]oxy}-1H-benzimidazol-1-yl)-2-thiophenecarboxamide

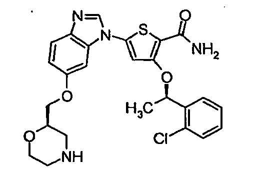

### Step A - 1,1-Dimethylethyl (2R)-2-{[(1-{4-{[(1R)-1-(2-chlorophenyl)ethyl]oxy}-5-[(methyloxy)carbonyl]-2-thienyl}-1H-benzimidazol-6-yl)oxy]methyl}-4-morpholinecarboxylate

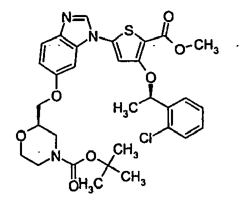

The title compound was prepared from methyl 3-{[(1*R*)-1-(2-chlorophenyl)ethyl]oxy}-5-(6-hydroxy-1*H-*benzimidazol-1-yl)-2-thiophenecarboxylate and 1,1-dimethylethyl (2*R*)-2-({[(4-methylphenyl)sulfonyl]oxy}methyl)-4-morpholinecarboxylate by a procedure analogous to **Example 23**, Step B. ¹H NMR (400 MHz, DMSO-*d₆*): δ 8.49 (s, 1H), 7.73 (dd, 1H, *J*= 1.5, 7.8 Hz), 7.65 (d, 1H, *J*= 8.8 Hz), 7.48-7.39 (m, 2H), 7.38 (s, 1H), 7.33 (m, 1H), 7.13 (d, 1H, *J*= 2.4 Hz), 6.98 (dd, 1H, *J*= 2.4, 8.8 Hz), 5.99 (q, 1H, *J*= 6.2 Hz), 4.08-4.03 (m, 2H), 3.88 (br s, 1H), 3.84 (br s, 1H), 3.81 (s, 3H), 3.76-3.68 (m, 2H), 3.46 (m, 1H), 3.03-2.70 (br m, 2H), 1.62 (d, 3H, *J*= 6.2 Hz), 1.39 (s, 9H); MS (ESI): 628 [M+H]⁺.

### Step B - 1,1-Dimethylethyl (2R)-2-({[1-(5-(aminocarbonyl)-4-{[(1R)-1-(2-chlorophenyl}ethyl]oxy}-2-thienyl)-1H-benzimidazol-6-yl]oxy}methyl}-4-morpholinecarboxylate

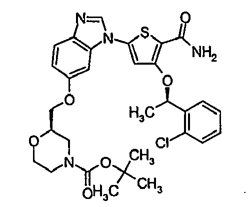

The title compound was prepared from 1,1-dimethylethyl (2*R*)-2-{[(1-{4-{[(1*R*)-1-(2-chlorophenyl)ethyl]oxy}-5-[(methyloxy)carbonyl]-2-thienyl}-1*H-*benzimidazol-6-yl)oxy]methyl}-4-morpholinecarboxylate by a procedure analogous to Example 23, Step C. ¹H NMR (400 MHz, DMSO-*d₆*): δ 8.38 (s, 1H), 7.80 (br s, 1H), 7.66 (dd, 1H, *J*= 1.6, 7.5), 7.62 (d, 1H, *J*= 8.8 Hz), 7.46 (m, 1H), 7.40(m,1H), 7.33 (m,1H), 7.13 (s,1H), 7.11 (br s, 1H), 7.01 (d, 1H, *J*= 2.2 Hz), 6.95 (dd, 1H, *J*= 2.4, 8.8 Hz), 5.98 (q, 1H, *J*= 6.2 Hz), 4.05-3.79 (m, 4H), 3.76-3.65 (m, 2H), 3.45 (m, 1H), 3.02-2.70 (br m, 2H), 1.70 (d, 3H, *J* = 6.4 Hz); 1.38 (s, 9H).

### Step C - 3-{[(1R)-1-(2-Chlorophenyl)ethyl]oxy}-5-(6-{[(2R)-2-morpholinylmethyl]-oxy}-1H-benzimidazol-1-yl)-2-thiophenecarboxamide (Title Compound)

1,1-Dimethylethyl (2*R*)-2-({[1-(5-(aminocarbonyl)-4-{[(1*R*)-1-(2-chlorophenyl)ethyl]oxy}-2-thienyl)-1*H*-benzimidazol-6-yl]oxy}methyl)-4-morpholinecarboxylate (0.563 g, 0.918 mmol) was dissolved in dichloromethane and cooled to 0ºC. Trifluoroacetic acid (1.4 mL, 18.2 mmol) was added, and the reaction was stirred for 1 h. At this point, additional trifluoroacetic acid (3-4 mL) was added, and the reaction was stirred for 1.5 h more. The reaction was quenched by the addition of 2 N ammonia in methanol (40 mL). The mixture was adsorbed onto Celite, and purification by flash chromatography provided 0.397 g (84%) of the title compound. ¹H NMR (400 MHz, DMSO-*d₆*): δ 8.37 (s, 1H), 7.80 (br s, 1H), 7.66 (dd, 1H, *J*= 1.6, 7.7 Hz), 7.61 (d, 1H, *J*= 8.8 Hz), 7.47 (dd, 1H, *J*= 1.1, 7.9 Hz), 7.40 (m, 1H), 7.33 (m, 1H), 7.12 (s, 1H), 7.11 (br s, 1H), 6.98 (d, 1H, *J*= 2.4 Hz), 6.93 (dd, 1H, *J*= 2.4, 9.0), 5.98 (q, 1H, *J*= 6.2 Hz), 3.95 (dd, 1H, *J*= 6.0, 10.3 Hz), 3.88 (dd, 1H, *J*= 4.1, 10.3 Hz), 3.77-3.66 (m, 2H), 3.47 (m, 1H), 2.86 (dd, 1H, *J*= 2.2, 12.1 Hz), 2.70-2.61 (m, 2H), 2.52 (dd, 1H, *J*= 10.3, 12.1 Hz), 1.70 (d, 3H, *J*= 6.2 Hz); MS (ESI): 513 [M+H]⁺.

### Intermediate Example 5: 2,2,12,12-tetramethyl-3,3,11,11-tetraphenyl-4,10-dioxa-3,11-disilatridecan-7-ol

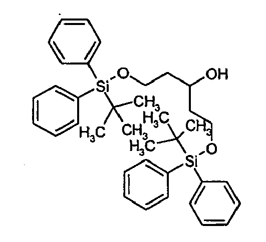

### Step A - diethyl 3-(tetrahydro-2H-pyran-2-yloxy)pentanedioate

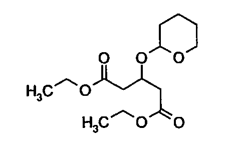

Diethyl 3-hydroxyglutarate (5.00 mL, 27.0 mmol) was dissolved in dichloromethane (150 mL) with stirring. 3,4-Dihydro-2*H-*pyran (3.00 mL, 32.9 mmol) was added followed by pyridinium *p*-toluenesulfonate (0.339 g, 1.35 mmol). The reaction was stirred overnight and poured into half-saturated aqueous sodium bicarbonate. The layers were separated, and the aqueous layer was washed with diethyl ether. The combined organic layers were dried over magnesium sulfate, filtered, and concentrated *in vacuo.* Toluene was added, and the mixture was concentrated *in vacuo* and carried directly into the next reaction.

### Step B - 3-(Tetrahydro-2H-pyran-2-yloxy)-1,5-pentanediol

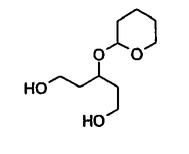

A 3-neck flask with a mechanical stirrer and addition funnel was charged with lithium aluminum hydride (4.10 g, 108 mmol) and diethyl ether (200 mL). The mixture was cooled to 0 ºC with stirring. Diethyl 3-(tetrahydro-2*H-*pyran-2-yloxy)pentanedioate from the prior step was dissolved in diethyl ether (80 mL) and added dropwise *via* addition funnel. The reaction was warmed to room temperature and stirred for 3 h. The reaction was quenched by the careful addition of water (4.1 mL), 1 N aqueous sodium hydroxide (4.1 mL), and water (12.3 mL). The solids were filtered and washed with diethyl ether and ethyl acetate. The filtrate was concentrated *in vacuo* and purification by flash chromatography afforded 5.70 g of the title compound along with unidentified impurities. ¹H NMR (400 MHz, DMSO-*d₆*): δ 4.56 (m, 1H), 4.33 (t, 1H, *J*= 5.1 Hz), 4.23 (t, 1H, *J*= 5.1 Hz), 3.80-3.69 (m, 2H), 3.51-3.33 (m, 5H), 1.73-1.30 (m, 10H).

### Step C-2,2,12,12-Tetramethyl-3,3,11,11-tetraphenyl-7-(tetrahydro-2H-pyran-2-yloxy)-4,10-dioxa-3;11-disilatridecane

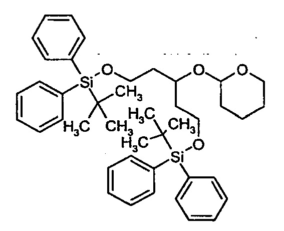

3-(Tetrahydro-2*H-*pyran-2-yloxy)-1,5-pentanediol from the prior reaction was dissolved in dichloromethane (200 mL) with stirring. Imidazole (9.19 g, 135 mmol) and *tert*-butylchlorodiphenylsilane (14.4 mL, 55.4 mmol) were added, and the reaction was stirred overnight. The reaction was quenched by the addition of a few milliliters of methanol. The reaction was poured into a separatory funnel, and the organic layer was washed with water and brine. The combined aqueous was further extracted with diethyl ether. The combined organic layers were dried over magnesium sulfate, filtered, and concentrated *in vacuo.* The title compound was carried directly into the next step. ¹H NMR (400 MHz, DMSO-*d₆*): δ 7.68-7.62 (m, 8H), 7.48-7.32 (m, 12H), 4.53 (m, 1H), 4.00 (m, 1H), 3.80-3.60 (m, 5H), 3.27 (m, 1H), 1.83-1.52 (m, 5H), 1.50-1.19 (m, 5H), 0.95 (s, 9H), 0.94 (s, 9H).

### Step D - 2,2,12,12-tetramethyl-3,3,11,11-tetraphenyl-4,10-dioxa-3,11-disilatridecan-7-ol (Title Compound)

2,2,12,12-Tetramethyl-3,3,11,11-tetraphenyl-7-(tetrahydro-2*H-*pyran-2-yloxy)-4,10-dioxa-3,11-disilatridecane from the prior step was dissolved in 100 mL of ethanol with stirring. Pyridinium *p*-toluenesulfonate (0.339 g, 1.35 mmol) was added and the solution was heated to 60 ºC overnight. The reaction was cooled to room temperature and poured into 50% ethyl acetate:hexanes. The organics were washed with water and brine, and the combined aqueous layers were extracted with 50% ethyl acetate:hexanes. The combined organic layers were dried over magnesium sulfate, filtered, and concentrated *in vacuo.* Purification by flash chromatography afforded 14.12 g (88% over four steps) of the title compound. ¹H NMR (400 MHz, CDCl₃): δ 7.71-7.63 (m, 8H), 7.45-7.33 (m, 12H), 4.14 (m, 1H), 3.89-3.76 (m, 4H), 1.81-1.62 (m, 4H), 1.04 (s, 18H).

### Example 25: 3-{[(1R)-1-(2-Chlorophenyl)ethyl]oxy}-5-{6-[(1-cyclopentyl-4-piperidinyl)oxy]-1H-benzimidazol-1-yl}-2-thiophenecarboxamide

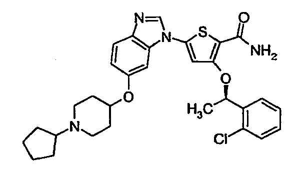

### Step A - Methyl 3-{[(1R)-1-(2-chlorophenyl)ethyl]oxy}-5-(6-{[3-{[(1,1-dimethylethyl)(diphenyl)silyl]oxy}-1-(2-{[(1,1-dimethylethyl)(diphenyl)-silyl]oxy}ethyl)propyl]oxy}-1H-benzimidazol-1-yl)-2-thiophenecarboxylate

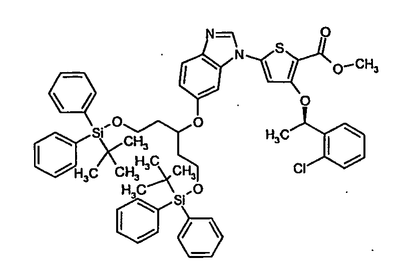

The title compound was prepared from methyl 3-{[(1*R*-1-(2-chlorophenyl)ethyl]oxy}-5-(6-hydroxy-1*H-*benzimidazol-1-yl)-2-thiophenecarboxylate and 2,2,12,12-tetramethyl-3,3,11,11-tetraphenyl-4,10-dioxa-3,11-disilatridecan-7-ol by a procedure analogous to Example 20, Step A. ¹H NMR (400 MHz, DMSO-*d₆*): δ 8.49 (s, 1H), 7.69 (dd, 1H, *J*= 1.3, 7.7 Hz), 7.62-7.51 (m, 5H), 7.47-7.14 (m, 20H), 7.09-7.02 (m, 2H), 5.88 (q, 1H, *J* = 6.2 Hz), 4.91 (m, 1H), 3.80-3.64 (m, 4H), 3.72 (s, 3H), 1.95-1.82 (m, 4H), 1.57 (d, 3H, *J*= 6.2 Hz), 0.89 (s, 9H), 0.86 (s, 9H); MS (ESI): 1007 [M+H]⁺.

### Step B - Methyl 3-{[(1R)-1-(2-chlorophenyl)ethyl]oxy}-5-(6-{[3-hydroxy-1-(2 hydroxyethyl)propyl]oxy}-1H-benzimidazol-1-yl)-2-thiophenecarboxylate

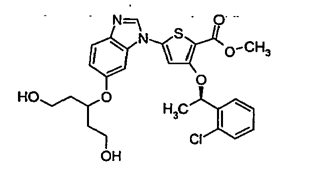

Methyl 3-{[(1*R*)-1-(2-chlorophenyl)ethyl]oxy}-5-(6-{[3-{[(1,1-dimethylethyl)(diphenyl)silyl]oxy}-1-(2-{[(1,1-dimethylethyl)(diphenyl)silyl]-oxy}ethyl)propyl]oxy}-1H-benzimidazol-1-yl)-2-thiophenecarboxylate (1.85 g, 1.84 mmol) was dissolved in tetrahydrofuran (20 mL) with stirring. Tetrabutylammonium fluoride (1.0 M in THF, 4.05 mL, 4.05 mmol) was added *via* syringe. The reaction was stirred for 1.5 h and adsorbed onto silica gel. Purification by flash chromatography afforded 0.870 g (89%) of the title compound. ¹H NMR (400 MHz, DMSO-*d₆*): δ 8.48 (s, 1H), 7.71 (dd, 1H, *J*= 1.6, 7.9 Hz), 7.63 (d, 1H, *J*= 8.8 Hz), 7.47-7.38 (m, 2H), 7.36-7.29 (m, 2H), 7.24 (d, 1H, *J*= 2.0 Hz), 6.99 (dd, 1H, *J=* 2.2, 8.8 Hz), 5.99 (q, 1H, *J*= 6.4 Hz), 4.62 (m, 1H), 4.56-4.49 (m, 2H), 3.81 (s, 3H), 3.57-3.44 (m, 4H), 1.88-1.72 (m, 4H), 1.62 (d, 3H, *J=* 6.4 Hz); MS (ESI): 531 [M+H]⁺.

### Step C - Methyl 5-(6-{[3-chloro-1-(2-chloroethyl)propyl]oxy}-1H-benzimidazol-1-yl)-3-{[(1R)-1-(2-chlorophenyl)ethyl]oxy}-2-thiophenecarboxylate

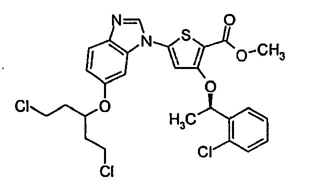

Methyl 3-{[(1*R*)-1-(2-chlorophenyl)ethyl]oxy}-5-(6-{[3-hydroxy-1-(2-hydroxyethyl)propyl]oxy}-1*H-*benzimidazol-1-yl)-2-thiophenecarboxylate (0.150 g, 0.282 mmol) was dissolved in dichloroethane (5 mL) with stirring. Thionyl chloride (0.21 mL, 2.9 mmol) was added and the reaction was heated to 60 ºC and stirred for 2 h. At this time, and additional amount of thionyl chloride (2.1 mL, 29 mmol) was added and the reaction was stirred at that temperature overnight. The reaction was cooled to room temperature and quenched by the slow addition of saturated aqueous sodium bicarbonate solution. The mixture was extracted with ethyl acetate (2x), and the organic layers were dried over magnesium sulfate, filtered, and concentrated in *vacuo.* Purification by flash chromatography provided 0.134 g (84%) of the title compound. ¹H NMR (400 MHz, DMSO-*d₆*): δ 8.51 (s, 1H), 7.71 (dd, 1H, *J*= 1.6, 7.7 Hz), 7.66 (d, 1H, *J*= 8.8 Hz), 7.45-7.37 (m, 2H), 7.36 (s, 1H), 7.32 (m, 1H), 7.24 (d, 1H, *J*= 2.2 Hz), 7.01 (dd, 1H, *J*= 2.4, 8.8 Hz), 5.94 (q, 1H, *J* = 6.2 Hz), 4.71 (m, 1H), 3.81-3.66 (m, 4H), 3.79 (s, 3H), 2.22-2.04 (m, 4H), 1.60 (d, 3H, *J*= 6.2 Hz); MS (ESI): 567 [M+H]⁺.

### Step D -Methyl 3-{[(1R)-1-(2-chlorophenyl)ethyl]oxy}-5-{6-[(1-cyclopentyl-4-piperidinyl)oxy]-1H-benzimidazol-1-yl}-2-thiophenecarboxylate

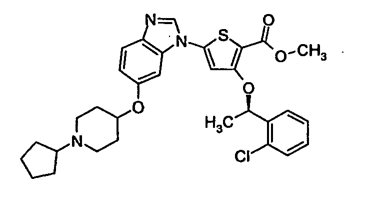

Methyl 5-(6-{[3-chloro-1-(2-chloroethyl)propyl]oxy}-1*H-*benzimidazol-1-yl)-3-{[(1*R*)-1-(2-chlorophenyl)ethyl]oxy}-2-thiophenecarboxylate (0.160 g, 0.282 mmol) and cyclopentylamine (0.30 mL, 2.8 mmol) were dissolved in 1,4-dioxane (8 mL) in a sealed tube and heated at 70 ºC overnight with stirring. Sodium iodide (0.126 g, 0.841 mmol) was added and the temperature was increased to 80 ºC. The reaction was stirred at that temperature for 2.5 days. At this point, additional cyclopentylamine was added, and stirring continued for 24 h more. The reaction was cooled to room temperature and poured into half-saturated aqueous sodium bicarbonate and extracted with ethyl acetate (3x). The combined organic layers were dried over magnesium sulfate, filtered, and concentrated *in vacuo.* Purification by flash chromatography provided the title compound. ¹H NMR (400 MHz, DMSO-*d₆*): δ 8.48 (s, 1H), 7.73 (dd, 1H, *J*= 1.7, 7.9 Hz), 7.63 (d, 1H, *J*=8.4 Hz), 7.47-7.40 (m, 2H), 7.38 (s, 1H), 7.34 (m, 1H), 7.11 (m, 1H), 6.99 (m, 1H), 5.98 (q, *J*= 6.4 Hz, 1H), 4.39 (br m, 1H), 3.81 (s, 3H), 2.79-2.65 (m, 2H), 2.55-2.38 (m, 2H), 2.29-2.13 (m, 2H), 2.00-1.85 (m, 2H), 1.83-1.69 (m, 2H), 1.69-1.62 (m, 3H), 1.62 (d, 3H, *J*= 6.4 Hz), 1.62-1.42 (m, 2H), 1.38-1.24 (m, 2H); MS (ESI): 580 [M+H]⁺.

### Step E - 3-{[(1R)-1-(2-Chlorophenyl)ethyl]oxy}-5-(6-[(1-cyclopentyl-4-piperidinyl)-oxy]-1H-benzimidazol-1-yl}-2-thiophenecarboxamide (Title Compound)

The title compound was prepared from methyl 3-{[(1*R*)-1-(2-chlorophenyl)ethyl]oxy}-5-{6-[(1-cyclopentyl-4-piperidinyl)oxy]-1*H-*benzimidazol-1-yl}-2-thiophenecarboxylate by a procedure analogous to Example 21, Step B. ¹H NMR (400 MHz, DMSO-*d₆*): δ 8.41 (s, 1H), 7.80 (br s, 1H), 7.70-7.60 (m, 2H), 7.49-7.30 (m, 3H), 7.25-6.94 (m, 4H), 5.97 (q, 1H, *J* = 6.4 Hz), 4.81-4.47 (br m, 1H), 3.64-3.23 (br m, 4H), 2.25-1.77 (m, 9H), 1.75-1.44 (m, 4H), 1.70 (d, 3H, *J*= 6.4 Hz); MS (ESI): 565 [M+H]⁺.

### Analog 3: 3-{[(1R)-1-(2-Chlorophenyl)ethyl]oxy}-5-(6-{[3-hydroxy-1-(2-hydroxyethyl)propyl]oxy}-1H-benzimidazol-1-yl)-2-thiophenecarboxamide

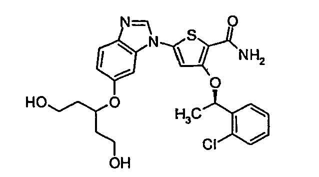

The title compound was prepared from methyl 3-{[(1*R*)-1-(2-chlorophenyl)ethyl]oxy}-5-(6-{[3-hydroxy-1-(2-hydroxyethyl)propyl]oxy}-1*H-*benzimidazol-1-yl)-2-thiophenecarboxylate by a procedure analogous to Example 21, Step B. ¹H NMR (400 MHz, DMSO-*d₆*): δ 8.37 (s, 1H), 7.79 (br s, 1H), 7.65 (dd, 1H, *J*= 1.8, 7.7 Hz), 7.60.(d, 1H, *J*= 8.8 Hz), 7.46-7.37 (m, 2H), 7.33 (m, 1H), 7.13 (d, 1H, *J*= 2.4 Hz), 7.10 (br s, 1H), 7.09 (s, 1H), 6.96 (dd, 1H, *J*= 2.2, 9.0 Hz), 5.99 (q, 1H, *J*= 6.4 Hz), 4.59 (m, 1H), 4.52 (t, 1H, *J* = 4.8 Hz), 4.49 (t, 1H, *J*= 4.8 Hz), 3.56-3.42 (m, 4H), 1.86-1.73 (m, 4H), 1.70 (d, 3H, *J*= 6.4 Hz).

### Example 26: 5-[7-Bromo-6-(4-piperidinyloxy)-1H-benzimidazol-1-yl]-3-{[(1R)-1-(2-chlorophenyl)ethyl]oxy}-2-thiophenecarboxamide

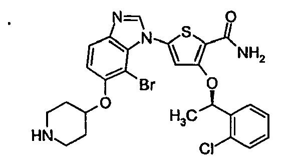

### Step A - Methyl 5-(7-bromo-6-hydroxy-1H-benzimidazol-1-yl)-3-{[(1R)-1-(2-chlorophenyl)ethyl]oxy}-2-thiophenecarboxylate and methyl 3-{(1R)-1-(2-chlorophenyl)ethyl)oxy}-5-(5,7-dibromo-6-hydroxy-1H-benzimidazol-1-yl)-2-thiophenecarboxylate

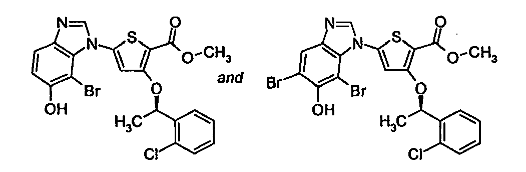

Methyl 3-{[(1*R*)-1-(2-chlorophenyl)ethyl]oxy}-5-(6-hydroxy-1*H-*benzimidazol-1-yl)-2-thiophenecarboxylate (0.250 g, 0.583 mmol) was dissolved in acetic acid (5 mL) with stirring. Bromine (0.36 mL, 1.78 M solution in acetic acid, 0.64 mmol) was added dropwise by syringe. The reaction was stirred for 30 min and quenched by the addition of aqueous 10% sodium thiosulfate solution (5 mL). The mixture was poured into aqueous 1 N sodium hydroxide (75 mL) and extracted with dichloromethane (1x) and ethyl acetate (1x). The combined organic layers were dried over magnesium sulfate, filtered, and concentrated *in vacuo.* Purification by flash chromatography provided both title compounds. For the 5,7-dibromide: ¹H NMR (400 MHz, DMSO-*d₆*): δ 9.74 (s, 1H), 8.32 (s, 1H), 7.94 (s, 1H), 7.65 (dd, 1H, *J*= 1.7, 7.9 Hz), 7.44-7.36 (m, 2H), 7.38 (s, 1H), 7.32 (m, 1H), 5.82 (q, 1H, *J*= 6.2 Hz), 3.80 (s, 3H), 1.58 (d, 3H, *J*= 6.2 Hz). For the 7-bromide (isolated as a mixture with unreacted starting material): ¹H NMR (400 MHz, DMSO-*d₆*): δ 10.21 (s, 1H), 8.21 (s, 1H), 7.66 (dd, 1H, *J*= 1.8, 7.7 Hz), 7.54 (d, 1H, *J*= 7.9 Hz), 7.48-7.29 (m, 4H), 6.95 (d, 1H, *J*= 8.6 Hz), 5.85 (q, 1H, *J*= 6.2 Hz), 3.81 (s, 3H), 1.59 (d, 3H, *J*= 6.2 Hz).

### Step B -1,1-Dimethylethyl 4-[(7-bromo-1-{4-{[(1R)-1-(2-chlorophenyl)ethy]oxy}-5-[(methyloxy)carbonyl]-2-thienyl}-1H-benzimidazol-6-yl)oxy]-1-piperidinecarboxylate

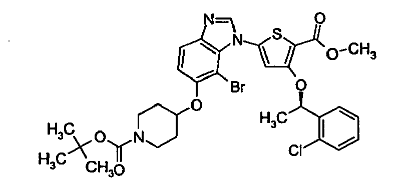

The title compound was prepared from methyl 5-(7-bromo-6-hydroxy-1*H-*benzimidazol-1-yl)-3-{[(1*R*)-1-(2-chlorophenyl)ethyl]oxy}-2-thiophenecarboxylate and *t*-butyl 4-hydroxy-1-piperidinecarboxylate by a procedure analogous to Example 20, Step A. ¹H NMR (400 MHz, DMSO-*d₆*): δ 8.34 (s, 1H), 7.68 (d, 1H, *J*= 8.8 Hz), 7.65 (dd, 1H, *J*= 2.0, 8.6 Hz, 1H), 7.45-7.37 (m, 2H), 7.35 (s, 1H), 7.30 (m, 1H), 7.21 (d, 1H, *J*= 9.0 Hz), 5.83 (q, 1H, *J*= 6.4 Hz), 4.65 (m, 1H), 3.82 (s, 3H), 3.64-3.42 (m, 2H), 3.34-2.26 (m, 2H), 1.87-1.73 (m, 2H), 1.58 (d, 3H, *J*= 6.4 Hz), 1.39 (s, 9H), 1.27-1.16 (m, 2H).

### Step C - 1,1-Dimethylethyl 4-{[1-(5-(aminocarbonyl)-4-{[(1R)-1-(2-chlorophenyl)ethyl]oxy}-2-thienyl)-7-bromo-1H-benzimidazol-6-yl]oxy}-1-piperidinecarboxylate

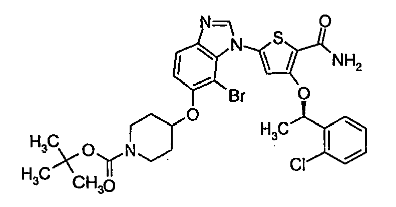

The title compound was prepared from 1,1-dimethylethyl 4-[(7-bromo-1-{4-{[(1*R*)-1-(2-chlorophenyl)ethyl]oxy}-5-[(methyloxy)carbonyl]-2-thienyl}-1*H-*benzimidazol-6-yl)oxy]-1-piperidinecarboxylate and *t*-butyl 4-hydroxy-1-piperidinecarboxylate by a procedure analogous to Example 21, Step B. ¹H NMR (400 MHz, DMSO-*d₆*): δ 8.30 (s, 1H), 7.90 (br s, 1H), 7.67 (d, 1H, *J*= 8.60 Hz), 7.59 (m, 1H), 7.43-7.37 (m, 2H), 7.31 (m, 1H), 7.20 (d, 1H, *J*= 8.8 Hz), 7.19 (br s, 1H), 7.11 (s, 1H), 5.84 (q, 1H, *J=* 6.4 Hz), 4.64 (m, 1H), 3.54-3.40 (m, 2H), 3.36-3.27 (m, 2H), 1.88-1.72 (m, 2H), 1.68 (d, 3H, *J*= 6.4 Hz), 1.40 (s, 9H), 1.27-1.15 (m, 2H).

### Step D - 5-[7-bromo-6-(4-piperidinyloxy)-1H-benzimidazol-1-yl]-3-{[(1R)-1-(2-chlorophenyl)ethyl]oxy}-2-thiophenecarboxamide (Title Compound)

The title compound was prepared from 1,1-dimethylethyl 4-{[1-(5-(aminocarbonyl)-4-{[(1*R*)-1-(2-chlorophenyl)ethyl]oxy}-2-thienyl)-7-bromo-1*H-*benzimidazol-6-yl]oxy}-1-piperidinecarboxylate by a procedure analogous to **Example 24**, Step C. ¹H NMR (400 MHz, DMSO-*d₆*): δ 8.28 (s, 1H), 7.89 (br s, 1H), 7.64 (d, 1H, *J*= 8.60 Hz), 7.60 (dd, 1H, *J*= 1.5, 7.7 Hz), 7.43 (m, 1H), 7.40 (m, 1H), 7.34 (m, 1H), 7.18 (br s, 1H), 7.17 (s, 1H), 7.14 (s, 1H), 5.84 (q, 1H, *J*= 6.4 Hz), 4.44 (m, 1H), 2.97-2.89 (m, 2H), 2.57-2.48 (m, 2H), 1.87-1.77 (m, 2H), 1.68 (d, 3H, *J*= 6.4 Hz), 1.55-1.44 (m, 2H); MS (ESI): 577 [M+2H]⁺.

### Example 27: 3-{[(1R)-1-(2-Chlorophenyl)ethyl]oxy}-5-[5,7-dibromo-6-(4-piperidinyloxy)-1H-benzimidazol-1-yl]-2-thiophenecarboxamide formic acid salt

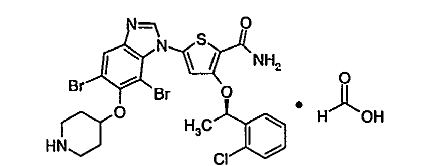

### Step A - 1,1-Dimethylethyl 4-[(5,7-dibromo-1-{4-{[(1R)-1-(2-chlorophenyl)-ethyl]-oxy}-5-[(methyloxy)carbonyl]-2-thienyl}-1H-benzimidazol-6-yl)oxy]-1-piperidinecarboxylate

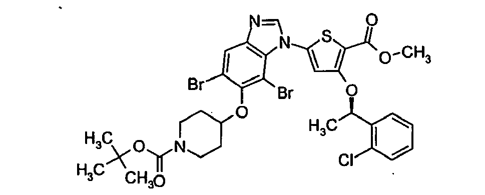

The title compound was prepared from methyl 3-{[(1*R)*-1-(2-chlorophenyl)ethyl]oxy}-5-(5,7-dibromo-6-hydroxy-1*H-*benzimidazol-1-yl)-2-thiophenecarboxylate and *t*-butyl 4-hydroxy-1-piperidinecarboxylate by a procedure analogous to Example 20, Step A. ¹H NMR (400 MHz, DMSO-*d₆*): δ 8.49 (s, 1H), 8.08 (s, 1H), 7.63 (dd, 1H, *J*= 1.7, 7.7 Hz), 7.45-7.37 (m, 2H), 7.34 (s, 1H), 7.32 (m, 1H), 5.83 (q, 1H, *J*= 6.2 Hz), 4.31 (m,1H), 3.94-3.84 (m, 2H), 3.82 (s, 3H), 2.97-2.81 (m, 2H), 1.95-1.78 (m, 2H), 1.69-1.56 (m, 2H), 1.59 (d, 3H, *J*= 6.2 Hz), 1.40 (s, 9H).

### Step B -1,1-Dimethylethyl 4-{[1-(5-(aminocarbonyl)-4-{[(1R)-1-(2 chlorophenyl)ethyl]oxy}-2-thienyl)-5,7-dibromo-1H-benzimidazol-6-yl]oxy}-1-piperidinecarboxylate

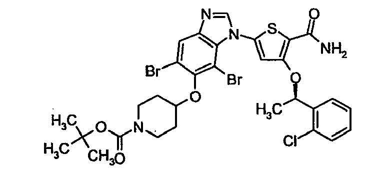

The title compound was prepared from 1,1-dimethylethyl 4-[(5,7-dibromo-1-{4-{[(1*R*)-1-(2-chlorophenyl)ethyl]oxy}-5-[(methyloxy)carbonyl]-2-thienyl}-1*H-*benzimidazol-6-yl)oxy]-1-piperidinecarboxylate by a procedure analogous to **Example 21**, Step B.¹H NMR (400 MHz, DMSO-*d₆*): δ 8.45 (s, 1H), 8.06 (s, 1H), 7.92 (brs, 1H), 7.57 (dd, 1H, *J*= 1.7, 7.5 Hz), 7.45-7.37 (m, 2H), 7.33 (m, 1H),7.21 (br s, 1H), 7.12(s, 1H), 5.83 (q, 1H, *J*= 6.4 Hz), 4.30 (m, 1H), 3.95-3.83 (m, 2H), 2.96-2.82 (m, 2H), 1.95-1.76 (m, 2H), 1.68 (d, 3H, *J*= 6.40 Hz), 1.66-1.55 (m, 2H), 1.40 (s, 9H).

### Step C - 3-{[(1R)-1-(2-Chlorophenyl)ethyl]oxy}-5-[5,7-dibromo-6-(4-piperidinyloxy)-1H-benzimidazol-1-yl]-2-thiophenecarboxamide formic acid salt (Title Compound)

The title compound was prepared from 1,1-dimethylethyl 4-{[1-(5-(aminocarbonyl)-4-{[(1*R*)-1-(2-chlorophenyl)ethyl]oxy}-2-thienyl)-5,7-dibromo-1*H-*benzimidazol-6-yl]oxy}-1-piperidinecarboxylate by a procedure analogous to **Example 24,** Step C. The material was further purified using reverse phase chromatography with an acetonitrile, water, and formic acid mobile phase. This led to isolation of the title compound as its formic acid salt. ¹H NMR (400 MHz, DMSO-*d₆*): δ 8.44 (s, 1H), 8.28 (s, 1H), 8.06 (s, 1H), 7.92 (br s, 1H), 7.58 (dd, 1H, *J*= 1.6, 7.5 Hz, 1H), 7.47-7.31 (m, 3H), 7.21 (br s, 1H), 7.15 (s, 1H), 5.84 (q, 1H, *J*= 6.4 Hz), 4.22 (m, 1H), 3.15-3.03 (m, 2H), 2.64-2.51 (m, 2H), 1.98-1.83 (m, 2H), 1.79-1.64 (m, 2H), 1.68 (d, 3H, *J*= 6.4 Hz); MS (ESI): 655 [M+H]⁺:

### Intermediate Example 6: 1,4-dibromo-2-({[4-(methylox)phenyl]methyl}oxy)-5-nitrobenzene

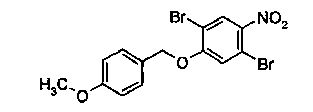

### Step A - N-(4-Bromo-5-fluoro-2-nitrophenyl)-2,2,2-trifluoroacetamide

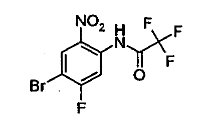

4-Bromo-3-fluoroaniline (5.00 g, 26.3 mmol) was dissolved in chloroform (200 mL) with stirring. Ammonium nitrate (5.05 g, 63.1 mmol) was added, and trifluoroacetic anhydride (26.7 mL, 189 mmol) was added dropwise *via* addition funnel over 10 minutes. The reaction was stirred for 2 h and quenched by the dropwise addition of saturated sodium bicarbonate until the pH was ^{∼}8. The mixture was poured into a separatory funnel, and the layers were separated. The organic layer was washed with brine, and the combined aqueous layers were extracted with ethyl acetate. The combined organic layers were dried over magnesium sulfate, filtered, and concentrated *in vacuo.* Purification by flash chromatography afforded 8.26 g (95%) of the title compound. ¹H NMR (400 MHz, DMSO-*d₆*): δ 11.88 (s, 1H), 8.51 (d, 1H, *J*= 6.8 Hz), 7.75 (d, 1H, *J*= 9.2 Hz).

### Step B - 4-Bromo-5-fluoro-2-nitroaniline

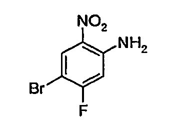

*N*-(4-Bromo-5-fluoro-2-nitrophenyl)-2,2,2-trifluoroacetamide was dissolved in 1,4-dioxane (80 mL) with stirring. Aqueous 1 N sodium hydroxide solution (50 mL) was added, and the mixture was stirred for 45 minutes. The reaction was heated to 50 ºC and was stirred at that temperature for 5 h. The reaction was cooled to room temperature and poured into diethyl ether and brine. The layers were separated, arid the aqueous was extracted further with ethyl acetate. The combined organic layers were dried over magnesium sulfate, filtered, and concentrated *in vacuo.* Purification by flash chromatography afforded 4.46 g (76%) of the title compound. ¹H NMR (300 MHz, DMSO-*d₆*): δ 8.2 (d, 1H, *J*= 7.30 Hz), 7.74 (br s, 2H), 6.97 (d, 1H, *J*= 10.7 Hz).

### Step C - 1,4-Dibromo-2-fluoro-5-nitrobenzene

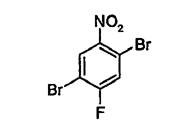

Copper (II) bromide (6.37 g, 28.5 mmol) was dissolved in acetonitrile (40 mL) with stirring. *t*-Butyl nitrite (5.0 mL, 42 mmol) was added *via* syringe, and the reaction was heated to 60 ºC. 4-Bromo-5-fluoro-2-nitroaniline (4.46 g, 19.0 mmol) was dissolved in acetonitrile (60 mL) and added *via* addition funnel over 15 minutes. The reaction was stirred an additional 10 minutes and cooled to room temperature. The reaction was poured onto aqueous 2 N HCl (400 mL). The mixture was extracted with diethyl ether, and the organic layer was washed with brine. The combined aqueous layer was extracted with ethyl acetate. The combined organic layers were dried over magnesium sulfate, filtered, and concentrated *in vacuo.* Purification by flash chromatography afforded 4.46 g (76%) of the title compound. ¹H NMR (300 MHz, DMSO-*d₆*): δ 8.59 (d, 1H, *J*= 6.5 Hz), 8.17 (d, 1H, *J*= 8.1 Hz).

### Step D - 1,4-dibromo-2-({[4-(methyloxy)phenyl]methyl}oxy)-5-nitrobenzene

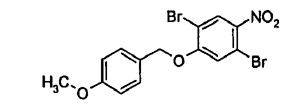

The title compound was prepared from 1,4-dibromo-2-fluoro-5-nitrobenzene and 4-methoxybenzyl alcohol by a procedure analogous to Example 1, Step A. ¹H NMR (400 MHz, DMSO-*d₆*): δ 8.35 (s, 1H), 7.67 (s, 1H), 7.41-7.35 (m, 2H), 6.98-6.93 (m, 2H), 5.26 (s, 2H), 3.73 (s, 3H).

### Example 28: 3-{[(1R)-1-(2-Chlorophenyl)ethyl]oxy}-5-[6-[(1-methyl-4-piperidinyl)oxy]-5-(1-methyl-1H-pyrazol-4-yl)-1H-benzimidazol-1-yl]-2-thiophenecarboxamide

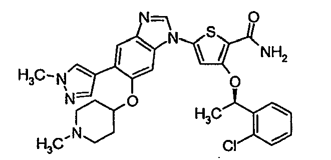

### Step A - Methyl 5-{[4-bromo-5-({[4-(methyloxy)phenyl]methyl}oxy)-2-nitrophenyl]amino}-3-{[(1R)-1-(2-chlorophenyl)ethyl]oxy}-2-thiophenecarboxylate

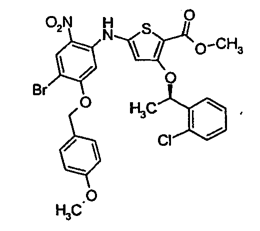

The title compound was prepared from methyl 5-amino-3-{[(1*R*)-1-(2-chlorophenyl)ethyl]oxy}-2-thiophenecarboxylate and 1,4-dibromo-2-({[4-(methyloxy)phenyl]methyl}oxy)-5-nitrobenzene by a procedure analogous to **Example 1,** Step B. ¹H NMR (400 MHz, DMSO-*d₆*): δ 9.90 (s, 1H), 8.31 (s, 1H), 7.68 (dd, 1H, *J*= 1.7, 7.9 Hz), 7.41-7.33 (m, 2H), 7.29-7.24 (m, 3H), 7.01 (s, 1H), 6.94-6.91 (m, 2H), 6.88 (s, 1H), 5.77 (q, 1H, *J*= 6.4 Hz), 5.06 (AB, 2H, [][]_{AB} = 19.0 Hz, *J*_{AB} = 11.6 Hz), 3.76 (s, 3H), 3.74 (s, 3H), 1.56 (d, 3H, *J*= 6.4 Hz).

### Step B - Methyl 5-{[2-amino-4-bromo-5-({[4-(methyloxy)phenyl]methyl}oxy)-phenyl]amino}-3-{[(1R)-(2-chlorophenyl)ethyl]oxy}-2-thiophenecarboxylate

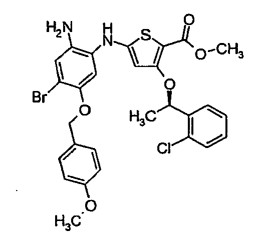

The title compound was prepared from methyl 5-{[4-bromo-5-({[4-(methyloxy)phenyl]methyl}oxy)-2-nitrophenyl]amino}-3-{[(1R)-1 -(2-chlorophenyl)ethyl]oxy}-2-thiophenecarboxylate by a procedure analogous to **Example 1**, Step C. ¹H NMR (400 MHz, DMSO-*d₆*): δ 8.66 (br s, 1H), 7.56 (dd, 1H, *J*= 1.7, 7.7 Hz), 7.44-7.22 (m, 6H), 6.95-6.88 (m, 4H), 5.62 (q, 1H, *J* = 6.2 Hz), 4.84 (s, 2H), 4.71 (br s, 2H), 3.71 (s, 3H), 3.64 (s, 3H), 1.51 (d, 3H, *J*= 6.2 Hz).

### Step C - Methyl 5-[5-bromo-6-({[4-(methyloxy)phenyl]methyl}oxy)-1H-benzimidazol-1-yl]-3-{[(1R)-1-(2-chlorophenyl)ethyl]oxy}-2 thiophenecarboxylate

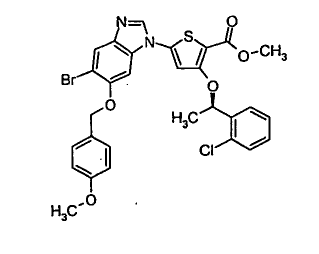

The title compound was prepared from methyl 5-{[2-amino-4-bromo-5-({[4-(methyloxy)phenyl]methyl}oxy)-phenyl]amino}-3-([(1*R*)-1-(2-chlorophenyl)ethyl]oxy}-2-thiophenecarboxylate by a procedure analogous to **Example 1**, Step D. ¹H NMR (400 MHz, DMSO-*d₆*): δ 8.53 (s, 1H), 8.00 (s, 1H), 7.73 (dd, 1H, *J*= 1.7, 7.8 Hz), 7.45-7.35 (m, 6H), 7.29 (m, 1H), 6.97-6.93 (m, 2H), 5.96 (q, 1H, *J*= 6.2 Hz), 5.12 (AB, 2H, J_{AB} = 19.1 Hz, *J*_{AB} = 11.5 Hz), 3.82 (s, 3H), 3.73 (s, 3H), 1.60 (d, 3H, *J*= 6.2 Hz).

### Step D - Methyl 5-(5-bromo-6-hydroxy-1H-benzimidazol-1-yl)-3-{[(1R)-1-(2-chlorophenyl)ethyl]oxy}-2-thiophenecarboxylate

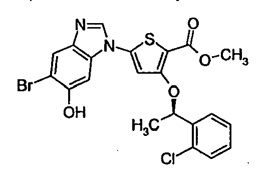

The title compound was prepared from methyl 5-[5-bromo-6-({[4-(methyloxy)phenyl]methyl}oxy)-1*H*-benzimidazol-1-yl]-3-{[(1*R*)-1-(2-chlorophenyl)ethyl]oxy}-2-thiophenecarboxylate by a procedure analogous to **Example 1**, Step E. ¹H NMR (400 MHz, DMSO-*d₆*): δ 10.42 (s, 1H); 8.55 (s, 1H), 7.89 (s, 1H), 7.76 (dd, 1H, *J*= 1.7, 7.7 Hz), 7.53 (s, 1H), 7.46-7.39 (m, 2H), 7.38 (s, 1H), 7.32 (m, 1H), 5.90 (q, 1H, *J*= 6.2 Hz), 3.79 (s, 3H), 1.60 (d, 3H, *J*= 6.2 Hz).

### Step E - 1,1-Dimethylethyl 4-[(5-bromo-1-{4-{[(1R)-1-(2-chlorophenyl)ethyl]oxy}-5-[(methyloxy)carbonyl]-2-thienyl}-1H-benzimidazole-6-yl)oxy]-1-piperidinecarboxylate

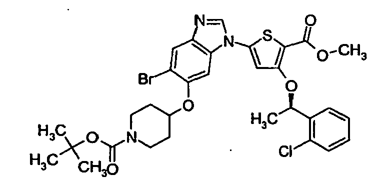

The title compound was prepared from methyl 5-(5-bromo-6-hydroxy-1*H-*benzimidazol-1-yl)-3-{[(1*R*)-1-(2-chlorophenyl)ethyl]oxy}-2-thiophenecarboxylate and *t*-butyl 4-hydroxy-1-piperidinecarboxylate by a procedure analogous to **Example 20,** Step A. ¹H NMR (400 MHz, DMSO-*d₆*): δ 8.54 (s, 1H), 8.02 (s, 1H), 7.73 (dd, 1H, *J* = 1.7, 7.9 Hz), 7.47-7.37 (m, 4H), 7.33 (m, 1H), 5.97 (q, 1H, *J*= 6.2 Hz), 4.75 (m, 1H), 3.82 (s, 3H), 3.59-3.45 (m, 2H), 3.40-3.29 (m, 2H), 1.90-1.80 (m, 2H), 1.72-1.58 (m, 2H), 1.62 (d, 3H, *J*= 6.2 Hz), 1.40 (s, 9H).

### Step F- 1,1-Dimethylethyl-4-{[1-(5-(aminocarbonyl)-4-{[(1R)-1-(2-chlorophenyl)ethyl]oxy}-2-thienyl)-5-bromo-1H-benzimidazol-6-yl]oxy}-1-piperidinecarboxylate

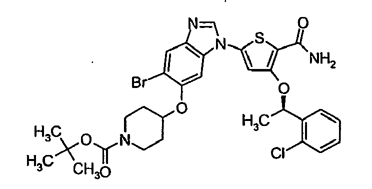

The title compound was prepared from 1,1-dimethylethyl 4-[(5-bromo-1-{4-{[(1*R*)-1-(2-chlorophenyl)ethyl]oxy}-5-[(methyloxy)carbonyl]-2-thienyl}-1*H-*benzimidazol-6-yl)oxy]-1-piperidinecarboxylate by a procedure analogous to **Example 21,** Step B. ¹H NMR (400 MHz, DMSO-*d₆*): δ 8.46 (s, 1H), 8.01 (s, 1H), 7.84 (br s, 1H), 7.67 (dd, 1H, *J*= 2.0, 7.9 Hz), 7.48-7.38 (m, 2H), 7.34 (m, 1H), 7.31 (s, 1H), 7.18 (s, 1H), 7.13 (br s, 1H), 5.99 (q, 1H, *J*= 6.2 Hz), 4.71 (m, 1H), 3.58-3.44 (m, 2H), 3.41-3.29 (m, 2H), 1.91-1.78 (m, 2H), 1.73-1.59 (m, 2H), 1.71 (d, 3H, *J*= 6.2 Hz), 1.40 (s, 9H).

### Step G - 1,1-Dimethylethyl 4-{[1-(5-(aminocarbonyl)-4-{[(1R)-1-(2-chlorophenyl)ethyl]oxy}-2-thienyl)-5-(1-methyl-1H-pyrazol-4-yl)-1H-benzimidazol-6-yl]oxy}-1-piperidinecarboxylate

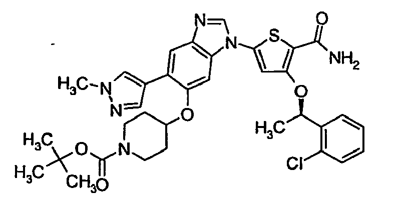

1,1-Dimethylethyl 4-{[1-(5-(aminocarbonyl)-4-{[(1*R*)-1-(2-chlorophenyl)ethyl]oxy}-2-thienyl)-5-bromo-1*H*-benzimidazol-6-yl]oxy}-1-piperidinecarboxylate (0.103 g, 0.152 mmol) was dissolved in 5 mL of *N,N-*dimethylacetamide with stirring. 1-Methyl-4-(4,4,5,5-tetramethyl-1,3,2-dioxaborolan-2-yl)-1*H*-pyrazole (0.0633 g, 0.304 mmol) was added followed by aqueous 1.0 N Na₂CO₃ solution (1 mL). Dichloro[1,1'-bis(diphenylphosphino)ferrocene]palladium (II) dichloromethane adduct (0.0222 g, 0.0303 mmol) was added, and the reaction was heated to 80 ºC. The reaction was stirred for 3 h and cooled to room temperature. The mixture was adsorbed onto silica gel. Purification by flash chromatography afforded an impure sample which was carried on to the next step. MS (ESI): 677 [M+H]⁺.

### Step H - 3-{[(1R)-1-(2-Chlorophenyl)ethyl]oxy}-5-[5-(1-methyl-1H-pyrazol-4-yl)-6-(4-piperidinyloxy)-1H-benzimidazol-1-yl]-2-thiophenecarboxamide

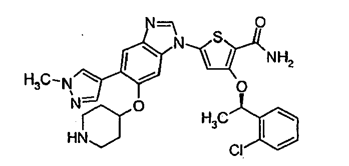

The title compound was prepared from 1,1-dimethylethyl 4-{[1-(5-(aminocarbonyl)-4-{[(1*R*)-1-(2-chlorophenyl)ethyl]oxy}-2-thienyl)-5-(1-methyl-1*H*-pyrazol-4-yl)-1*H*-benzimidazol-6-yl]oxy}-1-piperidinecarboxylate by a procedure analogous to **Example 24,** Step C. Reaction of the product from the prior step gave 0.0594 g (68% over 2 steps) following purification. ¹H NMR (400 MHz, DMSO-*d₆*): δ 8.37 (s, 1H), 8.08 (s, 1H), 7.93-7.88 (m, 2H), 7.79 (br s, 1H), 7.67 (dd, 1H, *J*= 1.6, 7.7 Hz), 7.44 (m, 1H), 7.41 (m, 1H), 7.34 (m, 1H), 7.18 (s, 1H), 7.15 (s, 1H), 7.09 (br s, 1H), 5.98 (q, 1H, *J*= 6.4 Hz), 4.50 (m, 1H), 3.85 (s, 3H), 3.35 (m, 1H), 2.97-2.83 (m, 2H), 2.65-2.50 (m, 2H), 1.99-1.88 (m, 2H), 1.70 (d, 3H, *J*= 6.4 Hz), 1.65-1.46 (m, 2H).

### Step I - 3-{[(1R)-1-(2-Chlorophenyl)ethyl]oxy}-5-[6-[(1-methyl-4-piperidinyl)oxy]-5-(1-methyl-1H-pyrazol-4-yl)-1H-benzimidazol-1-yl]-2 thiophenecarboxamide (Title Compound)

The title compound was prepared from 3-{[(1*R*)-1-(2-chlorophenyl)ethyl]oxy}-5-[5-(1-methyl-1*H*-pyrazol-4-yl)-6-(4-piperidinyloxy)-1*H*-benzimidazol-1-yl]-2-thiophenecarboxamide by a procedure analogous to **Example 21**, Step A. ¹H NMR (400 MHz, DMSO-*d₆*): δ 8.39 (s, 1H), 8.10 (s, 1H), 7.93 (s, 1H), 7.92 (s, 1H), 7.82 (br s, 1H), 7.69 (dd, 1H, *J*= 1.5, 7.9 Hz), 7.46 (m, 1H), 7.42 (m, 1H), 7.35 (m, 1H), 7.22-7.17 (m, 2H), 7.11 (br s, 1H), 6.00 (q, 1H, *J*= 6.4 Hz), 4.48 (m, 1H), 3.87 (s, 3H), 2.63-2.41 (m, 2H), 2.28-2.08 (m, 5H), 2.05-1.87 (m, 2H), 1.84-1.64 (m, 2H), 1.72 (d, 3H, *J*= 6.4 Hz); MS (ESI): 591 [M+H]⁺.

### Example 29: 3-{[(1R)-1-(2-chlorophenyl)ethyl]oxyl}-5-{6-[3-(dimethylamino)-propoxy]-1H-benzimidazol-1-yl}thiophene-2-carboxamide

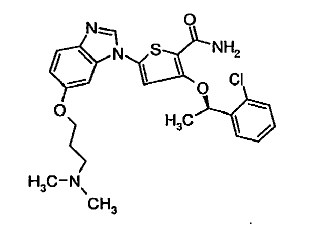

### Step A - 3-{[(1R)-1-(2-chlorophenyl)ethyl]oxy}-5-(6-hydroxy-1H-benzimidazol-1-yl)thiophene-2-carboxamide

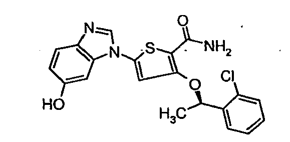

The title compound was prepared from methyl 3-{[(1R)-1-(2-chlorophenyl)ethyl]oxy}-5-(6-hydroxy-1H-benzimidazol-1-yl)thiophene-2-carboxylate by a procedure analogous to Example 5, Step D. MS (ESI): 414.13 [M+H]+.

### Step B - 3-{[(1R)-1-(2-chloropheny/)ethyl]oxy}-5-{6-[3-(dimethylamino)-propoxy]-1H-benzimidazo/-1-yl}thiophene-2-carboxamide (Title Compound)

To a solution of 3-{[(1R)-1-(2-chlorophenyl)ethyl]oxy}-5-(6-hydroxy-1H-benzimidazol-1-yl)thiophene-2-carboxamide (100 mg, 0.233 mmol) in N,N-dimethylformamide (5 mL) was added 3-chloro-N,N-dimethylpropan-1-amine (95.0mg, 0.582 mmol) and 368 mg (1.17 mmol) cesium carbonate, and the mixture stirred overnight at 60 oC. Water (20 mL) and ethyl acetate (50 mL) were added. The mixture was transferred to a separatory funnel and the organic layer was separated. The solvent was removed and purification by silica gel column chromatography (0 to 5% methanol:dichloromethane) provided 61.0 mg (52%) of the title compound as a tan foam. 1H NMR (400 MHz, DMSO-d6): δ 8.36 (s, 1H), 7.79 (brs, 1H), 7.66 (d, 1H, J = 8.0 Hz), 7.61 (d, 1H, J = 8.0 Hz), 7.45 (d, 1H, J = 8.0 Hz), 7.40 (t, 1H, 8.0 Hz), 7.32 (t, 1H, J = 8.0 Hz), 7.12 (s, 1H), 7.08 (br s, 1H), 6.95 (s, 1H), 6.91 (d, 1H, J = 8.0 Hz), 5.96 (q, 1H, J = 8.0 Hz), 4.02 - 3.93 (m, 2H) 2.37 (t, 2H, J = 8.0), 2.12 (s, 6H), 1.85 (t, 2H, J = 8.0,), 1.70 (d, 3H, J = 4.0 Hz); MS (ESI): 501.15 [M+H]+.

### Example 30: 1-[5-(Aminocarbonyl)-4-({(1R)-1-[2-(trifluoromethyl)phenyl]-ethyl}-oxy)-2-thienyl]-1H-benzimidazol-6-yl methanesulfonate

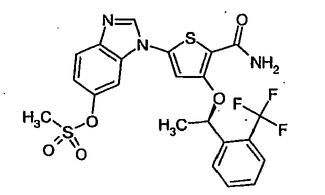

### Step A - 3-{[(1R)-1-(2-(Trifluoromethyl)phenyl)ethyl]oxy}-5-(6-hydroxy-1H-benzimidazol-1-yl)-2-thiophenecarboxamide

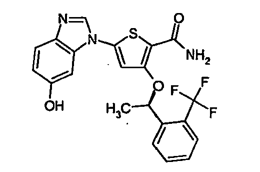

The title compound was prepared from methyl 5-(6-hydroxy-1*H*-benzimidazol-1-yl)-3-({(1*R*)-1-[2-(trifluoromethyl)phenyl)ethyl}oxy)-2-thiophenecarboxylate by a procedure analogous to **Example 49**, Step C. MS (ESI): 448.12 [M+H]⁺.

### Step B - 1-[5-(Aminocarbonyl)-4-({(1R)-1-[2-(trifluoromethyl)phenyl]ethyl}oxy)-2-thienyl]-1H-benzimidazol-6-yl methanesulfonate (Title Compound)

To a solution of 3-{[(1*R*)-1-(2-(trifluoromethyl)phenyl)ethyl]oxy}-5-(6-hydroxy-1*H*-benzimidazol-1-yl)-2-thiophenecarboxamide (62 mg, 0.14 mmol) in dichloromethane (2.8 mL) was added methanesulfonyl chloride (13 µL, 0.17 mmol). The reaction was stirred at room temperature for 19 h, and then triethylamine (0.50 mL) and of 4-*N,N*-dimethylaminopyridine (^{∼}5 mg) were added. After stirring for an additional 5 h at room temperature, the reaction was quenched by pouring into saturated aqueous ammonium chloride solution (25 mL). The aqueous layer was extracted with dichloromethane (2 x 20 mL). The combined organic fractions were concentrated onto silica gel. Purification by flash column chromatography (0 to 10% methanol:chloroform) followed by preparative HPLC (10 to 90% acetonitrile:0.1% formic acid/water) afforded 44 mg (60%) of the title compound. ¹H NMR (400 MHz, CDCl₃): δ 7.99 (s, 1H), 7.83 (d, 1H, *J*= 8.8 Hz), 7.75 (d, 1H, *J*= 8.0 Hz), 7.71-7.63 (m, 2H), 7.50 (t, 1H, *J*= 7.5 Hz), 7.31 (d, 1H, *J*= 2.2 Hz), 7.28-7.26 (m, 1H), 7.24 (br s, 1H), 6.66 (s, 1H), 6.45 (br s, 1H); 5.86 (q, 1H, *J*= 6.2 Hz), 3.17 (s, 3H), 1.81 (d, 3H, *J*= 6.2 Hz); MS (APCI): 525.87 [M+H]⁺.

### Example 31: 1-[5-(Aminocarbonyl)-4-({(1R)-1-[2-(trifluoromethyl)phenyl]-ethyl}oxy)-2-thienyl]-1H-benzimidazol-6-yl dimethylsulfamate

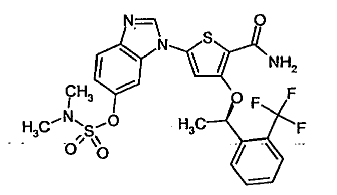

To a cooled (0 °C) solution of 3-{[(1*R*)-1-(2-chlorophenyl)ethyl]oxy}-5-(6-hydroxy-1*H*-benzimidazol-1-yl)-2-thiophenecarboxamide (60 mg, 0.13 mmol) in dichloromethane (2.7 mL) was added triethylamine (0.20 mL) and *N,N-*dimethylsulfamoyl chloride (37 µL, 0.35 mmol). The reaction was warmed to room temperature and stirred for 20 h. At that time, another portion of triethylamine (200 mL) and *N,N*-dimethylsulfamoyl chloride (36 µL, 0.34 mmol), along with 4-*N,N*-dimethylaminopyridine (^{∼}5 mg) were added. After stirring an additional 20.67 h at room temperature, the reaction was quenched by pouring into saturated aqueous ammonium chloride solution (25 mL). The aqueous layer was extracted with dichloromethane (2 x 20 mL). The combined organic fractions were concentrated dried over sodium sulfate, decanted, and concentrated onto silica gel. Purification by flash column chromatography (0 to 10% methanol:chloroform) followed by preparative HPLC (10 to 90% acetonitrile:0.1% formic acid in water) afforded 48 mg (65%) of the title compound. ¹H NMR (400 MHz, CDCl₃): δ 7.95 (s, 1H), 7.80 (d, 1H, *J*= 8.8 Hz), 7.75-7.69 (m, 2H), 7.64 (t, 1H, *J*= 7.6 Hz), 7.48 (t, 1H, *J*= 7.5 Hz), 7.38 (d, 1H, *J*= 2.2 Hz), 7.29-7.26 (m, 1H), 7.22 (br s, 1H), 6.65 (s, 1H), 5.89-5.85 (m, 2H), 3.01 (s, 6H), 1.81 (d, 3H, *J*= 6.2 Hz); MS (APCI): 554.90 [M+H]⁺.

### Example 32: 3-{[(1R)-1-(2-(Trifluoromethyl)phenyl)ethyl]oxy}-5-{6-[4S)-hexahydro-1H-azepin-4-yloxy]-1H-benzimidazol-1-yl}-2-thiophenecarboxamide trifluoroacetic acid salt

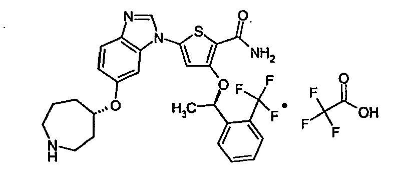

### Step A - 1,1-Dimethylethyl 4-oxohexahydro-1H-azepine-1-carboxylate

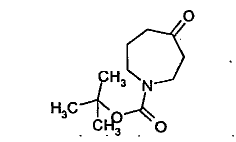

To a solution of hexahydro-4*H*-azepin-4-one hydrochloride (732 mg, 4.89 mmol) in tetrahydrofuran (24 mL) was added triethylamine (2.0 mL, 15 mmol) and di-*tert*-butyl dicarbonate (1.60 g, 7.34 mmol). The reaction was heated to 60°C for 4 h, and then additional triethylamine (0.68 mL, 4.9 mmol) and di-*tert*-butyl dicarbonate (1.07 g, 4.89 mmol) were added. After an additional 2 h of stirring at 60°C, the reaction mixture was poured into saturated aqueous ammonium chloride (100 mL) and extracted with ethyl acetate (3 x 30 mL). The combined organic fractions were washed with brine (1 x 50 mL), dried over magnesium sulfate, filtered, and concentrated. Purification by flash column chromatography (0 to 100% ethyl acetate:hexanes) afforded 970 mg (93%) of the title compound. ¹H NMR (300 MHz, CDCl₃): δ 3.58 (m, 4H), 2.65 (m, 4H), 1.79 (m, 2H), 1.46 (s, 9H).

### Step B-1,1-Dimethylethyl 4-hydroxyhexahydro-1H-azepine-1-carboxylate

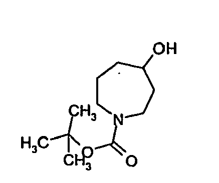

To a solution of 1,1-dimethylethyl 4-oxohexahydro-1*H*-azepine-1-carboxylate (970 mg, 4.55 mmol) in methanol (23 mL) was added polymer-supported borohydride resin (3.16 mmol/g, 1.15 g, 3.64 mmol). The reaction stirred at room temperature for 67.67 h and was then filtered to remove the resin, washing with dichloromethane (2 x 20 mL). The eluant was concentrated to afford 902 mg (92%) of the title compound. ¹H NMR (300 MHz, CDCl₃): δ 3.88 (m, 1H), 3.53-3.20 (m, 4H), 2.03-1.61 (m, 6H), 1.46 (s, 9H), 1.41 (br s, 1H).

### Step C - 1,1-Dimethy/ethy/ 4-[(methylsulfonyl)oxy]hexahydro-1H-azepine-1-carboxylate

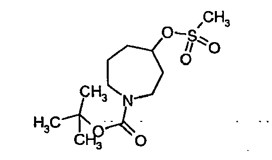

To a solution of 1,1-dimethylethyl 4-hydroxyhexahydro-1*H*-azepine-1-carboxylate (902 mg, 4.19 mmol) in dichloromethane (12.5 mL) was added triethylamine (875 []L, 6.28 mmol) and methanesulfonyl chloride (390 mL, 5.03 mmol). The reaction stirred at room temperature for 15 h and was then diluted with dichloromethane (20 mL). The organic solution was washed with saturated aqueous sodium bicarbonate (2 x 20 mL) and brine (20 mL), then dried over sodium sulfate, decanted, and concentrated to afford 1.19 g (97%) of the title compound. ¹H NMR (300 MHz, CDCl₃): δ 4.91 (m, 1H), 3.53-3.32 (m, 4H), 3.02 (s, 3H), 2.07-1.90 (m, 5H), 1.71 (m, 1H), 1.47 (s, 9H).

### Step D - 1,1-Dimethylethyl 4-({1-[5-[(methyloxy)carbonyl]-4-({(1R)-1-[2-(trifluoromethyl)phenyl]ethyl}oxy)-2-thienyl]-1H-benzimidazol-6-yl}oxy)hexahydro-1H-azepine-1-carboxylate

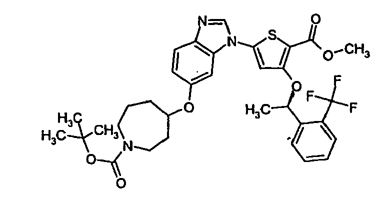

To a solution of methyl 3-{[(1*R*)-1-(2-(trifluoromethyl)phenyl)ethyl]oxy}-5-(6-hydroxy-1*H*-benzimidazol-1-yl)-2-thiophenecarboxylate (250 mg, 0.541 mmol) and 1,1-dimethylethyl 4-[(methylsulfonyl)oxy]hexahydro-1*H*-azepine-1-carboxylate (174 mg, 0.593 mmol) in *N,N*-dimethylformamide (5.5 mL) was added cesium carbonate (264 mg, 0.810 mmol). The reaction was heated to 60 °C for 26 h. Another portion of 1,1-dimethylethyl 4-[(methylsulfonyl)oxy]-hexahydro-1*H*-azepine-1-carboxylate (40 mg, 0.14 mmol) was then added, and the reaction stirred at 60 °C for a further 5 h. The mixture was then diluted with brine:water (1:1, 200 mL) and extracted with ethyl acetate (3 x 50 mL). The combined organic fractions were dried over sodium sulfate, filtered, and concentrated onto silica gel. Purification by flash column chromatography afforded 316 mg (89%) of the title compound. MS (ESI): 660.29 [M+H]⁺.

### Step E - 1,1-Dimethylethyl (4S)-4-({1-[5-(aminocarbonyl)-4-({(1R)-1-[2-(trifluoromethyl)phenyl]ethyl}oxy)-2-thienyl]-1H-benzimidazol-6-yl}oxy)hexahydro-1H-azepine-1-carboxylate

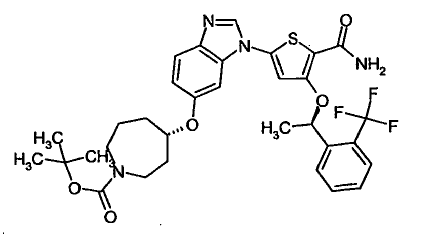

The title compound was prepared from 1,1-dimethylethyl 4-({1-[5-[(methyloxy)carbonyl]-4-({(1*R*)-1-[2-(trifluoromethyl)phenyl]ethyl}oxy)-2-thienyl]-1*H*-benzimidazol-6-yl}oxy)hexahydro-1*H*-azepine-1-carboxylate by a procedure analogous to **Example 49**, Step C. The compound was further purified by preparative HPLC (15% 2-propanol:hexanes) to separate the mixture of diastereomers into the individual diastereomers. Details for the other diastereomer are provided in **Example 33**. MS (ESI): 646.40 [M+H]⁺.

### Step F -3-{[(1R)-1-(2-(Trifluoromethyl)phenyl)ethyl]oxy}-5-{6-[(4S)-hexahydro-1H-azepin-4-yloxy]-1H-benzimidazol-1-yl}-2-thiophenecarboxamide trifluoroacetic acid salt (Title Compound)

The title compound was prepared from 1,1-dimethylethyl (4*S*)-4-({1-[5-(aminocarbonyl)-4-({(1*R*)-1-[2-(trifluoromethyl)phenyl)ethyl}oxy)-2-thienyl)-1*H-*benzimidazol-6-yl}oxy)hexahydro-1H-azepine-1-carboxylate by a procedure analogous to **Example 49**, Step D. ¹H NMR (400 MHz, CDCl₃): δ 9.81 (br s, 1H), 9.55 (br s, 1H), 7.99 (s, 1H), 7.74-7.61 (m, 4H), 7.47 (t, 1H, *J*= 7.5 Hz), 7.30 (br s, 1H), 7.05 (d, 1H, *J*= 2.2 Hz), 6.99 (dd, 1H, *J*= 2.1, 8.9 Hz), 6.85 (br s, 1H), 6.66 (s, 1H), 5.86 (q, 1H, *J*= 6.2 Hz), 4.76 (m, 1H), 3.44-3.26 (m, 4H), 2.29 (m, 2H), 2.20-2.03 (m, 3H), 1.93-1.84 (m, 1H), 1.82 (d, 3H, *J*= 6.5 Hz); MS (ESI): 545.25 [M+H]⁺.

### Example 33: 3-{[(1R)-1-(2-(Trifluoromethyl)phenyl)ethyl]oxy}-5-{6-[(4R)-hexahydro-1H-azepin-4-yloxy]-1H-benzimidazol-1-yl}-2-thiophenecarboxamide trifluoroacetic acid salt

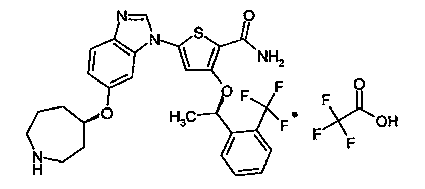

### Step A - 1,1-Dimethylethyl (4R)-4-({1-[5-(aminocarbonyl)-4-({(1R)-1-[2-(trifluoromethyl)phenyl]ethyl}oxy)-2-thienyl]-1H-benzimidazol-6-yl}oxy)hexahydro-1H-azepine-1-carboxylate

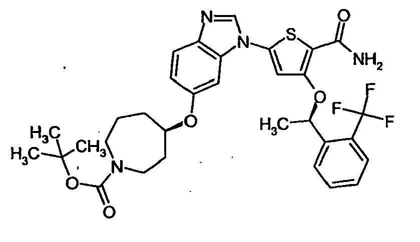

The title compound was prepared by separating from its diastereomer as described in **Example 32**, Step E. MS (ESI): 646.40 [M+H]⁺.

### Step B - 3-{[(1R)-1-(2-(Trifluoromethyl)phenyl)ethyl]oxy}-5-{6-[(4R)-hexahydro-1H-azepin-4-yloxy]-1H-benzimidazol-1-yl}-2-thiophenecarboxamide trifluoroacetic acid salt (Title Compound)

The title compound was prepared from 1,1-dimethylethyl (4*R*)-4-({1-[5-(aminocarbonyl)-4-({(1*R*)-1-[2-(trifluoromethyl)phenyl]ethyl}oxy)-2-thienyl]-1*H-*benzimidazol-6-yl}oxy)hexahydro-1*H*-azepine-1-carboxylate by a procedure analogous to **Example 49**, Step D. ¹H NMR (400 MHz, CDCl3): δ 9.51 (br s, 2H), 8.17 (s, 1H), 7.78-7.62 (m, 4H), 7.48 (t, 1H, *J*= 7.6 Hz), 7.32 (br s, 1H), 7.06-7.02 (m, 2H), 6.89 (br s, 1H), 6.70 (s, 1H), 5.86 (q, 1H, *J*= 6.2 Hz), 4.74 (m, 1H), 3.49-3.26 (m, 4H), 2.29 (m, 2H), 2.20-1.99 (m, 3H), 1.93-1.84 (m, 1H), 1.81 (d, 3H, *J*= 6.2 Hz); MS (ESI): 545.24 [M+H]⁺.

### Example 34: 5-{6-[(Cis-4-aminoclohexyl)oxy]-1H-benzimidazol-1-yl}-3-{[(1R)-1-2-chlorophenyl)ethyl]oxy}-2-thiophenecarboxamide

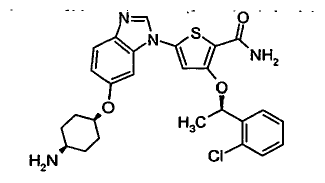

### Step A - Methyl 3-{[(1R)-1-(2-chlorophenyl)ethyl]oxy}-5-(6-{[cis-4-(1,3-dioxo-1,3-dihydro-2H-isoindol-2-yl)cyclohexyl]oxy}-1H-benzimidazol-1-yl)-2-thiophenecarboxylate

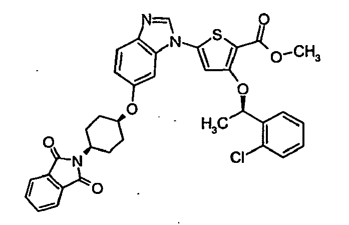

The title compound was prepared from methyl 3-{[(1*R*)-1-(2-chlorophenyl)ethyl]oxy}-5-(6-hydroxy-1*H*-benzimidazol-1-yl)-2-thiophenecarboxylate and the literature-known 2-(trans-4-hydroxycyclohexyl)-1*H*-bisoindole-1,3(2*H*)-dione by a procedure analogous to Intermediate **Example 3**, Step B. MS (ESI): 656 [M+H]⁺.

### Step B - 5-{6-[(Cis-4-aminocyclohexyl)oxy]-1H-benzimidazol-1-yl}-3-{[(1R)-1-(2-chlorophenyl)ethyl]oxy}-2-thiophenecarboxamide (Title Compound)

The title compound was prepared from methyl 3-{[(1*R*)-1-(2-chlorophenyl)ethyl]oxy}-5-(6-{[*cis*-4-(1,3-dioxo-1,3-dihydro-2*H*-isoindol-2-yl)cyclohexyl]oxy}-1*H*-benzimidazol-1-yl)-2-thiophenecarboxylate by a procedure analogous to **Example 5**, Step D. MS (ESI): 511 [M+H]⁺. ¹H NMR (400 MHz, DMSO-*d₆*): δ 8.42 (s, 1H), 7.68-7.61 (m, 2H), 7.47-7.30 (m, 3H), 7.25-7.04 (m, 4H), 5.95 (q, 1H, *J*= 6.4 Hz), 4.58 (br s, 1H), 3.18-3.05 (m, 2H), 1.95 (m, 2H), 1.79-1.55 (m, 9H), 1.20 (s, 2H).

### Intermediate Example 7: tert-butyl 4-mercaptopiperidine-1-carboxylate

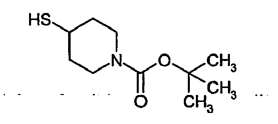

### Step A - Tert-butyl 4-{[(4-methylphenyl)sulfonyl]oxy}piperidine-1-carboxylate

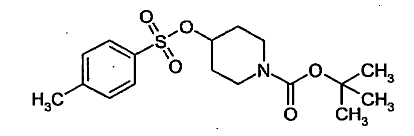

A solution of *tert*-butyl 4-hydroxypiperidine-1-carboxylate (10 g, 49.7mmol) and p-methylbenzenesulfonyl chloride (11.8 g, 62.1 mmol) in pyridine (50 mL) was allowed to stir at room temperature for 14 h. The solvent was removed *in vacuo* and the residue was partitioned between dichloromethane (200 mL) and water (200 mL). The organic layer was washed with water (2 x 100 mL), dried over sodium sulfate and concentrated *in vacuo.* Recrystallization from hexanes afforded 14.48 g (82%) of the title compound as a colorless crystalline solid. ¹H NMR (400 MHz, DMSO-*d₆*): δ 7.80-7.76 (m, 2H), 7.47-7.43 (m, 2H), 4.67-4.61 (m, 1H), 3.48-3.42 (m, 2H), 3.16-3.05 (m, 2H), 2.39 (s, 3H), 1.70-1.63 (m, 2H), 1.49-1.40 (m, 2H), 1.33 (s, 9H).

### Step B - Tert-butyl 4-(acetylthio)piperidine-1-carboxylate

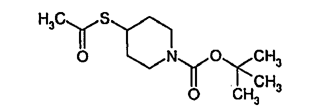

A solution of *tert*-butyl 4-{[(4-methylphenyl)sulfonyl]oxy}piperidine-1-carboxylate (14.4 g, 40.5 mmol) and potassium thioacetate (23.1 g, 202.6mmol) in *N,N-*dimethylformamide (150 mL) was allowed to stir at 50 °C for 14 h. The reaction was cooled to room temperature and diluted with ethyl acetate (300 mL). The organic layer was washed with water (5 x 150 mL), dried over sodium sulfate and concentrated *in vacuo.* Flash column chromatography (5% ethyl acetate:hexanes) afforded 7.52 g (72%) of the title compound as a yellow oil: ¹H NMR (400 MHz, DMSO-*d₆*): δ 3.73-3.64 (m, 2H), 3.55-3.48 (m, 1H), 3.09-2.92 (br s, 2H), 2.30 (s, 3H), 1.83-1.77 (m, 2H), 1.44-1.35 (m, 2H), 1.37 (s, 9H).

### Step C - Tert-butyl 4-mercaptopiperidine-1-carboxylate (Title Compound)

A solution of sodium methoxide (70 mL, 0.5 M in methanol, 35 mmol) was added dropwise to a stirring solution of *tert*-butyl 4-(acetylthio)piperidine-1-carboxylate (7.52 g, 29.0mmol) in methanol (120 mL) at 0°C. The reaction was allowed to stir at 0°C for 1 h and then warmed to room temperature and allowed to stir for an additional 4 h. The solvent was removed *in vacuo* and the residue was partitioned between ethyl acetate (150 mL) and water (150 mL). The organic layer was washed with water (2 x 100 mL), dried over sodium sulfate and concentrated *in vacuo.* Flash column chromatography (10% ethyl acetate:hexanes) afforded 5.35 g (85%) of the title compound as a pale yellow solid. ¹H NMR (400 MHz, DMSO-*d₆*): δ 3.82-3.74 (m, 2H), 2.95-2.75 (m, 3H), 2.64 (d, 1H, *J*= 10 Hz), 1.89-1.81 (m, 2H), 1.42-1.27 (m, 2H), 1.37 (s, 9H).

### Example 35: 3-{[(1R)-1-(2-Chlorophenyl)ethyl]oxy}-5-{6-[(piperidin-4-ysulfonyl)methyl]-1H-benzimidazol-1-yl}thiophene-2-carboxamide

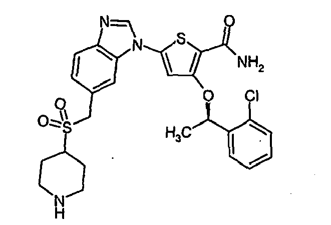

### Step A - Tert-buty/ 4-[({1-[4-{[(1R)-1-(2-ch/orophenyl)ethy/]oxy}-5-(methoxycarbonyl)thien-2-yl]-1H-benzimidazol-6-yl}methyl)thio]piperidine-1-carboxylate

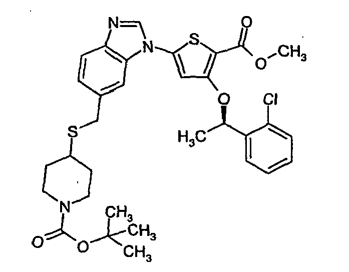

A mixture of methyl 5-[6-(chloromethyl)-1*H*-benzimidazol-1-yl)-3-{[(1*R*)-1-(2-chlorophenyl)ethyl]oxy}thiophene-2-carboxylate (0.843 g, 1.83 mmol), *tert-*butyl 4-mercaptopiperidine-1-carboxylate (0.456 g, 2.10 mmol), and potassium carbonate (0.694 g, 5.02 mmol) in *N,N*-dimethylformamide (10 mL) was stirred at 60°C for 14 h. The reaction was cooled to room temperature and partitioned between ethyl acetate (100 mL) and water (100 mL). The layers were separated and the organic layer was washed with water (5 x 50 mL), dried over sodium sulfate and concentrated *in vacuo* to afford 1.23 g (100%) of the title compound as a colorless solid. ¹H NMR (400 MHz, DMSO-*d₆*): δ 8.63 (s, 1H), 7.74-7.69 (m, 2H), 7.60 (br s, 1H), 7.48-7.41 (m, 3H), 7.36-7.32 (m, 2H), 5.96 (q, 1H, *J*= 6.2 Hz), 3.94 (br s, 2H), 3.82 (s, 3H), 3.80-3.73 (m, 2H), 2.88-2.70 (m, 3H), 1.91-1.82 (m, 2H), 1.62 (d, 3H, *J*= 6.2 Hz), 1.40-1.25 (m, 2H), 1.36 (s, 9H).

### Step B - Tert-butyl 4-[({1-[4-{[(1R)-1-(2-chlorophenyl)ethyl]oxy}-5-(methoxycarbony/)thien-2-y/]-1H-benzimidazo/-6-yl}methy/)su/fonyl]piperidine-1-carboxylate

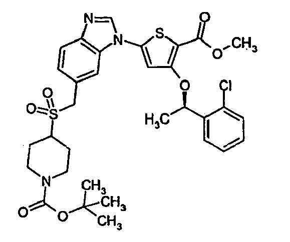

A solution of *tert-*butyl 4-[({1-[4-{[(1*R*)-1-(2-chlorophenyl)ethyl]oxy}-5-(methoxycarbonyl)thien-2-yl]-1*H*-benzimidazol-6-yl}methyl)thio]piperidine-1-carboxylate (1.23 g, 1.92 mmol) in dichloromethane (35 mL) at -10 °C was treated with *m*-chloroperoxybenzoic acid (0.662 g, 3.84 mmol) in portions. After stirring for 1 h at -10 °C the reaction was warmed quickly to room temperature and diluted with dichloromethane (100 mL). The acidic reaction components were extracted into saturated aqueous sodium bicarbonate and the organic layer was washed with water (1 x 50mL), dried over sodium sulfate and concentrated *in vacuo* to afford the 1.26 g (98%) of the title compound as a colorless solid. ¹H NMR (400 MHz, DMSO-*d₆*): δ 8.71 (s, 1H), 7.80 (s, 1H), 7.78-7.74 (m, 2H), 7.52 (s, 1H), 7.47-7.32 (m, 4H), 5.94 (q, 1H, *J*= 6.2 Hz), 4.65 (br s, 2H), 4.08-3.99 (br s, 2H), 3.82 (s, 3H), 2.86-2.72 (br s, 2H), 2.10-2.02 (br s, 2H), 1.63 (d, 3H, *J*= 6.2 Hz), 1.53-1.43 (m, 2H), 1.38 (s, 9H).

### Step C - Methyl 3-{[(1R)-1-(2-chlorophenyl)ethyl]oxy}-5-{6-[(piperidin-4-ylsulfonyl)methyl]-1H-benzimidazol-1-yl}thiophene-2-carboxylate

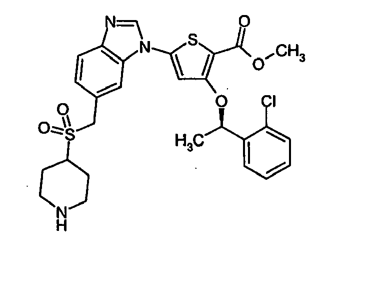

A solution of *tert-*butyl 4-[({1-[4-{[(1*R*)-1-(2-chlorophenyl)ethyl]oxy}-5-(methoxycarbonyl)thien-2-yl]-1*H*-benzimidazol-6-yl}methyl)sulfonyl]piperidine-1-carboxylate (1.26 g, 1.87 mmol) in dichloromethane (20 mL) was treated with trifluoroacetic acid (6 mL) dropwise *via* syringe at room temperature. After stirring for 1 h at room temperature the reaction was quenched with saturated aqueous sodium bicarbonate (15 mL) and the aqueous layer was extracted with dichloromethane (3 x 15 mL). The combined organic extracts were dried over sodium sulfate and concentrated *in vacuo to* afford 1.23 g (100%) the title compound as a colorless solid. ¹H NMR (400 MHz, DMSO-*d₆*): δ 8.69 (s, 1H), 7.80-7.73 (m, 3H), 7.49 (s, 1H), 7.45-7.40 (m, 2H), 7.36-7.30 (m, 2H), 5.92 (q, 1H, *J*= 6.3 Hz), 4.59 (br s, 2H), 3.81 (s, 3H), 3.14-3.00 (br s, 3H), 1.99-1.92 (br s, 2H), 1.61 (d, 3H, *J*= 6.3 Hz), 1.56-1.44 (m, 2H).

### Step D - 3-{[(1R)-1-(2-chlorophenyl)ethyl]oxy}-5-{6-(piperidin-4-ylsulfonyl)-methyl]-1H-benzimidazol-1-yl}thiophene-2-carboxamide (Title Compound)

The title compound was prepared from methyl 3-{[(1*R*)-1-(2-chlorophenyl)ethyl]oxy}-5-{6-[(piperidin-4-ylsulfonyl)methyl]-1*H*-benzimidazol-1-yl}thiophene-2-carboxylate by a procedure analogous to **Example 5**, Step D. ¹H NMR (400 MHz, DMSO-*d₆*): δ 8.60 (s, 1H), 7.83 (br s, 1H), 7.78-7.76 (m, 1H), 7.70-7.66 (m, 2H), 7.48-7.46 (m, 1H), 7.43-7.39 (m, 1H), 7.36-7.32 (m, 2H), 7.27 (s, 1H), 7.11 (br s, 1H), 5.95 (q, 1H, *J*= 6.4 Hz), 4.60-4.52 (m, 2H), 3.14-2.96 (m, 3H), 2.48-2.38 (m, 2H), 1.98-1.90 (br s, 2H), 1.72 (d, 3H, *J* = 6.4 Hz), 1.54-1.44 (m, 2H); MS (ESI): 559 [M+H]⁺.

### Example 36: 3-{[(1R)-1-(2-Chlorophenyl)ethyl]oxy)-5-[6-({[3-(dimethylamino)propyl]sulfonyl}methyl)-1H-benzimidazol-1-yl]thiophene-2-carboxamide

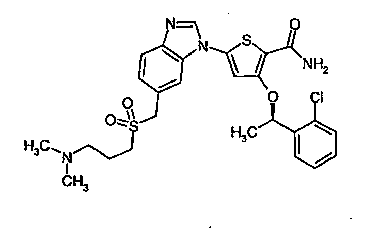

### Step A - Methyl 3-{[(1R)-1-(2-chloropheny/)ethyl]oxy}-5-(6-{[(3-hydroxypropyl)thio]methyl}-1H-benzimidazo/-1-yl)thiophene-2-carboxy/ate

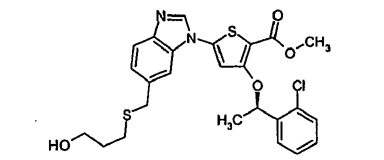

The title compound was prepared from methyl 5-[6-(chloromethyl)-1*H-*benzimidazol-1-yl]-3-{[(1*R*)-1-(2-chlorophenyl)ethyl]oxy}thiophene-2-carboxylate and 3-mercapto-1-propanol by a procedure analogous to **Example 35**, Step A. ¹H NMR (400 MHz, DMSO-*d₆*): δ 8.62 (s, 1H), 7.73-7.69 (m, 2H), 7.57-7.55 (m, 1H), 7.48-7.41 (m, 2H), 7.39 (s, 1H), 7.36-7.30 (m, 2H), 5.96 (q, 1H, *J*= 6.2 Hz), 4.44 (t, 1H, *J*= 5.2 Hz), 3.87 (s, 2H), 3.82 (s, 3H), 3.43-3.38 (m, 2H), 2.45-2.41 (m, 2H), 1.66-1.59 (m, 2H), 1.62 (d, 3H, *J*= 6.2 Hz).

### Step B - Methyl 3-{[(1R)-1-(2-chlorophenyl)ethyl]oxy}-5-(6-{[(3-hydroxypropyl)sulfonyl]methyl}-1H-benzimidazol-1-yl)thiophene-2-carboxylate

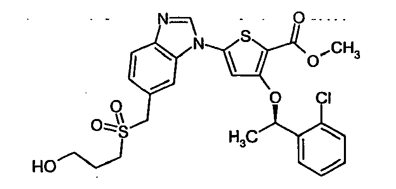

The title compound was prepared from methyl 3-{[(1*R*)-1-(2-chlorophenyl)ethyl]oxy}-5-(6-{[(3-hydroxypropyl)thio]methyl}-1H-benzimidazol-1-yl)thiophene-2-carboxylate by a procedure analogous to **Example 35,** Step B. ¹H NMR (400 MHz, DMSO-*d₆*): δ 8.69 (s, 1H), 7.79 (s, 1H), 7.77-7.73 (m, 2H), 7.48 (s, 1H), 7.46-7.30 (m, 4H), 5.93 (q, 1H, *J*= 6.2 Hz), 4.66-4.61 (m, 3H), 3.80 (s, 3H), 3.46-3.41 (m, 2H), 3.07-3.01 (m, 2H), 1.84-1.76 (m, 2H), 1.61 (d, 3H, *J*= 6.2 Hz).

### Step C - Methyl 3-{[(1R)-1-(2-chlorophenyl)ethyl]oxy}-5-{6-[({3-[(methylsulfonyl)oxy]propyl}sulfonyl)methyl]-1H-benzimidazol-1-yl}thiophene-2-carboxylate

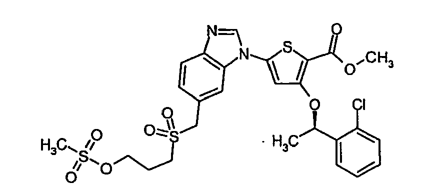

Methanesulfonic anhydride (0.699 g, 4.02mmol) was added to a stirring solution of methyl 3-{[(1*R*)-1-(2-chlorophenyl)ethyl]oxy}-5-(6-{[(3-hydroxypropyl)sulfonyl]methyl}-1*H*-benzimidazol-1-yl)thiophene-2-carboxylate (1.47 g, 2.68 mmol) and triethylamine (1.1 mL, 8.03 mmol) in dichloromethane (30 mL) at 0 °C. After stirring for 0.5 h the reaction was quenched with water (15 mL) and the layers were separated. The aqueous layer was extracted with dichloromethane (3 x 15 mL) and the combined organic extracts were dried over sodium sulfate, filtered, and concentrated *in vacuo* to afford 1.73 g (100%) of the title compound as a colorless solid. ¹H NMR (400 MHz, DMSO-*d₆*): δ 8.70 (s, 1H), 7.80-7.73 (m, 3H), 7.48 (s, 1H), 7.45-7.37 (m, 3H), 7.34-7.29 (m, 1H), 5.93 (q, 1H, *J*= 6.2 Hz), 4.73-4.65 (m, 2H), 4.27-4.24 (m, 2H), 3.80 (s, 3H), 3.19-3.13 (m, 5H), 2.11-2.04 (m, 2H), 1.61 (d, 3H, *J*= 6.2 Hz).

### Step D - Methyl 3-{[(1R)-1-(2-chlorophenyl)ethyl]oxy}-5-[6-({[3-(dimethylamino)propyl]sulfonyl}methyl)-1H-benzimidazol-1-yl]thiophene-2-carboxylate

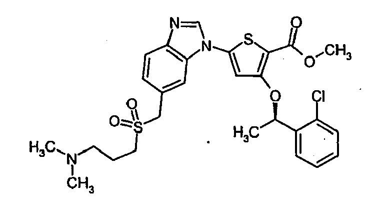

A solution of methyl 3-{[(1*R*)-1-(2-chlorophenyl)ethyl]oxy}-5-{6-[({3-[(methylsulfonyl)oxy]propyl}sulfonyl)methyl]-1*H*-benzimidazol-1-yl}thiophene-2-carboxylate (0.250 g, 0.40 mmol) in dimethylamine (2 N in THF, 8 mL, 4 mmol) was heated at 60 °C for 14 h. The solvent was removed *in vacuo* and the residue was purified by flash chromatography to afford 0.230 g (100%) of the title compound as a pale yellow solid: MS (APCI): 576 [M+H]⁺.

### Step E- 3-{[(1R)-1-(2-chlorophenyl)ethyl]oxy}-5-[6-({[3-(dimethylamino)propyl]sulfonyl}methyl)-1H-benzimidazol-1-yl]thiophene-2-carboxamide (Title Compound)

The title compound was prepared from methyl 3-{[(1*R*)-1-(2-chlorophenyl)ethyl]oxy}-5-[6-({[3-(dimethylamino)propyl]sulfonyl}methyl)-1*H-*benzimidazol-1-yl]thiophene-2-carboxylate by a procedure analogous to **Example 5,** Step D. ¹H NMR (400 MHz, DMSO-*d₆*): δ 8.59 (s, 1H), 7.81 (br s, 1H), 7.77-7.75 (m, 1H), 7.70 (br s, 1H), 7.68-7.65 (m, 1H), 7.47-7.45 (m, 1H), 7.41-7.31 (m, 3H), 7.25(s, 1H), 7.09 (br s, 1H), 5.93 (q, 1H, *J*= 6.3Hz), 4.65-4.56 (m, 2H), 3.02-2.98 (m, 2H), 2.23-2.20 (m, 2H), 2.02 (s, 6H), 1.78-1.70 (m, 5H); MS (ESI): 561 [M+H]⁺.

### Example 37: 3-{[(1R)-1-(2-Chlorophenyl)ethyl]oxy}-5-(6-{[(methylamino)sulfonyl]methyl}-1H-benzimidazol-1-yl)thiophene-2-carboxamide

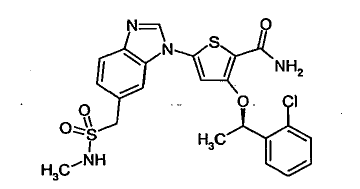

### Step A - Sodium {1-[4-{[(1R)-1-(2-chloropheny/)ethyl]oxy}-5-(methoxycarbonyl)thien-2-yl]-1H-benzimidazol-6-yl}methanesulfonate

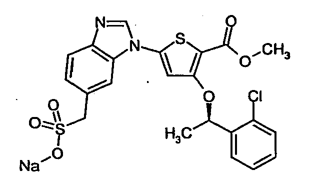

A solution of sodium sulfite (0.50 g, 3.94 mmol) in water (10 mL) was added to a solution of methyl 5-[6-(chloromethyl)-1*H*-benzimidazol-1-yl]-3-{[(1*R*)-1-(2-chlorophenyl)ethyl]oxy}thiophene-2-carboxylate (1.82 g, 3.94 mmol) in acetone (10 mL) and the resultant solution was heated at reflux for 14 h. The solvent was removed *in vacuo* to afford 2.12 g (100%) of the title compound as a pale yellow solid which was used without further purification: ¹H NMR (400 MHz, DMSO-*d₆*): δ 8.61 (s, 1H), 7.75-7.73 (m, 1H), 7.64-7.62 (m, 2H), 7.47-7.41 (m, 3H), 7.35-7.27 (m, 2H), 5.95 (q, 1H, *J*= 6.4 Hz), 3.84 (br s, 2H), 3.82 (s, 3H), 1.62 (d, 3H, *J*= 6.4 Hz).

### Step B - Methyl 3-{[(1R)-1-(2-chloropheny/)ethyl]oxy}-5-(6-{[(methylamino)sulfonyl]methyl}-1H-benzimidazo/-1-y/)thiophene-2-carboxylate

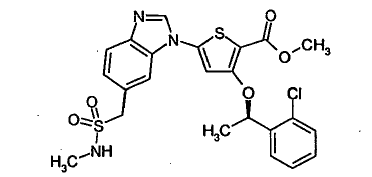

### A solution of sodium {1-[4-{[(1R)-1-(2-chlorophenyl)ethyl]oxy}-5-(methoxycarbonyl)thien-2-yl]-1H-benzimidazol-6-yl}methanesulfonate (0.26 g, 0.49 mmol) in thionyl chloride (5 mL) was heated at reflux for 1 h. The reaction was cooled to room temperature and the solvent was removed in vacuo. The residue was dissolved in dichloromethane (5 mL) and treated sequentially with methylamine (0.2 mL, 2 N in THF, 0.4 mmol) and diisopropylethylamine (0.2 mL, 0.74 mmol) at room temperature. Flash chromatography afforded 23 mg (9%) of the title compound. ¹H NMR (400 MHz, CDCl₃): δ 8.01 (s, 1H), 7.82-7.80 (m, 1H), 7.66-7.64 (m, 1H), 7.50 (br s, 1H), 7.39-7.23 (m, 4H), 6.71 (s, 1H), 5.81 (q, 1H, J= 6.4 Hz), 4.35 (s, 2H), 3.99 (q, 1H, J= 5.1 Hz), 3.91 (s, 3H), 2.68 (d, 3H, J= 5.1 Hz), 1.73 (d, 3H, J= 6.4Hz).

### Step C - 3-{[(1R)-1-(2-Chlorophenyl)ethyl]oxy}-5-(6-{[(methylamino)sulfonyl]methyl}-1H-benzimidazol-1-yl)thiophene-2-carboxamide

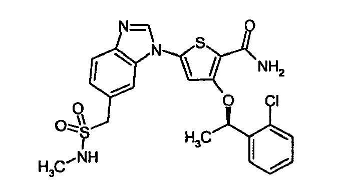

The title compound was prepared from methyl 3-{[(1*R*)-1-(2-chlorophenyl)ethyl]oxy}-5-(6-{[(methylamino)sulfonyl]methyl}-1*H-*benzimidazol-1-yl)thiophene-2-carboxylate by a procedure analogous to **Example 5**, Step D. ¹H NMR (400 MHz, CD₃OD): δ 8.39 (s, 1H), 7.74-7.72 (m, 1H), 7.61-7.59 (m, 1H), 7.55 (br s, 1H), 7.49-7.32 (m, 4H), 7.04 (s, 1H), 6.05 (q, 1H, *J*= 6.4 Hz), 4.48-4.40 (m, 2H), 2.65 (s, 3H), 1.79 (d, 3H, *J*= 6.4 Hz); MS (ESI): 505 [M+H]⁺.

### Example 38: 5-(6-{[(Methylsulfonyl)amino]methyl}-1H-benzimidazol-1-yl)-3-({(1R)-1-[2-(trifluoromethyl)phenyl]ethyl}oxy)thiophene-2-carboxamide

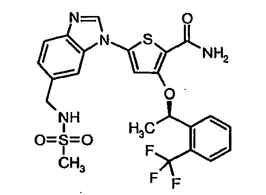

### Step A - 5-[6-(Aminomethyl)-1H-benzimidazol-1-yl]-3-({(1R)-1-[2-(trifluoromethyl)pheny/]ethyl}oxy)thiophene-2-carboxamide

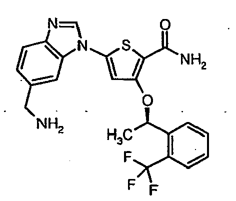

A mixture of methyl 5-[6-(chloromethyl)-1*H*-benzimidazol-1-yl]-3-({(1*R*)-1-[2-(trifluoromethyl)phenyl]ethyl}oxy)thiophene-2-carboxylate (250 mg, 0.50 mmol) and 7 N ammonia in methanol (100 mL, 700.0 mmol) in a sealed high pressure glass reaction vessal was heated at 80°C for ^{∼}36 h. The reaction was then cooled to room temperature, concentrated under vacuum and purified by silica gel chromatography (0 to 10% methanol:dichloromethane, with 1% ammonium hydroxide) to give 214 mg (92%) of the title compound as a white solid. ¹H NMR (400 MHz, DMSO-*d₆*): δ 8.45 (s, 1H), 7.93 (d, 1H, *J* =7.87 Hz), 7.84 (s, 1H), 7.77 (m, 2H), 7.66 (d, 1H, *J*= 8.23 Hz), 7.56 (t, 1H, *J* = 7.59 Hz), 7.46 (s, 1H), 7.30 (d, 1H, *J*= 9.15 Hz), 7.14 (s, 1H), 7.09 (s, 1H), 5.94 (m, 1H), 3.81 (s, 2H), 1.87 (s, 2H), 1.75 (d, 3H, *J*= 6.22 Hz).

### Step B - 5-(6-{[(Methylsulfonyl)amino]methyl}-1H-benzimidazol-1-yl)-3-({(1R)-1-[2-(trifluoromethyl)pheny/]ethy/}oxy)thiophene-2-carboxamide (Title Compound)

To a stirred, cooled (0 °C) solution of 5-[6-(aminomethyl)-1*H*-benzimidazol-1-yl]-3-({(1*R*)-1-(2-(trifluoromethyl)phenyl]ethyl}oxy)thiophene-2-carboxamide (78.5 mg, 0.17 mmol) in dichloromethane (1.0 mL) was added triethylamine (30.0 µL, 0.21 mmol) followed by methanesulfonyl chloride (15.0 µL, 0.19 mmol) in dichloromethane (1 mL). The reaction was stirred 1 h, then diluted with ethyl acetate and washed with water, brine, dried over sodium sulfate, concentrated under vacuum and purified by silica gel chromatography (0 to 10% methanol:dichloromethane, with 1% ammonium hydroxide) to give 17 mg (18%) of the title compound as a white solid. ¹H NMR (400 MHz, DMSO-*d₆*): δ 8.51 (s, 1H), 7.95 (d, 1H, *J*=8.05 Hz), 7.85 (s, 1H), 7.80-7.71 (m, 3H), 7.63-7.51 (m, 3H), 7.34 (d, 1H, *J*= 8.23 Hz), 7.14 (s, 2H), 5.95-5.90 (m, 1H), 4.26 (d, 2H, *J* = 5.12 Hz), 2.83 (s, 3H), 1.75 (d, 3H, *J* = 6.22 Hz); MS (ESI): 538 [M+H]⁺.

### Example 39: 5-{6-[(methylsulfonyl)methyl]-1H-benzimidazol-1-yl}-3-({(1R)-1-[2-(trifluoromethyl)phenyl]ethyl}oxy)-2-thiophenecarboxamide

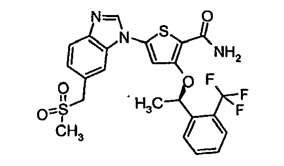

### Route 1: Step A - methy/ 5-{6-[(methylthio)methy/]-1H-benzimidazol-1-yl}-3-({(1R)-1-[2-(trifluoromethyl)phenyl]ethyl}oxy)thiophene-2-carboxy/ate

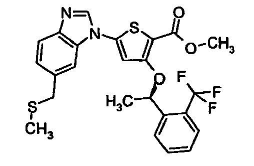

To a stirred mixture of methyl 5-[6-(chloromethyl)-1*H*-benzimidazol-1-yl]-3-({(1*R*)-1-[2-(trifluoromethyl)phenyl]ethyl}oxy)thiophene-2-carboxylate (200 mg, 0.40 mmol) in *N,N*-dimethylformamide (2.5 mL) was added sodium thiomethoxide (37 mg, 0.52 mmol). The reaction was stirred 30 min, then diluted with ethyl acetate, washed with water (5x), brine, dried over sodium sulfate, concentrated under vacuum and purified by silica gel chromatography (0 to 60% ethyl acetate:hexanes) to give 147 mg (72%) of the title compound as a white solid. MS (ESI): 507 [M+H]⁺.

### Step B - 5-{6-[(Methylthio)methyl]-1H-benzimidazol-1-yl}-3-({(1R)-1-[2-(trifluoromethyl)phenyl]ethyl}oxy)thiophene-2-carboxamide

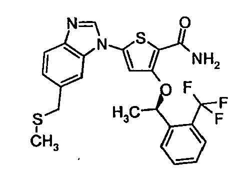

A mixture of methyl 5-{6-[(methylthio)methyl]-1H-benzimidazol-1-yl}-3-({(1*R*)-1-[2-(trifluoromethyl)phenyl]ethyl}oxy)thiophene-2-carboxylate (144.0 mg, 0.28 mmol) and 7 N ammonia in methanol (18 mL, 126.0 mmol) was added to a high-pressure glass reaction flask. The flask was sealed, then heated to 80 °C for ^{∼}16 h. The flask was cooled to room temperature, opened, and the reaction mixture concentrated under vacuum, then purified by silica gel chromatography (0 to 3% methanol:dichloromethane w/ 1% ammonium hydroxide to give 130 mg (93%) of the title compound as a white gold solid. ¹H NMR (400 MHz, DMSO-*d₆*): δ 8.49 (s, 1H), 7.93 (d, 1H, *J*= 7.68 Hz), 7.85 (br s, 1H), 7.80-7.75 (m, 2H), 7.69 (d, 1H, *J*= 8.23 Hz), 7.56 (t, 1H, *J*= 7.68 Hz), 7.39 (s, 1H), 7.29 (d, 1H, *J*= 8.42 Hz), 7.15 (br s, 1H), 7.08 (s, 1H), 5.94 (m, 1H), 3.79 (s, 2H), 1.93 (s, 3H), 1.75 (d, 3H, *J*= 6.22 Hz); MS (ESI): 492 [M+H]⁺.

### Step C - 5-{6-[(Methylsulfonyl)methyl]-1H-benzimidazol-1-yl}-3-({(1R)-1-[2-(trifluoromethyl)phenyl]ethyl}oxy)thiophene-2-carboxamide

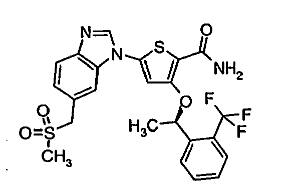

To a stirred, cooled (-10°C) solution of 5-{6-[(methylthio)methyl]-1*H-*benzimidazol-1-yl}-3-({(1*R*)-1-[2-(trifluoromethyl)phenyl]ethyl}oxy)thiophene-2-carboxamide (76 mg, 0.15 mmol) in dichloromethane (3.0 mL) was added m-chloroperoxybenzoic acid (70 mg, 0.31 mmol). The reaction was stirred 30 min, warmed to room temperature and stirred 15 min, then was concentrated under vacuum. The residue was diluted with chloroform and washed with aqueous saturated sodium bicarbonate, water, dried over sodium sulfate, concentrated under vacuum, and purified by silica gel chromatography (0 to 5% methanol:dichloromethane w/ 1% ammonium hydroxide), to give 78 mg (96%) of the title compound as a white solid. ¹H NMR (400 MHz, DMSO-*d₆*): δ 8.57 (s, 1H), 7.95 (d, 1H, *J*= 7.87 Hz), 7.85 (br s, 1H), 7.80-7.73 (m, 3H), 7.69 (s, 1H), 7.55 (t, 1H, *J*= 7.69 Hz), 7.38 (d, 1H, *J*= 8.42 Hz), 7.19 (s, 1H), 7.12 (br s, 1H), 5.95-5.89 (m, 1H), 4.61-4.58 (m, 2H), 2.89 (s, 3H), 1.75 (d, 3H, *J*= 6.04 Hz); MS (ESI): 524 [M+H]⁺.

### Route 2: Step A - methyl 5-{6-[(methylsulfonyl)methyl]-1H-benzimidazol-1-yl}-3-({(1R)-1-[2-(trifluoromethyl)phenyl]ethyl}oxy)-2-thiophenecarboxylate

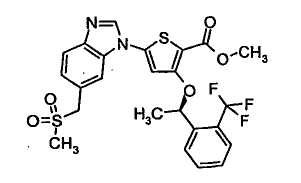

A mixture of methyl 5-[6-(chloromethyl)-1*H*-benzimidazol-1-yl]-3-({(1*R*)-1-[2-(trifluoromethyl)phenyl]ethyl}oxy)thiophene-2-carboxylate (4.53 g, 9.17 mmol), methanesulfonic acid sodium salt (2.81 g, 27.5 mmol) and ethanol (40.0 mL) was added to a high-pressure glass reaction flask. The flask was sealed, then heated to 85 °C for ^{∼}16 h. The flask was cooled to room temperature, opened, and the reaction mixture concentrated under vacuum, then purified by silica gel chromatography (5 to 35% ethyl acetate:hexanes) to give 4.54 g (92%) of the title compound as a light yellow solid. ¹H NMR (400 MHz, DMSO-*d₆*): δ 8.67 (s, 1H), 8.01 (d, 1H, *J*= 7.87 Hz), 7.82-7.70 (m, 4H), 7.53 (t, 1H, *J*= 7.69 Hz), 7.45 (s, 1H), 7.40 (d, 1H, *J*= 8.42 Hz), 5.95 (m, 1H), 4.63 (m, 2H), 3.83 (s, 3H), 2.90 (s, 3H), 1.65 (d, 3H, *J*= 6.204 Hz); MS (ESI): 539 [M+H]⁺.

### Step B - 5-{6-[(Methylsulfonyl)methyl]-1H-benzimidazol-1-yl}-3-({(1R)-1-[2-(trifluoromethyl)phenyl]ethyl}oxy)thiophene-2-carboxamide

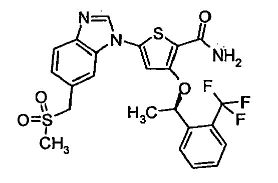

A mixture of methyl 5-{6-[(methylsulfonyl)methyl]-1*H*-benzimidazol-1-yl}-3-({(1*R*)-1-[2-(trifluoromethyl)phenyl]ethyl}oxy)-2-thiophenecarboxylate (4.53 g, 8.42 mmol) and 7 N ammonia in methanol (250.0 mL, 1.75 mol) was added to a high-pressure glass reaction flask. The flask was sealed, then heated to 85 °C for ^{∼}36 h. The flask was cooled to room temperature, opened, and the reaction mixture combined with a second batch of methyl 5-{6-[(methylsulfonyl)methyl]-1*H*-benzimidazol-1-yl}-3-({(1*R*)-1-[2-(trifluoromethyl)phenyl]ethyl}oxy)-2-thiophenecarboxylate (4.11 g, 7.63 mmol), which had also been treated with 7 N ammonia in methanol (200.0 mL, 1.40 mol) in a high-pressure glass reaction flask at 85 °C for ^{∼}36 hrs, then cooled to room temperature and opened. The combined reaction mixtures were concentrated under vacuum, then puriified by silica gel chromatography (0 to 5% methanol:dichloromethane, w/ 1% ammonium hydroxide), to give 7.47g (89%) of the title compound as an off-white solid. MS (ESI): 524 [M+H]⁺.

### Example 40: 5-{6-[(4-methylpiperazin-1-yl)methyl-1H-benzimidazol-1-yl}-3-{(1R)-1-[2-(trifluoromethyl)phenyl]ethoxy}thiophene-2-carboxamide

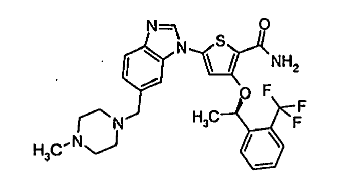

### Route 1:

### Step A - methy/ 3-({(1R)-1-[2-(trifluoromethy/)phenyl]ethyl}oxy)-5-(6-{[(trifluoromethyl)sulfonyl]oxy}-1H-benzimidazol-1-yl)-2-thiophenecarboxy/ate

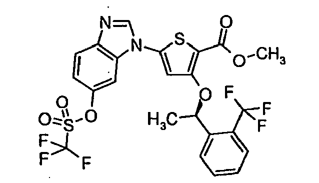

To a stirred, cooled (0 °C) solution of methyl 5-(6-hydroxy-1*H*-benzimidazol-1-yl)-3-({(1*R*)-1-(2-(trifluoromethyl)phenyl]ethyl}oxy)-2-thiophenecarboxylate (2.49 g, 5.38 mmol) and *N*-phenyltrifluoromethanesulfonimide (2.06 g, 5.76 mmol) in dichloromethane (30 mL) was added diisopropylethylamine (2.0 mL, 11.5 mmol). The reaction was allowed to warm to room temperature and stirred for 12 h. The reaction mixture was then concentrated under vacuum, and chromatographed on silica gel (5 to 40% ethyl acetate:hexanes) to give 3.12 g (98%) of the title compound as a light yellow solid. ¹H NMR (400 MHz, DMSO-*d₆*): δ 8.76 (s, 1H), 8.01-7.94 (m, 2H), 7.80-7.70 (m, 3H), 7.56-7.43 (m, 3H), 5.98 (q, 1H, *J*= 6.10 Hz), 3.84 (s, 3H), 1.65 (d, 3H, *J*= 6.22 Hz); MS (ESI): 595 [M+H]⁺.

### Step B - 3-{(1R)-1-[2-(trifluoromethyl)phenyl]ethoxy}-5-(6-vinyl-1H-benzimidazol-1-yl)thiophene-2-carboxylate

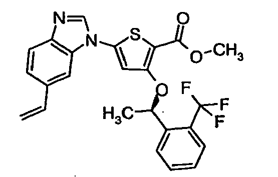

To a mixture of methyl 3-({(1*R*)-1-[2-(trifluoromethyl)phenyl]ethyl}oxy)-5-(6-{[(trifluoromethyl)sulfonyl]oxy}-1*H*-benzimidazol-1-yl)-2-thiophenecarboxylate (20.69 g, 34.83 mmol), potassium vinyltrifluoroborate (5.6 g, 42.10 mmol) and triethylamine (4.85 mL, 34.86 mmol), stirred at room temperature in *n-*propanol (175 mL) was added [1,1'-bis(diphenylphosphino)-ferrocene]dichloropalladium(II) dichloromethane complex (570 mg, 0.70 mmol). The mixture was then heated to reflux and stirred for 3 h, then cooled to room temperature, poured into water and extracted with ethyl acetate (3x). The combined organic layers were washed with brine, dried over magnesium sulfate, concentrated under vacuum and purified by silica gel chromatography (10 to 50% ethyl acetate:hexanes) to give 12.98 g (79%) of the title compound as a light yellow foam solid. ¹H NMR (400 MHz, DMSO-*d₆*): δ 8.59 (s, 1H), 7.98 (d, 1H, *J*= 7.87 Hz), 7.80-7.71 (m, 3H), 7.59 (s, 1H), 7.56-7.52 (m, 2H), 7.38 (s, 1H), 6.85 (dd, 1H, *J*= 10.90 and 17.67 Hz), 6.00 (q, 1H, *J*= 5.98 Hz), 5.86 (d, 1H, *J*= 17.58 Hz), 5.31 (d, 1H, *J*= 10.99 Hz), 3.83 (s, 3H), 1.65 (d, 3H, *J*= 6.04 Hz); MS (ESI): 473 [M+H]⁺.

### Step C - methyl 5-[6-(1,2-dihydroxyethyl-1H-benzimidazol-1-yl]-3-{(1R)-1-[2-(trifluoromethyl)phenyl]ethoxy}thiophene-2-carboxylate

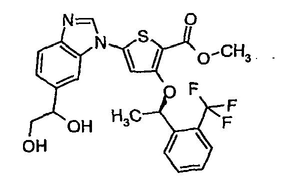

To a stirred solution of methyl 3-{(1*R*)-1-[2-(trifluoromethyl)phenyl]ethoxy}-5-(6-vinyl-1*H*-benzimidazol-1-yl)thiophene-2-carboxylate (12.97 g, 27.47 mmol) in 3:1 acetone:water (275 mL) was added 4-methylmorpholine N-oxide (3.87 g, 32.95 mmol) followed by a 2.5 wt% solution of osmium tetroxide in 2-methyl-2-propanol (6.88 mL, 0.55 mmol). The reaction was stirred at room temperature for 60 h, then quenched with saturated aqueous sodium sulfite. The mixture was extracted with ethyl acetate (3x). The combined organic layers were washed with brine, dried over magnesium sulfate, and concentrated under vacuum to give 14.3 g of the crude title compound as an off white foam. ¹H NMR (400 MHz, DMSO-*d₆*): δ 8.58 (d, 1H, *J*= 1.10 Hz), 7.99 (d, 1H, *J*= 8.06 Hz), 7.81-7.68 (m, 3H), 7.60-7.51 (m, 2H), 7.36-7.31 (m, 2H), 5.95 (q, 1H, *J*= 5.98 Hz), 5.35 (t, 1H, *J*= 3.57 Hz), 4.73-4.64 (m, 2H), 3.83 (s, 3H), 3.47-3.42 (m, 2H), 1.66 (d, 3H, *J*= 6.23 Hz); MS (ESI): 50[M+H]⁺.

### Step D - methyl 5-(6-formyl-1H-benzimidazol-1-yl)-3-{(1R)-1-[2-(trifluoromethyl)phenyl]ethoxy}thiophene-2-carboxylate

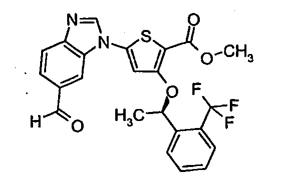

To a solution of methyl 5-[6-(1,2-dihydroxyethyl)-1*H*-benzimidazol-1-yl]-3-{(1*R*)-1-[2-(trifluoromethyl)phenyl]ethoxy}thiophene-2-carboxylate (13.9 g, 27.47 mmol) in 1:1:1 dichloromethane:water:methanol (180 mL) was added sodium periodate (8.81 g, 41.20 mmol). The resulting slurry was stirred for 45 min, then was poured through filter paper, washing the solid with dichloromethane. The filtrate was then poured into water and extracted with ethyl acetate (3x). The combined organic layers were washed with brine, dried over magnesium sulfate and concentrated under vacuum to give 13.09 g of the crude title compound as a light yellow solid. ¹H NMR (400 MHz, DMSO-*d₆*): δ 10.09 (s, 1H), 8.87 (s, 1H), 8.18 (s, 1H), 8.02-7.89 (m, 3H), 7.81-7.72 (m, 2H), 7.57-7.51 (m, 2H), 5.98 (q, 1H, *J*= 6.10 Hz), 3.84 (s, 3H), 1.66 (d, 3H, *J*= 6.22 Hz); MS (ESI): 475 [M+H]⁺.

### Step E - methyl 5-{6-[(4-methylpiperazin-1-yl)methyl]-1H-benzimidazol-1-yl}-3-{(1R)-1-[2-(trifluoromethyl)phenyl]ethoxy}thiophene-2-carboxylate

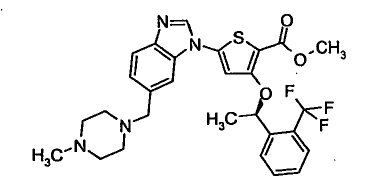

To a stirred solution of methyl 5-(6-formyl-1*H*-benzimidazol-1-yl)-3-{(1*R*)-1-[2-(trifluoromethyl)phenyl]ethoxy}thiophene-2-carboxylate (11.15 g, 23.52 mmol), *N*-methylpiperazine (4.32 mL, 47.01 mmol) and acetic acid (1.61 mL, 37.4 mmol) in dichloroethane (120 mL) was added sodium triacetoxyborohydride (7.50 g, 35.39 mmol). The reaction was stirred for 3.0 hrs, then aqueous 5% potassium carbonate was added. The mixture was then diluted with ethyl acetate and water. The aqueous layer was extracted with ethyl acetate (3x). The combined organic layers were washed with brine, dried over magnesium sulfate, and concentrated under vacuum to give 13.1 g (100%) of the crude title compound as a light yellow solid. ¹H NMR (400 MHz, DMSO-*d₆*): δ 8.59 (s, 1H), 7.98 (d, 1H, *J*= 7.87 Hz), 7.81-7.69 (m, 3H), 7.55 (t, 1H, *J*= 7.59 Hz), 7.47 (s, 1H), 7.35-7.29 (m, 2H), 5.97 (q, 1H, *J*= 6.16 Hz), 3.83 (s, 3H), 3.57 (s, 2H), 2.60-2.30 (m, 8H), 2.27 (s, 3H), 1.65 (d, 3H, *J*= 6.22 Hz); MS (ESI): 559 [M+H]⁺.

### Step F- 5-{6-[4-methylpiperazin-1-yl)methyl]-1H-benzimidazol-1-yl}-3-{(1R)-1-[2-(trifluoromethyl)pheny/]ethoxy}thiophene-2-carboxamide (Title Compound)

A mixture of methyl 5-{6-[(4-methylpiperazin-1-yl)methyl]-1*H*-benzimidazol-1-yl}-3-{(1*R*)-1-[2-(trifluoromethyl)phenyl]ethoxy}thiophene-2-carboxylate (15.82 g, 28.35 mmol) and 7 N ammonia in methanol (250 mL, 1.75 mol) was added to a high-pressure glass reaction flask. The flask was sealed, then heated to 80 °C for ^{∼}40 h. The flask was cooled to room temperature, opened, and the reaction mixture concentrated under vacuum, then puriified by silica gel chromatography, (2 to 8% methanol:dichloromethane w/ 1% ammonium hydroxide) to give 14.11 g (92%) of the title compound as a white foam solid. ¹H NMR (400 MHz, DMSO-*d₆*): δ 8.49 (s, 1H), 7.93 (d, 1H, *J*= 7.87 Hz), 7.86 (br s, 1H), 7.80-7.75 (m, 2H), 7.68 (d, 1H, *J*= 8.23 Hz), 7.56 (t, 1H, *J*= 7.68 Hz), 7.33 (s, 1H), 7.28 (d, 1H, *J*= 8.42 Hz), 7.15 (br s, 1H), 7.06 (s, 1H), 5.94 (q, 1H, *J*= 6.10 Hz), 3.52 (s, 2H), 2.45-2.20 (m, 8H), 2.13 (s, 3H), 1.74 (d, 3H, *J*= 6.22 Hz); MS (ESI): 544 [M+H]⁺.

### Route 2:

### Step A - Methyl 5-{6-[(4-methylpiperazin-1-y/)methy/]-1H-benzimidazol-1-y/}-3-({(1R)-1-[2-(trifluoromethyl)phenyl]ethyl}oxy)thiophene-2-carboxylate

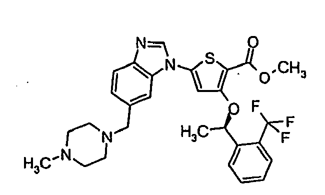

To a stirred, solution of methyl 5-[6-(chloromethyl)-1*H*-benzimidazol-1-yl]-3-({(1 R)-1-[2-(trifluoromethyl)phenyl]ethyl}oxy)thiophene-2-carboxylate (150 mg, 0.30 mmol) in dioxane (1.0 mL) was added *N*-methylpiperazine (50 µL, 0.45 mmol). The reaction was heated at 60 °C for 18 h, cooled to room temperature and concentrated under vacuum. The residue was dissolved in ethyl acetate and water. The aqueous layer was extracted with ethyl acetate. The combined organic layers were washed with brine, dried over sodium sulfate, concentrated under vacuum, and purified by silica gel chromatography (0 to 10% methanol:dichloromethane w/ 1% ammonium hydroxide), to give 134 mg (79%) of the title compound as a white solid. MS (ESI): 559 [M+H]⁺.

### Step B - 5-{6-[(4-methylpiperazin-1-yl)methyl]-1H-benzimidazol-1yl}-3-{(1R)-1-[2-(trifluoromethyl)phenyl]ethoxy}thiophene-2-carboxamide (Title Compound)

The title compound can be prepared by using a procedure analogous to **Example 40**, Route 1, Step F. MS (ESI): 544 [M+H]⁺.

### Route 3:

### Step A - 4-[bis(methyloxy)methyl]-2-bromo-1-nitrobenzene

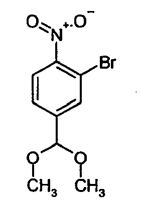

A solution of 3-bromo-4-nitrobenzaldehyde (7.97 g, 34.6 mmol), which was prepared in a manner analogous to the literature procedure (Katritzky, A.R.; Xie, L. Tetrahedron Letters 1996, 37, 347-350), trimethyl orthoformate (11.4 mL, 104 mmol), and p-toluenesulfonic acid hydrate (329 mg, 1.73 mmol) in methanol (69 mL) was refluxed for 3 h. The reaction was then quenched by addition of saturated aqueous ammonium hydroxide (1 mL) and concentrated onto silica gel. Purification by column chromatography (10 to 25% ethyl acetate:hexanes) provided 8.76 g (92%) of the title compound as an orange oil. ¹H NMR (300 MHz, CDCl₃): δ 7.89 (m, 2H), 7.59 (m, 1H), 5.47 (s, 1H), 3.38 (s, 6H).

### Step B - methyl 5-({5-[bis(methyloxy)methyl]-2-nitrophenyl}amino)-3-({(1R)-1-[2-(trifluoromethy/)phenyl]ethyl}oxy)-2-thiophenecarboxylate

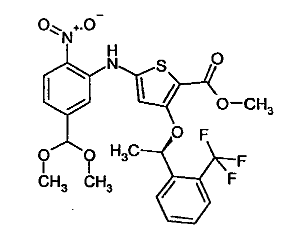

A solution of tris(dibenzylideneacetone) dipalladium(0) (117 mg, 0.127 mmol), 9,9-dimethyl-4,5-bis(diphenylphosphino)xanthene (162 mg, 0.280 mmol), methyl 5-amino-3-({(1*R*)-1-[2-(trifluoromethyl)phenyl]ethyl}oxy)-2-thiophenecarboxylate (2.31 g, 6.69 mmol), 4-[bis(methyloxy)methyl]-2-bromo-1-nitrobenzene (1.76 g, 6.37 mmol), and cesium carbonate (10.39 g, 31.89 mmol) in 1,4-dioxane (25 mL) was prepared in a round-bottom flask under nitrogen. The flask was evacuated and refilled three times with nitrogen and then stirred at 60 °C for 16 h. The reaction mixture was then diluted with tetrahydrofuran (100 mL) and concentrated onto silica gel. Purification by column chromatography (5 to 75% ethyl acetate:hexanes) provided 2.79 g (81%) of the title compound as a red foam. ¹H NMR (300 MHz, CDCl₃): δ 9.63 (br s, 1H), 8.21 (m, 1H), 7.94 (m, 1H), 7.62 (m, 2H), 7.48 (s, 1H), 7.40 (m, 1H), 7.02 (m, 1H), 6.47 (s, 1H), 5.73 (q, 1H, *J*= 6.2 Hz), 3.88 (s, 3H), 3.34 (s, 1H), 3.31 (s, 3H), 3.28 (s, 3H), 1.72 (d, 3H, *J*= 6.2 Hz); MS (ESI): 541 [M+H]⁺.

### Step C - methyl 5-{6[bis(methyloxy)methyl]-1H-benzimidazol-1-yl}-3-({(1R)-1-[2-(trifluoromethyl)phenyl]ethyl}oxy)-2-thiophenecarboxylate

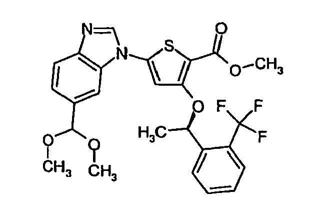

To a solution of methyl 5-({5-[bis(methyloxy)methyl]-2-nitrophenyl}amino)-3-({(1R-1-[2-(trifluoromethyl)phenyl]ethyl}oxy)-2-thiophenecarboxylate (2.71 g, 5.01 mmol) in trimethyl orthoformate (50 mL) in a Fischer-Porter bottle was added pyridinium p-toluenesulfonate (126 mg, 0.501 mmol) and sulfided platinum on carbon (5 wt% Pt, 977 mg, 0.250 mmol Pt). The mixture was hydrogenated on a Fischer-Porter hydrogenation apparatus at 50 psi of hydrogen until the uptake of hydrogen had ceased (17 h). The reaction mixture was filtered through a sintered glass filter to remove the catalyst, washing with dichloromethane (75 mL). The eluant was concentrated to afford 2.61 g (100%) of the crude title compound as an orange oil, which was carried on to the next step without purification. MS (ESI): 521 [M+H]⁺.

### Step D-methy/5-(6-formyl-1H-benzimidazol-1-yl)-3-({(1R)-1-[2-(trifluoromethyl)phenyl]ethyl}oxy)-2-thiophenecarboxylate

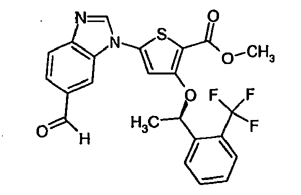

To a solution of crude methyl 5-{6-[bis(methyloxy)methyl]-1*H*-benzimidazol-1-yl}-3-({(1*R*)-1-(2-(trifluoromethyl)phenyl]ethyl}oxy)-2-thiophenecarboxylate (2.61 g, 5.01 mmol) (from *Step C,* above) in acetone (20 mL) and water (5 mL) was added pyridinium p-toluenesulfonate (126 mg, 0.501 mmol). The reaction stirred for 2 h at room temperature and was then poured into water (30 mL) and saturated aqueous sodium bicarbonate (30 mL). The mixture was extracted with dichloromethane (2 x 30 mL). The combined organic fractions were dried over sodium sulfate, filtered, and concentrated onto silica gel. Purification by column chromatography (30 to 100% ethyl acetate:hexanes) provided 1.37 g (58%, 2 steps) of the title compound as a light yellow solid. MS (ESI): 475 [M+H]⁺.

### Step E-methyl5-{6-[(4-methyl-1-piperazinyl)methyl]-1H-benzimidazol-1-yl}-3-({(1R)-1-[2-(trifluoromethyl)phenyl]ethyl}oxy)-2-thiophenecarboxylate

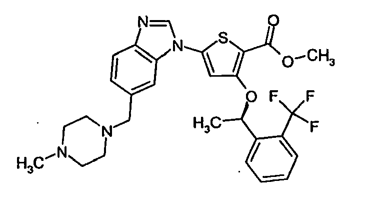

The title compound can be prepared by using a procedure analogous to **Example 40**, Route 1, Step E.

### Step F - 5-{6-[(4-methylpiperazin-1-yl)methyl]-1H-benzimidazol-1-yl}-3-{(1R)-1-[2-(trifluoromethyl)phenyl]ethoxy}thiophene-2-carboxamide (Title Compound)

The title compound can be prepared by using a procedure analogous to **Example 40**, Route 1, Step F.

### Example 41: 5-[6-(Piperazin-1-ylmethyl)-1H-benzimidazol-1-yl]-3-({(1R)-1-[2-(trifluoromethyl)phenyl]ethyl}oxy)thiophene-2-carboxamide

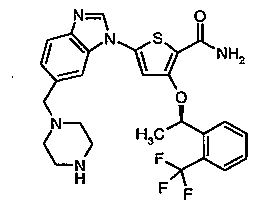

### Step A - Methy/5-[6-(piperazin-1-y/methyl)-1H-benzimidazo/-1-yl]-3-({(1R)-1-[2-(trifluoromethyl)phenyl]ethyl}oxy)thiophene-2-carboxy/ate

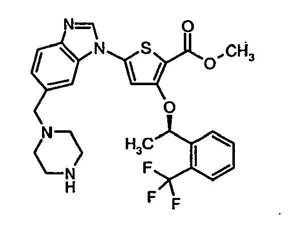

The title compound was prepared from methyl 5-[6-(chloromethyl)-1H-benzimidazol-1-yl]-3-({(1R)-1-[2-(trifluoromethyl)phenyl]ethyl}oxy)thiophene-2-carboxylate and piperazine by a procedure analogous to Example 40, Route 2, Step A. MS (ESI): 545 [M+H]+.

### Step B - 5-[6-(piperazin-1-y/methy/)-1H-benzimidazo/-1-yl]-3-({(1R)-1-[2-(trifluoromethyl)phenyl]ethyl}oxy)thiophene-2-carboxamide (Title Compound)

The title compound was prepared from methyl 5-[6-(piperazin-1-ylmethyl)-1H-benzimidazol-1-yl]-3-({(1R)-1-[2-(trifluoromethyl)phenyl]ethyl}oxy)thiophene-2-carboxylate by a procedure analogous to Example 38, Step A. 1H NMR (400 MHz, DMSO-d6): δ 8.48 (s, 1H), 7.93 (d, 1H, J = 7.87 Hz), 7.85 (s, 1H), 7.80-7.75 (m, 2H), 7.68 (d, 1H, J = 8.24 Hz), 7.56 (t, 1H, J = 7.69 Hz), 7.33 (s, 1H), 7.28 (d, 1H, J = 8.42 Hz), 7.15 (s, 1H), 7.05 (s, 1H), 5.96-5.91 (m, 1H), 3.49 (s, 2H), 2.67-2.63 (m, 4H), 2.29-2.24 (m, 4H), 2.15-2.05 (m, 1H), 1.75 (d, 3H, J = 6.23 Hz); MS (ESI): 530 [M+H]+.

### Example 42: 3-{[(1R)-1-(2-Chlorophenyl)ethyl]oxyl}-5-{6-[(methylthio)methyl]-1H-benzimidazol-1-yl}thiophene-2-carboxamide

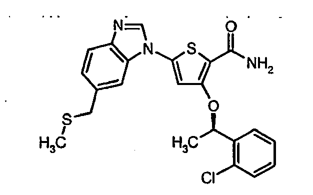

### Step A - Methy/ 3-{[(1R)-1-(2-ch/orophenyl)ethyl]oxy}-5-{6-[(methy/thio)methyl]-1H-benzimidazo/-1-yl}thiophene-2-carboxylate

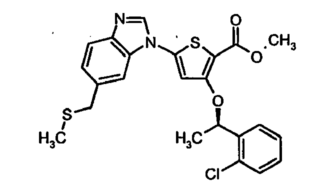

The title compound was prepared from methyl 5-[6-(chloromethyl)-1H-benzimidazol-1-yl]-3-{[(1R)-1-(2-chlorophenyl)ethyl]oxy}thiophene-2-carboxylate by a procedure analogous to Example 39, Route 1, Step A. MS (ESI): 473 [M+H]+.

### Step B - 3-{[(1R)-1-(2-Chlorophenyl)ethyl]oxy}-5-{6[(methylthio)methyl]-1H-benzimidazol-1-yl}thiophene-2-carboxamide (Title Compound)

The title compound was prepared from methyl 3-{[(1R)-1-(2-chlorophenyl)ethyl]oxy}-5-{6-[(methylthio)methyl]-1H-benzimidazol-1-yl}thiophene-2-carboxylate by a procedure analogous to Example 38, Step A. 1H NMR (400 MHz, DMSO-d6): δ 8.53 (s, 1H), 7.83 (s, 1H), 7.71-7.66 (m, 2H), 7.49 (d, 1H, J = 7.87 Hz), 7.45-7.35 (m, 3H), 7.29 (d, 1H, J = 8.42 Hz), 7.16-7.11 (m, 2H), 6.01-5.95 (m,1H), 3.82 (s, 2H), 1.94 (s, 3H), 1.73 (d, 3H, J = 6.41 Hz); MS (ESI): 458 [M+H]+.

### Example 43: 3-{[(1R)-1-(2-Chlorophenyl)ethyl]oxy}-5-[6-(piperazin-1-ylmethyl)-1H-benzimidazol-1-yl]thiophene-2-carboxamide

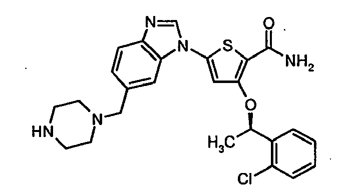

### Step A - Methyl 3-{[(1R)-1-(2-chropheny/)ethyl]oxy}-5-[6-(piperazin-1-ylmethyl)-1H-benzimidazo/-1-yl]thiophene-2-carboxylate

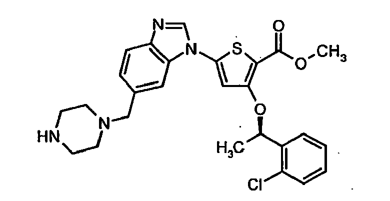

The title compound was prepared from methyl 5-[6-(chloromethyl)-1H-benzimidazol-1-yl]-3-{[(1R)-1-(2-chlorophenyl)ethyl]oxy}thiophene-2-carboxylate and piperazine by a procedure analogous to **Example 40, Route 2,** Step A. MS (ESI): 511 [M+H]⁺.

### Step B - 3-{[(1R)-1-(2-Chlorophenyl)ethyl]oxy}-5-[6-(piperazin-1-ylmethyl)-1H-benzimidazol-1-y/]thiophene-2-carboxamide (Title Compound)

The title compound was prepared from methyl 3-{[(1R)-1-(2-chlorophenyl)ethyl]oxy}-5-[6-(piperazin-1-ylmethyl)-1H-benzimidazol-1-yl]thiophene-2-carboxylate by a procedure analogous to Example 38, Step A. ¹H NMR (400 MHz, DMSO-d₆): δ 8.52 (s, 1H), 7.83 (s, 1H), 7.71-7.67 (m, 2H), 7.52-7.48 (m, 1H), 7.46-7.35 (m, 3H), 6.00-5.95 (m, 1H), 3.52 (s, 2H), 2.68-2.63 (m, 4H), 2.31-2.25 (m, 4H), 2.15-2.05 (m, 1H), 1.73 (d, 3H, *J*= 6.23 Hz); MS (ESI): 496 [M+H]⁺.

### Example 44: 3-{[(1R)-1-(2-Chlorophenyl)ethyl]oxy}-5-{6-[(4-methylpiperazin-1-yl)methyl]-1H-benzimidazol-1-yl}thiophene-2-carboxamide

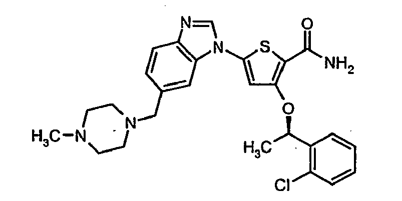

### Step A - Methy/3-{[(1R)-1-(2-ch/orophenyl)ethyl]oxy}-5-{6-[(4-methylpiperazin-1-yl)methyl]-1H-benzimidazo/-1-yl}thiophene-2-carboxylate

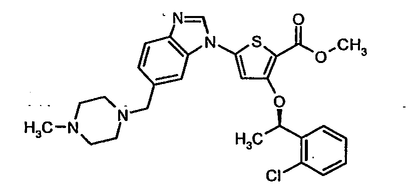

The title compound was prepared from methyl 5-[6-(chloromethyl)-1H-benzimidazol-1-yl]-3-{[(1R)-1-(2-chlorophenyl)ethyl]oxy}thiophene-2-carboxylate and N-methylpiperazine by a procedure analogous to **Example 40**, Route 2, Step A. MS (ESI): 525 [M+H]+.

### Step B - 3-{[(1R)-1-(2-Chlorophenyl)ethyl]oxy}-5-{6-[(4-methylpiperazin-1-yl)methyl]-1H-benzimidazol-1-yl}thiophene-2-carboxamide (Title Compound)

The title compound was prepared from methyl 3-{[(1R)-1-(2-chlorophenyl)ethyl]oxy}-5-{6-[(4-methylpiperazin-1-yl)methyl]-1H-benzimidazol-1-yl}thiophene-2-carboxylate by a procedure analogous to **Example 38**, Step A. 1H NMR (400 MHz, DMSO-d6): δ 8.52 (s, 1H), 7.83 (s, 1H), 7.71-7.67 (m, 2H), 7.51-7.35 (m, 4H), 7.28 (d, 1H, J = 8.24 Hz), 7.16-7.11 (m, 2H), 6.00-5.95 (m, 1H), 3.55 (s, 2H), 2.42-2.22 (m, 8H), 2.13 (s, 3H), 1.72 (d, 3H, J = 6.23 Hz); MS (ESI): 510 [M+H]+.

### Example 45: 3-{[(1R)-1-(2-chlorophenyl)ethyl]oxy}-5-{6-[(methylsulfonyl)methyl]-1H-benzimidazol-1-yl}thiophene-2-carboxamide

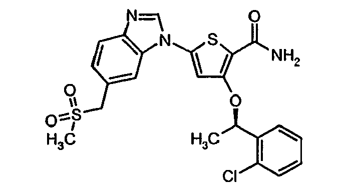

The title compound was prepared from 3-{[(1R)-1-(2-chlorophenyl)ethyl]oxy}-5-{6-[(methylthio)methyl]-1H-benzimidazol-1-yl}thiophene-2-carboxamide by a procedure analogous to **Example 39**, **Route 1,** Step C. ¹H NMR (400 MHz, DMSO-*d₆*): δ 8.61 (s, 1H), 7.84 (s, 1H), 7.79 (d, 1H, *J*= 8.24 Hz), 7.73 (s, 1H), 7.70-7.67 (m, 1H), 7.49-7.47 (m, 1H), 7.44-7.33 (m, 3H), 7.26 (s, 1H), 7.12 (s, 1H), 5.97-5.93 (m, 1H), 4.64-4.60 (m, 2H), 2.90 (s, 3H), 1.73 (d, 3H, *J* = 6.41 Hz); MS (ESI): 490 [M+H]⁺.

### Example 46: 5-[6-({[2-(Dimethylamino)ethyl]sulfonyl}methyl)-1H-benzimidazol-1-yl]-3-({(1R)-1-[2-(trifluoromethyl)phenyl]ethyl}oxy)thiophene-2-carboxamide

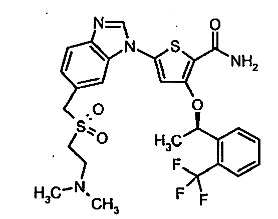

### Step A - Methyl 5-(6-{[(2-hydroxyethyl)thio]methyl}-1H-benzimidazol-1-yl)-3-({(1R)-1-[2-(trifluoromethyl)phenyl]ethyl}oxy)thiophene-2-carboxylate

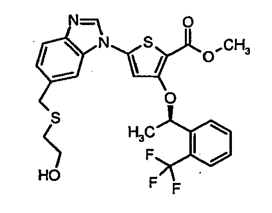

To a stirred solution of methyl 5-[6-(chloromethyl)-1H-benzimidazol-1-yl]-3-({(1R)-1-[2-(trifluoromethyl)phenyl]ethyl}oxy)thiophene-2-carboxylate (494 mg, 1.0 mmol) in N,N-dimethylformamide (4.0 mL) was added 2-mercaptoethanol (90 µL, 1.3 mmol) and potassium carbonate (240 mg, 1.7 mmol). The reaction was heated at 60 °C for 2 h, then cooled to room temperature. The reaction was diluted with ethyl acetate and washed with water (5x), brine, dried over magnesium sulfate, and concentrated under vacuum to give 429 mg (80%) of the title compound as a light yellow foam solid. MS (ESI): 537 [M+H]⁺.

### Step B - 5-(6-{[(2-Hydroxyethyl)thio]methyl}-1H-benzimidazol-1-yl)-3-({(1R)-1-[2-(trifluoromethyl)phenyl]ethyl}oxy)thiophene-2-carboxamide

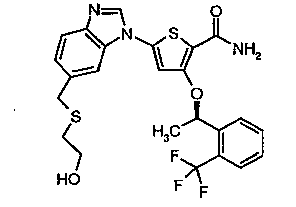

The title compound was prepared from methyl 5-(6-{[(2-hydroxyethyl)thio]methyl}-1H-benzimidazol-1-yl)-3-({(1R)-1-[2-(trifluoromethyl)phenyl]ethyl}oxy)thiophene-2-carboxylate by a procedure analogous to **Example 38**, Step A. MS (ESI): 522 [M+H]⁺.

### Step C - 5-(6-{[(2-Hydroxyethyl)sulfonyl]methyl}-1H-benzimidazol-1-yl)-3-({(1R)-1-[2-(trifluoromethyl)phenyl]ethyl}oxy)thiophene-2-carboxamide

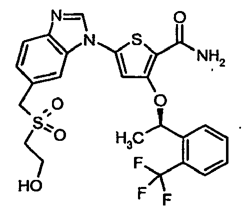

The title compound was prepared from 5-(6-{[(2-hydroxyethyl)thio]methyl}-1H-benzimidazol-1-yl)-3-({(1R)-1-[2-(trifluoromethyl)phenyl]ethyl}oxy)thiophene-2-carboxamide by a procedure analogous to **Example 39, Route 1**, Step C. MS (ESI): 554 [M+H]⁺.

### Step D - 3-({(1R)-1-[2-(Trifluoromethy/)phenyl]ethyl}oxy)-5-{6-[(vinylsulfonyl)methyl]-1H-benzimidazol-1-yl}thiophene-2-carboxamide

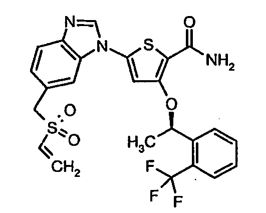

To a stirred, cooled (0 °C) solution of 5-(6-{[(2-hydroxyethyl)sulfonyl]methyl}-1H-benzimidazol-1-yl)-3-({(1R)-1-[2-(trifluoromethyl)phenyl]ethyl}-oxy)thiophene-2-carboxamide (310 mg, 0.56 mmol) and methanesulfonyl chloride (50 µL, 0.65 mmol) in dichloromethane (3.0 mL) was added triethylamine (345 µL, 2.46 mmol). The reaction was stirred 15 min at 0 °C, then 15 min at room temperature. The reaction was then diluted with ethyl acetate and water. The aqueous layer was extracted with ethyl acetate. The combined organic layers were washed with brine, dried over magnesium sulfate, concentrated under vacuum and purified by silica gel chromatography (0 to 7% methanol:dichloromethane, with 1% ammonium hydroxide) to give 274 mg (92%) of the title compound as a light tan solid. MS (ESI): 536 [M+H]⁺.

### Step E - 5-[6-({[2-(dimethylamino)ethyl]sulfonyl}methyl)-1H-benzimidazol-1-yl]-3-({(1R)-1-[2-(trifluoromethyl)phenyl]ethyl}oxy)thiophene-2-carboxamide (Title Compound)

A mixture of 3-({(1*R*)-1-[2-(trifluoromethyl)phenyl]ethyl}oxy)-5-{6-[(vinyisulfonyl)methyl]-1H-benzimidazol-1-yl}thiophene-2-carboxamide (141 mg, 0.26 mmol) and 2 N dimethylamine in tetrahydrofuran (14 mL, 28.0 mmol) in a sealed high pressure glass reaction vessel was heated at 75 °C for ~2 h. The reaction was then cooled to room temperature, concentrated under vacuum and purified by silica gel chromatography (0 to 7% methanol:dichloromethane, with 1% ammonium hydroxide) to give 142 mg (93%) of the title compound as a white solid. ¹H NMR (400 MHz, DMSO-d₆): δ 8.57 (s, 1H), 7.95 (d, 1H, *J*=7.87 Hz), 7.86 (s, 1H), 7.80-7.73 (m, 3H), 7.67 (s, 1H), 7.5 (t, 1H, *J*= 7.60 Hz), 7.36 (d, 1H, *J*= 8.42 Hz), 7.19 (s, 1H), 7.12 (s, 1H), 5.94-5.88 (m, 1H), 4.65-4.61 (m, 2H), 3.19 (t, 2H, *J*= 7.05 Hz), 2.63 (t, 2H, *J*= 7.05 Hz), 2.13 (s, 6H),1.75 (d, 3H, *J*= 6.04 Hz); MS (ESI): 581 [M+H]⁺.

### Example 47: 3-{[(1R)-1-(2-Chlorophenyl)ethyl]oxy}-5-{6-[difluoro(piperidin-4-ylsulfonyl)methyl]-1H-benzimidazol-1-yl}thiophene-2-carboxamide

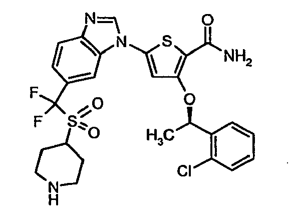

### Step A - Tert-buty/ 4-{[{1-[4-{[(1R)-1-(2-chlorophenyl)ethyl]oxy}-5-(methoxycarbonyl)thien-2-yl]-1H-benzimidazol-6-yl}(difluoro)methyl]sulfonyl}piperidine-1-carboxylate

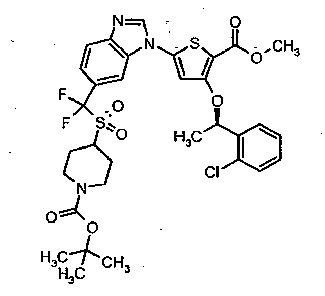

To a stirred, cooled (-78 °C) solution of tert-butyl 4-[({1-[4-{[(1R)-1-(2-chlorophenyl)ethyl]oxy}-5-(methoxycarbonyl)thien-2-yl]-1H-benzimidazol-6-yl}methyl)sulfonyl]piperidine-1-carboxylate (913 mg, 1.35 mmol) and N-fluorobenzenesulfonimide (1.28 g, 4.06 mmol) in tetrahydrofuran (30 mL) was added sodium bis(trimethylsilyl)amide (1.0 M in tetrahydrofuran, 3.0 mL, 3.0 mmol) dropwise over 1 h. The reaction was stirred for 5 h at -78 °C, then 1 h at room temperature. The reaction was diluted with tetrahydrofuran and aqueous saturated ammonium chloride solution. The aqueous layer was extracted with dichloromethane and ethyl acetate. The combined organic layers were dried over magnesium sulfate, concentrated under vacuum and purified by silica gel chromatography (0 to 50% ethyl acetate:hexanes) to give to give 163 mg (17%) of the title compound as a tan solid. MS (ESI): 710 [M+H]⁺.

### Step B - Methyl 3-{[(1R)-1-(2-chlorophenyl)ethyl]oxy}-5-{6-[difluoro(piperidin-4-ylsulfonyl)methyl]-1H-benzimidazol-1-yl}thiophene-2-carboxylate

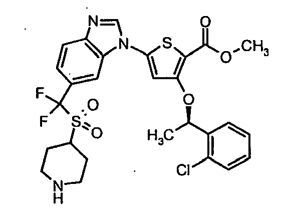

A mixture of tert-butyl 4-{[{1-[4-{[(1R)-1-(2-chlorophenyl)ethyl]oxy}-5-(methoxycarbonyl)thien-2-yl]-1H-benzimidazol-6-yl}(difluoro)-methyl]-sulfonyl}piperidine-1-carboxylate (160 mg, 0.22 mmol) and 10% trifluoroacetic acid in dichloromethane (15 mL) was stirred at room temperature for 45 minutes, then was concentrated under vacuum and purified by silica gel chromatography (0 to 10% methanol:dichloromethane, with 1% ammonium hydroxide) to give 93 mg (67%) of the title compound as a tan solid. MS (ESI): 610 [M+H]⁺.

### Step C - 3-{[(1R)-1-(2-chlorophenyl)ethyl]oxy}-5-{6-[difluoro(piperidin-4-ylsulfonyl)methyl]-1H-benzimidazol-1-yl}thiophene-2-carboxamide (Title Compound)

The title compound was prepared from methyl 3-{[(1R)-1-(2-chlorophenyl)ethyl]oxy}-5-{6-[difluoro(piperidin-4-ylsulfonyl)methyl]-1H-benzimidazol-1-yl}thiophene-2-carboxylate by a procedure analogous to **Example 38**, Step A. ¹H NMR (400 MHz, DMSO-d₆): δ 8.80 (s, 1H), 8.00 (d, 1H, *J=* 8.42), 7.88 (s, 1H), 7.73 (s, 1H), 7.69 (dd, 1H, *J* = 1.47, 7.69 Hz), 7.58 (dd, 1H, *J* = 1.47, 8.61 Hz), 7.48-7.33 (m, 3H), 7.27 (s, 1H), 7.15 (s, 1H), 5.98-5.93 (m, 1H), 3.87-3.79 (m, 1H), 3.06-3.00 (m, 2H), 2.60-2.53 (m, 2H), 2.25-2.15 (m, 1H), 2.01-1.94 (m, 2H), 1.72 (d, 3H, *J*= 6.41 Hz), 1.69-1.58 (m, 2H); MS (ESI): 595 [M+H]⁺.

### Example 48: 5-[6-(2-Amino-2-iminoethyl)-1H-benzimidazol-1-yl]-3-({(1R)-1-[2-(trifluoromethyl)phenyl]ethyl}oxy)thiophene-2-carboxamide

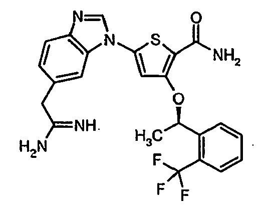

### Step A - Methy/ 5-[6-(cyanomethy/)-1H-benzimidazo/-1-yl]-3-({(1R)-1-[2-(trifluoromethyl)phenyl]ethyl}oxy)thiophene-2-carboxylate

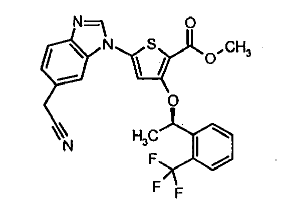

A mixture of methyl 5-[6-(chloromethyl)-1H-benzimidazol-1-yl]-3-({(1R)-1-[2-(trifluoromethyl)phenyl]ethyl}oxy)thiophene-2-carboxylate (848 mg, 1.71 mmol) and sodium cyanide (100 mg, 2.04 mmol) in N,N-dimethylformamide (10 mL) was stirred at 55 °C for 2 h, then at room temperature for 18 h. The reaction was diluted with ethyl acetate and washed with water, brine, dried over magnesium sulfate, concentrated under vacuum and purified by silica gel chromatography (20 to 60% ethyl acetate:hexanes) to give to give 720 mg (86%) of the title compound as a white solid. MS (ESI): 486 [M+H]⁺.

### Step B - 5-[6-(Cyanomethyl)-1H-benzimidazol-1-yl]-3-({(1R)-1-[2-(trifluoromethyl)phenyl]ethyl}oxy)thiophene-2-carboxamide

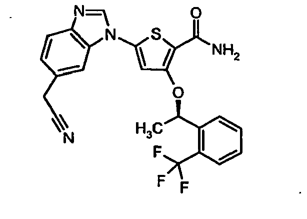

The title compound was prepared from methyl 5-[6-(cyanomethyl)-1H-benzimidazol-1-yl]-3-({(1R)-1-[2-(trifluoromethyl)phenyl]ethyl}oxy)thiophene-2-carboxylate by a procedure analogous to **Example 38**, Step A. MS (ESI): 471 [M+H]⁺.

### Step C - 5-{6-[(2Z)-2-Amino-2-(hydroxyimino)ethyl]-1H-benzimidazol-1-yl}-3-({(1R)-1-[2-(trifluoromethyl)phenyl]ethyl}oxy)thiophene-2-carboxamide

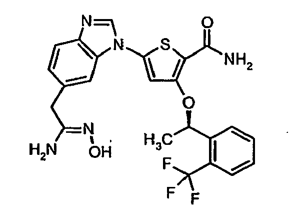

To a mixture of 5-[6-(cyanomethyl)-1H-benzimidazol-1-yl]-3-({(1R)-1-[2-(trifluoromethyl)phenyl]ethyl}oxy)thiophene-2-carboxamide (250 mg, 0.53 mmol) and hydroxylamine hydrochloride (110 mg, 1.58 mmol) in ethanol (3 mL) was added sodium carbonate (168 mg, 1.58 mmol) dissolved in water (1 mL). The reaction was then heated at 65°C for 42 hrs, cooled to room temperature, diluted with water and neutalized to pH 7 by addition of aqueous 1 N HCl solution. The mixture was then extracted with ethyl acetate. The combined organic layers were washed with brine, dried over sodium sulfate, concentrated under vacuum and purified by silica gel chromatography (0 to 8% methanol:dichloromethane, with 1 % ammonium hydroxide) to give 251 mg (94%) of the title compound as a white solid. MS (ESI): 504 [M+H]⁺.

### Step D - 5-(6-{(2Z)-2-[(acetyloxy)imino]-2-aminoethyl}-1H-benzimidazol-1-yl)-3-({(1R)-1-[2-(trifluoromethyl)phenyl]ethyl}oxy)thiophene-2-carboxamide

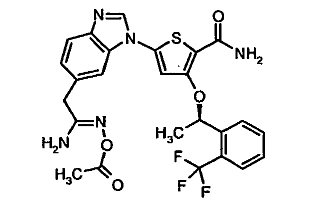

To a stirred solution of 5-{6-[(2Z)-2-amino-2-(hydroxyimino)ethyl]-1H-benzimidazol-1-yl}-3-({(1R)-1-[2-(trifluoromethyl)phenyl]ethyl}oxy)thiophene-2-carboxamide (179 mg, 0.35 mmol) in acetic acid (3.5 mL) was added acetic anhydride (50 µL, 0.53 mmol). The reaction was stirred for 4 h at room temperature, then diluted with water and neutalized to pH 7 by addition of aqueous saturated sodium bicarbonate solution. The reaction mixture was then extracted with ethyl acetate. The combined organic layers were washed with brine, dried over magnesium sulfate, concentrated under vacuum to give 140 mg (72%) of the title compound as a white solid. MS (ESI): 546 [M+H]⁺.

### Step E - 5-[6-(2-amino-2-iminoethyl)-1H-benzimidazol-1-yl]-3-({(1R)-1-[2-(trifluoromethyl)phenyl]ethyl}oxy)thiophene-2-carboxamide (Title Compound)

To a solution of 5-(6-{(2Z)-2-[(acetyloxy)imino]-2-aminoethyl}-1H-benzimidazol-1-yl)-3-({(1R)-1-[2-(trifluoromethyl)phenyl]ethyl}oxy)thiophene-2-carboxamide (95 mg, 0.17 mmol) in ethanol (4.0 mL) and acetic acid (3.0 mL) was added palladium (10 wt% on carbon, 20 mg, 0.018 mmol). The reaction vessel was purged with vacuum and nitrogen gas (3x), then purged with vacuum and hydrogen gas (3x). Then hydrogen gas was applied at 50 psi for 2 h. The reaction was then filtered through Celite, washing with ethyl acetate and methanol. The filtrate was concentrated under vacuum and the title compound recrystallized from water to give 51 mg (61 %) of the title compound as a white solid. ¹H NMR (400 MHz, DMSO-d₆): δ 8.49 (s, 1H), 7.94 (d, 1H, *J*= 7.87), 7.85 (s, 1H), 7.80-7.75 (m, 2H), 7.70 (d, 1H, *J*= 8.42 Hz), 7.58-7.51 (m, 2H), 7.29 (d, 1H, *J*= 8.42Hz), 7.16-7.10 (m, 5H), 5.96-5.90 (m, 1H), 3.53 (s, 2H), 1.75 (d, 3H, *J*= 6.23 Hz); MS (ESI): 488 [M+H]⁺.

### Intermediate Example 8: 1,1-dimethylethyl 4-[1-(trimethylstannanyl)ethenyl]-1-piperidinecarboxylate

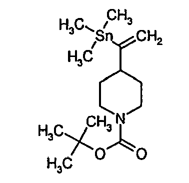

### Step A - 1, 1-Dimethy/ethy/ 4-(1-{[(trifluoromethy/)su/fony/]oxy}etheny/)-1-piperidinecarboxylate

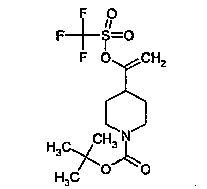

To a cooled (-78 °C) solution of sodium bis(trimethylsilyl)amide (1.0 M in tetrahydrofuran, 6.1 mL, 6.1 mmol) in tetrahydrofuran (11 mL) was added a solution of 1,1-dimethylethyl 4-acetyl-1-piperidinecarboxylate (1.25 g, 5.50 mmol) (which may be prepared according to the literature procedure: Ivobe, A.; Uchida, M.; Kamata, K.; Hotei, Y.; Kusama, H.; Harada, H.; Chemical and Pharmaceutical Bulletin 2001, 49, 822-829) in tetrahydrofuran (11 mL) dropwise by cannula. After stirring for 30 min at -78 °C, a solution of *N-*phenyltrifluoromethanesulfonimide (2.10 g, 5.89 mmol) in tetrahydrofuran (11 mL) was added to the reaction mixture by cannula. The reaction was warmed to 0 °C and stirred 3 h at that temperature. The reaction was then filtered directly through a pad of neutral alumina, eluting with 10% ethyl acetate:hexanes (200 mL). The eluant was concentrated to afford 1.98 g (98%) of the title compound. ¹H NMR (400 MHz, CDCl3): δ 5.13 (d, 1H, *J*= 5.1 Hz), 4.92 (dd, 1H, *J*= 1.1, 4.0 Hz), 1.67 (m, 2H), 2.71 (m, 2H), 2.35 (m, 1H), 1.89 (d, 2H, *J*= 13.4 Hz), 1.45 (s, 9H), 1.45-1.35 (m, 2H).

### Step B - 1,1-Dimethylethyl 4-[1-(trimethylstannanyl)ethenyl]-1-piperidinecarboxylate

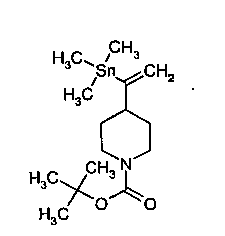

To a solution of hexamethylditin (1.77 g, 5.40 mmol), lithium chloride (1.55 g, 36.5 mmol), and 1,1-dimethylethyl 4-(1-{[(trifluoromethyl)sulfonyl]oxy}-ethenyl)-1-piperidinecarboxylate (1.94 g, 5.40 mmol) was added tetrakis(triphenyl-hosphine)palladium (0) (62 mg, 0.054 mmol). The reaction was purged with a stream of nitrogen for 30 min, then heated to 60 °C for 22 h. The reaction mixture was then cooled to room temperature and filtered through Celite to remove insoluble material, eluting with diethyl ether. The filtered solution was washed with water (100 mL) and brine (100 mL), then dried over sodium sulfate, filtered, and concentrated. Purification by column chromatography on Florisil (0 to 100% diethyl ether:petroleum ether) afforded 1.01 g (50%) of the title compound. ¹H NMR (300 MHz, CDCl₃): δ 5.68 (dd, 1H, *J*= 1.3, 2.3 Hz), 5.15 (dd, 1H, *J=* 0.9, 2.2 Hz), 1.89 (m, 2H), 2.70 (t, *2H, J* = 12.6 Hz), 2.29 (tt, 1H, *J*= 3.7, 11.8 Hz), 1.67-1.56 (m, 2H), 1.46 (s, 9H), 1.38-1.24 (m, 2H), 0.15 (s, 9 H).

### Example 49: 3-{[(1R)-1-(2-Chlorophenyl)ethyl]oxy}-5-{6-[1-(4-piperidinyl)ethenyl]-1H-benzimidazol-1-yl}-2-thiophenecarboxamide trifluoroacetic acid salt

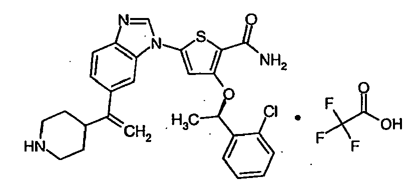

### Step A - Methy/ 3-{[(1R)-1-(2-ch/orophenyl)ethyl]oxy}-5-(6-{[(trifluoromethyl)sulfonyl]oxy}-1H-benzimidazol-1-yl)-2-thiophenecarboxylate

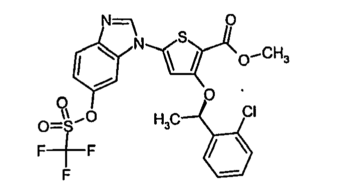

To a cooled (0 °C) solution of methyl 3-{[(1*R*)-1-(2-chlorophenyl)ethyl]oxy}-5-(6-hydroxy-1H-benzimidazol-1-yl)-2-thiophenecarboxylate (250 mg, 0.58 mmol) in dichloromethane (4.1 mL) and triethylamine (1.6 mL) was added *N-*phenyltrifluoromethanesulfonimide (375 mg, 1.05 mmol). The reaction was warmed to room temperature and stirred for 5 h. The reaction mixture wasthen diluted with dichloromethane (25 mL) and extracted with 1 N sodium hydroxide (25 mL) and brine (25 mL). The organic layer was dried over sodium sulfate, decanted, and concentrated onto silica gel. Purification by flash column chromatography (0 to 50% ethyl acetate:hexanes) provided 308 mg (95%) of the title compound. MS (APCI): 560.85 [M+H]⁺.

### Step B - 1,1-Dimethylethyl 4-[1-(1-{4-{[(1R)-1-(2-chlorophenyl)ethyl]oxy}-5-[(methy/oxy)carbonyl]-2-thienyl}-1H-benzimidazol-6-yl)ethenyl]-1-piperidinecarboxylate

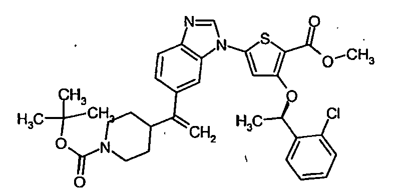

To a solution of methyl 3-{[(1*R*)-1-(2-chlorophenyl)ethyl]oxy}-5-(6-{[(trifluoromethyl)sulfonyl]oxy}-1H-benzimidazol-1-yl)-2-thiophenecarboxylate (890 mg, 1.59 mmol) and 1,1-dimethylethyl 4-[1-(trimethylstannanyl)ethenyl]-1-piperidinecarboxylate (653 mg, 1.74 mmol) in *N*,*N*-dimethylformamide (4.0 mL) was added cesium fluoride (483 mg, 3.18 mmol), tetrakis(triphenylphosphine)palladium (0) (92 mg, 0.080 mmol), and copper **(I)** iodide (30 mg, 0.16 mmol). The reaction vessel was evacuated and refilled with nitrogen five times in succession and then heated to 45 °C for 17 h. The reaction mixture was then poured into water (50 mL) and dichloromethane (100 mL) and the whole was vigorously shaken together. The layers were separated, and the organic layer was filtered through a pad of Celite, eluting with dichloromethane:ethyl acetate (1:1, 200 mL). The eluant was dried over sodium sulfate, filtered, and concentrated onto silica gel. Purification by flash column chromatography (0 to 100% ethyl acetate:hexanes) afforded 777 mg (79%) of the title compound. MS (ESI): 622.26 [M+H]⁺.

### Step C - 1,1-Dimethylethyl 4-{1-[1-(5-(aminocarbonyl)-4-{[(1R)-1-(2-ch/orophenyl)ethyl]oxy}-2-thieny/)-1H-benzimidazol-6-yl]ethenyl}-1-piperidinecarboxylate

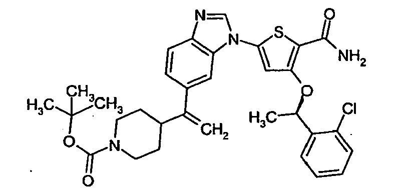

A solution of 1,1-dimethylethyl 4-[1-(1-{4-{[(1*R*)-1-(2-chlorophenyl)ethyl]oxy}-5-[(methyloxy)carbonyl]-2-thienyl}-1H-benzimidazol-6-yl)ethenyl]-1-piperidinecarboxylate (75 mg, 0.11 mmol) in 7 N ammonia in methanol (2.3 mL) was heated in a sealed tube at 80 °C for 42.25 h. The reaction mixture was then cooled to room temperature and concentrated. Purification by flash column chromatography (0 to 10% methanol:chloroform) provided 67 mg (97%) of the title compound. MS (ESI): 607.34 [M+H]⁺.

### Step D - 3-{[(1R)-1-(2-Chlorophenyl)ethyl]oxy}-5-{6-[1-(4-piperidinyl)ethenyl]-1H -benzimidazol-1-yl}-2-thiophenecarboxamide trifluoroacetic acid salt (Title Compound)

To a solution of 1,1-dimethylethyl 4-{1-[1-(5-(aminocarbonyl)-4-{[(1*R*)-1-(2-chlorophenyl)ethyl]oxy}-2-thienyl)-1H-benzimidazol-6-yl]ethenyl}-1-piperidinecarboxylate (56 mg, 0.092 mmol) in dichloromethane (4.0 mL) was added trifluoroacetic acid (1.0 mL). After stirring for 1 h at room temperature, the reaction mixture was concentrated. The residue was triturated with diethyl ether (3 x 5 mL), with sonication applied and the ether decanted away from the precipitated solid after each trituration. The residual solid was then dried under vacuum to afford 51 mg (89%) of the title compound. ¹H NMR (400 MHz, CDCl₃): δ 9.63 (br s, 1H), 9.19 (br s, 1H), 8.14 (br s, 1H), 7.81 (m, 1H), 7.47 (d, 1H, *J*= 7.5 Hz), 7.41-7.24 (m, 6H), 6.67 (s, 1H), 6.58 (br s, 1H), 5.88 (q, 1H, *J*= 6.2 Hz), 5.22 (s, 1H), 5.16 (s, 1H), 3.49 (d, 2H, *J*= 12.3 Hz), 3.03-2.92 (m, 2H), 2.70 (t, 1H, *J* = 11.5 Hz), 2.00 (d, 2H, *J* = 14.1 Hz), 1.85-1.77 (m, 5H); MS (ESI): 507.24 [M+H]⁺.

### Example 50: 3-{[(1R)-1-(2-Chlorophenyl)ethyl]oxy}-5-[6-(4-piperidnylcarbonyl)-1H-benzimidazol-1-yl]-2-thiophenecarboxamide trifluoroacetic acid salt

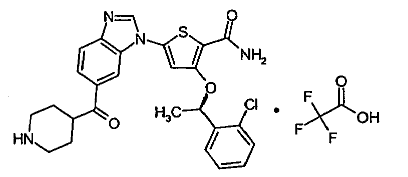

### Step A - 1,1-Dimethylethyl 4-[1-(1-{4-{[(1R)-1-(2-chrophenyl)ethyl]oxy}-5-[(methyloxy)carbonyl]-2-thienyl}-1H-benzimidazol-6-yl)-1,2-dihydroxyethyl]-1-piperidinecarboxylate

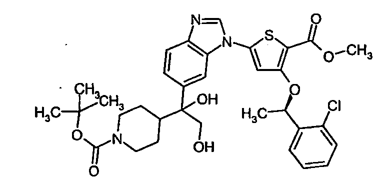

To a solution of 1,1-dimethylethyl 4-[1-(1-{4-{[(1*R*-1-(2-chlorophenyl)ethyl}oxy}-5-[(methyloxy)carbonyl]-2-thienyl}-1*H*-benzimidazol-6-yl)ethenyl]-1-piperidinecarboxylate (120 mg, 0.193 mmol) and methanesulfonamide (18 mg, 0.19 mmol) in 2-methyl-2-propanol (1.0 mL) and water (1.0 mL) was added commercially available AD-Mix α (Aldrich, 418 mg, ^{∼}0.289 mmol). The reaction mixture was stirred vigorously at room temperature for 17 h. The reaction was then quenched by the addition of sodium sulfite (500 mg) and stirred vigorously for 10 min. Following concentration to remove the majority of the 2-methyl-2-propanol, the residue was diluted with 1:1 brine:water (25 mL) and extracted with ethyl acetate (3 x 10 mL). The combined organic fractions were dried over sodium sulfate, filtered, and concentrated to provide 130 mg (∼100%) of the title compound, which was carried on crude into the next step. MS (APCI): 656.31 [M+H]⁺.

### Step B - 1,1-Dimethy/ethy/ 4-[(1-{4-{[(1R)-1-(2-chlorophenyl)ethy]oxy}-5-[(methy/oxy)carbony/]-2-thieny/}-1H-benzimidazo/-6-y/)carbony/]-1-piperidinecarboxylate

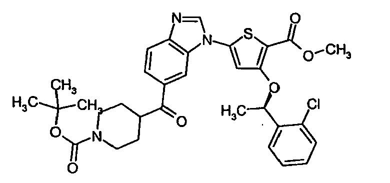

To a solution of crude 1,1-dimethylethyl 4-[1-(1-{4-{[(1*R*)-1-(2-chlorophenyl)ethyl]oxy}-5-[(methyloxy)carbonyl]-2-thienyl}-1H-benzimidazol-6-yl)-1,2-dihydroxyethyl]-1-piperidinecarboxylate (^{∼}130 mg, 0.19 mmol) in methanol (1.9 mL) was added a solution of sodium periodate (50 mg, 0.23 mmol) in water:methanol (1:1, 1.2 mL). The reaction was stirred vigorously at room temperature for 2.5 h and then poured into brine:water (1:1, 75 mL). The whole was extracted was ethyl acetate (3 x 20 mL). The combined organic fractions were dried over sodium sulfate, filtered, and concentrated to afford 107 mg (89%, 2 steps) of the title compound. MS (ESI): 624.24 [M+H]⁺.

### Step C - 1,1-Dimethy/ethy/ 4-{[1-(5-(aminocarbonyl)-4-{[(1R)-1-(2-chlorophenyl)ethyl]oxy}-2-thieny/)-1H-benzimidazol-6-yl]carbonyl}-1-piperidinecarboxylate

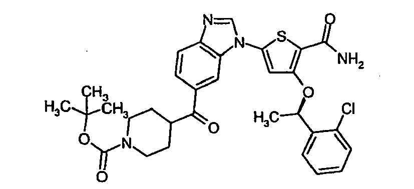

The title compound was prepared from 1,1-dimethylethyl 4-[(1-{4-{[(1*R*)*-1-(2-*chlorophenyl)ethyl]oxy}-5-[(methyloxy)carbonyl]-2-thienyl}-1H-benzimidazol-6-yl)carbonyl]-1-piperidinecarboxylate by a procedure analogous to **Example 49**, Step C. MS (APCI): 609.04 [M+H]⁺.

### Step D - 3-{[(1R)-1-(2-Chlorophenyl)ethyl]oxy}-5-[6-(4-piperidinylcarbonyl)-1H-benzimidazol-1-yl]-2-thiophenecarboxamide trifluoroacetic acid salt (Title Compound)

The title compound was prepared from 1,1-dimethylethyl 4-{[1-(5-(aminocarbonyl)-4-{[(1*R*)-1-(2-chlorophenyl)ethyl]oxy}-2-thienyl)-1*H-*benzimidazol-6-yl]carbonyl}-1-piperidinecarboxylate by a procedure analogous to **Example 49**, Step D. ¹H NMR (400 MHz, CDCl₃): δ 10.23 (br s, 1H), 9.35 (br s, 1H), 8.21 (s, 1H), 8.11 (s, 1H), 7.93-7.88 (m, 2H), 7.48-7.41 (m, 2H), 7.38-7.28 (m, 3H), 7.07 (br s, 1H), 6.67 (s, 1H), 5.88 (q, 1H, *J*= 6.3 Hz); MS (APCI): 508.96 [M+H]⁺.

### Example 51: 3-{[(1R)-1-(2-Chlorophenyl)ethyl]oxy}-5-{6-difluoro(4-piperidinyl)methyl]-1H-benzimidazol-1-yl}-2-thiophenecarboxamide trifluoroacetic acid salt

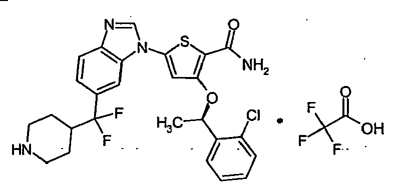

### Step A - 1,1-Dimethylethyl 4-[(1-{4-{[(1R)-1-(2 chlorophenyl)ethyl]oxy}-5-[(methy/oxy)carbonyl]-2-thienyl}-1H-benzimidazol-6-yl)(difluoro)methyl]-1-piperidinecarboxylate

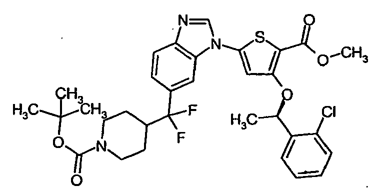

To a cooled (-78 °C) solution of 1,1-dimethylethyl 4-[(1-{4-{[(1R)-1-(2-chlorophenyl)ethyl]oxy}-5-[(methyloxy)carbonyl]-2-thienyl}-1H-benzimidazol-6-yl)carbonyl]-1-piperidinecarboxylate (145 mg, 0.232 mmol) and antimony (III) chloride (5.3 mg, 0.023 mmol) in dichloromethane (250 µL) was added [bis(2-methoxyethyl)amino]sulfur trifluoride (300 µL, 1.62 mmol). The reaction was warmed to room temperature over 1 h, and then stirred at room temperature for 3 days. Another portion of [bis(2-methoxyethyl)amino]sulfur trifluoride (300 µL, 1.62 mmol) was added, and the reaction stirred for an additional 3 days. The reaction was then slowly quenched by pouring into saturated aqueous sodium bicarbonate (25 mL). The mixture was extracted with dichloromethane (3 x 10 mL). The combined organic fractions were dried over sodium sulfate, filtered, and concentrated onto silica gel. Purification by flash column chromatography afforded 43 mg (30%) of recovered 1,1-dimethylethyl 4-[(1-{4-{[(1R)-1-(2-chlorophenyl)ethyl]oxy}-5-[(methyloxy)carbonyl]-2-thienyl}-1H-benzimidazol-6-yl)carbonyl]-1-piperidinecarboxylate and 51 mg (34%) of the title compound. MS (APCI): 668.07 [M+Na]⁺.

### Step B - 1,1-Dimethylethy/ 4-[[1-(5-(aminocarbony/)-4-{[(1R)-1-(2-chlorophenyl)ethyl]oxy}-2-thienyl)-1H-benzimidazol-6-yl](difluoro)methyl]-1-piperidinecarboxylate

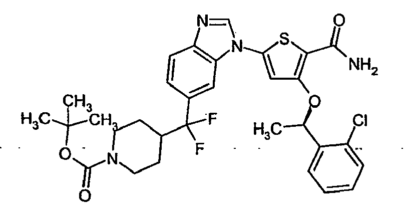

The title compound was prepared from 1,1-dimethylethyl 4-[(1-{4-{[(1*R*)*-1-(2-*chlorophenyl)ethyl]oxy}-5-[(methyloxy)carbonyl]-2-thienyl}-1*H*-benzimidazol-6-yl)(difluoro)methyl]-1-piperidinecarboxylate by a procedure analogous to **Example 49,** Step C. MS (ESI): 631.27 [M+H]⁺.

### Step C - 3-{[(1R)-1-(2-Chlorophenyl)ethyl]oxy}-5-{6-[difluoro(4-piperidinyl)methyl]-1H-benzimidazol-1-yl}-2-thiophenecarboxamide trifluoroacetic acid salt (Title Compound)

The title compound was prepared from 1,1-dimethylethyl 4-[[1-(5-(aminocarbonyl)-4-{[(1R)-1-(2-chlorophenyl)ethyl]oxy}-2-thienyl)-1*H-*benzimidazol-6-yl](difluoro)methyl]-1-piperidinecarboxylate by a procedure analogous to **Example 49**, Step D. ¹H NMR (400 MHz, CDCl₃): δ 9.70 (br s, 1H), 9.15 (br s, 1H), 8.14 (s, 1H), 7.91 (d, 1H, *J*= 8.6 Hz), 7.52-7.29 (m, 8H), 6.68 (s, 1H), 5.88 (q, 1H, *J*= 6.5 Hz), 3.53-3.45 (m, 2H), 1.58 (m, 2H), 1.94-1.83 (m, 4H), 1.78 (d, 3H, *J*= 6.4 Hz), 1.34-1.20 (m, 1H); MS (ESI): 531.23 [M+H]⁺.

### Example 52: 3-{[(1R)-1-(2-Chlorophenyl)ethyl]oxy}-5-{6-[difluoro(1-methyl-4-piperidinyl)methyl]-1H-benzimidazol-1-yl}-2-thiophenecarboxamide

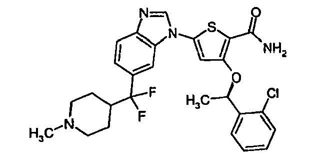

To a solution of 3-{[(1*R*)-1-(2-chlorophenyl)ethyl]oxy}-5-{6-[difluoro(4-piperidinyl)methyl]-1*H*-benzimidazol-1-yl}-2-thiophenecarboxamide trifluoroacetic acid salt (41 mg, 0.077 mmol) in methanol (0.5 mL) and dichloromethane (1.0 mL) was added formaldehyde (37% aqueous solution, 7 µL, 0.09 mmol), acetic acid (5 µL, 0.09 mmol) and sodium cyanoborohydride (7.5 mg, 0.12 mmol). The reaction was stirred 15 min at room temperature, the quenched by pouring into saturated aqueous sodium bicarbonate (25 mL). The mixture was extracted with dichloromethane (2 x 20 mL). The combined organic fractions were dried over sodium sulfate, filtered, and concentrated. Purification by flash column chromatography (40 to 100% 1/10/89 ammonium hydroxide/methanol/ chloroform:chloroform) afforded 32 mg (76%) of the title compound. ¹H NMR (300 MHz, CDCl₃): δ 8.03 (s, 1H), 7.84 (d, 1H, *J*= 8.4 Hz), 7.49-7.27 (m, 6H), 7.22 (br s, 1H), 6.65 (s, 1H), 5.99 (br s, 1H), 5.88 (q, 1H, *J*= 6.3 Hz), 2.94-2.86 (m, 2H), 2.26 (s, 3H), 1.98-1.82 (m, 3H), 1.78 (d, 3H, *J*= 6.3 Hz), 1.70-1.52 (m, 4H); MS (APCI): 545.04 [M+H]⁺.

### Example 53: 3-{[(1R)-1-(2-Chlorophenyl)ethyl]oxy}-5-{6-[(4-methylhexahydro-1H-1,4-diazepin-1-yl)methyl]-1H-benzimidazol-1-yl}-2-thiophenecarboxamide

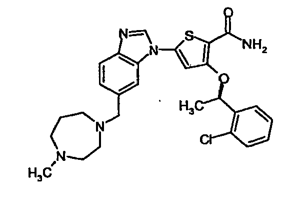

Methyl 5-[6-(chloromethyl)-1*H*-benzimidazol-1-yl]-3-{[(1*R*)-1-(2-chlorophenyl) ethyl]oxy}-2-thiophenecarboxylate (0.28 g, 0.647 mmol), 1-methylhexahydro-1*H*-1,4-diazepine (0.147 g, 1.3 mmol), and cesium carbonate (0.623 g, 1.94 mmol) were dissolved in *N,N*-dimethylformamide (10 mL). The reaction was heated to 60 °C and stirred for 2 h. The mixture was concentrated *in vacuo.* Purification by flash chromatography provided 0.239 g (38%) of methyl 3-{[(1*R*)-1-(2-chlorophenyl)ethyl]oxy}-5-{6-[(4-methylhexahydro-1*H*-1,4-diazepin-1-yl)methyl]-1*H*-benzimidazol-1-yl}-2-thiophenecarboxylate. Methyl 3-{[(1*R*)-1-(2-chlorophenyl)ethyl]oxy}-5-{6-[(4-methylhexahydro-1*H*-1,4-diazepin-1-yl)methyl]-1*H*-benzimidazol-1-yl}-2-thiophenecarboxylate (0.139 g, 0.257 mmol) was then placed in a sealed tube and dissolved in 10 mL of 7 N ammonia in methanol. The reaction was heated to 65 °C and stirred for 72 h. The mixture was concentrated *in vacuo.* Purification by flash chromatography afforded 0.076 g (56%) of the title compound. ¹H NMR (400 MHz, CD₃OD): δ 8.35 (s, 1H), 7.68 (d, 1H, *J*= 8.42 Hz), 7.61 (dd, 1H, *J*= 1.65, 7.68 Hz), 7.48(dd, 1H, *J*= 1.28, 7.87 Hz), 7.45 (s, 1H), 7.42-7.33 (m, 3H), 7.01 (s, 1H), 6.06 (q, 1H, *J*= 6.40 Hz), 3.75 (s, 2H), 2.76-2.71 (m, 6H), 2.68-2.65 (m, 2H), 2.35 (s, 3H), 1.86-1.78 (m, 5H); MS (ESI): 524 [M+H]⁺.

### Example 54: 5-{6-[(4-Amino-1-piperidinyl)methyl]-1H-benzimidazol-1-yl}-3-({(1R)-1-[2-(trifluoromethyl)phenyl]ethyl}oxy)-2-thiophenecarboxamide hydrochloride

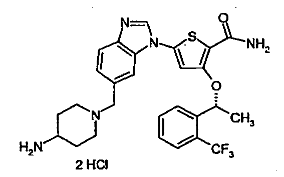

### Step A - methyl 5-(6-{[4-({[(1,1-dimethylethyl)oxy]carbonyl}amino)-1-piperidinyl]methyl}-1H-benzimidazo/-1-yl)-3-({(1R)-1-[2-(trifluoromethy/)phenyl]ethyl}oxy)-2-thiophenecarboxylate

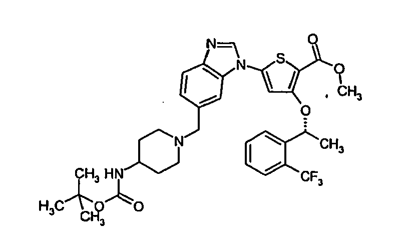

A solution of methyl 5-[6-(chloromethyl)-1H-benzimidazol-1-yl]-3-({(1R)-1-[2-(trifluoromethyl)phenyl]ethyl}oxy)-2-thiophenecarboxylate (0.100 g, 0.20 mmol), 4-(N-Boc-amino)piperidine (0.073 g, 0.36 mmol), and triethylamine (0.08 mL, 0.60 mmol) in dioxane (1.0 mL) was stirred for 18 h after which time the mixture was cooled, diluted with dichloromethane, and washed with water twice. The organic layer was dried over magnesium sulfate, filtered, and concentrated onto silica gel. Purification by column chromatography (0 to 10% methanol:dichloromethane) provided 0.13 g (100%) of the title compound as a yellow residue. MS (ESI): 659 [M+H]⁺.

### Step B - 5-{6-[(4-Amino-1-piperidinyl)methyl]-1H-benzimidazol-1-yl}-3-({(1R)-1-[2-(trifluoromethyl)pheny/]ethyl}oxy)-2-thiophenecarboxamide hydrochloride (Title Compound)

Methyl 5-(6-{[4-({[(1,1-dimethylethyl)oxy]carbonyl}amino)-1-piperidinyl]-methyl}-1H-benzimidazol-1-yl)-3-({(1R)-1-[2-(trifluoromethyl)phenyl]ethyl}oxy)-2-thiophenecarboxylate (crude 0.13 g, 0.20 mmol) in 7 N ammonia in methanol (2 mL) was heated while stirring in a sealed tube at 90 °C) overnight. The mixture was cooled, concentrated, and then purified by column chromatography (0 to 10% methanol:dichloromethane). The resulting residue (0.13 g) was dissolved in methanol (2.0 mL) and 4 N HCl in 1,4-dioxane (2.0 mL) and stirred for 0.5 h. Concentrating to dryness afforded 0.13 g (100%) of the title compound as a yellow solid. ¹H NMR (400 MHz, CD₃OD): δ 9.48 (s, 1H), 8.18 (s, 1H), 7.96 (d, *J*= 8.4 Hz, 1H), 7.86 (dd, 2H, *J*= 8.0, 14.4 Hz), 7.70 (t, 2H, *J*= 7.6 Hz), 7.51 (t, 1H, *J=* 7.6 Hz), 7.33 (s, 1H), 6.13-6.08 (m, 1H), 4.54 (s, 2H), 3.60-3.46 (m, 3H), 3.28-3.22 (m, 2H), 2.25-2.22 (m, 2H), 2.12-2.03 (m, 2H), 1.82 (d, 3H, *J*= 6.0 Hz); MS (ESI): 544 [M+H]⁺.

### Example 55: 1-{[1-5-(Aminocarbonyl)-4-{[(1R)-1-(2-chlorophenyl)ethyl]oxy}-2-thienyl)-1H-benzimidazol-6-yl]methyl}-4-piperidinecarboxamide

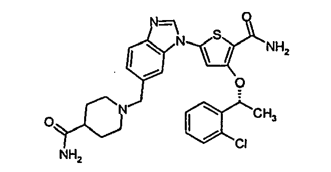

### Step A - 3-{[(1R)-1-(2-chlorophenyl)ethyl]oxy}-5-[6-(hydroxymethyl)-1H-benzimidazol-1-yl]-2-thiophenecarboxamide

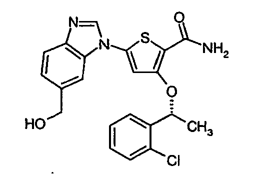

The title compound can be prepared by using a procedure analogous to **Example 84**, Step A.

### Step B - 5-[6-(Chloromethyl)-1H-benzimidazol-1-yl]-3-{[(1R)-1-(2-chlorophenyl)ethyl]oxy}-2-thiophenecarboxamide

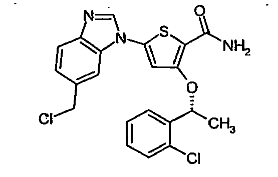

The title compound was prepared from 3-{[(1*R*)-1-(2-chlorophenyl)ethyl]oxy}-5-[6-(hydroxymethyl)-1*H*-benzimidazol-1-yl]-2-thiophenecarboxamide by a procedure analogous to **Example 84**, Step B.

### Step C - 1-{[1-(5-(Aminocarbony/)-4-{[(1R)-1-(2-chloropheny/)ethyl]oxy}-2-thieny/)-1H-benzimidazol-6-yl]methyl}-4-piperidinecarboxamide (Title Compound)

A mixture of 5-[6-(chloromethyl)-1H-benzimidazol-1-yl]-3-{[(1R)-1-(2-chlorophenyl)ethyl]oxy}-2-thiophenecarboxamide (0.100 g, 0.22 mmol), diisopropylethylamine (0.10 mL) and isonipecotamide (0.40 g, 0.31 mmol) in dioxane (2 mL) was stirred in a sealed tube while heating at 75 ºC overnight.

The reaction mixture was concentrated onto silica gel and purified by column chromatography (0 to 20% methanol:dichloromethane) to afford 0.090 g (76%) of the title compound as an off-white powder. ¹H NMR (400 MHz, CD₃OD): δ 8.37 (s, 1H), 7.70 (d, 1H, *J*= 8.4 Hz), 7.60 (d, 1H, *J*= 7.6 Hz), 7.49-7.40 (m, 2H), 7.38-7.33 (m, 4H), 7.02 (s, 1H), 6.07-6.03 (m, 1H), 5.47 (s, 1H), 3.77 (br s, 2H), 3.21-3.15 (m, 3H), 3.03-2.99 (m, 2H), 2.28-2.23 (m, 2H), 1.85-1.70 (m, 4H), 1.31-1.27 (t, 3H); MS (ESI): 538 [M+H]⁺.

### Example 56: 3-{[(1R)-1-(2-Chlorophenyl)ethyl]oxy}-5-(6-{[4-(ethyl)sulfonyl)-1-piperazinyl]methyl}-1H-benzimidazol-1-yl)-2-thiophenecarboxamide

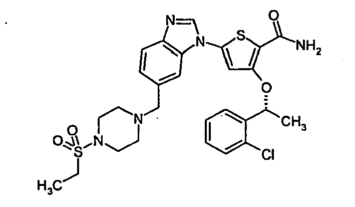

The title compound was prepared from 5-[6-(chloromethyl)-1H-benzimidazol-1-yl]-3-{[(1R)-1-(2-chlorophenyl)ethyl]oxy}-2-thiophenecarboxamide and 1-(ethylsulfonyl)piperazine by a procedure analogous to **Example 55,** Step C. ¹H NMR (400 MHz, CD₃OD): δ 7.93 (s, 1H), 7.74 (d, 1H, J= 8.0 Hz), 7.48-7.26 (m, 7H), 6.60 (s, 1H), 5.88-5.84 (m, 1H), 5.70 (br s, 1H), 3.60 (br s, 2H), 3.30-3.28 (m, 4H), 2.94 (q, 2H, *J*= 12.4, 20.0 Hz), 2.51-2.49 (m, 4H), 1.76 (d, 3H, *J*= 6.4 Hz), 1.36 (t, 3H, *J*= 7.6 Hz); MS (ESI): 588 [M+H]⁺.

### Example 57: 3-{[(1R)-1-(2-chlorophenyl)ethyl]oxy}-5-(5-{6-[[2-(dimethylamino)ethyl](methyl)amino]-3-pyridinyl}-1H-benzimidazol-1-yl)-2-thiophenecarboxamide

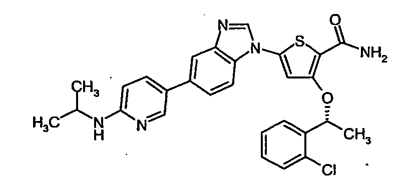

A mixture of 3-{[(1*R*)-1-(2-chlorophenyl)ethyl]oxy}-5-[5-(6-fluoropyridin-3-yl)-1*H*-benzimidazol-1-yl]thiophene-2-carboxamide (0.050 g, 0.10 mmol), isopropylamine (2 mL) and ethanol (1 mL) was stirred in the microwave at 180 °C for 1 h. The mixture was concentrated to dryness, dissolved in dichloromethane, and washed with saturated aqueous sodium bicarbonate and brine. The organic layer was concentrated onto silica gel and purified by column chromatography (0 to 10% methanol:dichloromethane) to give 0.048 g (90%) of the title compound as a tan solid. ¹H NMR (400 MHz, CDCl₃): δ 8.34 (d, 1H, *J*= 2.0 Hz), 7.96 (s, 1H), 7.90 (s, 1H), 7.69 (dd, 1H, *J*= 2.4, 8.8 Hz), 7.47 - 7.41 (m, 4H), 7.35 - 7.27 (m, 2H), 7.19 (br s, 1H), 6.62 (s, 1H), 6.45 (d, 1H, *J*= 8.8 Hz), 5.89 - 5.85 (m, 1H), 5.74 (br s, 1H), 4.52 (br s, 1H), 3.95 - 3.89 (m, 1H), 1.76 (d, 3H, *J*= 6.4 Hz), 1.26 (d, 6H, *J*= 6.4 Hz); MS (ESI): 532 [M+H]⁺.

### Example 58: 3-{[(1R)-1-(2-Chlorophenyl)ethyl]oxy}-5-(5-{6-[(1-methyl-4-piperidinyl)amino]-3-pyridinyl}-1H-benzimidazol-1-yl)-2-thiophenecarboxamide

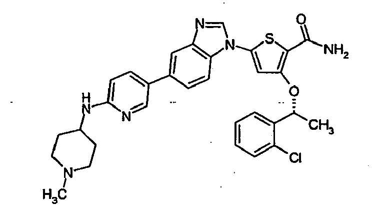

The title compound was prepared from 3-{[(1*R*)-1-(2-chlorophenyl)ethyl]oxy}-5-[5-(6-fluoropyridin-3-yl)-1*H*-benzimidazol-1-yl]thiophene-2-carboxamide and 1-methyl-4-piperidinamine by a procedure analogous to **Example 57**. ¹H NMR (400 MHz, CDCl₃): δ 8.35 (d, 1H, *J*= 2.0 Hz), 7.96 (s, 1H), 7.90 (s, 1H), 7.68-7.66 (m, 1H), 7.50-7.41 (m, 4H), 7.35-7.20 (m, 3H), 6.61 (s, 1H), 6.46 (d, 1H, *J*= 8.4 Hz), 5.88-5.75 (m, 1H), 4.47-4.45 (m, 1H), 3.75 (br s, 1H), 2.96-2.88 (m, 2H), 2.36 (s, 3H), 2.30-2.08 (m, 6H), 1.77 (d, 3H, *J*= 6.4 Hz); MS (ESI): 587 [M+H]⁺.

### Example 59: 5-[5-(6-Piperazin-1-ylpyridin-3-yl)-1H-benzimidazol-1-yl]-3-{(1R)-1-[2-(trifluoromethyl)phenyl]ethoxy}thiophene-2-carboxamide

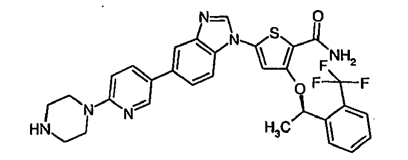

### Step A - 1,1-Dimethy/ethy/ 4-(5-{1-[5-[(methyloxy)carbonyl]-4-({(1R)-1-[2-(trifluoromethyl)phenyl]ethyl}oxy)-2-thienyl]-1H-benzimidazol-5-yl}-2-pyridinyl)-1-piperazinecarboxylate

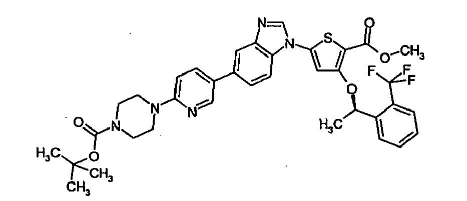

1,1-Dimethylethyl 4-[5-(4,4,5,5-tetramethyl-1,3,2-dioxaborolan-2-yl)-2-pyridinyl]-1-piperazinecarboxylate (1.0 g, 2.5 mmol) was suspended in *N*,*N-*dimethylacetamide (14 mL) and treated with aqueous 1 N sodium carbonate (6 mL, 6 mmol), methyl 5-(5-bromo-1H-benzimidazol-1-yl)-3-({(1*R*)-1-[2-(trifluoromethyl)phenyl]ethyl}oxy)-2-thiophenecarboxylate (800 mg, 1.5 mmol) and 1,1'-bis diphenylphosphinoferracene dichloropalladium(II) dichloromethane adduct (210 mg, 0.26 mmol) and heated to 80 °C for 1.5 h. The reaction was allowed to cool to room temperature overnight. The reaction mixture was partitioned between 5:1 chloroform:methanol and saturated aqueous sodium bicarbonate. Toluene was added and the mixture concentrated (2x) to remove remaining *N*,*N*-dimethylacetamide. The crude compound was purified by column chromatography (hexanes:ethyl acetate, with 0.5% triethylamine) to yield 1.0 g of the title compound as an oil. MS (APCI): 708.15 [M+H]⁺.

### Step B - 1,1-Dimethylethyl 4-(5-{1-[5-(aminocarbonyl)-4-({(1R)-1-[2-(trifluoromethyl)phenyl]ethyl}oxy)-2-thienyl]-1H-benzimidazol-5-yl}-2-pyridiny/)-1-piperazinecarboxylate

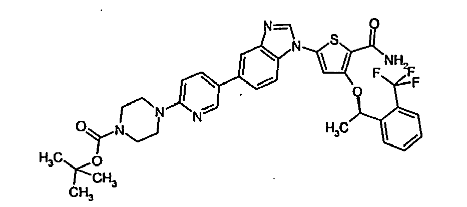

1,1-Dimethylethyl 4-(5-{1-[5-[(methyloxy)carbonyl]-4-({(1*R*)*-1-[2-*(trifluoromethyl)phenyl]ethyl}oxy)-2-thienyl]-1H-benzimidazol-5-yl}-2-pyridinyl)-1-piperazinecarboxylate (1.0 g, 1.41mmol) was dissolved in 7 N ammonia in methanol (15 mL) and placed in a sealed vessel. The mixture was heated at 80 °C for 16 h and then cooled to room temperature. The crude mixture was purified by column chromatography (hexanes:ethyl acetate, with 0.5% triethylamine) to yield 810 mg of the title compound as a yellow foam. MS (APCI): 693.11 [M+H]⁺.

### Step C - 5-[5-(6-Piperazin-1-ylpyridin-3-yl)-1H-benzimidazol-1-yl]-3-{(1R)-1-[2-(trifluoromethyl)phenyl]ethoxy}thiophene-2-carboxamide (Title Compound)

1,1-Dimethylethyl 4-(5-{1-[5-(aminocarbonyl)-4-({(1*R*)-1-[2-(trifluoromethyl)phenyl]ethyl}oxy)-2-thienyl]-1*H*-benzimidazol-5-yl}-2-pyridinyl)-1-piperazinecarboxylate (810 mg, 1.17mmol) in chloroform (20 mL) was treated with trifluoroacetic acid (3 mL) and stirred at room temperature for 1.5 h. The reaction was quenched by the slow addition of saturated aqueous sodium bicarbonate until the pH was >7. The reaction mixture was partitioned between 5:1 chloroform:methanol and saturated aqueous sodium bicarbonate. The organic layer was dried over sodium sulfate. The mixture was filtered, concentrated and purified by column chromatography (90/9/1 dichloromethane/methanol/ammonium hydroxide:dichloromethane). The product was triturated in diethyl ether and filtered to yield 277 mg of the title compound as a light yellow solid. ¹H NMR (400 MHz, DMSO-d₆): δ 8.53 (s, 1H), 8.47 (d, 1H, *J*= 2.38Hz), 7.95-7.87 (m, 3H), 7.82 (br s, 1H), 7.77-7.74 (m, 2H), 7.61-7.50 (m, 3H), 7.12 (br s, 2H), 6.86 (d, 1H, *J*= 8.79), 5.94 (q, 1H, *J*= 6.04 Hz), 3.42 (t, 4H, *J*= 4.85 Hz), 2.76 (t, 4H, *J*= 4.94 Hz), 1.73 (d, 3H, *J*= 6.04 Hz); HRMS C₃₀H₂₈N₆O₂F₃S: [M+H]⁺ calc'd. 593.1947, found 593.1942.

### Example 60: 3-[(1R)-1-(2-Chlorophenyl)ethoxy)-5-(5-{1-[2-(dimethylamino)ethyl]-1H-pyrazol-4-yl}-1H-benzimidazol-1-yl)thiophene-2-carboxamide

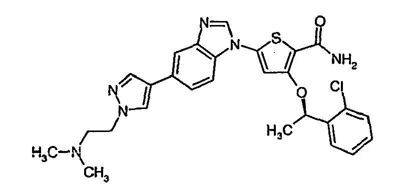

### Step A - methy/ 3-{[(1R)-1-(2-Chloropheny/)ethyl]oxy}-5-[5-(1H-pyrazol-4-yl)-1H-benzimidazol-1-yl]-2-thiophenecarboxylate

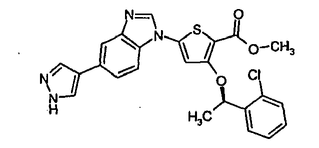

4-(4,4,5,5-Tetramethyl-1,3,2-dioxaborolan-2-yl)-1*H*-pyrazole (355 mg, 1.83 mmol) in *N*,*N*-dimethylacetamide (15 mL) was treated with aqueous 1 N sodium carbonate (4.5 mL, 4.5 mmol), methyl 5-(5-bromo-1H-benzimidazol-1-yl)-3-{[(1*R*)-1-(2-chlorophenyl)ethyl]oxy}thiophene-2-carboxylate (600 mg, 1.22 mmol) and 1,1'-bis diphenylphosphinoferrocene dichloropalladium(II) dichloromethane adduct (149 mg, 0.18 mmol) and heated to 80 °C overnight. After cooling to room temperature the reaction mixture was partitioned between 5:1 chloroform:methanol and saturated aqueous sodium bicarbonate. The layers were separated and the organic layer was dried over sodium sulfate. The solution was filtered and concentrated and placed on a vacuum pump overnight to remove remaining *N*,*N*-dimethylacetamide. The crude compound was purified by column chromatography (hexanes:ethyl acetate, with 0.5% triethylamine) to yield 349 mg of the title compound as a foam. MS (APCI): 478.94 [M+H]⁺.

### Step B - Methyl 3-{[(1R)-1-(2-chlorophenyl)ethyl]oxy}-5-(5-{1-[2-(dimethylamino)ethyl]-1H-pyrazol-4-yl}-1H-benzimidazol-1-yl)-2-thiophenecarboxylate

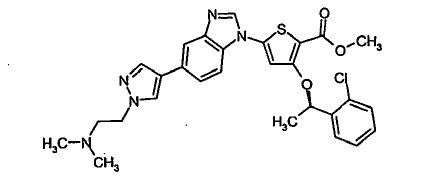

To methyl 3-{[(1*R*)-1-(2-chlorophenyl)ethyl]oxy}-5-[5-(1H-pyrazol-4-yl)-1*H-*benzimidazol-1-yl]-2-thiophenecarboxylate (50 mg, 0.10 mmol) in *N*,*N-*dimethylformamide (1 mL) was added cesium carbonate (67.7 mg, 0.21 mmol) and stirred for 20 min, Commercially available (2-chloroethyl)dimethylamine hydrochloride and more cesium carbonate (67.7 mg, 0.21 mmol) were added to the reaction and stirred at room temperature for 1.5 h. No reaction was noted. The reaction was heated to 65 °C; product began forming at this temperature. The reaction was heated at 68 °C overnight. The reaction mixture was partitioned between 5:1 chloroform:methanol and saturated aqueous sodium bicarbonate. The layers were separated and the organic layer was dried over sodium sulfate. The organic layer was filtered and concentrated. The residue was purified by column chromatography (90/9/1 dichloromethane/methanol/ammonium hydroxide:dichloromethane) to provide 35 mg of title compound as an oil. MS (APCI): 550.00 [M+H]⁺.

### Step C: 3-[(1R)-1-(2-chlorophenyl)ethoxy]-5-(5-{1-[2-(dimethylamino)ethyl]-1H-pyrazol-4-yl}-1H-benzimidazol-1-yl)thiophene-2-carboxamide (Title Compound)

The title compound was prepared from methyl 3-{[(1*R*)-1-(2-chlorophenyl)ethyl]oxy}-5-(5-{1-[2-(dimethylamino)ethyl]-1H-pyrazol-4-yl}-1*H-*benzimidazol-1-yl)-2-thiophenecarboxylate by a procedure analogous to **Example 59**, Step B. ¹H NMR (400 MHz, DMSO-d₆): δ 8.52 (s, 1H), 8.23 (s, 1H), 7.94 (s, 1H), 7.92 (s, 1H), 7.79 (br s, 1H), 7.66 (d, *J*= 6.59, 1H), 7.57 (d, 1H, *J*= 7.69), 7.49 (d, 1H, *J*= 7Hz), 7.47 (d, 1H, *J*= 8.4 Hz), 7.43-7.33 (m, 2H), 7.16 (s, 1H), 7.09 (br s, 1H), 5.97 (q, 1H, *J*= 6.35 Hz), 4.18 (t, 2H, *J*= 6.50 Hz), 2.65-2.60 (m, 2H), 2.17 (s, 6H), 1.71 (d, 3H, J= 6.41 Hz); HRMS C₂₇H₂₈N₆O₂SCl: [M+H]⁺ calc'd. 535.1683, found 535.1680.

### Example 61: 3-{[(1R)-(2-Chlorophenyl)ethyl]oxy}-5-(5-{1-[3-(4-ethyl-1-piperazinyl)propyl]-1H-pyrazol-4-yl}-1H-benzimidazol-1-yl)-2-thiophenecarboxamide

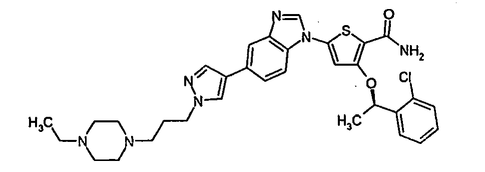

### Step A - Methy/ 3-{[(1R)-1-(2-chlorophenyl)ethy/]oxy}-5-(5-{1-[3-(4-ethy/-1-piperazinyl)propy/]-1H-pyrazol-4-yl}-1H-benzimidazo/-1-yl)-2-thiophenecarboxylate

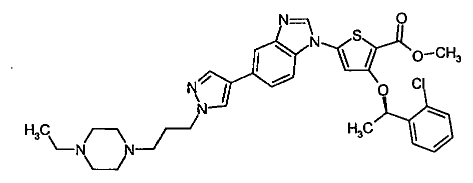

To methyl 3-{[(1*R*)-1-(2-chlorophenyl)ethyl]oxy}-5-[5-(1*H*-pyrazol-4-yl)-1*H-*benzimidazol-1-yl]-2-thiophenecarboxylate (125 mg, 0.26 mmol) in *N,N-*dimethylformamide (2 mL) was added cesium carbonate (136 mg, 0.42 mmol) and stirred for 30 min. Literature-known (Journal of the Chemical Society, Abstracts 1961, 2404-2418) 1-(3-bromopropyl)-4-ethylpiperazine dihydrobromide (165 mg, 0.42 mmol) and more cesium carbonate (136 mg, 0.42 mmol) were added to the reaction and stirred at 70 °C for 1 h. No reaction was noted. More cesium carbonate (170 mg) was added and then the reaction was complete within 1.5 hours. The reaction mixture was concentrated onto silica gel and purified by column chromatography (hexane then dichloromethane:90/9/1 dichloromethane/methanol/ammonium hydroxide) to afford 140 mg of the title compound as a yellow oil. MS (ESI): 633.31 [M+H]⁺.

### Step B - 3-{[(1R)-1-(2-Chlorophenyl)ethyl]oxy}-5-(5-{1-[3-(4-ethyl-1-piperazinyl)propyl]-1H-pyrazol-4-yl}-1H-benzimidazol-1-yl)-2-thiophenecarboxamide (Title Compound)

Methyl-3-{[(1*R*)-1-(2-chlorophenyl)ethyl]oxy}-5-(5-{1-[3-(4-ethyl-1-piperazinyl)propyl]-1H-pyrazol-4-yl}-1*H*-benzimidazol-1-yl)-2-thiophenecarboxylate (140 mg, 0.22 mmol) was dissolved in 7 N ammonia in methanol (6mL) and placed in a sealed vessel. The reaction was heated at 80 °C overnight and cooled to room temperature, at which time the reaction was incomplete. More 7 N ammonia in methanol (1.5 mL) was added and the reaction was heated at 80 °C for another 4 hours. Silica gel was then added, and the mixture was concentrated and purified by column chromatography (90/9/1 dichloromethane/methanol/ammonium hydroxide:dichloromethane) to afford 45 mg of the title compound as a beige solid. ¹H NMR (300 MHz, DMSO-d₆): δ 8.58 (s, 1H), 8.28 (s, 1H), 8.01 (s, 1H), 7.98 (s, 1H), 7.80 (br s, 1H), 7.73 (dd, 1H, *J* = 1.75, 7.51 Hz), 7.64 (d, 1H, *J*= 9.69 Hz), 7.56-7.39 (m, 4H), 7.22 (s, 1H), 7.18 (br s, 1H), 6.04 (q, 1H, *J*= 6.32), 4.17 (t, 2H, *J*= 6.95), 2.56-2.50 (m, 2H), 2.45-2.25 (m, 10H), 2.06-1.94 (m, 2H), 1.77 (d, 3H, *J*= 6.32 Hz), 1.01 (t, 3H, *J*= 7.09 Hz); HRMS C₃₂H₃₇N₇O₂SCl: [M+H]⁺ calc'd. 618.24125, found 618.24109.

### Example 62: 3-{[(1R)-1-(2-Chlorophenyl)ethyl]oxy}-5[5-(2-piperazin-1-ylpyridin-4-yl)-1H-benzimidazol-1-yl]thiophene-2-carboxamide

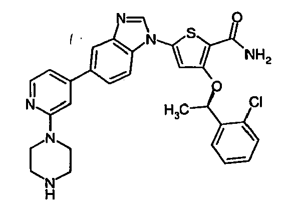

3-{[(1*R*)-1-(2-chlorophenyl)ethyl]oxy}-5-[5-(2-fluoropyridin-4-yl)-1*H-*benzimidazol-1-yl]thiophene-2-carboxamide (0.025 g, 0.05 mmol) and piperazine (0.109 g, 1.27 mmol) were combined in 95% ethanol (0.3 mL). The reaction mixture was heated in a Personal Chemistry microwave at 180 °C for 20 min after which time the mixture was diluted with dichloromethane and washed with water. The aqueous layer was extracted twice with dichloromethane. The organics were combined, dried over magnesium sulfate, filtered and concentrated onto silica gel. Purification by column chromatography (10 to 100% 1/9/90 ammonium hydroxide/methanol/dichloromethane:dichloromethane) provided 0.009 g (32%) as an off-white solid. ¹H NMR (400 MHz, CDCl₃): δ 8.24 (d, 1H, *J*= 5.3 Hz), 8.03 (d, 1H, *J*= 1.3 Hz), 7.99 (s, 1H), 7.56 (dd, 1H, *J*= 1.7, 8.6 Hz), 7.45 (m, 3H), 7.32 (m, 2H), 7.20 (br s, 1H), 6.88 (d, 1H, *J*= 5.2 Hz), 6.85 (s, 1H), 6.65 (s, 1H), 5.97 (br s, 1H), 5.87 (q, 1H, *J*= 6.4 Hz), 3.58 (t, 4H, *J*= 5.0 Hz), 3.01 (t, 4H, *J*= 5.0 Hz), 1.77 (d, 3H, *J*= 6.4 Hz), 1.73 (br s, 1H); MS (ESI): 559.2 [M+H]⁺.

### Examples 63: 5-[5-(1-Oxidopyridin-4-yl)-1H-benzimidazol-1-yl]-3-({(1R)-1-[2-(trifluoromethyl)phenyl]ethyl}oxy)thiophene-2-carboxamide

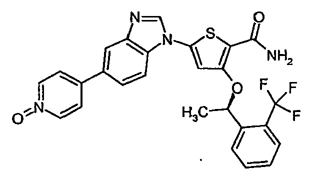

### Step A - Methyl 5-(5-pyridin-4-yl-1H-benzimidazol-1-yl)-3-({(1R)-1-[2-(trifluoromethyl)phenyl]ethyl}oxy)thiophene-2-carboxylate

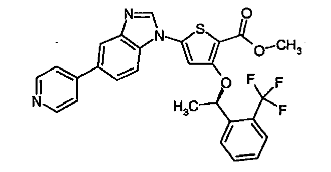

The title compound was prepared from methyl 5-(5-bromo-1*H*-benzimidazol-1-yl)-3-({(1*R*-1-[2-(trifluoromethyl)phenyl]ethyl}oxy)thiophene-2-carboxylate and pyridine-4-boronic acid by a procedure analogous to Example 10. MS (ESI): 524.2 [M+H]⁺.

### Step B -5-(5-Pyridin-4-yl-1H-benzimidazol-1-yl)-3-({(1R)-1-[2-(trifluoromethyl)phenyl]ethyl}oxy)thiophene-2-carboxamide

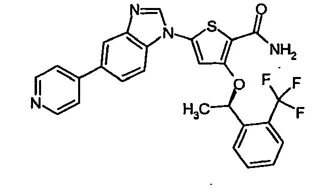

The title compound was prepared from methyl 5-(5-pyridin-4-yl-1*H-*benzimidazol-1-yl)-3-({(1*R*)-1-[2-(trifluoromethyl)phenyl]ethyl}oxy)thiophene-2-carboxylate by a procedure analogous to Example 5, Step D. MS (ESI): 509.2 [M+H]⁺.

### Step C - 5-[5-(1-oxidopyridin-4yl)-1H-benzimidazol-1-yl]-3-({(1R)-1-[2-(trifluoromethyl)phenyl]ethyl}oxy)thiophene-2-carboxamide (Title Compound)

5-(5-pyridin-4-yl-1*H*-benzimidazol-1-yl)-3-({(1*R*)-1-[2-(trifluoromethyl)phenyl]-ethyl}oxy)thiophene-2-carboxamide (0.230 g, 0.45 mmol) and 77% *m-*chloroperoxybenzoic acid (0.39 g, 2.3 mmol) were combined in dichloromethane (20 mL). The reaction mixture was stirred under nitrogen for 2 h, after which time the reaction was quenched with aqueous saturated sodium bicarbonate and extracted three times with dichloromethane. The organics were combined, dried over magnesium sulfate, filtered and concentrated onto silica gel. Purification by column chromatography (0 to 90% 1/9/90 ammonium hydroxide/methanol/ dichloromethane: dichloromethane) provided 0.170 g (72%) of the title compound as a tan solid. ¹H NMR (400 MHz, CDCl₃): δ 8.26 (d, 2H, *J*= 7.3 Hz), 8.01 (s, 1H), 7.98 (s, 1H), 7.71 (m, 2H), 7.63 (t, 1H, *J*= 7.6 Hz), 7.55 (s, 1H), 7.53 (s, 3H), 7.47 (t, 1H, *J*= 7.6 Hz), 7.19 (br s, 1H), 6.67 (s, 1H), 5.85 (q, 1H, *J*= 6.4 Hz), 5.80 (br s, 1H), 1.80 (d, 3H, *J*= 6.4 Hz); MS (ESI): 525.2 [M+H]⁺.

### Example 64: 5-[5-(2-Oxo-1,2-dihydropyridin-4-yl)-1H-benzimidazol-1-yl]-3-({[(1R)-1-[2-(trifluoromethyl)phenyl]ethyl}oxy)thiophene-2-carboxamide

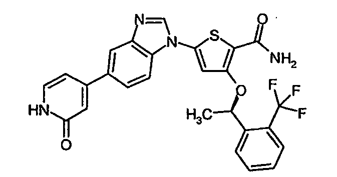

5-[5-(1-oxidopyridin-4-yl)-1*H*-benzimidazol-1-yl]-3-({(1*R*)-1-[2-(trifluoromethyl)phenyl]ethyl}oxy)thiophene-2-carboxamide (0.145 g, 0.276 mmol) and acetic anhydride (5 mL) were combined and heated at reflux overnight. The reaction mixture was cooled and concentrated *in vacuo.* The black residue was diluted with methanol and made basic by the addition of solid KOH. The reaction mixture was stirred at room temperature for 2 h after which time the reaction was acidified with 10% aqueous HCl and extracted three times with dichloromethane. The organic fractions were combined, dried over magnesium sulfate, filtered and concentrated onto silica gel. Purification by column chromatography (0 to 20% methanol:dichloromethane) provided 0.023 g (16%) of the title compound as an off-white solid. ¹H NMR (400 MHz, CDCl₃): δ 12.63 (br s, 1H), 8.05 (s, 1H), 7.99 (s, 1H), 7.72 (dd, 2H, *J*= 8.2, 11.7 Hz), 7.65 (t, 1H, *J*= 7.9 Hz), 7.56 (m, 1H), 7.47 (m, 3H), 7.23 (br s, 1H), 6.84 (s, 1H), 6.69 (s, 1H), 6.62 (d, 1H, *J*= 6.9 Hz), 6.33 (br s, 1H), 5.86 (q, 1H, *J*= 6.1 Hz), 1.82 (d, 3H, *J*= 6.2 Hz); MS (ESI): 525.2 [M+H]⁺.

### Example 65: 5-(5-{2-[(1-Methylpiperidin-4-yl)amino]pyridin-4-yl}-1H-benzimidazol-1-yl)-3-({(1R)-1-[2-(trifluoromethyl)phenyl]ethyl}oxy)thiophene-2-carboxamide

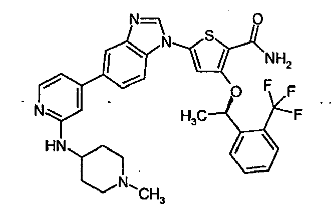

The title compound was prepared from fluoropyridin-4-yl)-1*H*-benzimidazol-1-yl]-3-({(1*R*)-1-[2-(trifluoromethyl)phenyl]ethyl}oxy)thiophene-2-carboxamide and 4-amino-1-methyl-piperidine by a procedure analogous to **Example 62**, with the exception that the reaction mixture was heated in the microwave at 180 °C for 80 minutes. ¹H NMR (400 MHz, CDCl₃): δ 8.13 (d, 1H, *J*= 5.3 Hz), 8.02 (d, 1H, *J*= 1.1 Hz), 7.96 (s, 1H), 7.72 (m, 2H), 7.64 (t, 1H, *J*= 7.6 Hz), 7.56 (dd, 1H, *J*= 1.5, 8.6 Hz), 7.48 (m, 2H), 7.21 (br s, 1H), 6.82 (dd, 1H, *J*= 1.3, 5.3 Hz), 6.67 (s, 1H), 6.59 (s, 1H), 6.09 (br s, 1H), 5.87 (q, 1H, *J*= 6.2 Hz), 4.53 (d, 1H, *J*= 8.1 Hz), 3.73 (m, 1H), 2.84 (d, 2H, *J*= 10.3 Hz), 2.32 (s, 3H), 2.21 (t, 2H, *J*= 10.9 Hz), 2.12 (d, 2H, *J*= 13.0 Hz), 1.81 (d, 3H, *J*= 6.2 Hz), 1.60 (m, 2H); MS (ESI): 621.1 [M+H]⁺.

### Example 66: 5-[5-(2-Piperazin-1-ylpyrimidin-4-yl)-1H-benzimidazol-1-yl]-3-{(1R)-1-[2-(trifluoromethyl)phenyl]ethoxy}thiophene-2-carboxamide

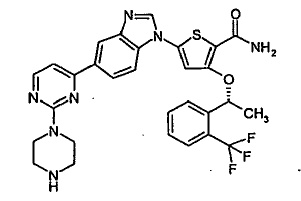

### Step A - Methy/5-[(4-acety/-2-nitrophenyl)amino]-3-{(1R)-1-[2-(trifluoromethyl)phenyl]ethoxy}thiophene-2-carboxy/ate

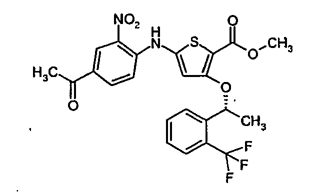

The title compound was prepared from methyl 5-amino-3-{(1*R*)-1-[2-(trifluoromethyl)phenyl]ethoxy}thiophene-2-carboxylate and 4-bromo-3-nitroacetophenone by a procedure analogous to Example 1, Step B. ¹H NMR (400 MHz, DMSO-*d₆*): δ 9.99 (s,1H), 8.55 (d, 1H, *J*= 2.0 Hz), 7.99 (dd, 1H, *J* = 2.1, 8.9 Hz), 7.88 (d, 1H, *J*= 7.9 Hz), 7.75 (d, 1H, *J*=7.7 Hz), 7.71 (d, 1H, *J* = 7.3 Hz), 7.52 (t, 1H, *J*= 7.6 Hz), 7.28 (d, *J* = 9.0 Hz), 6.72 (s, 1H), 5. 76 (q, 1H, *J*= 6.0 Hz), 3.75 (s, 3H), 2.54 (s, 3H), 1.58 (d, 3H, *J*= 6.2 Hz).

### Step B - Methyl 5-[(4-acetyl-2-aminophenyl)amino]-3-{(1R)-1-[2-(trifluoromethyl)phenyl]ethoxy}thiophene-2-carboxylate

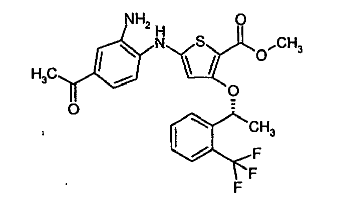

The title compound was prepared from methyl 5-[(4-acetyl-2-nitrophenyl)amino]-3-{(1*R*)-1-[2-(trifluoromethyl)phenyl]ethoxy}thiophene-2-carboxylate (4.0 g, 7.87 mmol) dissolved in acetic acid (50 mL, 873 mmol) by adding iron (2.5 g, 44.8 mmol) and heating at 50°C for 16 h. The reaction was poured over 1:1 ice:water (500 mL), neutralized with saturated aqueous sodium bicarbonate solution and extracted with dichloromethane (3 x 250 mL). The combined extracts were filtered and concentrated *in vacuo* to give 3.45 g (92%) the title compound. ¹H NMR (400 MHz, DMSO-*d₆*): δ 8.80 (s, 1H), 7.81 (d, 1H, *J*= 8.1 Hz), 7.73-7.69 (m, 2H), 7.50 (t, 1H, *J*= 7.7 Hz), 7.27 (d, 1H, *J*= 2.0 Hz), 7.12 (dd, 1H, *J*= 1.9, 8.3 Hz), 7.06 (d, 1H, *J*= 8.4 Hz), 5.65 (q, 1H, *J*= 6.0 Hz), 5.09 (s, 2H), 3.67 (s, 3H), 2.43 (s, 3H), 1.55 (d, 3H, *J* = 6.2 Hz).

### Step C - Methyl 5-(5-acetyl-1H -benzimidazol-1-yl)-3-{(1R)-1-[2-(trifluoromethyl)phenyl]ethoxy}thiophene-2-carboxylate

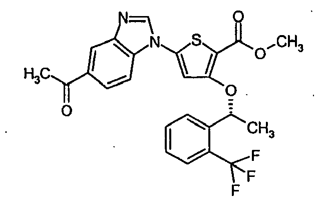

The title compound was prepared from methyl 5-[(4-acetyl-2-aminophenyl)amino]-3-{(1*R*)-1-[2-(trifluoromethyl)phenyl]ethoxy}thiophene-2-carboxylate by a procedure analogous to **Example 1**, Step D. ¹H NMR (400 MHz, DMSO-*d₆*): δ 8.74 (s, 1H), 8.4 (d, 1H, *J* = 1.1 Hz), 7.98-7.94 (m, 2H), 7.76 (t, 1H, *J*= 7.6 Hz), 7.71 (d, 1H, *J*= 7.9 Hz), 7.68 (d, 1H, *J*= 8.6 Hz), 7.52 (t, 1H, *J*= 7.6 Hz), 7.43 (s, 1H), 5.96 (q, 1H, *J*= 6.1 Hz), 3.81 (s, 3H), 2.64 (s, 3H), 1.63 (d, 3H, *J*= 6.2 Hz).

### Step D - 5-(5-Acetyl-1H-benzimidazol-1-yl)-3-{(1R)-1-[2 (trifluoromethyl)phenyl]ethoxy}thiophene-2-carboxamide

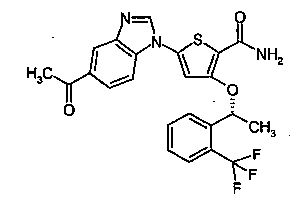

The title compound was prepared methyl 5-(5-acetyl-1*H*-benzimidazol-1-yl)-3-{(1*R*)-1-[2-(trifluoromethyl)phenyl]ethoxy}thiophene-2-carboxylate by a procedure analogous to **Example 5**, Step D. ¹H NMR (400 MHz, DMSO-*d₆*): δ 8.65 (s, 1H), 8.39 (d, 1H, *J*= 1.1 Hz), 7.94-7.91 (m, 2H), 7.84 (s, 1H), 7.77-7.74 (m, 2H), 7.57-7.52 (m, 2H), 7.15 (s, 1H), 7.12 (s, 1H), 5.93 (q, 1H, *J*= 6.2 Hz), 2.64 (s, 3H), 1.73 (d, 3H, *J=* 6.2 Hz).

### Step E - 5-{5-[(2E)-3-(Dimethylamino)prop-2-enoyl]-1H-benzimidazol-1-yl}-3-{(1R)-1-[2(trifluoromethyl)phenyl]ethoxy}thiophene-2-carboxamide

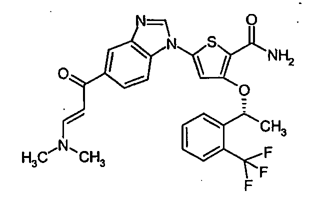

To s stirring solution of 5-(5-acetyl-1*H*-benzimidazol-1-yl)-3-{(1*R*)-1-(2-(trifluoromethyl)phenyl]ethoxy}thiophene-2-carboxamide (0.48 g, 1.0 mmol) in *N,N*-dimethylformamide (10 mL) was added *N,N*-dimethylformamide-di-*tert-*butyl acetal (0.50 g, 2.46 mmol). The mixture was heated for 2.5 h at 100°C. Additional *N,N*-dimethylformamide-di-*tert*-butyl acetal (0.35 g,1.7 mmol) was added and the reaction heated for 68 h. The reaction was cooled and poured in 2:1 ethyl acetate:water (75 mL). The separated aqueous-phase was extracted with ethyl acetate (2 x 25 mL). The combined organic phase was washed with brine, filtered, and concentrated *in vacuo* to yield 0.386 g (73%) of the title compound as golden residue. ¹H NMR (400 MHz, DMSO-*d₆*): δ 8.67 (s,1H), 8.62 (s, 1H), 8.37 (s, 1H), 8.18 (d, 1H, *J*= 7.9 Hz), 7.99 (dd, 1H, J = 1.3, 8.5 Hz), 7.8-7.73 (m, 4H), 7.61 (d, 1H, *J*= 8.6 Hz), 7.55 (t,1H, *J*= 7.7 Hz), 7.31 (s, 1H), 6.09-6.05 (m, 1H), 6.01 (d, 2H, *J*=12.2 Hz), 3.19 (s, 1H), 2.99 (s, 1H), 1.67 (d, 3H, *J*=6.2 Hz).

### Step F- 5-[5-(2-Piperazin-1-ylpyrimidin-4-yl)-1H-benzimidazol-1-yl]-3-{(7R)-1-[2-(trifluoromethyl)phenyl]ethoxy}thiophene-2-carboxamide (Title Compound)

A reaction mixture of 5-{5-[(2*E*)-3-(dimethylamino)prop-2-enoyl]-1*H-*benzimidazol-1-yl}-3-{(1*R*)-1-[2-(trifluoromethyl)phenyl]ethoxy}thiophene-2-carboxamide (0.128 g, 0.24 mmol), piperazine-1-carboxamidinamide sulfate (0.157 g, 0.69 mmol) and potassium carbonate (0.104 g, 0.75 mmol) in ethanol (6 mL) was heated to reflux. *N,N*-Dimethylformamide (6 mL) was added to solubilize the reaction and heating was continued at 100 °C for 16 h. The reaction was concentrated *in vacuo* to a oily residue. The residue was dissolved in *N,N*-dimethylformamide and heated at 110 °C for 16 h. The reaction was poured into 2:1 ethyl acetate:water (30mL). After separating the phases, the aqueous phase was extracted with ethyl acetate (2 x 15 mL). The combined organic fractions were washed with brine, filtered, and concentrated *in vacuo* to a crude residue. Purification by column chromatography (0 to 100% 40/9/1 dichloromethane/methanol/ammonia:dichloromethane) provided 0.023g (16%) of the title compound as a canary yellow solid. ¹H NMR (400 MHz, DMSO-*d₆*): δ 8.61 (s, 1H), 8.54 (d, 1H, *J*= 1.1 Hz), 8.40 (d, 1H, *J*= 5.3 Hz), 8.16 (dd, 1H, *J*= 1.5, 8.6 Hz), 7.94 (d, 1H, *J*= 7.7 Hz), 7.85 (s, 1H), 7.79-7.75 (m, 2H), 7.63 (d, 1H, *J*= 8.6 Hz), 7.55 (t, 1H, *J*=7.6 Hz), 7.29 (d, 1H, *J*=5.1 Hz), 7.18 (s, 1H), 7.12 (s, 1H), 5.95 (q, 1H, *J*= 6.0 Hz), 3.75 (t, 4H, *J*= 4.8 Hz), 2.76 (t, 4H, *J*= 4.9 Hz), 1.74 (d, 3H, *J*= 6.2 Hz), 1.21 (s, 1H); MS (ESI): 594.28 [M+H]⁺.

### Example 67: 3-{[(2-Chloro-3-pyridinyl)methyl]oxy}-5-{6-[(1-methyl-4-piperidinyl)oxyl-1H-benzimidazol-1-yl}-2-thiophenecarboxamide

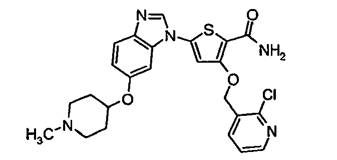

### Step A - 1,1-Dimethylethyl 4-[(1-{4-{[(2-chloro-3-pyridinyl)methyl]oxy}-5-[(methy/oxy)carbonyl]-2-thienyl}-1H-benzimidazo/-6-yl)oxy]-1-piperidinecarboxylate

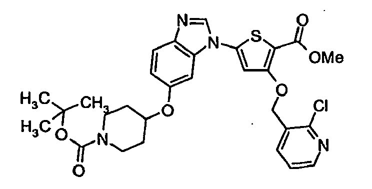

The title compound was prepared from 1,1-dimethylethyl 4-[(1-{4-hydroxy-5-[(methyloxy)carbonyl]-2-thienyl}-1*H*-benzimidazol-6-yl)oxy]-1-piperidinecarboxylate and (2-chloro-3-pyridinyl)methanol by a procedure analogous to Intermediate **Example 1**, Step A. MS (ESI): 599 [M+H]⁺.

### Step B - 1,1-Dimethylethyl 4-{[1-(5-(aminocarbonyl)-4-{[(2-chloro-3-pyridinyl)methy]oxy}-2-thienyl)-1H-benzimidazol-6-yl]oxy}-1-piperidinecarboxylate

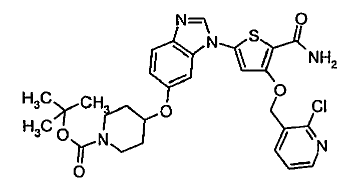

The title compound was prepared from 1,1-dimethylethyl 4-[(1-{4-{[(2-chloro-3-pyridinyl)methyl]oxy}-5-[(methyloxy)carbonyl]-2-thienyl}-1*H*-benzimidazol-6-yl)oxy]-1-piperidinecarboxylate by a procedure analogous to **Example 5**, Step D. MS (ESI): 584 [M+H]⁺.

### Step C - 3-{[(2-Chloro-3-pyridinyl)methyl]oxy}-5-[6-(4-piperidinyloxy)-1H-benzimidazol-1-yl]-2-thiophenecarboxamide

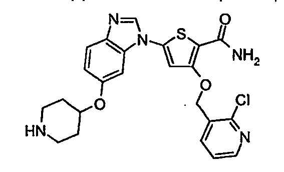

The title compound was prepared from 1,1-dimethylethyl 4-{[1-(5-(aminocarbonyl)-4-{[(2-chloro-3-pyridinyl)methyl]oxy}-2-thienyl)-1*H-*benzimidazol-6-yl]oxy}-1-piperidinecarboxylate by a procedure analogous to **Example 20**, Step B. MS (ESI): 484 [M+H]⁺.

### Step D - 3-{[(2-Chloro-3-pyridinyl)methyl]oxy)-5-{6-[(1-methyl-4-piperidinyl)oxy]-1H-benzimidazol-1-yl}-2-thiophenecarboxamide (Title Compound)

The title compound was prepared from 3-{[(2-chloro-3-pyridinyl)methyl]oxy}-5-[6-(4-piperidinyloxy)-1*H*-benzimidazol-1-yl]-2-thiophenecarboxamide by a procedure analogous to **Example 21**, Step A. ¹H NMR (400 MHz, DMSO-*d₆*): δ 8.47 (s, 1H), 8.43 (dd, 1H, *J*= 1.9, 4.9 Hz), 8.09 (dd, 1H, *J*= 1.8, 7.6 Hz), 7.73 (s, 1H), 7.66-7.62 (m, 2H), 7.51 (m, 1H), 7.22 (d, 1H, *J*= 2.3 Hz), 7.01-6.95 (m, 2H), 5.47 (s, 2H), 4.41 (m, 1H), 2.58 (m, 2H), 2.11 (m, 5H), 1.92 (m, 2H), 1.65 (m, 2H); MS (ESI): 498 [M+H]⁺.

### Intermediate Example 9: (1S)-1-(2-chloro-3-nitrophenyl)ethanol

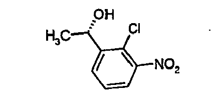

The enantiomers of the literature-known 1-(2-chloro-3-nitrophenyl)ethanol were separated using packed column supercritical fluid chromatography (SFC) on a 3 x 25cm Daicel® AD-H column with a 90 g/min total flow (81 g/min CO₂-90%) (9 g/min MeOH-10%) to give the title compound as a yellow oil. ¹H NMR (400 MHz, DMSO-*d₆*): δ 7.88-7.85 (m, 2H), 7.58 (t, 1H, *J*= 8.0 Hz), 5.62 (d, 1H, *J*= 4.0 Hz), 5.07-5.04 (m, 1H), 1.30 (d, 3H, *J*= 6.4 Hz).

### Example 68: 3-[(1R)-1-(3-Amino-2-chlorophenyl)ethoxy]-5-[5-(1-methyl-1H-pyrazol-4-yl)-1H-benzimidazol-1-yl]thiophene-2-carboxamide

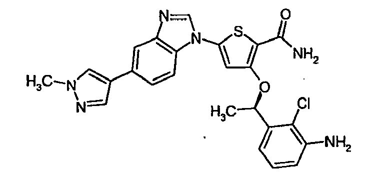

### Step A - Methyl 3-[(1R)-1-(2-chloro-3-nitrophenyl)ethoxy]-5-[5-(1-methy/-1H-pyrazol-4-yl)-1H-benzimidazo/-1-yl]thiophene-2-carboxylate

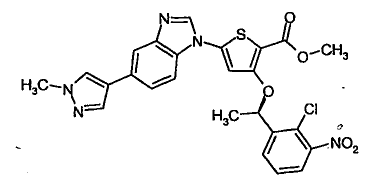

To a solution of methyl 3-hydroxy-5-[5-(1-methyl-1*H*-pyrazol-4-yl)-1*H-*benzimidazol-1-yl]thiophene-2-carboxylate (485 mg, 1.4 mmol) in methylene chloride (14 mL) was added (1*S*)-1-(2-chloro-3-nitrophenyl)ethanol (310 mg, 1.5 mmol), triphenylphosphine (734 mg, 2.8 mmol) and di-*tert* butylazodicarboxylate (645 mg, 2.8 mmol). After 16 h, the reaction was concentrated onto silica and purified by column chromatography (0 to 100% 5/95 methanol/dichloromethane:dichloromethane) to give the title compound as a yellow foam (592 mg, 79%). ¹H NMR (400 MHz, DMSO-*d₆*): δ 8.65 (s, 1H), 8.19 (s, 1H), 8.07-8.05 (m, 1H), 8.01 (dd, 1H, *J*= 1.2, 8.0 Hz), 7.97 (s, 1H), 7.93 (s, 1H), 7.73-7.68 (m, 2H), 6.07 (m, 1H), 3:86 (s, 3H), 3.83 (s, 3H), 1.66 (d, 3H, *J*= 6.0 Hz).

### Step B - 3-{[(1R)-1-(2-Chloro-3-nitrophenyl)ethyl]oxy}-5-[5-(1-methyl-1H-pyrazol-4-yl)-1H-benzimidazol-1-yl]-2-thiophenecarboxamide

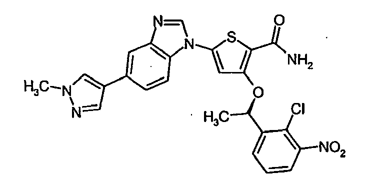

The title compound was prepared as a yellow foam from methyl 3-[(1*R)-1-(2-*chloro-3-nitrophenyl)ethoxy]-5-[5-(1-methyl-1*H*-pyrazol-4-yl)-1*H-*benzimidazol-1-yl]thiophene-2-carboxylate (382 mg, 1 mmol) by a procedure analogous to **Example 5**, Step D. ¹H NMR (400 MHz, DMSO-*d₆*): δ 8.52 (s, 1H), 8.16 (s, 1H), 8.01-7.97 (m, 2H), 7.93 (s, 1H), 7.89 (s, 1H), 7.81 (br s, 1H), 7.67 (t, 1H, *J*= 8.0 Hz), 7.57-7.51 (m, 2H), 7.22 (s, 1H), 7.13 (br s,1H), 6.05 (m, 1H), 3.84 (s, 3H), 1.74 (d, 3H, *J*= 6.4 Hz).

### Step C - 3-{[(1R)-1-(3-amino-2-chlorophenyl)ethyl]oxy}-5-[5-(1-methyl-1H-pyrazol-4-yl)-1H-benzimidazol-1-yl]-2-thiophenecarboxamide (Title Compound)

To a solution of 3-{[(1*R*)-1-(2-chloro-3-nitrophenyl)ethyl]oxy}-5-[5-(1-methyl-1*H*-pyrazol-4-yl)-1*H*-benzimidazol-1-yl]-2-thiophenecarboxamide (520 mg, 1.0 mmol) in acetic acid (3 mL) was added iron powder (279 mg, 5.0 mmol) and the reaction was heated at 60°C. When the starting material was consumed, the reaction was diluted with ethyl acetate and neutralized with a 50% weight solution of aqueous sodium hydroxide. The mixture was filtered through Celite, rinsing well with ethyl acetate. The organic phase was concentrated and purified by column chromatography to give 328 mg (76%) of the title compound as a white solid. ¹H NMR (400 MHz, DMSO-*d₆*) δ 8.52 (s, 1H), 8.16 (s, 1H), 7.92 (d, 2H, *J*= 7.2 Hz), 7.77 (br s, 1H), 7.57 (d, 1H, *J*= 8.4 Hz), 7.41 (d, 1H, *J*= 8.8 Hz), 7.08-7.02 (m, 3H), 6.74 (m, 1H), 5.89 (m, 1H), 5.49 (s, 2H), 3.84 (s, 3H), 1.66 (d, 3H, *J*= 6.0 Hz); MS (ESI): 493 [M+H]⁺.

### Intermediate Example 10: (1S)-1-(2-chloro-3-{[(1,1-dimethylethyl)(dimethyl)-silyl]-oxy}phenyl)ethanol

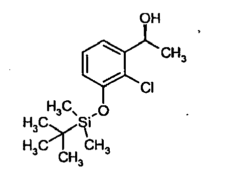

### Step A - 1-(2-Chloro-3-{[(1,1-dimethylethyl)(dimethyl)silyl]oxy}phenyl)-ethanone

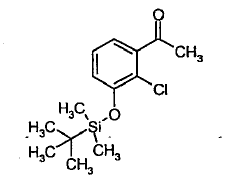

To a solution of 1-(2-chloro-3-hydroxyphenyl)ethanone (8.4 g, 50 mmol) and imidazole (3.8 g, 55 mmol) in dichloromethane (100 mL) was added chloro(tert-butyl)dimethylsilane (8.3 g, 55 mmol). The solution was stirred 1 h and silica gel (20 g) was added. The volatiles were evaporated under reduced pressure, and the residue was purified by flash column chromatography (0 to 10% ethyl acetate:hexanes) to give 7.1 g (50%) of the title compound. ¹H NMR (400 MHz, CDCl₃): δ 7.16 (dd, 1H, *J*= 7.7, 8.0 Hz), 7.04 (dd, 1H, *J*= 1.5, 7.7 Hz), 6.96 (dd, 1H, *J*= 1.5, 8.0 Hz), 2.60 (s, 3H), 1.02 (s, 9H), 0.23 (s, 6H).

### Step B - (1S)-1-(2-Chloro-3-{[(1,1-dimethylethyl)(dimethyl)sily]oxy}phenyl)-ethanol (Title Compound)

To a solution of borane-dimethylsulfide complex (1.8 mL, 30 mmol) in tetrahydrofuran (10 mL) was added a 1 M solution of (*R*)-1-methyl-3,3-diphenyltetrahydro-3H-pyrrolo[1,2-c][1,3,2]oxazaborole in toluene (0.25 mL, 0.25 mmol). To this mixture was slowly added over 2 h a solution of 1-(2-chloro-3-{[(1,1-dimethylethyl)(dimethyl)silyl]oxy}phenyl)ethanone from Step A in tetrahydrofuran (50 mL). The solution was stirred an additional 18 h, then methanol was added dropwise to quench any excess borane. The volatiles were evaporated under reduced pressure, and dichloromethane was added (50 mL). The resulting white solid was removed by filtration and the silica gel was added to the filtrate. The volatiles were evaporated under reduced pressure and the residue was purified by flash column chromatography (0 to 20% ethyl acetate:hexanes) to give 6.8 g of the title compound as a white solid. ¹H NMR (400 MHz, CDCl₃): δ 7.19-7.12 (m, 2H), 6.81-6.79 (m,1H), 5.30-5.25 (m, 1H), 1.93 (d, 1H, *J*= 3.6 Hz) 1.47 (d, 3H, *J*= 6.4 Hz), 1.02 (s, 9H), 0.21 (s, 3H), 0.21 (s, 3H).

### Example 69: 3-{[(1R)-1-(2-chloro-3-hydroxyphenyl)ethyl]oxy)-5-[5-(1-methyl-1H-pyrazol-4-yl)-1H-benzimidazol-1-yl]-2-thiophenecarboxamide

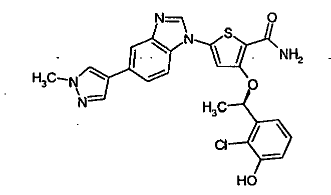

### Step A - methyl 5-[5-(1-methyl-1H-pyrazo/-4-yl)-1H-benzimidazol-1-yl]-3-[(phenylmethyl)oxy]-2-thiophenecarboxylate

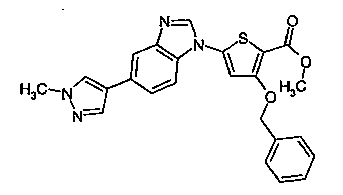

To a solution of methyl 5-(5-bromo-1*H*-benzimidazol-1-yl)-3-[(phenylmethyl)oxy]-2-thiophenecarboxylate (2.8 g, 6.3 mmol) in *N,N-*dimethylacetamide (60 mL) and 1 N aqueous sodium carbonate (20 mL) was added 1-methyl-4-(4,4,5,5-tetramethyl-1,3,2-dioxaborolan-2-yl)-1*H*-pyrazole (1.6 g, 7.5 mmol), followed by 1,1'-bis diphenylphosphinoferracene dichloropalladium(II) (0.60 g, 0.75 mmol), and the reaction mixture was heated to 80°C for 1 h. The solution was filtered, cooled to room temperature, diluted with ethyl acetate (250 mL) and washed with water (3 x 200 mL). The organic layer was dried over magnesium sulfate, filtered, and silica gel (10 g) was added. The volatiles were evaporated under reduced pressure, and the residue was purified by flash column chromatography (0 to 100% 90/10/1 dichloromethane/methanol/ammonium hydroxide:dichloromethane) to give 1.6 g (57%) of the title compound. ¹H NMR (400 MHz, CDCl₃): δ 8.03 (s, 1H), 7.90 (s, 1H), 7.79 (s, 1H), 7.64 (s, 1H), 7.54-7.32 (m, 7H), 6.88 (s, 1H), 5.32 (s, 2H), 3.96 (s, 3H), 3.90 (s, 3H).

### Step B - methyl 3-hydroxy-5-[5-(1-methyl-1H-pyrazol-4-yl)-1H-benzimidazol-1-yl]-2-thiophenecarboxylate

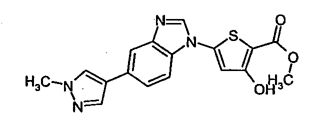

To methyl 5-[5-(1-methyl-1*H*-pyrazol-3-yl)-1*H*-benzimidazol-1-yl]-3-[(phenylmethyl)oxy]-2-thiophenecarboxylate-(1.6g, 3.6 mmol) was added trifluoroacetic acid (20 mL) and the mixture was stirred at room temperature for 18 h. The solution was concentrated to give an oil and dichloromethane (20 mL) was added resulting in the precipitation of a solid. The acid was neutralized by addition of 7 N ammonia in methanol and the solution diluted with dichloromethane and methanol so that all the solid dissolved. Silica gel (10 g) was added and the volatiles were evaporated under reduced pressure. The residue was purified by flash column chromatography (0 to 100% 90/10/1 dichloromethane/methanol/ammonium hydroxide:dichloromethane) to give 1.3 g (100%) of the title compound as a white solid. ¹H NMR (400 MHz, DMSO-*d₆*): δ 8.64 (s, 1H), 8.17 (s, 1H), 7.96 (d, 1H, *J* =1.1 Hz), 7.91 (d, 1H, J = 0.7 Hz), 7.74 (d, 1H, *J*=8.4 Hz), 7.60 (dd, 1 H, *J*=1.7, 8.4 Hz), 7.11 (s, 1 H), 3.84 (s, 3H), 3.76 (s, 3H).

### Step C - methyl3-{[(1R)-1-(2-chloro-3-{[(1,1-dimethylethyl)(dimethyl)silyl]oxy}-phenyl)ethyl]oxy}-5-[5-(1-methyl-1H-pyrazo/-4-yl}-1H-benzimidazol-1-yl]-2-thiophenecarboxylate

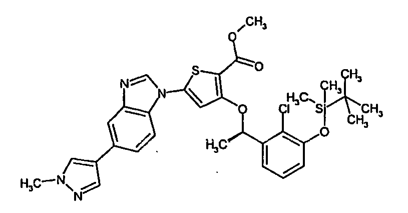

To a slurry of methyl 3-hydroxy-5-[5-(1-methyl-1*H*-pyrazol-3-yl)-1*H-*benzimidazol-1-yl]-2-thiophenecarboxylate (0.71 g, 2.0 mmol) and (15)-1-(2-chloro-3-{[(1,1-dimethylethyl)(dimethyl)silyl]oxy}phenyl)ethanol (0.63 g, 2.2 mmol) in dichloromethane (20 mL) was added triphenylphosphine (1.1 g, 4.0 mmol) and di-*tert*-butylazodicarboxylate (0.92 g, 4.0 mmol). The clear, yellow solution was stirred for 1 h, then silica gel (5 g) was added. The volatiles were evaporated under reduced pressure and the residue was purified by flash column chromatography (0 to 100% 90/10/1 dichloromethane/methanol/ammonium hydroxide:dichloromethane) to give 1.1 g (1.8 mmol) of the title compound as a white solid: ¹H NMR (400 MHz, CDCl₃): δ 7.98 (s, 1H), 7.87 (s, 1H), 7.78 (s, 1H), 7.64 (s, 1H), 7.46-7.44 (m, 2H), 7.26-7.23 (m, 1H), 7.16 (dd, 1H, *J*= 7.8, 7.9 Hz), 6.85-6.83 (m, 1H), 5.82 (q, 1H, *J*= 6.3 Hz), 3.96 (s, 3H), 3.91 (s, 3H), 1.72 (d, 3H, *J*= 6.3 Hz), 1.01 (s, 9H), 0.21 (s, 3H), 0.19 (s, 3H).

### Step D - Methyl 3-{[(1R)-1-(2-chloro-3-hydroxyphenyl)ethyl]oxy}-5-[5(1-methyl-1H-pyrazo/-4-yl)-1H-benzimidazo/-1-yl]-2-thiophenecarboxylate

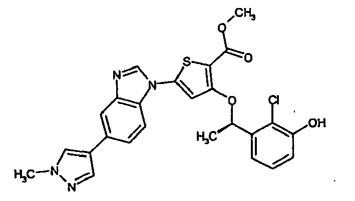

To a solution of methyl 3-{[(1*R*)-1-(2-chloro-3-{[(1,1-dimethylethyl)(dimethyl)silyl]oxy}phenyl)ethyl]oxy}-5-[5-(1-methyl-1*H-*pyrazol-4-yl)-1*H*-benzimidazol-1-yl]-2-thiophenecarboxylate (0.72 g, 1.2 mmol) in tetrahydrofuran (5 mL) was added a solution of 1 N tetrabutylamonium fluoride in tetrahydrofuran (1.4 mL, 1.4 mmol). After 10 min, silica (5 g) was added, the volatiles were evaporated under reduced pressure and the residue was purified by flash column chromatography (0 to 100% 80/20/1 dichloromethane/methanol/ammonium hydroxide:dichloromethane) to give 0.53 g (83%) of the title compound as a light yellow foam. ¹H NMR (400 MHz, CDCl₃): δ 7.97 (s, 1H), 7.87 (s, 1H), 7.78 (s, 1H), 7.63 (s, 1H), 7.46-7.44 (m, 1H), 7.36 (d, 1H, *J*= 7.8 Hz), 7.24-7.20 (m, 2H), 7.01-6.97 (m, 1H), 6.64 (s, 1H) 5.73 (q, 1H, *J*= 6.4 Hz), 3.95 (s, 3H), 3.91 (s, 3H), 1.73 (d, 3H, *J*= 6.4 Hz).

### Step E - 3-{[(1R)-1-(2-Chloro-3-hydroxyphenyl)ethyl]oxy}-5-[5(1-methyl-1H-pyrazol-4-yl)-1H-benzimidazol-1-yl]-2-thiophenecarboxamide (Title compound)

To methyl 3-{[(1*R*)-1-(2-chloro-3-hydroxyphenyl)ethyl]oxy}-5-[5-(1-methyl-1*H-*pyrazol-4-yl)-1*H*-benzimidazol-1-yl]-2-thiophenecarboxylate (0.25 g, 0.050 mmol) was added a solution of 7 N ammonia in methanol (10 mL). The mixture was heated in a sealed tube to 80 °C for 40 h and then cooled to room temperature. Diethyl ether was added (20 mL) and the resulting white solid collected by vacuum filtration. The solid was washed with diethyl ether to give 0.012 g (48%) of the title compound as a white solid. ¹H NMR (400 MHz, CDCl₃): δ 8.02 (s, 1H), 7.94 (s, 1H), 7.84 (s, 1H), 7.74 (s, 1H), 7.60 (s, 1H), 7.44-7.35 (m, 2H), 7.21-7.17 (m, 2H), 7.03-6.97 (m, 2H), 6.59 (s, 1H) 5.92 (br s, 1H), 5.81 (q, 1H, *J*= 6.4 Hz), 3.91 (s, 3H), 1.76 (d, 3H, *J*= 6.4 Hz); MS (ESI): 494 [M+H]⁺.

### Example 70: 3-{[(1R)-1-(2-chlorophenyl)ethyl]oxy}-5-[6-(2,3-dihydroxypropyl)-1H-benzimidazol-1-yl]-2-thiophenecarboxamide

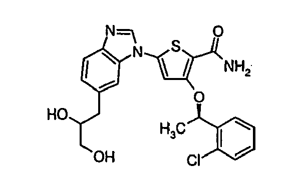

### Step A - methyl 3-{[(1R)-1-(2-chloropheny/)ethy/]oxy}-5-[6-(2-propen-1-y/)-1H benzimidazol-1-yl]-2-thiophenecarboxylate

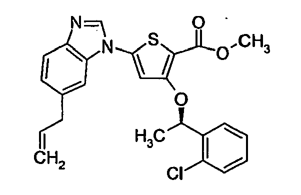

To a stirred solution of 3-{[(1*R*)-1-(2-chlorophenyl)ethyl]oxy}-5-(6-{[(trifluoromethyl)sulfonyl]oxy}-1*H*-benzimidazol-1-yl)-2-thiophenecarboxylate (1.0 g, 1.78 mmol) and lithium chloride (226 mg, 5.33 mmol) in *N,N-*dimethylformamide (5.0 mL) was added allyltributyltin (580 µL, 1.87 mmol), then bis(triphenylphosphine)palladium dichloride (25 mg, 0.035 mmol). The reaction was then heated at 90 °C for 1 h, cooled to room temperature, diluted with ethyl acetate, and poured through Celite, washing with ethyl acetate. The filtrate was washed with water (5x), saturated aqueous sodium chloride, dried over magnesium sulfate, and concentrated under vacuum. Flash chromatography (5 to 40% ethyl acetate:hexanes) afforded 778 mg (96%) of the title compound as a clear yellow oil. MS (ESI): 453 [M+H]⁺.

### Step B - methyl 3-{[(1R)-1-(2-chlorophenyl)ethyl]oxy}-5-[6(2,3-dihydroxypropyl)-1H-benzimidazol-1-yl]-2-thiophenecarboxylate

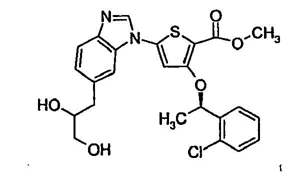

A mixture of methyl 3-{[(1*R*)-1-(2-chlorophenyl)ethyl]oxy}-5-[6-(2-propen-1-yl)-1*H*-benzimidazol-1-yl]-2-thiophenecarboxylate (770 mg, 1.70 mmol)) was stirred in 3:1 acetone:water (17 mL), then 4-methylmorpholine *N*-oxide (240 mg, 2.04 mmol) was added, followed by osmium tetroxide as a 2.5% by weight solution in t-butanol (640 µL, 0.05 mmol). The reaction was stirred 16 h, then quenched with saturated aqueous sodium sulfite solution. The aqueous layer was extracted with ethyl acetate. The combined organic layers were washed with saturated aqueous sodium chloride, dried over magnesium sulfate, and concentrated under vacuum to afford 800 mg (97%) of the crude title compound as a yellow oil. MS (ESI): 487 [M+H]⁺.

### Step C - 3-{[(1R)-1-(2-chlorophenyl)ethyl]oxy}-5-[6-(2,3-dihydroxypropyl)-1H-benzimidazol-1-yl]-2-thiophenecarboxamide (Title Compound)

A mixture of methyl 3-{[(1*R*)-1-(2-chlorophenyl)ethyl]oxy)-5-[6-(2,3-dihydroxypropyl)-1*H*-benzimidazol-1-yl]-2-thiophenecarboxylate (126.0 mg, 0.25 mmol) and 7 N ammonia in methanol (30 mL, 210 mmol) was added to a high-pressure glass reaction flask. The flask was sealed, then heated to 85 °C for ~24 h. The flask was cooled to room temperature, opened, and the reaction mixture concentrated under vacuum. Flash chromatography (0 to 8% methanol:dichloromethane with 1% ammonium hydroxide) afforded 106 mg (87%) of the title compound as an off-white solid. ¹H NMR (400 MHz, DMSO-*d₆*): δ 8.48 (s, 1H), 7.81 (br s, 1H), 7.70-7.61 (m, 2H), 7.54-7.50 (m, 1H), 7.45-7.31 (m, 3H), 7.22-7.18 (m, 1H), 7.13 (br s, 2H), 6.00-5.94 (m, 1H), 4.61-4.54 (m, 2H), 3.66-3.59 (m, 1H), 3.32-3.29 (m, 2H), 2.94-2.85 (m, 1H), 2.70-2.61 (m, 1H), 1.73 (d, 3H, *J*= 6.23 Hz); MS (ESI): 472 [M+H]+.

### Example 71: 3{[(1R)-1-(2-chlorophenyl)ethyl]oxy}-5-{6-[2-(1,3-dioxolan-2-yl)ethyll-1H-benzimidazol-1-yl}-2-thiophenecarboxamide

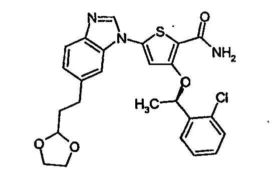

### Step A - methyl 3-{[(1R)-1-(2-chlorophenyl)ethy/]oxy}-5-{6-[2-(1,3-dioxolan-2-yl)ethyl]-1H-benzimidazol-1-yl}-2-thiophenecarboxylate

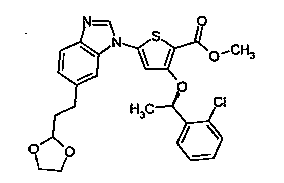

To a mixture of methyl 3-{[(1R)-1-(2-chlorophenyl)ethyl]oxy}-5-(6-{[(trifluoromethyl)sulfonyl]oxy}-1H-benzimidazol-1-yl)thiophenecarboxylate (2.3 g, 4.1 mmol), dichloro[1,1'-bis(diphenylphosphino)ferrocene]palladium (II) dichloromethane adduct (0.40 g, 0.49 mmol), and copper iodide (0.19 g, 1.0 mmol) in tetrahydrofuran (30 mL) which had been purged and refilled under N₂ was added 2-[2-(1,3-dioxolanyl)ethyl]zinc bromide (0.5 M in tetrahydrofuran, 20 mL, 10 mmol) via syringe over 5 min. The reaction was heated at reflux for 1.5 h. The reaction was cooled and diluted with dichloromethane (200 mL). The reaction was quenched with 1:1 saturated aqueous ammonium chloride:water (50 mL) and the phases separated using a separatory funnel. The organic layer was flushed through a Celite pad to remove insoluble material. The pad was washed with 4:1 dichloromethane:methanol. The solution was filtered and concentrated *in vacuo* to a crude residue. Purification by flash column chromatography (0 to 100% ethyl acetate:hexanes) yielded 1.5 g (71 %) of the title compound as a fluffy white solid. ¹H NMR (400 MHz, CDCl₃): δ 8.02 (br s, 1H), 7.74 (d, 1H, *J* = 6.8 Hz), 7.63 (dd, 1H, *J*= 1.6, 7.6), 7.42 (dd, 1H, *J*= 1.3, 7.9 Hz), 7.34-7.25 (m, 2H), 7.22 (s, 1H), 7.20 (s, 1H), 6.68 (s, 1H), 5.81 (q, 1H, *J*= 6.4 Hz), 4.91 (t, 1H, *J*= 4.6 Hz), 4.07-3.98 (m, 2H), 3.91 (s, 3H), 3.89 (t, 2H, *J*= 1.7 Hz), 2.89-2.80 (m, 2H), 2.03-1.93 (m, 2H), 1.74 (d, 3H, *J*= 6.4 Hz).

### Step B - 3-{[(1R)-1-(2-chlorophenyl)ethyl]oxy}-5-{6-[2-(1,3-dioxolan-2-yl)ethyl]-1H-benzimidazol-1-yl}-2-thiophenecarboxamide (Title Compound)

The title compound was prepared from methyl 3-{[(1*R*)-1-(2-chlorophenyl)ethyl]oxy}-5-{6-[2-(1,3-dioxolan-2-yl)ethyl]-1*H*-benzimidazol-1-yl}-2-thiophenecarboxytate by a procedure analogous to **Example 49**, Step C. ¹H NMR (300 MHz, CDCl₃): δ 7.92 (s, 1H), 7.72 (m, 1H), 7.48 (m, 2H), 7.35 (m, 2H), 7.20 (m, 3H), 6.61 (s, 1H), 5.87 (m, 2H), 4.93 (t, 1H, *J*= 4.6 Hz), 4.06-3.88 (m, 4H), 2.85 (m, 2H), 2.00 (m, 2H), 1.78 (d, 3H, *J*= 6.3 Hz); MS (APCI): 498.29 [M+H]⁺.

### Example 72: 3-{[(1R)-1-(2-Chlorophenyl)ethyl]oxy)-5-[6-(3-oxopropyl)-1H-benzimidazol-1-yl]1-2-thiophenecarboxamide

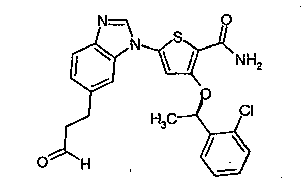

To a solution of 3-{[(1*R*)-1-(2-chlorophenyl)ethyl]oxy}-5-{6-[2-(1,3-dioxolan-2-yl)ethyl]-1*H*-benzimidazol-1-yl}-2-thiophenecarboxamide (710 mg, 1.43 mmol) in 4:1 acetone:water (7.1 mL) was added pyridinium p-toluenesulfonate (36 mg, 0.14 mmol). The reaction stirred for 18 h at room temperature, then 3 h at reflux. At that time, p-toluenesulfonic acid hydrate (53 mg, 0.28 mmol) was added and the reaction was refluxed for another 24 h. The reaction was quenched by pouring into 1:1 water:saturated aqueous sodium bicarbonate (50 mL). The mixture was extracted with dichloromethane (2 x 20 mL). The combined organic fractions were dried over sodium sulfate, filtered, and concentrated onto silica gel. Purification by column chromatography (0 to 7% methanol:chloroform) provided 500 mg (77%) of the title compound. ¹H NMR (300 MHz, CDCl₃): δ 9.85 (t, 1H, *J*= 1.3 Hz), 7.93 (s, 1H), 7.73 (d, 1H, *J*= 7.9 Hz), 7.46 (m, 2H), 7.33 (m, 2H), 7.19 (m, 3H), 6.62 (s, 1H), 5.88 (q, 1H, *J*= 6.2 Hz), 5.84 (br s, 1H), 3.05 (m, 2H), 2.80 (m, 2H), 1.78 (d, 3H, *J*= 6.5 Hz); . MS(APCI): 454.22 [M+H]⁺.

### Example 73: 3-{[(1R)-1-(2-Chlorophenyl)ethyl]oxy}-5-[6-(2-{[2-(methylsulfonyl)ethyl]amino}ethyl)-1H-benzimidazol-1-yl]-2-thiophenecarboxamide

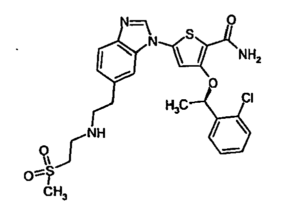

### Step A - 3-{[(1R)-1-(2-chlorophenyl)ethyl]oxy}-5-[6-(2-oxoethy/)-1H-benzimidazol-1-yl]-2-thiophenecarboxamide

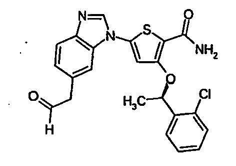

To a solution of 3-{[(1*R*)-1-(2-chlorophenyl)ethyl]oxy}-5-[6-(2,3-dihydroxypropyl)-1*H*-benzimidazol-1-yl]-2-thiophenecarboxamide (640 mg, 1.36 mmol) in methanol (22 mL) and water (5 mL) was added sodium periodate (349 mg, 1.63 mmol). The reaction was stirred vigorously at room temperature for 24 h. The reaction mixture was then poured into a 1:1 mixture of saturated aqueous sodium chloride and water (125 mL). The aqueous layer was extracted with ethyl acetate (3 x 30 mL). The combined organic fractions were dried over sodium sulfate, filtered, and concentrated to afford 460 mg (77%) of a mixture of the title compound and its mono-methyl hemiacetal, which were carried together crude into the next step. MS (ESI): 472.03 [M+H]⁺ (for hemiacetal).

### Step B - 3-{[(1R)-1-(2-chlorophenyl)ethyl]oxy}-5-[6-(2-{[2-(methylsulfonyl)-ethyl]amino}ethyl)-1H-benzimidazol-1-yl]-2-thiophenecarboxamide (Title Compound)

To a solution of the crude mixture from **Example 72**, Step A above (110 mg, ~0.25 mmol) and 2-(methylsulfonyl)ethanamine (34 mg, 0.28 mmol) in 10:1 1,2-dichloroethane:methanol (2.0 mL) was added acetic acid (14 mL, 0.25 mmol) and sodium triacetoxyborohydride (74 mg, 0.35 mmol). The reaction stirred at room temperature for 16 h and was then poured into saturated aqueous sodium bicarbonate (25 mL). The aqueous layer was extracted with ethyl acetate (2 x 20 mL). The combined organic fractions were dried over sodium sulfate, filtered, and concentrated onto silica gel. Purification by flash column chromatography (0 to 100% 1/10/89 ammonium hydroxide/methanol/chloroform:chloroform) afforded 60 mg (44%) of the title compound. ¹H NMR (300 MHz, CDCl₃): δ 7.93 (s, 1H), 7.74 (d, 1H, *J* = 8.4 Hz), 7.47 (m, 2H), 7.34 (m, 3H), 7.20 (m, 2H), 6.63 (s, 1H), 5.89 (q, 1H *J* = 6.4 Hz), 5.83 (br s, 1H), 3.15 (m, 4H), 2.94 (s, 3H), 2.91 (m, 4H), 1.78 (d, 3H, *J* = 6.3 Hz), 1.63 (br s, 1H); MS (ESI): 547.11 [M+H]⁺.

### Example 74: 1-[5-(aminocarbonyl)-4-({(1R)-1-[2-(trifluoromethyl)phenyl]-ethyl}oxy)-2-thienyl]-1H-benzimidazol-6-yl 2-propanesulfonate

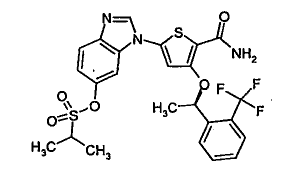

The title compound was prepared from 5-(6-hydroxy-1*H*-benzimidazol-1-yl)-3-({(1*R*)-1-[2-(trifluoromethyl)phenyl]ethyl}oxy)-2-thiophenecarboxamide and 2-propanesulfonyl chloride by a procedure analogous to **Example 30**, Step B. ¹H NMR (400 MHz, CDCl₃): δ 7.95 (s, 1H), 7.81 (d, 1H, *J*= 8.8 Hz), 7.75-7.62 (m, 3H), 7.49 (m, 1H), 7.35 (m, 1H), 7.24 (m, 2H), 6.64 (s, 1H), 5.92 (br s, 1H), 5.86 (q, 1H, *J*= 6.0 Hz), 3.50 (m, 1H), 1.81 (d, 3H, *J*= 6.1 Hz), 1.59 (m, 6H); MS (APCI): 553.95 [M+H]⁺.

### Example 75: 1-[5-(aminocarbonyl)-4({(1R)-1-[2-(trifluoromethyl)phenyl]-ethyl}oxy)-2-thienyl]-1H-benzimidazol-6-yl benzenesulfonate

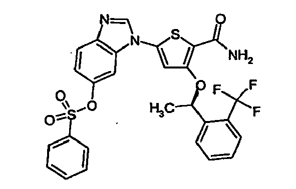

The title compound was prepared from 5-(6-hydroxy-1*H*-benzimidazol-1-yl)-3-({(1*R*)-1-[2-(trifluoromethyl)phenyl]ethyl}oxy)-2-thiophenecarboxamide and benzenesulfonyl chloride by a procedure analogous to **Example 30**, Step B. ¹H NMR (400 MHz, CDCl₃): δ 7.94 (s, 1H), 7.81 (m, 3H), 7.62 (m, 3H), 7.55 (m, 4H), 7.22 (br s, 1H), 7.07 (s, 1H), 6.81 (d, 1H, *J*= 8.2 Hz), 6.61 (s, 1H), 6.11 (br s, 1H), 5.85 (q, 1H, *J*= 5.7 Hz), 1.82 (s, 3H, *J*= 5.5 Hz); MS (ESI): 588.12 [M+H]⁺.

### Example 76: 1-[5-(aminocarbonyl)-4-({(1R)-1-(2-(trifluoromethyl)phenyl]-ethyl}oxy)-2-thienyl]-1H-benzimidazol-6-yl trifluoromethanesulfonate

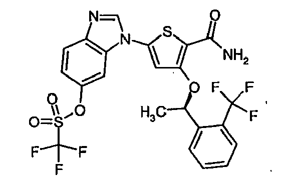

The title compound was prepared from 5-(6-hydroxy-1*H*-benzimidazol-1-yl)-3-({(1*R*)-1-[2-(trifluoromethy)phenyl]ethyl}oxy)-2-thiophenecarboxamide and trifluoromethanesulfonylchloride by a procedure analogous to **Example 30**, Step B. ¹H NMR (400 MHz, CDCl₃): δ 8.05 (s, 1H), 7.85 (d, 1H, *J*= 8.6 Hz), 7.76 (d, 1H, *J*= 7.5 Hz), 7.68 (m, 2H), 7.50 (m, 1H), 7.38-7.25 (m, 3H), 6.68 (s, 1H), 6.53 (br s, 1H), 5.87 (q, 1H, *J*= 5.9 Hz), 1.83 (d, 3H, *J*= 5.9 Hz); MS (APCI): 579.87 [M+H]⁺.

### Example 77: 1-(5-(Aminocarbonyl)-4-{(1R)-1-(2-chlorophenyl)ethyl]oxy}-2-thienyl)-1H-benzimidazol-6-yl methanesulfonate

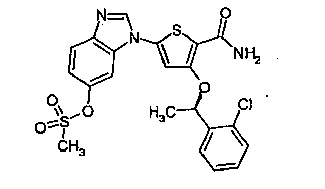

### Step A - 3-{[(1R)-1-(2-chlorophenyl)ethyl]oxy}-5-(6-hydroxy-1H-benzimidazol 1-yl)-2-thiophenecarboxamide

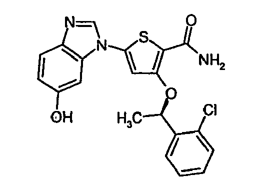

The title compound was prepared from methyl 3-{[(1*R*)-1-(2-chlorophenyl)ethyl]oxyl-5-(6-hydroxy-1*H*-benzimidazol-1-yl)-2-thiophenecarboxylate by a procedure analogous to **Example 30**, Step A. ¹H NMR (300 MHz, DMSO-*d₆*): δ 9.59 (s, 1H), 8.34 (s, 1H), 7.79 (br s, 1H), 7.68 (dd, 1H, *J*= 1.8, 7.5 Hz), 7.55-7.32 (m, 4H), 7.20 (s, 1H), 7.08 (br s, 1H), 7.00 (d, 1H, *J*= 2.0 Hz), 6.80 (dd, 1H, *J*= 2.3, 8.7 Hz), 5.96 (q, 1H, *J*= 6.4 Hz), 1.72 (d, 3H, *J*= 6.5 Hz); MS (APCI): 413.94 [M+H]⁺.

### Step B - 1-(5-(aminocarbonyl)-4-{[(1R)-1-(2-chlorophenyl)ethyl]oxy}-2-thienyl)-1H-benzimidazol-6-yl methanesulfonate (Title Compound)

The title compound was prepared from 3-{[(1*R*)-1-(2-chlorophenyl)ethyl]oxy}-5-(6-hydroxy-1*H*-benzimidazol-1-yl)-2-thiophenecarboxamide by a procedure analogous to **Example 30**, Step B. ¹H NMR (300 MHz, CDCl₃): δ 8.02 (s, 1H), 7.84 (d, 1H, *J*= 8.7 Hz), 7.47 (m, 2H), 7.36 (m, 3H), 7.28 (m, 1H), 7.22 (br s, 1H), 6.64 (s, 1H), 6.10 (br s, 1H), 5.88 (q, 1H, *J*= 6.4 Hz), 3.17 (s, 3H), 1.79 (d, 3H); MS (ESI): 492.14 [M+H]⁺.

### Example 78: 1-[5-(Aminocarbonyl)-4-({(1R)-1-[2-(trifluoromethyl)phenyl]-ethyl}oxy)-2-thienyl]-1H-benzimidazol-6-yl ethanesulfonate

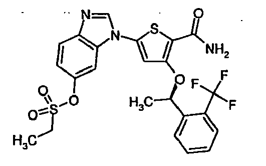

The title compound was prepared from 5-(6-hydroxy-1*H*-benzimidazol-1-yl)-3-({(1*R*)-1-[2-(trifluoromethyl)phenyl]ethyl}oxy)-2-thiophenecarboxamide and ethanesulfonyl chloride by a procedure analogous to **Example 30**, Step B. ¹H NMR (300 MHz, CDCl₃): δ 7.97 (s, 1H), 7.83-7.65 (m, 4H), 7.49 (m, 1H), 7.33 (d, 1H, *J*= 2.0 Hz), 7.27-7.21 (m, 2H), 6.65 (s, 1H), 6.19 (br s, 1 H), 5.86 (q, 1H, *J*= 6.2 Hz), 3.31 (q, 2H, *J*= 7.4 Hz), 1.81 (d, 3H, *J*= 6.3 Hz), 1.57 (t, 3H, *J*= 7.4 Hz); MS (ESI): 540.19 [M+H]⁺.

### Example 79: -1-[5-(Aminocarbonyl)-4-({(1R)-1-[2-(trifluoromethyl)phenyl]-ethyl)oxy)-2-thienyl]-1H-benzimidazol-6-yl 1-propanesulfonate

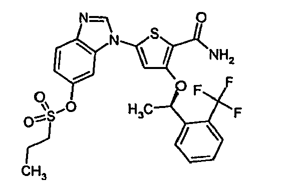

The title compound was prepared from 5-(6-hydroxy-1*H*-benzimidazol-1-yl)-3-({(1*R*)-1-[2-(trifluoromethyl)phenyl]ethyl}oxy)-2-thiophenecarboxamide and 1-propanesulfonyl chloride by a procedure analogous to **Example 30**, Step B. ¹H NMR (300 MHz, CDCl₃): δ 7.97 (s, 1H), 7.83-7.62 (m, 4H), 7.49 (m, 1H), 7.32 (d, 1H, *J*=2.3 Hz), 7.26-7.21 (m, 2H), 6.65 (s, 1H), 6.01 (br s, 1H), 5.86 (q, 1H, *J*= 6.3 Hz), 3.25 (m, 2H), 2.05 (m, 2H), 1.81 (d, 3H, *J*= 6.3 Hz), 1.15 (t, 3H, *J*= 7.4 Hz); MS (ESI): 554.20 [M+H]⁺.

### Example 80: 1-[5-(aminocarbonyl)-4-({(1R)-1-[2-(trifluoromethyl)phenyl]ethyl}oxy)-2-thienyl-1H-benzimidazol-6-yl[3-(dimethylamino)propyl]methylsulfamate formic acid salt

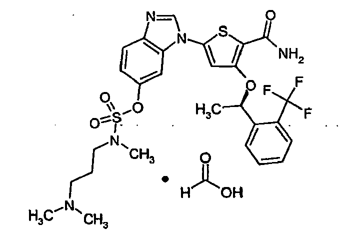

### Step A - [3-(dimethylamino)propyl]methylsulfamoyl chloride

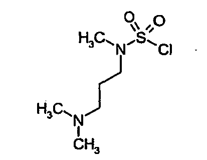

To a cooled (-5 °C) suspension of sulfuryl chloride (1.61 mL, 20.0 mmol) and MP-Carbonate resin (3.82 g, 2.62 mmol/g, 10.0 mmol) in dichloromethane (40 mL) was added a solution of *N*,*N*,*N*'-trimethyl-1,3-propanediamine (1.47 mL, 10.0 mmol) in dichloromethane (5 mL) dropwise via addition funnel over a period of 15 min. Upon completion of the addition, the reaction stirred a further 2 h, maintaining the temperature between -5 °C and 0 °C. The reaction mixture was then filtered through a sintered glass funnel to remove the MP-Carbonate resin, washing with dichloromethane (50 mL). The reaction was concentrated to dryness. The residual material was triturated with ethyl acetate (50 mL), causing precipitation of a solid. The mixture was filtered to collect the solid, which was dried under vacuum to provide 1.25 g (58%) of the crude title compound. ¹H NMR (300 MHz, DMSO-*d₆*): δ 3.36 (t, 2H, *J* = 7.0 Hz), 3.06 (m, 2H), 3.00 (s, 3H), 2.72 (s, 3H), 2.70 (s, 3H), 2.07 (m, 2H).

### Step B - 1-[5-(aminocarbonyl)-4-({(1R)-1-[2-(trifluoromethyl)phenyl]ethyl}oxy)-2-thienyl]-1H-benzimidazol-6-yl[3-(dimethylamino)propyl]methylsulfamate formic acid salt (Title Compound)

To a cooled (0 °C) suspension of 5-(6-hydroxy-1*H*-benzimidazol-1-yl)-3-({(1*R*)-1-[2-(trifluoromethyl)phenyl]ethyl}oxy)-2-thiophenecarboxamide (60 mg, 0.13 mmol) and MP-Carbonate resin (130 mg, 2.62 mmol/g, 0.34 mmol) in dichloromethane (2.7 mL) was added [3-(dimethylamino)propyl]methylsulfamoyl chloride (72 mg, 0.34 mmol). After stirring for 10 min at 0 °C, the reaction was warmed to room temperature and stirred for 24 h. Another portion of MP-Carbonate and [3-(dimethylamino)propyl]methylsulfamoyl chloride were then added in the amounts described above, plus a small amount (~5 mg) of 4-*N,N*-dimethylaminopyridine. After a further 24 h of stirring, another portion of [3-(dimethylamino)propyl]methylsulfamoyl chloride (144 mg, 0.68 mmol) and 4-*N,N*-dimethylaminopyridine (~5 mg) was added. The reaction stirred a further 20 h. The reaction was then filtered to remove the MP-Carbonate resin and concentrated. Purification by flash column chromatography (0 to 10% methanol:chloroform) afforded 68 mg of the crude title compound. The crude material was further purified by preparative HPLC (10 to 90% acetonitrile:water with 0.1 % formic acid) to afford 19 mg (21 %) of the title compound. ¹H NMR (300 MHz, CDCl₃): δ 8.37 (s, 1H), 7.96 (s, 1H), 7.82-7.69 (m, 4H), 7.49 (m, 1H), 7.40 (d, 1H, *J*= 2.1 Hz), 7.24 (m, 1H), 6.66 (s, 1H), 6.20 (br s, 1H), 5.86 (m, 2H), 3.37 (m, 2H), 3.02 (s, 3H), 2.90 (m, 2H), 2.65 (s, 6H), 2.06 (m, 2H), 1.81 (d, 3H, *J*= 6.3 Hz); MS (APCI): 626.04 [M+H]⁺.

### Example 81: 5-{6-[Difluoro(4-piperidinylsulfonyl)methyl]-1H-benzimidazol-1-yl}-3-({(1R)-1-[2-(trifluoromethyl)phenyl]ethyl}oxy)-2-thiophenecarboxamide trifluoroacetic acid salt

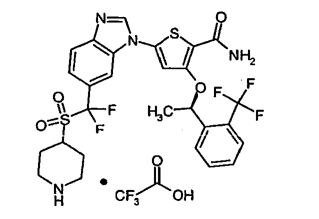

### Step A - 1, 1-dimethy/ethy/4-[({1-[5-[(methy/oxy)carbonyl]-4-({(1R)-1-[2-(trifluoromethyl)phenyl)ethyl}oxy)-2-thienyl]-1H-benzimidazol-6-yl}methyl)thio]-1-piperidinecarboxylate

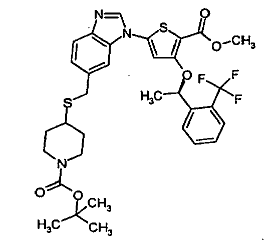

The title compound was prepared from methyl 5-[6-(chloromethyl)-1*H-*benzimidazol-1-yl]-3-({(1*R*)-1-[2-(trifluoromethyl)phenyl]ethyl}oxy)-2-thiophenecarboxylate and 1,1-dimethylethyl 4-mercapto-1-piperidinecarboxylate by a procedure analogous to **Example 35**, Step A. ¹H NMR (400 MHz, DMSO-*d₆*): δ 8.68 (s, 1H), 8.01 (d, 1H, *J*= 7.5 Hz), 7.79 (m, 3H), 7.72 (d, 1H, *J*= 7.1 Hz), 7.54 (m, 1H), 7.47 (s, 1H), 7.38 (dd, 1H, *J*= 1.5, 8.2 Hz), 5.95 (q, 1H, *J*= 6.2 Hz), 4.63 (s, 2H), 4.04 (m, 2H), 3.83 (s, 3H), 3.27 (m, 1H), 2.78 (m, 2H), 2.05 (m, 2H), 1.65 (d, 3H, *J*= 6.2 Hz), 1.48 (m, 2H), 1.39 (s, 9H); MS (ESI): 676.29 [M+H]⁺.

### Step B - 1,1-dimethylethyl 4-[({1-5-[(methyloxy)carbonyl-4-({(1R)-1-[2-(trifluoromethyl)phenyl]ethyl}oxy)-2-thienyl]-1H-benzimidazol-6-yl}methyl)sulfonyl]-1-piperidinecarboxylate

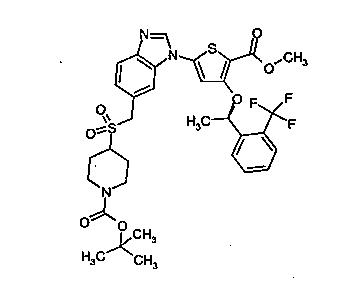

The title compound was prepared from 1,1-dimethylethyl 4-[({1-[5-(aminocarbonyl)-4-({(1*R*)-1-[2-(trifluoromethyl)phenyl]ethyl)oxy)-2-thienyl]-1*H-*benzimidazol-6-yl}methyl)thio]-1-piperidinecarboxylate by a procedure analogous to **Example 35**, Step B. ¹H NMR (400 MHz, DMSO-*d₆*): δ 8.68 (s, 1H), 8.01 (d, 1H, *J*= 7.9 Hz), 7.78 (m, 3H), 7.72 (d, 1H, *J*= 7.9 Hz), 7.54 (m, 1H), 7.47 (s, 1H), 7.38 (dd, 1H, *J*= 1.5, 8.2 Hz), 5.95 (q, 1H, *J*= 6.3 Hz), 4.63 (s, 2H), 4.04 (m, 2H), 3.83 (s, 3H), 3.26 (m, 1H), 2.78 (m, 2H), 2.06 (m, 2H), 1.65 (d, 3H, *J*= 6.2 Hz), 1.50 (m, 2H), 1.39 (s, 9H); MS (ESI): 708.27 [M+H]⁺.

### Step C - 1,1-dimethylethyl 4-[(difluoro{1-[5-[(methyloxy)carbonyl]-4-({(1R)-1-[2-(trifluoromethyl)phenyl]ethyl}oxy)-2-thienyl]-1H-benzimidazol-6-yl}methyl)sulfonyl]-1-piperidinecarboxylate

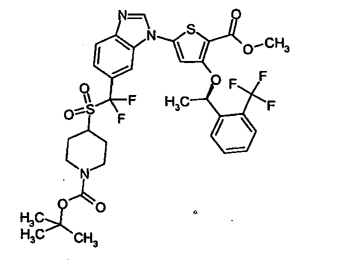

To a cooled (-78 °C) solution of 1,1-dimethylethyl 4-[({1-[5-[(methyloxy)carbonyl]-4-({(1*R*)-1-[2-(trifluoromethyl)phenyl]ethyl}oxy)-2-thienyl]-1*H*-benzimidazol-6-yl}methyl)sulfony]-1-piperidinecarboxylate (50 mg, 0.071 mmol) and N-fluorobenzenesulfonimide (67 mg, 0.21 mmol) in tetrahydrofuran (1.4 mL) was added sodium bis(trimethylsilyl)amide (1.0 M in tetrahydrofuran, 0.15 mL, 0.15 mmol) dropwise over 2 min. The reaction mixture was stirred for 2 h at -78 °C and then for 14 h at room temperature. The reaction quenched by addition of saturated aqueous ammonium chloride (10 mL). The mixture was extracted with dichloromethane (3 x 10 mL). The combined organic fractions were dried over sodium sulfate, filtered, and concentrated onto silica gel. Purification by flash column chromatography afforded 10 mg (19%) of the title compound. ¹H NMR (300 MHz, CDCl₃): δ 8.10 (s, 1H), 7.94 (m, 2H), 7.79 (s, 1H), 7.70-7.59 (m, 3H), 7.41 (m, 1H), 6.79 (s, 1H), 5.83 (q, 1H, *J*= 6.3 Hz), 4.32 (m, 2H), 3.96 (s, 3H), 3.60 (m, 1H), 2.87 (m, 2H), 2.26 (m, 2H), 2.02-1.88 (m, 2H), 1.78 (d, 3H, *J*= 6.3 Hz), 1.48 (s, 9H); MS (APCl): 766.03 (M+Na]⁺.

### Step D - 1,1-dimethylethyl 4-{[{1-5[(aminocarbonyl)-4-({(1R)-1-[2-(trifluoromethyl)phenyl]ethyl}oxy)-2-thienyl]-1H-benzimidazole-6-yl}(difluoro)methyl]su/fonyl}-1-piperidinecarboxy/ate

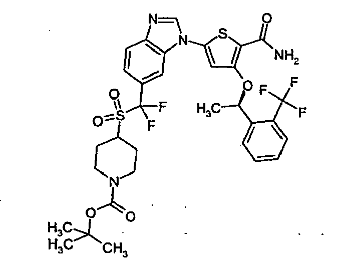

The title compound was prepared from 1,1-dimethylethyl 4-[(difluoro{1-[5-[(methyloxy)carbonyl]-4-({(1*R*)-1-[2-(trifluoromethyl)phenyl]ethyl}oxy)-2-thienyl]-1*H*-benzimidazol-6-yl}methyl)sulfonyl]-1-piperidinecarboxylate by a procedure analogous to **Example 49**, Step C. ¹H NMR (300 MHz, CDCl₃): δ 8.06 (s, 1H), 7.94 (d, 1H, *J*= 8.6 Hz), 7.78-7.58 (m, 5H), 7.53-7.43 (m, 1H), 7.23 (br s, 1H), 6.68 (s, 1H), 5.95 (br s, 1H), 5.87 (q, 1H, *J*= 6.3 Hz), 4.31 (m, 2H), 3.59 (m, 1H), 2.87 (m, 2H), 2.25 (m, 2H), 1.94 (m, 2H), 1.81 (d, 3H, *J*= 6.3 Hz), 1.48 (s, 9H); MS (APCI): 751.06 [M+Na]⁺.

### Step E - 5-{6-[difluoro(4-piperidinylsulfonyl)methyl]-1H-benzimidazol-1-yl}-3-({(1R)-1-[2-(trifluoromethyl)phenyl]ethyl}oxy)-2-thiophenecarboxamide trifluoroacetic acid salt (Title Compound)

The title compound was prepared from 1,1-dimethylethyl 4-{[{1-[5-(aminocarbonyl)-4-({(1*R*)-1-[2-(trifluoromethyl)phenyl]ethyl}oxy)-2-thienyl]-1*H* benzimidazol-6-yl}(difluoro)methyl]sulfonyl}-1-piperidinecarboxylate by a procedure analogous to **Example 49**, Step D. ¹H NMR (400 MHz, CDCl₃): δ 8.07 (s, 1H), 7.95 (d, 1H, *J*= 8.8 Hz), 7.80 (s, 1H), 7.71-7.60 (m, 5H), 7.45 (m, 1H), 7.21 (br s, 1H), 6.68 (s, 1H), 6.40 (br s, 1H), 5.85 (q, 1H, *J*= 6.0 Hz), 3.74 (m, 1H), 3.65 (m, 2H), 3.18 (m, 2H), 2.49 (m, 4H), 1.81 (d, 3H, *J*= 6.0 Hz); MS (APCI): 629.20 [M+H]⁺.

### Example 82: 3-{[(1R)-1-(2-chlorophenyl)ethyl]oxy}-5-{6-[1-methyl-4-piperidinyl)carbonyl]-1H-benzimidazol-1-yl}-2-thiophenecarboxamide

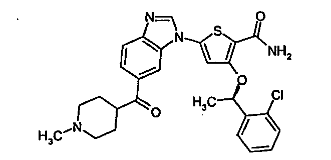

The title compound was prepared from 3-{[(1*R*)-1-(2-chlorophenyl)ethyl]oxy}-5-(6-(4-piperidinylcarbonyl)-1*H*-benzimidazol-1-yl]-2-thiophenecarboxamide by a procedure analogous to **Example 52**. ¹H NMR (400 MHz, CDCl₃): δ 8.07 (s, 1H), 7.89 (m, 2H), 7.49-7.28 (m, 5H), 7.23 (br s, 1H), 6.70 (s, 1H), 5.99 (br s, 1H), 5.89 (q, 1H, *J*= 6.2 Hz), 3.26 (m, 1H), 2.95 (m, 2H), 2.33 (s, 3H), 2.14 (m, 2H), 1.89 (m, 4H), 1.79 (d, 3H, *J*= 6.4 Hz); MS (APCI): 523.03 [M+H]⁺.

### Example 83: 3-{([1R)-1-(2-Chlorophenyl)ethyl]oxy}-5-(6-{difluoro[1-(1-methylethyly-4-piperidinyl]methyl}-1H-benzimidazol-1-yl)-2-thiophenecarboxamide

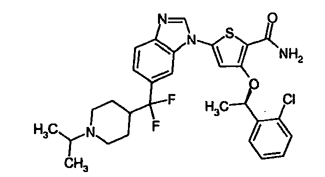

A solution of 3-{[(1*R*)-1-(2-chlorophenyl)ethyl]oxy}-5-{6-[difluoro(4-piperidinyl)methyl]-1*H*-benzimidazol-1-yl}-2-thiophenecarboxamide (46 mg, 0.087 mmol), 2-bromopropane (16 mL, 0.17 mmol), and sodium carbonate (46 mg, 0.43 mmol) in acetonitrile (1.7 mL) was heated to 80°C in a sealed tube for 17 h. The crude reaction mixture was then concentrated onto silica gel. Purification by flash column chromatography (30 to 100% 1/10/89 ammonium hydroxide/methanol/chloroform:chloroform) afforded 23 mg (46%) of the title compound. ¹H NMR (300 MHz, CDCl₃): δ 8.03 (s, 1H), 7.84 (d, 1H, *J*= 8.6 Hz), 7.49-7.30 (m, 6H), 7.23 (br s, 1H), 6.64 (s, 1H), 6.00 (br s, 1H), 5.88 (q, 1H, *J*= 6.3 Hz), 2.92 (m, 2H), 2.69 (m, 1H), 2.10-1.94 (m, 3H), 1.78 (d, 3H, *J*= 6.3 Hz), 1.70 (m, 2H), 1.58-1.46 (m, 2H), 1.02 (d, 6H, *J*= 6.6 Hz); MS (APCI): 573.11 [M+H]⁺.

### Example 84: 5- 6- chloromethyl) -1H-benzimidazol-1-yl]-3-{[(1R)-1-(2-chlorophenyl)ethyl]oxy}-2-thiophenecarboxamide

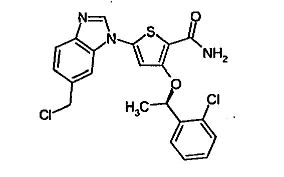

### Step A - 3-{[(1R)-1-(2-chlorophenyl)ethyl]oxy}-5-[6-(hydroxymethyl)-1H-benzimidazol-1-yl]-2-thiophenecarboxamide

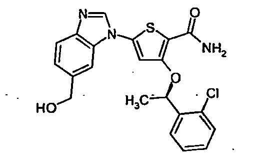

The title compound was prepared from methyl 3-{[(1*R*)-1-(2-chlorophenyl)ethyl]oxy}-5-[6-(hydroxymethyl)-1*H*-benzimidazol-1-yl]-2-thiophenecarboxylate by a procedure analogous to Example 21, Step B. ¹H NMR (300 MHz, CDCl₃): δ 7.96 (s, 1H), 7.79 (d, 1H, *J*= 8.3 Hz), 7.46 (m, 3H), 7.33 (m, 3H), 7.20 (br s, 1H), 6.64 (s, 1H), 5.88 (m, 2H), 4.80 (d, 2H, *J*= 5.9 Hz), 1.83 (t, 1H, *J*= 6.0 Hz), 1.78 (d, 3H, *J*= 6.5 Hz); MS (ESI): 428.15 [M+H]⁺.

### Step B - 5-[6-(chloromethyl)-1H-benzimidazol-1-yl]-3-{[(1R)-1-(2-chlorophenyl)ethyl]oxy}-2-thiophenecarboxamide (Title Compound)

To a suspension of 3-{[(1*R*)-1-(2-chlorophenyl)ethyl]oxy}-5-[6-(hydroxymethyl)-1*H*-benzimidazol-1-yl]-2-thiophenecarboxamide (720 mg, 1.68 mmol) and PS-triphenylphosphine (1.02 g, 2.15 mmol/g, 2.19 mmol) in dichloromethane (17 mL) was added *N*-chlorosuccinimide (292 mg, 2.19 mmol). The reaction was heated to reflux for 4 h, then was filtered to remove the PS-triphenylphosphine resin, washing with dichloromethane (50 mL). The filtrate was washed with 1:1 saturated aqueous sodium chloride:water (50 mL), dried over sodium sulfate, filtered, and concentrated. Purification by flash column chromatography (0 to 50% 1/10/89 ammonium hydroxide/methanol/chloroform:chloroform) afforded 610 mg (81%) of the title compound. ¹H NMR (300 MHz, CDCl₃): δ 8.00 (s, 1H), 7.80 (d, 1H, *J*= 8.1 Hz), 7.48 (m, 2H), 7.34 (m, 4H), 7.21 (br s, 1H), 6.64 (s, 1H), 6.11 (br s, 1H), 5.89 (q, 1H, *J*= 6.3 Hz), 4.68 (m, 2H), 1.79 (d, 3H, *J*= 6.3 Hz); MS (ESI): 446.21.

### Example 85: N-{[1-(5-(Aminocarbonyl)-4-{[(1R)-1-(2-chlorophenyl)ethyl]oxy}-2-thienyl)-1H-benzimidazol-6-yl]methyl}-β-alanine bis(trifluoroacetic acid salt

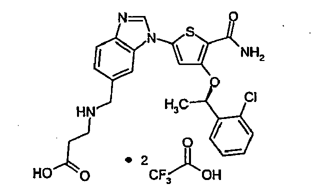

### Step A - 1,1-dimethylethyl N-{[1-(5-(aminocarbonyl)-4-{[(1R)-1-(2-chlorophenyl)ethyl]oxy}-2-thienyl)-1H-benzimidazol-6-y/]methyl}-β-alaninate

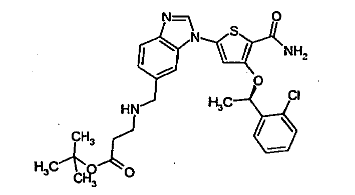

The title compound was prepared from 5-[6-(chloromethyl)-1*H*-benzimidazol-1-yl]-3-{[(1*R*)-1-(2-chlorophenyl)ethyl]oxy}-2-thiophenecarboxamide and 1,1-dimethylethyl β-alaninate by a procedure analogous to **Example 54**, Step A. MS (ESI): 555.25 [M+H]⁺.

### Step B-N-{[1-(5-(aminocarbonyl)-4-{[(1R)-1-(2-chlorophenyl)ethyl]oxy}-2 thienyl)-1H-benzimidazol-6-yl]methyl}-β-alanine bis(trifluoroacetic acid) salt (Title Compound)

To a solution of 1,1-dimethylethyl *N*-{[1-(5-(aminocarbonyl)-4-{[(1*R*)-1-(2-chlorophenyl)ethyl]oxy}-2-thienyl)-1*H*-benzimidazol-6-yl]methyl}-β-alaninate (70 mg, 0.13 mmol) in dichloromethane (1.7 mL) was added trifluoroacetic acid (0.8 mL). The reaction was stirred at room temperature for 2.5 h and was then concentrated. The crude reaction was triturated with diethyl ether (3 x 10 mL) and the resultant precipitate was dried under vacuum to afford 38 mg (41%) of the title compound. ¹H NMR (300 MHz, DMSO-*d₆*) δ 12.70 (s, 1H), 8.77 (br s, 2H), 8.66 (s, 1H) 7.86 (m, 2H), 7.75-7.24 (m, 8H), 7.13 (br s, 1H), 5.95 (q, 1H, *J*= 6.2 Hz), 4.32 (m, 2H), 3.13 (m, 2H), 2.64 (t, 2H, *J*= 7.0 Hz), 1.74 (d, 3H, *J*= 6.3 Hz); MS (ESI): 499.16 [M+H]⁺.

### Example 86: N-{3-[1-5-(aminocarbonyl)-4-{[(1R)-1-(2-chlorophenyl)ethyl]oxy}-2-thienyl)-1H-benzimidazol-6-yl]propyl}-L-alanine trifluoroacetic acid salt

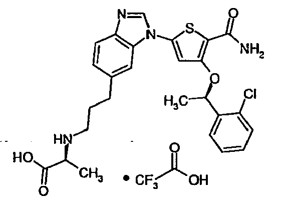

### Step A -1,1-dimethylethyl N-{3-[1-(5-(aminocarbonyl)-4-{[(1R)-1-(2-chlorophenyl}ethyl]oxy}-2-thienyl}-1H-benzimidazo/-6-yl]propyl}-L-alaninate

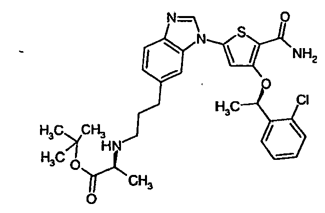

A solution of 3-{[(1*R*)-1-(2-chlorophenyl)ethyl]oxy}-5-[6-(3-oxopropyl)-1*H-*benzimidazol-1-yl]-2-thiophenecarboxamide (82 mg, 0.18 mmol), 1,1-dimethylethyl L-alaninate hydrochloride (36 mg, 0.20 mmol), and acetic acid (10 µL, 0.18 mmol) in 1 ,2-dichloroethane (0.72 mL) was stirred for 5 min, and then sodium triacetoxyborohydride (53 mg, 0.25 mmol) was added. The reaction stirred 71 h at room temperature and was then poured into saturated aqueous sodium bicarbonate (25 mL). The mixture was extracted with ethyl acetate (2 x 20 mL). The combined organic fractions were dried over sodium sulfate, filtered, and concentrated onto silica gel. Purification by flash column chromatography (0 to 100% 1/10/89 ammonium hydroxide/methanol/chloroform:chloroform) afforded 60 mg (57%) of the title compound. ¹H NMR (300 MHz, CDCl₃): δ 7.92 (s, 1H), 7.70 (d, 1H, *J*= 8.3 Hz), 7.47 (m, 2H), 7.33 (m, 2H), 7.20 (m, 3H), 6.63 (s, 1H), 6.09 (br s, 1H), 5.89 (q, 1H, *J*= 6.9 Hz), 3.20 (q, 1H, *J*= 6.9 Hz), 2.75 (m, 2H), 2.66 (m, 1H), 2.52 (m, 1H), 1.78 (m, 5H), 1.44 (s, 9H), 1.26 (m, 4H); MS (APCI): 584.08 [M+H]⁺.

### Step B -N-{3-[1-(5-(aminocarbonyl)-4-{[(1R)-1-(2-chlorophenyl)ethyl]oxy}-2-thienyl)-1H-benzimidazol-6-yl]propyl}-L-alanine trifluoroacetic acid salt(Title Compound)

The title compound was prepared from 1,1-dimethylethyl *N*-{3-[1-(5-(aminocarbonyl)-4-{[(1*R*)-1-(2-chlorophenyl)ethyl]oxy}-2-thienyl)-1*H-*benzimidazol-6-yl]propyl}-L-alaninate by a procedure analogous to **Example 85**, Step B. Purification by preparative HPLC (0 to 50% acetonitrile:water with 0.1 % trifluoroacetic acid) afforded 24 mg (24%) of the title compound. ¹H NMR (300 MHz, DMSO-*d₆*): δ 8.87 (br s, 2H), 8.54 (s, 1H), 7.83 (br s, 1H), 7.69 (m, 2H), 7.52-7.35 (m, 5H), 7.22 (m, 1H), 7.13 (br s, 1H), 5.97 (q, 1H, *J*= 6.5 Hz), 4.03 (m, 1H), 4.03 (m, 1H), 2.94 (m, 2H), 2.79 (t, 2H, *J*= 7.2 Hz), 1.94 (m, 2H), 1.73 (d, 3H, *J*= 6.5 Hz), 1.41 (d, 3H, *J*= 7.2 Hz); MS (ESI): 527.32 [M+H]⁺.

### Examples 87: N-{3-[1-(5-(aminocarbonyl)-4-{[(1R)-1-(2-chlorophenyl)ethyl]-oxy}-2-thienyl)-1H-benzimidazol-6-yl]propyl}-L-valine trifluoroacetic acid salt

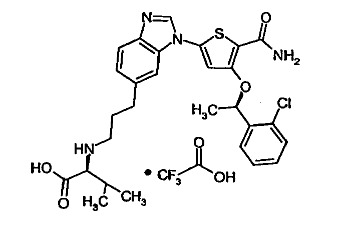

### Step A - 1,1-dimethylethyl N-{3-[1-(5-(aminocarbony/)-4-{[(1R)-1-(2-chlorophenyl)ethyl]oxy}-2-thienyl)-1H-benzimidazol-6-yl]propyl}-L-valinate

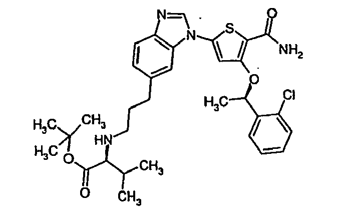

The title compound was prepared from 3-{[(1*R*)-1-(2-chlorophenyl)ethyl]oxy}-5-[6-(3-oxopropyl)-1*H*-benzimidazol-1-yl]-2-thiophenecarboxamide and 1,1-dimethylethyl L-valinate hydrochloride by a procedure analogous to **Example 86**, Step A. ¹H NMR (300 MHz, CDCl₃): δ 7.91 (s, 1H), 7.70 (d, 1H, *J*= 8.3 Hz), 7.47 (m, 2H), 7.35 (m, 3H), 7.19 (m, 2H), 6.62 (s, 1H), 5.90 (m, 2H), 3.17 (d, 1H, *J*= 4.8 Hz), 2.83-2.63 (m, 3H), 2.45 (m, 1H), 1.99 (m, 1H), 1.78 (m, 4H), 1.44 (s, 9H), 0.99-0.88 (m, 8H); MS (APCI): 611.36 [M+H]⁺.

### Step B -N-{3-[1-(5-(aminocarbonyl)-4-{[(1R)-1-(2-chlorophenyl)ethyl]oxy}-2-thienyl)-1H-benzimidazol-6-yl]propyl}-L-valine trifluoroacetic acid salt (Title Compound)

The title compound was prepared from 1,1-dimethylethyl *N-*{3-[1-(5-(aminocarbonyl)-4-{[(1*R*)-1-(2-chlorophenyl)ethyl]oxy}-2-thienyl)-1*H-*benzimidazol-6-yl]propyl}-L-valinate by a procedure analogous to **Example 85**, Step B. Purification by preparative HPLC (0 to 50% acetonitrile:water with 0.1% trifluoroacetic acid) afforded 5 mg (4%) of the title compound. ¹H NMR (300 MHz, DMSO-*d₆*): δ 8.86 (br s, 2H), 8.53 (s, 1H), 7.83 (br s, 1H), 7.69 (m, 2H), 7.52-7.35 (m, 4H), 7.21 (m, 2H), 7.13 (br s, 1H), 5.97 (q, 1H, *J*= 6.5 Hz), 3.89 (m, 1H), 2.92 (m, 1H), 2.76 (m, 2H), 2.22 (m, 1H), 1.98 (m, 2H), 1.73 (d, 3H, *J*= 6.3 Hz), 1.03 (d, 3H, *J*= 6.9 Hz), 0.93 (d, 3H, *J*= 6.9 Hz); MS (ESI): 555.28 [M+H]⁺.

### Example 88: N-{[1-(5-(Aminocarbonyl)-4-{[(1R)-1-(2-chlorophenyl)ethyl]oxy}-2-thienyl)-1H-benzimidazol-6-yl]methyl}-L-alanine trifluoroacetic acid salt

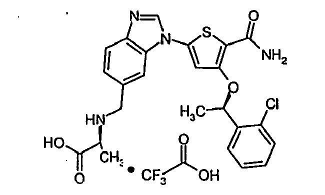

### Step A - 1,1-dimethylethyl N-{[1-(5-(aminocarbonyl)-4-{[(1R)-1-(2 chlorophenyl)ethyl]oxy}-2-thienyl)-1H-benzimidazol-6-yl]methyl}-L-alaninate

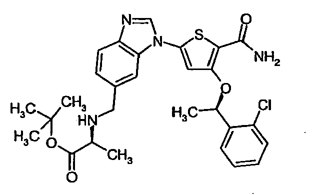

The title compound was prepared from 5-[6-(chloromethyl)-1*H-*benzimidazol-1-yl]-3-{[(1*R*)-1-(2-chlorophenyl)ethyl]oxy}-2-thiophenecarboxamide and 1,1-dimethylethyl L-alaninate hydrochloride by a procedure analogous to

### Example 54, Step A. ¹H NMR (300 MHz, CDCl₃): δ 7.93 (s, 1H), 7.75 (d, 1H, J= 8.3 Hz), 7.45 (m, 3H), 7.33 (m, 3H), 7.20 (br s, 1H), 6.62 (s, 1H), 5.89 (q, 1H, J= 6.3 Hz), 5.78 (br s, 1H), 3.92 (m, 1H), 3.76 (m, 1H), 3.26 (q, 1H, J= 7.0 Hz), 1.78 (d, 3H, J= 6.5 Hz), 1.48 (m, 10H), 1.29 (d 3H, J= 6.9 Hz); MS (ESI): 555.24 [M+H]⁺.

### Step B - N-{[1-(5-(aminocarbonyl)-4-{[(1R)-1-(2-chlorophenyl)ethyl]oxy}-2-thienyl)-1H-benzimidazol-6-yl]methyl}-L-alanine trifluoroacetic acid salt (Title Compound)

The title compound was prepared from 1,1-dimethylethyl *N*-{[1-(5-(aminocarbonyl)-4-{[(1*R*)*-*1-(2-chlorophenyl)ethyl]oxy}-2-thienyl)-1*H-*benzimidazol-6-yl]methyl}-L-alaninate by a procedure analogous to **Example 85**, Step B. Purification by preparative HPLC (0 to 50% acetonitrile:water with 0.1 % trifluoroacetic acid) afforded 79 mg (60%) of the title compound. ¹H NMR (300 MHz, DMSO-*d₆*): δ 9.29 (br s, 2H), 8.67 (s, 1H), 7.86 (m, 3H), 7.69 (dd, 1H, *J*= 1.8, 7.6 Hz), 7.50-7.36 (m, 4H), 7.33 (s, 1H), 7.13 (br s, 1H), 5.95 (q, 1H, *J*= 6.5 Hz), 4.32 (m, 2H), 4.00 (m, 1H), 1.74 (d, 3H, *J*= 6.3 Hz), 1.49 (d, 3H, *J*= 7.2 Hz); MS (ESI): 499.14 [M+H]⁺.

### Example 89: N-{2-[1-(5-(Aminocarbonyl)-4-{[(1R)-1-(2-chlorophenyl)ethyl]-oxy}-2-thienyl)-1H-benzimidazol-6-yl]ethyl}-L-alanine trifluoroacetic acid salt

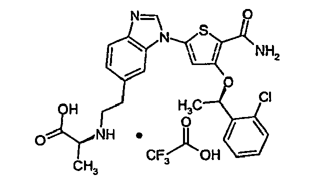

### Step A - 1,1-dimethylethyl N-{2-[1-(5-(aminocarbonyl)-4-{[(1R)-1-(2-chlorophenyl)ethyl]oxy}-2-thienyl)-1H-benzimidazol-6-yl]ethyl}-L-alaninate

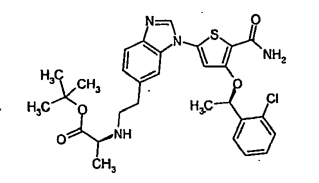

The title compound was prepared from 3-{[(1*R*)-1-(2-chlorophenyl)ethyl]oxy}-5-[6-(2-oxoethyl)-1*H-*benzimidazol-1-yl]-2-(thiophenecarboxamide and 1,1-dimethylethyl L-alaninate hydrochloride by a procedure analogous to **Example 86,** Step A. ¹H NMR (300 MHz, CDCl₃): δ 7.92 (s, 1H), 7.73 (d, 1H, *J*= 8.3 Hz), 7.47 (m, 2H), 7.34 (m, 3H), 7.25 (br s, 1H), 7.20 (dd, 1H, *J*= 1.5, 8.3 Hz), 6.62 (s, 1H), 5.88 (q, 1H, *J*= 6.3 Hz), 5.76 (br s, 1H), 3.23 (q, 1H, *J*= 7.0 Hz), 2.97-2.75 (m, 3H), 1.78 (d, 3H, *J*= 6.3 Hz), 1.59 (m, 2H), 1.41 (s, 9H), 1.25 (d, 3H, *J*= 7.0 Hz); MS (ESI): 569.26 [M+H]⁺.

### Step B - N-{2-[1-(5-(aminocarbonyl)-4-{[(1R)-1-(2-chlorophenyl)ethyl]oxy}-2-thienyl)-1H-benzimidazol-6-yl]ethyl}-L-alanine trifluoroacetic acid salt (Title Compound)

The title compound was prepared from 1,1-dimethylethyl *N-*{2-[1-(5-(aminocarbonyl)-4-{[(1*R*)-1-(2-chlorophenyl)ethyl]oxy}-2-thienyl)-1*H-*benzimidazol-6-yl]ethyl}-L-alaninate by a procedure analogous to **Example 85**, Step B. Purification by preparative HPLC (0 to 50% acetonitrile:water with 0.1% trifluoroacetic acid) afforded 50 mg (39%) of the title compound. ¹H NMR (300 MHz, DMSO-*d₆*): δ 8.98 (br s, 2H), 8.57 (s, 1H), 7.83 (br s, 1H), 7.75 (d, 1H, *J*= 8.3 Hz), 7.69 (dd, 1H, *J*= 1.8, 7.6 Hz), 7.53-7.34 (m, 4H), 7.25 (dd, 1H, *J*= 1.5, 8.3 Hz), 7.22 (s, 1H), 7.13 (br s, 1H), 5.97 (q, 1H, *J*= 6.5 Hz), 4.06 (m, 1H), 3.22 (m, 2H), 3.09 (m, 2H), 1.73 (d, 3H, *J*= 6.3 Hz), 1.45 (d, 3H, *J*= 7.2 Hz); MS (ESI): 513.19 [M+H]⁺.

### Example 90: 3-{[(1R)-1-(2-chlorophenyl)ethyl]oxy}-5-[6-({[2-(methylsulfonyl)ethyl]amino}methyl)-1H-benzimidazol-1-yl]-2-thiophenecarboxamide

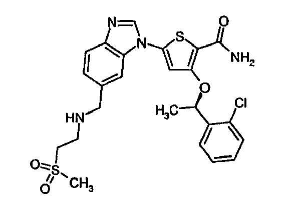

The title compound was prepared from 5-[6-(chloromethyl)-1*H*-benzimidazol-1-yl]-3-{[(1*R*)-1-(2-chlorophenyl)ethyl]oxy}-2-thiophenecarboxamide and 2-(methylsulfonyl)ethanamine by a procedure analogous to **Example 54**, Step A. ¹H NMR (300 MHz, DMSO-*d₆*): δ 8.54 (s, 1H), 7.82 (br s, 1H), 7.69 (m, 2H), 7.56-7.32 (m, 5H), 7.21 (s, 1H), 7.12 (br s, 1H), 5.97 (q, 1H, *J*= 6.5 Hz), 3.88 (m, 2H), 3.31 (s, 3H), 3.29 (m, 3H), 2.95 (m, 2H), 1.73 (d, 3H, *J*= 6.3 Hz); MS (ESI): 533.17 [M+H]⁺.

### Example 91: 5-(6-{[(2-aminoethyl)amino]methyl}-1H-benzimidazol-1-yl)-3-{[(1R)-(2-chlorophenyl)ethyl]oxy}-2-thiophenecarboxamide bis(trifluoroacetic acid) salt

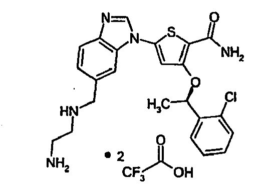

### Step A - 1,1-dimethylethyl [2-({[1-(5-(aminocarbonyl)-4-{[(1R)-1-(2-chlorophenyl)ethyl]oxy}-2-thienyl)-1H-benzimidazol-6-yl]methyl}amino)ethyl]carbamate

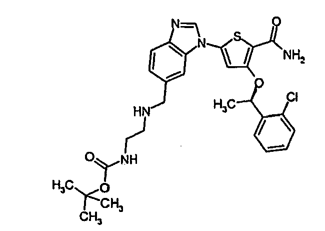

The title compound was prepared from 5-[6-(chloromethyl)-1*H*-benzimidazol-1-yl]-3-{[(1*R*)-1-(2-chlorophenyl)ethyl]oxy}-2-thiophenecarboxamide and 1,1-dimethylethyl (2-aminoethyl)carbamate by a procedure analogous to **Example 54,** Step A. ¹H NMR (300 MHz, CDCl₃): δ 7.94 (s, 1H), 7.76 (d, 1H, *J*= 8.3 Hz), 7.50-7.34 (m, 6H), 7.21 (br s, 1H), 6.63 (s, 1H), 5.89 (m, 2H), 4.91 (br s, 1H), 3.88 (s, 2H), 3.41 (m, 1H), 3.25 (m, 2H), 2.75 (t, 2H, *J*= 6.0 Hz), 1.78 (d, 3H, *J*= 6.5 Hz), 1.44 (s, 9H); MS (ESI): 570.28 [M+H]⁺.

### Step B -5-(6-{[(2-aminoethyl)amino]methyl)-1H-benzimidazol-1-yl)-3-{[(1R)-1-(2-chlorophenyl)ethyl]oxy}-2-thiophenecarboxamide bis(trifluoroacetic acid) salt (Title Compound)

The title compound was prepared from 1,1-dimethylethyl [2-({[1-(5-(aminocarbonyl)-4-{[(1*R*-1-(2-chlorophenyl)ethyl]oxy}-2-thienyl)-1*H-*benzimidazol-6-yl]methyl}amino)ethyl]carbamate by a procedure analogous to **Example 49**, Step D. ¹H NMR (300 MHz, DMSO-*d₆*): δ 9.01 (br s, 2H), 8.68 (s, 1H), 7.88 (m, 5H), 7.69 (m, 2H), 7.50-7.33 (m, 5H), 7.13 (br s, 1H), 5.95 (q, 1H, *J*= 6.0 Hz), 4.37 (s, 2H), 3.81 (m, 2H), 3.29 (m, 1H), 2.88 (m, 1H), 1.74 (d, 3H, *J*= 6.5 Hz); MS (ESI): 470.19 [M+H]⁺.

### Example 92: 5-(6-{[(2-aminoethyl)(methyl)amino]methyl}-1H-benzimidazol-1-yl)-3-{[(1R)-1-(2-chlorophenyl)ethyl]oxy}-2-thiophenecarboxamide bis(trifluoroacetate) salt

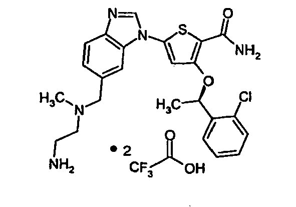

### Step A - 1,1-dimethylethyl {2-[{[1-(5-(aminocarbony/)-4-{[(1R)-1-(2-chlorophenyl)ethyl]oxy}-2-thienyl)-1H-benzimidazol-6-yl]methyl}(methyl)amino]ethyl}carbamate

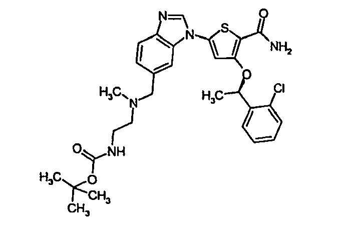

The title compound was prepared from 1,1-dimethylethyl [2-({[1-(5-(aminocarbonyl)-4-{[(1*R*)-1-(2-chlorophenyl)ethyl]oxy)-2-thienyl)-1*H-*benzimidazol-6-yl]methyl}amino)ethyl]carbamate by a procedure analogous to **Example 52.** ¹H NMR (300 MHz, CDCl₃): δ 7.95 (s, 1H), 7.76 (d, 1H, *J*= 8.8 Hz), 7.50-7.30 (m, 6H), 7.22 (br s, 1H), 6.64 (s, 1H), 6.19 (br s, 1H), 5.89 (q, 1H, *J*= 6.3 Hz), 4.91 (br s, 1H), 3.60 (s, 2H), 3.22 (m, 2H), 2.50 (t, 2H, *J*= 6.0 Hz), 2.21 (s, 3H), 1.78 (d, 3H, *J*= 6.5 Hz), 1.42 (s, 9H); MS (ESI): 584.28 [M+H]⁺.

### Step B -5-(6-{[(2-aminoethyl)(methyl)amino]methyl}-1H-benzimidazol-1-yl)-3-{[(1R)-1-(2-chlorophenyl)ethyl]oxy}-2-thiophenecarboxamide bis(trifluoroacetate) salt (Title Compound)

The title compound was prepared from 1,1-dimethylethyl {2-[{[1-(5-(aminocarbonyl)-4-{[(1*R*)-1-(2-chlorophenyl)ethyl]oxy}-2-thienyl)-1*H-*benzimidazol-6-yl]methyl}(methyl)amino]ethyl}carbamate by a procedure analogous to **Example 49**, Step D. ¹H NMR (300 MHz, DMSO-*d₆*): δ 10.13 (br s, 1H), 8.68 (s, 1H), 7.99 (m, 3H), 7.82 (m, 3H), 7.69 (dd, 1H, *J*= 1.8, 7.6 Hz), 7.50-7.33 (m, 5H), 7.13 (br s, 1H, 5.95 (q, 1H, *J*=6.4 Hz), 4.47 (br s, 3H), 3.20 (br m, 4H), 2.71 (br s, 2H), 1.73 (d, 3H, *J*= 6.3 Hz); MS (ESI): 484.23 [M+H]⁺.

### Example 93: 3-{[(1R)-1-(2-chlorophenyl)ethyl]oxy}-5-(6-{[[2-(dimethylamino)ethyl](methyl)amino]methyl}-1H-benzimidazol-1-yl)-2-thiophenecarboxamide bis(trifluoroacetate) salt

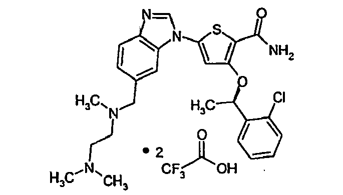

### The title compound was prepared from 5-(6-{[(2-aminoethyl)(methyl)amino]-methyl}-1H-benzimidazol-1-yl)-3-{[(1R)-1-(2-chlorophenyl)ethyl]oxy}-2-thiophenecarboxamide bis(trifluoroacetate) salt by a procedure analogous to Example 52. ¹H NMR (300 MHz, DMSO-d₆): δ 8.66 (br s, 1H), 7.85 (m, 3H), 7.69 (dd, 1H, J= 1.8, 7.6 Hz), 7.50-7.31 (m, 6H), 7.30 (br s, 1H), 7.13 (br s, 1H), 5.95 (q, 1H, J= 6.3 Hz), 3.37 (br m, 4H), 2.78 (m, 9H), 2.45-2.25 (m, 2H), 1.73 (d, 3H, J= 6.3 Hz); MS (ESI): 512.25 [M+H]⁺.

### Example 94: 3-{[(1R)-1-(2-Chlorophenyl)ethyl]oxy}-5-{6-[1-(2-hydroxyethyl)-4-piperidinyl]-1H-benzimidazol-1-yl)-2-thiophenecarboxamide

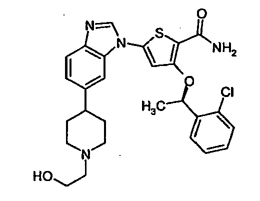

### Step A - (1-{[(1,1-Dimethylethyl)oxy]carbonyl}-4-piperidinyl)(iodo)zinc

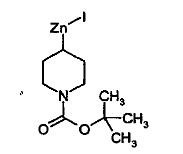

The procedure followed was analogous to that described in Journal of Organic Chemistry 2004, 69, 5120-5123. To a slurry of zinc dust (4.22 g, 64.5 mmol) and Celpure P65 (0.83 g) in *N,N-*dimethylformamide (10.4 mL) was added a 7:5 v/v mixture of chlorotrimethylsilane:1,2-dibromoethane (1.25 mL) over a 10 min period at room temperature. The internal temperature was maintained below 65 °C during the addition. The slurry was stirred for 15 min and a solution of 1,1-dimethylethyl 4-iodo-1-piperidinecarboxylate (16.50 g, 52.0 mmol, prepared as in the literature reference cited above) in *N,N-*dimethylformamide (26 mL) was added slowly at a rate to maintain temperature below 65 °C. The reaction mixture was heated at 65 °C for 5 min and allowed to cool to room temperature with stirring for 30 min. The mixture was filtered to afford a solution of the desired product in *N,N-*dimethylformamide of undetermined concentration.

### Step B - 1,1-Dimethylethyl 4-(1-{4-{[(1R)-1-(2-chlorophenyl)ethyl]oxy}-5-[(methyloxy)carbonyl]-2-thienyl}-1H-benzimidazol-6-yl)-1-piperidinecarboxylate

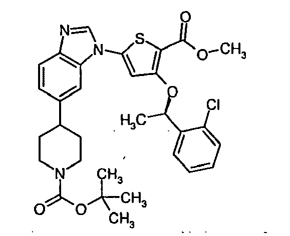

To a solution of methyl 3-{[(1*R*)-1-(2-chlorophenyl)ethyl]oxy}-5-(6-{[(trifluoromethyl)sulfonyl]oxy}-1*H-*benzimidazol-1-yl)-2-thiophenecarboxylate (100 mg, 0.18 mmol), [1,1-bis(diphenylphosphino)-ferrocene]dichloropalladium(II)-dichloromethane complex (7.4 mg, 0.009 mmol), and cuprous iodide (3.4 mg, 0.018 mmol) in *N*,*N*-dimethylacetamide (0.24 mL) was added (1-{[(1,1-dimethylethyl)oxy]carbonyl}-4-piperidinyl)(iodo)zinc solution (0.72 mL). The resulting mixture was purged with nitrogen and heated to 80 °C for 2 h. Ethyl acetate was added and the mixture was filtered through a pad of Celite, washing with ethyl acetate. The organic layer was washed with water and dried over sodium sulfate. Silica was added and the volatiles were evaporated under reduced pressure. The residue purified by flash chromatography (0 to 100% ethyl acetate:hexanes) to afford 94 mg (87%) of the title compound. MS (ESI): 596 [M+H]⁺.

### Step C - 1,1-Dimethylethyl 4-[1-(5-(aminocarbonyl)-4-{[(1R)-1-(2 chlorophenyl)ethyl]oxy}-2-thienyl)-1H-benzimidazol-6-yl]-1-piperidinecarboxylate

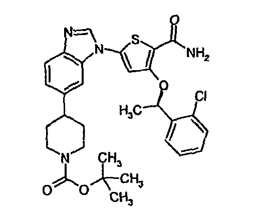

To 1,1-dimethylethyl4-(1-{4-{[(1*R*)-1-(2-chlorophenyl)ethyl]oxy}-5-[(methyloxy)carbonyl]-2-thienyl}-1*H*-benzimidazo)-6-yl)-1-piperidinecarboxylate (0.50 g, 0.84 mmol) was added 7 N ammonia in methanol (10 mL)). The mixture was heated in a sealed tube to 70 °C for 48 h and then cooled to room temperature. Silica was added and the volatiles evaporated under reduced pressure. The residue was purified by flash chromatography (0 to 100% 89/10/1 dichloromethane/methanol/ammonium hydroxide:dichloromethane) to afford 320 mg (66%) of the title compound. MS (ESI): 581 [M+H]⁺.

### Step D - 3-{[(1R)-1-(2-Chlorophenyl)ethyl]oxy}-5-[6(4-piperidinyl)- 1H - benzimidazol-1-yl]-2-thiophenecarboxamide

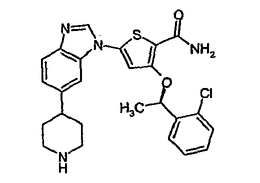

To a solution of 1,1-dimethylethyl 4-[1-(5-(aminocarbonyl)-4-{[(1*R*)-1-(2-chlorophenyl)ethyl]oxy}-2-thienyl)-1*H-*benzimidazol-6-yl]-1-piperidinecarboxylate (0.23 g, 0.40 mmol) in dichloromethane (10 mL) was added trifluoroacetic acid (5 mL) and the mixture stirred for 20 min. Silica was added and the volatiles were evaporated under reduced pressure. The residue was purified by flash chromatography (0 to 100% 89/10/1 dichloromethane/methanol/ammonium hydroxide:dichloromethane) to afford 180 mg (97%) of the title compound. 1H NMR (400 MHz, DMSO-*d₆*): δ 8.50 (s, 1H), 7.84 (br s, 1H), 7.74-7.63 (m, 2H), 7.59-7.50 (m, 1H), 7.49-7.33 (m, 2H), 7.28-7.19 (m, 2H), 7.15 (s, 1H), 7.12 (s, 1H), 5.99 (q, 1H, *J*= 6.2 Hz), 3.09 (d, 2H, *J*= 11.4 Hz), 2.80-2.54 (m, 2H), 1.77-1.67 (m, 5H), 1.64-1.50 (m, 2H), 1.24 (br s, 2H); MS (ESI): 481 [M+H]⁺.

### Step E -3-{[(1R)-7-(2-Chlorophenyl)ethyl]oxy}-5-{6-[1-(2-hydroxyethyl)-4-piperidinyl]-1H-benzimidazol-1-y/}-2-thiophenecarboxamide (Title Compound)

To a solution of 3-{[(1*R*)-1-(2-chlorophenyl)ethyl]oxy}-5-[6-(4-piperidinyl)-1*H-*benzimidazol-1-yl]-2-thiophenecarboxamide (600 mg, 0.13 mmol) and hydroxyacetaldehyde (360 mg, 0.63 mmol) in 2:1:0.5 dichloromethane:methanol:water (1 mL) was added sodium triacetoxyborohydride (0.093 g, 0.44 mmol) and acetic acid (0.036 g, 0.63 mmol) and the reaction stirred at room temperature for 3 h. Silica was added and the volatiles were evaporated under reduced pressure and the residue was purified by flash chromatography ((0 to 100% 89/10/1 dichloromethane/methanol/ammonium hydroxide:dichloromethane) to afford 0.059 g (90%) of the title compound. ¹H NMR (400 MHz, CDCl₃): δ 7.93 (s, 1H), 7.74 (d, 1H, *J*=8.4 Hz), 7.49 (dd, 1H, *J* = 1.6, 7.7 Hz), 7.43 (dd, 1H, *J*= 1.2, 7.6 Hz), 7.40-7.29 (m, 3H), 7.27-7.18 (m, 2H), 6.63 (s, 1H), 6.22 (br s, 1H), 5.89 (q, 1H, *J*= 6.2 Hz), 3.68 (t, 2H, *J*= 5.4 Hz), 3.10 (d, 2H, *J*= 11.3 Hz), 2.72-2.58 (m, 3H), 2.48-2.17 (m, 4H), 1.95-1.80 (m, 4H), 1.78 (d, 3H, *J*= 6.2 Hz); MS (ESI): 525 [M+H]⁺.

### Example 95: 3-{[(1R)-1-(2-Chlorophenyl)ethyl]oxy}-5-(6-{1-[2-(methylsulfonyl)ethyl]-4-piperinyl}-1H-benzimidazol-1-yl)-2-thiophenecarboxamide

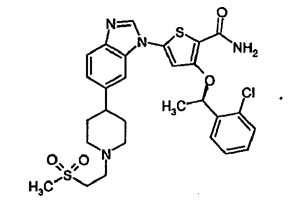

To 3-{[(1*R*)-1-(2-chlorophenyl)ethyl]oxy}-5-(6-(4-piperidinyl)-1*H-*benzimidazol-1-yl]-2-thiophenecarboxamide in tetrahydrofuran (2 mL) was added methyl vinyl sulfone (16 mg, 0.15 mmol) and the mixture stirred at room temperature overnight. Silica was added and the volatiles were evaporated under reduced pressure. The residue was purified by flash chromatography (0 to 100% 89/10/1 dichloromethane/methanol/ammonium hydroxide:dichloromethane) to afford 26 mg (35%) of the title compound. ¹H NMR (400 MHz, DMSO-*d₆*): δ 8.50 (s, 1H), 7.84 (br s, 1H), 7.74-7.63 (m, 2H), 7.53 (d, 1H, *J* = 7.9 Hz), 7.49-7.35 (m, 2H), 7.30-7.21 (m, 2H), 7.16 (br s, 1H), 7.10 (s, 1H), 5.99 (q, 1H, *J*= 5.9 Hz), 3.39-3.26 (m, 5H), 3.13-2.99 (m, 5H), 2.78 (t, 2H, *J*= 6.4 Hz), 2.62 (t, 1H, *J=* 11.8 Hz), 2.12 (t, 2H, *J=* 11.1 Hz), 1.85-1.57 (m, 6H); MS (ESI): 587 [M+H]⁺.

### Example 96: 3-{[(1R)-1-(2-Chlorophenyl)ethyl]oxy}-5-[6-(4-piperidinylmethyl)-1H-benzimidazol-1-yl]-2-thiophenecarboxamide

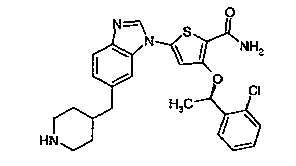

### Step A- 1,1-Dimethylethyl 4-(iodomethyl)-1-piperidinecarboxylate

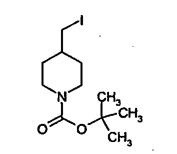

To a solution 1,1-dimethylethyl 4-(hydroxymethyl)-1-piperidinecarboxylate (10 g, 46.4 mmol), triphenylphosphine (15 g, 55.7 mmol) and imidazole (4 g, 55.7 mmol) in tetrahydrofuran (25 mL) at 0 °C was added via an addition funnel iodine (14 g, 55.7 mmol) in tetrahydrofuran (25 mL) over 10 min. The reaction was allowed to warm to room temperature and stirred for 3 h. The reaction was diluted with 20% ethyl acetate:hexane and filtered through a pad of silica with copious 20% ethyl acetate:hexane washings. Silica was added and the volatiles were evaporated under reduced pressure and the residue was purified by flash column chromatography (0 to 25% ethyl acetate:hexane) to afford 13 g (84%) of the title compound. ¹H NMR (400 MHz, CDCl₃): δ 4.19-3.96 (m, 2H), 3.05 (d, 2H, *J*= 6.4 Hz), 2.71-2.56 (m, 2H), 1.78 (d, 2H, *J*= 13.4 Hz), 1.64-1.50 (m, 1H), 1.40 (s, 9H), 1.16-1.01 (m, 2H).

### Step B - [(1-{[(1,1-Dimethylethyl)oxy]carbonyl}-4-piperidinyl)methyl](iodo)zinc

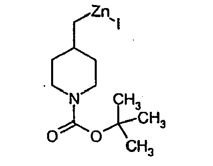

The title compound was prepared from 1,1-dimethylethyl 4-(iodomethyl)-1-piperidinecarboxylate by a procedure analogous to **Example 94**, Step A.

### Step C -1,1-Dimethylethyl 4-[(1-{4-{[(1R)-1-(2-chlorophenyl)ethyl]oxy}-5-[(methyloxy)carbonyl]-2-thienyl}-1H-benzimidazol-6-yl)methyl]-1-piperidinecarboxylate

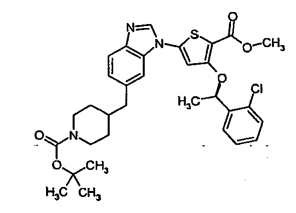

The title compound was prepared from methyl 3-{[(1*R*)-1-(2-chlorophenyl)ethyl]oxy}-5-(6-{[(trifluoromethyl)sulfonyl]oxy}-1*H*-benzimidazol-1-yl)-2-thiophenecarboxylate and [(1-{[(1,1-dimethylethyl)oxy]carbonyl}-4-piperidinyl)methyl](iodo)zinc by a procedure analogous to **Example 94**, Step B. ¹H NMR (400 MHz, DMSO-*d₆*) δ 8.58 (s, 1H), 7.61 - 7.80 (m, 2 H), 7.40 - 7.51 (m, 2 H), 7.31 - 7.38 (m, 2 H), 7.30 (s, 1H), 7.16 (d, *J*= 8.2 Hz, 1H), 5.99 (q, *J*= 6.2 Hz, 1H), 3.91 (d, *J*= 11.7 Hz, 2H), 3.83 (s, 3 H), 2.63 (d, *J*= 7.1 Hz, 3H), 1.66 - 1.73 (m, 2 H), 1.64 (d, *J*= 6.2 Hz, 3H), 1.52 (d, *J*= 13.2 Hz, 2H), 1.38 (s, 9 H), 1.05 (d, *J*= 12.3 Hz, 2H), MS (ESI): 610 [M+H]⁺. *Step D -3-{[(1*R*)-1-(2-Chlorophenyl)ethyl]oxy}-5-[6-(4-piperidinylmethyl)-1*H-*benzimidazol-1-yl]-2-thiophenecarboxamide (Title Compound)* To 1,1-dimethylethyl4-[(1-{4-{[(1*R*)-1-(2-chlorophenyl)ethyl]oxy}-5-[(methyloxy)carbonyl]-2-thienyl}-1*H*-benzimidazol-6-yl)methyl]-1-piperidinecarboxylate (1.6 g, 2.6 mmol) was add 7 N ammonia in methanol (25 mL) and the reaction mixture was stirred at 70 oC for 48 h. The reaction was cooled to room temperature and the volatiles were evaporated under reduced pressure. To the residue were added dichloromethane (26 mL) and trifluoroacetic acid (6 mL) and the reaction stirred for 0.5 h. Silica was added and the volatiles were evaporated under reduced pressure and the residue was purified by flash chromatography (0 to 100% 84/15/1 dichloromethane/methanol/ammonium hydroxide:dichloromethane) to afford 1.1 g, (83%) of the title compound. ¹H NMR (400 MHz, DMSO-*d₆*) δ 8.51 (s, 1H), 7.85 (s, 1H), 7.63 - 7.72 (m, 2 H), 7.51 (dd, *J*= 7.8, 1.4 Hz, 1H), 7.33-7.48 (m, 2 H), 7.23 (s, 1H), 7.15 (dd, *J*= 8.1, 1.4 Hz, 2 H), 7.11 (s, 1H), 6.00 (q, *J*= 6.4 Hz, 1H), 3.02 (d, *J*= 12.1 Hz, 2 H), 2.62 (d, *J*= 7.0 Hz, 2 H), 2.51 - 2.58 (m, 2 H), 1.73 (d, *J*= 6.4 Hz, 3 H), 1.59 - 1.69 (m, 1H), 1.49 -1.60 (m, 2 H), 1.17 (m, 3 H), MS (ESI): 495 [M+H]⁺.

### Example 97: 3-{[(1R)-1-(2-Chlorophenyl)ethyl]oxy}-6-[(1-methyl-4-piperidinyl)methyl]-1H-benzimidazol-1-yl}-2-thiophenecarboxamide

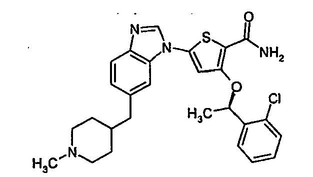

To a solution of 3-{[(1*R*)-1-(2-chlorophenyl)ethyl]oxy}-5-[6-(4-piperidinylmethyl)-1*H-*benzimidazol-1-yl]-2-thiophenecarboxamide (0.1 g, 0.2 mmol) in dichloromethane (1 mL) and methanol (0.5 mL) was added acetic acid (42 mg, 0.71 mmol) and 37% w/v formaldehyde in water (82 mg, 1.0 mmol). The reaction was stirred and sodium triacetoxyborohydride (150 mg, 0.71 mmol) was added and stirring continued for 3 h. Silica was added and the volatiles were evaporated under reduced pressure. The residue was purified by flash chromatography (0 to 100% 84/15/1 dichloromethane/methanol/ammonium hydroxide:dichloromethane) to afford 72 mg (70%) of the title compound. ¹H NMR (400 MHz, DMSO-*d₆*): δ 8.49 (s, 1H), 7.84 (s, 1H), 7.67 (dd, 2H, *J*= 8.0, 13.3 Hz), 7.55-7.32 (m, 3H), 7.29-7.00 (m, 4H), 6.00 (q, 1H, *J*= 6.6 Hz), 2.73 (d, 2H, *J*= 10.8 Hz), 2.61 (d, 2H, *J*= 6.2 Hz), 2.13 (s, 3H), 1.86-1.65 (m, 5H), 1.58-1.35 (m, 3H), 1.08-1.32 (m, 2H); MS (ESI): 509 [M+H]⁺.

### Example 98: 5-[5-(2-thienyl)-1H-benzimidazol-1-yl]-3-({(1R)-1[2-(trifluoromethyl)phenyl]ethyl)oxy)-2-thiophenecarboxamide

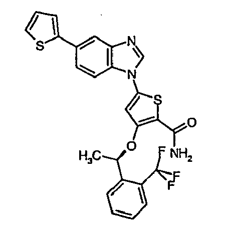

### Step A - methyl 5-[5-(2-thienyl)-1H-benzimidazol-1-yl]-3-({(1R)-1-[2-(trifluoromethyl)phenyl]ethyl}oxy)-2-thiophenecarboxylate

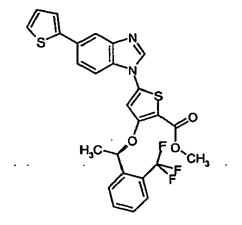

2-Thienylboronic acid (182 mg, 1.39 mmol) in *N,N*-dimethylacetamide (7 mL) was treated with aqueous 1 N sodium carbonate (1.6 mL, 1.6 mmol), methyl 5-(5-bromo-1*H-*benzimidazol-1-yl)-3-({1*R*)-1-[2-(trifluoromethyl)phenyl]ethyl}oxy)-2-thiophenecarboxylate (220 mg, 0.42 mmol) and 1,1'-bisdiphenylphosphinoferrocene dichloropalladium(II) dichloromethane adduct (70 mg, 0.09 mmol) and heated to 80 °C for 14 h. After cooling to room temperature the reaction was partitioned between 5:1 chloroform:methanol and saturated aqueous sodium bicarbonate. The organic layer was washed with saturated aqueous sodium chloride. The layers were separated and the organic layer was dried over sodium sulfate. The solution was filtered and concentrated. The crude compound was purified by column chromatography (hexanes:ethyl acetate) to yield 41 mg (18%) of the title compound as an oil. MS (APCl): 529.02 [M+H]⁺.

### Step B - 5-[5-(2-thienyl)-1H-benzimidazol-1-yl]-3-({(1R)-1-[2-(trifluoromethyl)phenyl]ethyl}oxy)-2-thiophenecarboxamide (Title Compound)

Methyl 5-[5-(2-thienyl)-1*H-*benzimidazol-1-yl]-3-({(1*R*)-1-[2-(trifluoromethyl)phenyl]ethyl}oxy)-2-thiophenecarboxylate (41 mg, 0.08 mmol) was dissolved in 7 N ammonia in methanol (5 mL) and placed in a sealed vessel. The mixture was heated to 80 °C for 14 h and then cooled to room temperature. The residue was partially purified by silica gel chromatography (hexanes:ethyl acetate) and then further purified by reverse phase preparative HPLC (10 to 90% acetonitrile with 0.1% formic acid:water with 0.1% formic acid) to yield 8 mg (19%) of the title compound as a white solid. ¹H NMR (400 MHz, CDCl₃): δ 8.04 (s, 1H), 7.95 (s, 1H), 7.72 (dd, 2H, *J*= 7.87, 13.90 Hz), 7.64 (t, 1H, *J*= 7.50 Hz), 7.59 (d, 1H, *J*= 8.60 Hz), 7.51-7.41 (m, 2H), 7.34 (d, 1H, *J*= 3.48 Hz) 7.29 (d, 1H, *J*= 5.12 Hz) 7.21 (br s, 1H), 7.10 (dd, 1H, *J*= 3.66, 5.12 Hz), 6.66 (s, 1H), 5.96-5.82 (m, 2H), 1.81 (d, 3H, *J*= 6.3 Hz); MS (APCl): 513.98 [M+H]⁺.

### Example 99: 5-[5-(3-pyridinyl)-1H-benzimidazol-1-yl]-3-({(1R)-1-[2-(trifluoromethyl)phenyl]ethyl}oxy)-2-thiophenecarboxamide

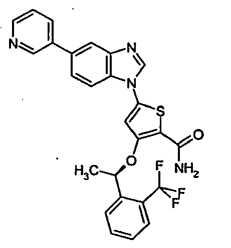

### Step A -methyl 5-[5-(3-pyridinyl)-1H-benzimidazo/-1-yl]-3-({(1R)-1-[2-(trifluoromethyl)phenyl]ethy}oxy)-2-thiophenecarboxylate

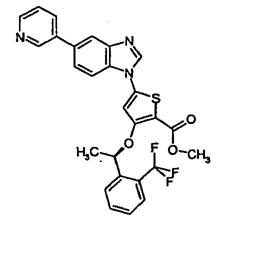

3-Pyridylboronic acid (103 mg, 0.84 mmol) in *N,N-*dimethylacetamide (7 mL) was treated with aqueous 1 N sodium carbonate (1.6 mL, 1.6 mmol), methyl 5-(5-bromo-1*H-*benzimidazol-1-yl)-3-({(1*R*)-1-[2-(trifluoromethyl)phenyl]-ethyl}oxy)-2-thiophenecarboxylate (220 mg, 0.42 mmol) and 1,1'-bisdiphenylphosphinoferrocene dichloropalladium(II) dichloromethane adduct (69 mg, 0.09 mmol) and heated to 80 °C for 2 h. After cooling to room temperature the reaction was partitioned between 5:1 chloroform:methanol and saturated aqueous sodium chloride. The organic layer was washed with saturated aqueous sodium chloride (3x). The layers were separated and the organic layer was dried over sodium sulfate. The solution was filtered and concentrated. The crude compound was purified by column chromatography (hexanes:ethyl acetate) to yield 142 mg (65%) of the title compound as a yellow foam. MS (ESI): 524.08 [M+H]⁺.

### Step B: 5-[5-(3-pyridinyl)-1H-benzimidazo/-1-yl]-3-({(1R)-1-[2-(trifluoromethyl)phenyl]ethyl}oxy)-2-thiophenecarboxamide (Title Compound)

Methyl 5-[5-(3-pyridinyl)-1*H-*benzimidazol-1-yl]-3-({(1*R*)-1-[2-(trifluoromethyl)phenyl]ethyl}oxy)-2-thiophenecarboxylate (142 mg, 0.27 mmol) was dissolved in 7 N ammonia in methanol (5 mL) and placed in a sealed vessel. The mixture was heated to 80 °C overnight and then cooled to room temperature. The residue was purified by silica gel chromatography (hexanes:ethyl acetate, with 0.5% triethylamine) to yield 59 mg of the title compound as a white foam. ¹H NMR(400 MHz, CDCl₃) : δ 8.89 (d, 1H, *J*=1.83 Hz), 8.60 (d, 1H, *J*=4.76 Hz), 8.01-7.94 (m, 3H), 7.71 (dd, 2H, *J*=7.97, 11.99 Hz), 7.63 (t, 1H, *J*= 7.60 Hz), 7.56-7.40 (m, 4H), 7.14 (br s, 1H), 6.67 (s, 1H), 5.86 (q, 1H, *J*= 6.23 Hz), 5.75 (s, 1H), 1.80 (d, 3H, *J*=6.23 Hz); HRMS calc'd. for C₂₆H₂₀F₃N₄O₂S [M+H]⁺ 509.1259, found 509.1231.

### Example 100: 3-{[(1R)-1-(2-chlorophenyl)ethyl]oxy}-5-[5-(4-pyridinyl)-1H-benzimidazol-1-yl]-2-thiophenecarboxamide

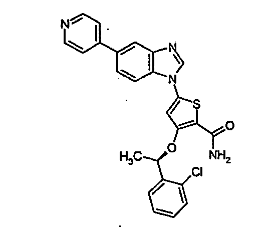

### Step A - methyl 3-{[(1R)-1-(2-chlorophenyl}ethyl]oxy}-5-[5-(4-pyridinyl}-1H-benzimidazol-1-yl]-2-thiophenecarboxylate

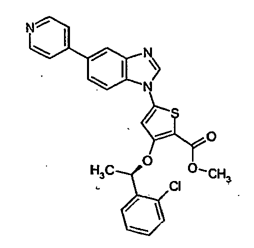

4-Pyridylboronic acid (103 mg, 0.84 mmol) in *N,N-*dimethylacetamide (6 mL) was treated with aqueous 1 N sodium carbonate (1.6 mL, 1.6 mmol), methyl 5-(5-bromo-1*H-*benzimidazol-1-yl)-3-({(1*R*)-1-(2-(trifluoromethyl)phenyl]-ethyl)oxy)-2-thiophenecarboxylate (207 mg, 0.42 mmol) and 1,1'-bisdiphenylphosphinoferrocene dichloropalladium(II) dichloromethane adduct (69 mg, 0.09 mmol) and heated to 80 °C for 2 h. After cooling to room temperature the reaction was partitioned between 5:1 chloroform:methanol and saturated aqueous sodium chloride. The organic layer was washed with water. The layers were separated and the organic layer was dried over sodium sulfate. The solution was filtered and concentrated. The crude compound was purified by silica gel chromatography (hexanes:ethyl acetate, with 0.5% triethylamine) to yield 189 mg (92%) of the title compound as a brown film. MS (APCI) 489.99 [M+H]⁺.

### Step B - 3-{[(1R)-1-(2-chlorophenyl)ethyl]oxy}-5-[5-(4-pyridinyl)-1H-benzimidazol-1-yl]-2-thiophenecarboxamide (Title Compound)

Methyl 3-{[(1*R*)-1-(2-chlorophenyl)ethyl]oxy}-5-(5-(4-pyridinyl)-1*H* benzimidazol-1-yl]-2-thiophenecarboxylate (189 mg, 0.39 mmol) was dissolved in 7 N ammonia in methanol (6 mL) and placed in a sealed vessel.

The mixture was heated to 80 °C for 14 h and then cooled to room temperature. The residue was purified by silica gel chromatography (hexanes:ethyl acetate, with 0.5% triethylamine) to yield 135 mg (73%) of the title compound as a white solid. ¹H NMR(400 MHz, CDCl₃): δ 8.67 (d, 2H, *J*= 5.86 Hz), 8.09 (s, 1H), 8.02 (s, 1H), 7.63-7.55 (m, 3H), 7.54-7.40 (m, 3H), 7.37-7.28 (m, 2H), 7.20 (br s, 1H), 6.65 (s, 1H), 5.88 (q, 1H, *J*= 6.35 Hz), 5.74 (br s, 1H) 1.78 (d, 3H, *J*= 6.41 Hz); MS (ESI): 475.11 [M+H]⁺.

### Example 101: 3-{([(1R)-1-(2-chlorophenyl)ethyl]oxy}-5-(5-phenyl-1H-benzimidazol-1-yl)-2-thiophenecarboxamide

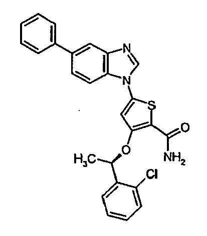

### Step A - methyl 3-{[(1R)-1-(2-chlorophenyl)ethyl]oxy}-5-(5-phenyl-1H-benzimidazol-1-yl}-2-thiophenecarboxylate

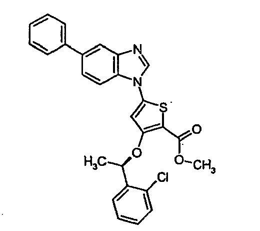

Phenylboronic acid (102 mg, 0.84 mmol) in *N,N-*dimethylacetamide (7 mL) was treated with aqueous 1N sodium carbonate (1.6 mL, 1.6 mmol), methyl 5-(5-bromo-1*H-*benzimidazol-1-yl)-3-({(1*R*)-1-[2-(trifluoromethyl)phenyl]ethyl}oxy)-2-thiophenecarboxylate (220 mg, 0.42 mmol) and 1,1'-bisdiphenylphosphinoferrocene dichloropalladium(II) dichloromethane adduct (69 mg, 0.09 mmol) and heated to 80 °C for 4 h. After cooling to room temperature the reaction was partitioned between 5:1 chloroform:methanol and saturated aqueous sodium bicarbonate. The organic layer was washed with saturated aqueous sodium bicarbonate (2x).

The layers were separated and the organic layer was dried over sodium sulfate. The solution was filtered and concentrated. The crude compound was purified by silica gel chromatography (hexanes:ethyl acetate, with 0.5% triethylamine) to yield 105 mg (48%) of the product as an oil. MS (APCI): 522.93 [M+H]⁺.

### Step B - 3-{[(1R)-7-(2-chlorophenyl)ethyl]oxy}-5-(5-phenyl-1H-benzimidazol-1-yl)-2-thiophenecarboxamide (Title Compound)

Methyl 3-{[(1*R*-1-(2-chlorophenyl)ethyl]oxy}-5-(5-phenyl-1*H*-benzimidazol-1-yl)-2-thiophenecarboxylate (105 mg, 0.20 mmol) was dissolved in 7 N ammonia in methanol (5 mL) and placed in a sealed vessel. The mixture was heated to 80 °C for 14 h and then cooled to room temperature. The residue was purified by silica gel chromatography (hexanes:ethyl acetate, with 0.5% triethylamine) to yield 25 mg (25%) of the title compound as a yellow solid. ¹H NMR (400 MHz, CDCl₃): δ 8.01 (s, 1H), 7.97 (s, 1H), 7.71 (dd, 2H, *J*= 7.78, 11.99 Hz), 7.66-7.60 (m, 3H), 7.57 (d, 1H, *J*= 8.42 Hz), 7.51-7.42 (m, 4H), 7.35 (t, 1H, *J*= 7.32 Hz), 7.19 (br s, 1H), 6.66 (s, 1H), 5.86 (q, 1H, *J*= 6.04 Hz), 5.75 (br s, 1H), 1.80 (d, 3H, *J*= 6.23 Hz); HRMS calc'd for C₂₇H₂₁F₃N₃O₂S [M+H]⁺ 508.1307, found 508.1299.

### Example 102: 5-[5-(1H-pyrrol-2-yl)-1H-benzimidazol-1-yl]-3-({(1R)-1-[2-(trifluoromethyl)phenyl)ethy}oxy)-2-thiophenecarboxamide

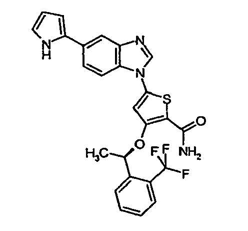

### Step A - 1,1-dimethylethyl 2{1-[5-(methyloxy)carbonyl]-4-({(1R)-1-[2-(trifluoromethy/)phenyl]ethyl}oxy)-2-thienyl]-1H-benzimidazol-5-yl}- 1H-pyrrole-1-carboxylate

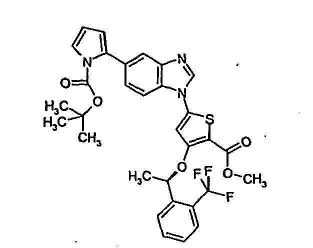

(1-{[(1,1-dimethylethyl)oxy]carbonyl}-1*H-*pyrro)-2-yl)boronic acid (161 mg, 0.76 mmol) dissolved in *N,N-*dimethylacetamide (7 mL) was treated with aqueous 1 N sodium carbonate (1.5 mL, 1.5 mmol), methyl 5-(5-bromo-1*H-*benzimidazol-1-yl)-3-({(1*R*)-1-[2-(trifluoromethyl)phenyl]ethyl}oxy)-2-thiophenecarboxylate (200 mg, 0.38 mmol), and 1,1'-bisdiphenylphosphinoferrocene dichloropalladium(II) dichloromethane adduct (62 mg, 0.08 mmol) and heated to 80 °C for 3 h. After cooling to room temperature the reaction was partitioned between 5:1 chloroform: methanol and saturated aqueous sodium chloride. The organic layer was washed with saturated aqueous sodium chloride (2x). The layers were separated and the organic layer was dried over sodium sulfate. The crude compound was purified by silica gel chromatography (hexanes:ethyl acetate) to yield 216 mg (93%) of the product as an orange oil. MS (APCl): 611.98 [M+H]⁺.

### Step B - 1,1-Dimethylethyl 2-{1-[5-(aminocarbonyl)-4-({(1R)-1-[2-(trifluoromethyl)phenyl]ethyl}oxy)-2-thienyl]-1H-benzimidazol-5-yl}-1H-pyrrole-1-carboxylate

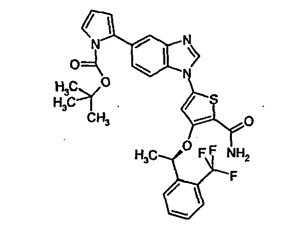

1,1-Dimethylethyl 2-{1-[5-[(methyloxy)carbonyl]-4-({(1*R*)-1-[2-(trifluoromethyl)phenyl]ethyl)oxy}-2-thienyl]- 1*H-*benzimidazol-5-yl}-1*H-*pyrrole-1-carboxylate (216 mg, 0.35 mmol) was dissolved in 7N ammonia in methanol (5 mL) and placed in a sealed vessel. The mixture was heated to 80 °C for 13.5 h and then cooled to room temperature. The residue was purified by silica gel chromatography (hexanes:ethyl acetate, with 0.5% triethylamine) to yield 63 mg (30%) of the title compound as an oil. MS (APCI): 597.01 [M+H]⁺.

### Step C - 5-[5-(1H-pyrrol-2-yl)-1H-benzimidazol-1-yl]-3-({(1R)-1-[2-(trifluoromethyl)phenyl]ethyl}oxy)-2-thiophenecarboxamide (Title Compound)

1,1-Dimethylethyl2-{1-[5-(aminocarbonyl)-4-({(1*R*)-1-(2-(trifluoromethyl)-phenyl]ethyl}oxy)-2-thienyl]-1*H*-benzimidazol-5-yl}-1*H*-pyrrole-1-carboxylate (63 mg, 0.11 mmol) was dissolved in chloroform (10 mL), treated with trifluoroacetic acid (1 mL, 13 mmol) and stirred overnight. The reaction was incomplete, and so was concentrated to a residue. Chloroform (10 mL) and trifluoroacetic acid (2 mL, 26 mmol) were added. No further change was evident after additional stirring. The reaction was neutralized with saturated aqueous sodium bicarbonate and extracted with chloroform. The organic layer was dried with soldium sulfate, filtered, and concentrated. Purification by flash column chromatography (hexanes:ethyl acetate, with 0.5% triethylamine) yielded 27 mg (49%) of the title compound as a white solid. ¹H NMR (400 MHz, CDCl₃): δ 8.67 (br s, 1H), 8.11 (s, 1H), 7.91 (s, 1H), 7.74-7.66 (m, 2H), 7.63 (t, 1H, *J*= 7.69 Hz), 7.54 (d, 1H, *J*= 8.61 Hz) 7.51-7.44 (m, 1H) 7.41 (d, 1H, *J*= 8.79 Hz), 7.19 (br s, 1H), 6.90 (s, 1H), 6.68 (s, 1H), 6.55 (br s, 1H), 6.32-6.29 (m, 1H), 5.89-5.75 (m, 2 H), 1.80 (d, 3H, *J*= 6.23 Hz); HRMS calc'd for C₂₅H₂₀F₃N₄O₂S [M+H]⁺ 497.1259, found 497.1261.

### Example 103: 5-[5-(2-pyridinyl)- 1H-benzimidazol-1-yl]-3-({(1R)-1-[2-(trifluoromethyl)phenyl]ethy}oxy)-2-thiophenecarboxamide

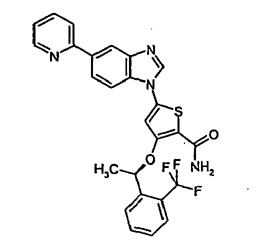

### Step A - methyl 5-[5-(2-pyridinyl)-1H-benzimidazol-1-yl]-3-({(1R)-1-[2-(trifluoromethyl)phenyl]ethyl}oxy)-2-thiophenecarboxylate

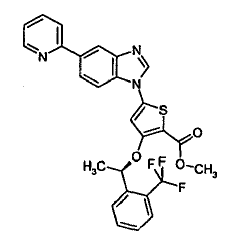

2-Pyridylboronic acid (103 mg, 0.84 mmol) in *N,N*-dimethylacetamide (6 mL) was treated with aqueous 1 N sodium carbonate (1.5 mL, 1.5 mmol), methyl 5-(5-bromo-1*H-*benzimidazol-1-yl)-3-({(1*R*)-1-[2-(trifluoromethyl)phenyl]-ethyl}oxy)-2-thiophenecarboxylate (207 mg, 0.42 mmol) and 1,1'-bisdiphenylphosphinoferrocene dichloropalladium(II) dichloromethane adduct (69 mg, 0.09 mmol) and heated to 80 °C for 8 h. After cooling to room temperature the reaction was partitioned between 5:1 chloroform: methanol and saturated aqueous sodium bicarbonate. The organic layer was washed with saturated aqueous sodium chloride. The layers were separated and the organic layer was dried over sodium sulfate. The solution was filtered and concentrated. The crude compound was purified by silica gel chromatography (hexanes:ethyl acetate, with 0.5% triethylamine) to yield 50 mg (23%) of the title compound as an oil. MS (APCI): 523.91 [M+H]⁺.

### Step B - 5-[5-(2-pyridiny/}-1H-benzimidazol-1-yl]-3-({(1R)-1-[2 - (trifluoromethyl)phenyl]ethyl}oxy)-2-thiophenecarboxamide (Title Compound)

Methyl 5-[5-(2-pyridinyl)-1H-benzimidazol-1-yl]-3-({(1*R*-1-[2-(trifluoromethyl)phenyl]ethyl}oxy)-2-thiophenecarboxylate (50 mg, 0.1 mmol) was dissolved in 7 N ammonia in methanol (6 mL) and placed in a sealed vessel. The mixture was heated to 80 °C for 14 hours and then cooled to room temperature. The crude reaction mixture was concentrated to a residue. The residue was purified by silica gel chromatography (hexanes:ethyl acetate, with 0.5% triethylamine) to yield 9 mg (18%) of the title compound as an orange solid. ¹H NMR (400 MHz, DMSO-*d₆*): δ 8.65 (d, 1H, *J*= 4.21 Hz), 8.58 (s, 1H), 8.44 (s, 1H), 8.13 (d, 1H, *J*= 8.61 Hz), 8.07 (d, 1H, *J*= 8.06 Hz), 7.92 (d, 1H *J*= 8.24 Hz), 7.90-7.80 (m, 2H), 7.80-7.70 (m, 2H), 7.57-7.52 (m, 2H), 7.33 (dd, 1H, *J*= 5.04, 7.05 Hz), 7.13 (s, 2H), 5.95 (q, 1H, *J*= 5.92 Hz), 1.73 (d, 3H, *J*= 6.04 Hz); HRMS calc'd. for C₂₆H₂₀F₃N₄O₂S [M+H]⁺ 509.1259, found 509.1253.

### Example 104: 5-[5-(6-amino-3-pyridazinyl)-1H-benzimidazol-1-yl]-3-{[(1R)-1-(2-chlorophenyl)ethyl]oxy}-2-thiophenecarboxamide

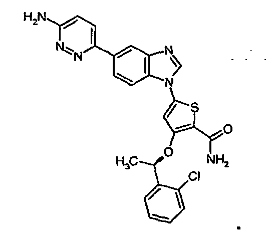

### Step A - 6-iodo-3-pyridazinamine

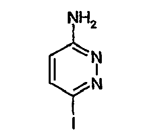

6-Chloro-3-pyridazinamine (4.0 g, 31 mmol) was dissolved in hydriodic acid (57% in water, 32 mL, 244 mmol) and heated to 100 °C overnight. The reaction was cooled to room temperature and ethyl acetate was added. The reaction mixture was sonicated, filtered, and washed with more ethyl acetate. The resulting solid was dissolved in methanol and treated with sodium hydroxide pellets (1.3 g, 33 mmol), refluxed for 5 minutes, then cooled to room temperature. The solvent was concentrated, then water (140 mL) was added and the mixture stirred for 15 minutes. The title compound was then filtered off as an off-white solid of 5.2 g (76%). ¹H NMR (300 MHz, DMSO-*d₆*): δ 7.54 (d, 1H, *J*= 9.16 Hz), 6.58-6.52 (m, 3H).

### Step B - bis(1,1-dimethylethyl) (6 iodo-3-pyridazinyl)imidodicarbonate

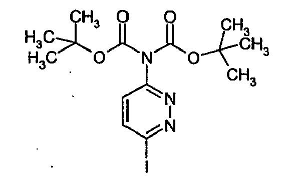

6-iodo-3-pyridazinamine (1.0 g, 4.5 mmol) dissolved in dichloromethane (15 mL) was treated with 4-*N,N-*dimethylaminopyridine (28 mg, 0.23 mmol) and di-*tert-*butyldicarbonate (3.0 g, 14 mmol). The reaction mixture was stirred at room temperature overnight. The reaction mixture was concentrated and the residue was purified by silica gel chromatography (hexanes:ethyl acetate) to yield 1.22 g (64%) of the title compound as a white solid. ¹H NMR (400 MHz, DMSO-*d₆*): δ 8.22 (d, 1H, *J*= 8.97 Hz), 7.61 (d, 1H, *J*= 8.79 Hz), 1.36 (s, 18H).

### Step C - bis(1,1-dimethylethyl) {6-[1-(5-(aminocarbony/)-4-{[(1R)-1-(2-chlorophenyl)ethyl]oxy}-2-thienyl)-1H-benzimidazol-5-yl]-3-pyridazinyl)imidodicarbonate

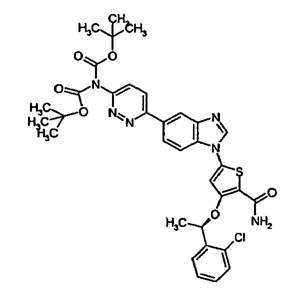

3-{[(1*R*-1-(2-chlorophenyl)ethyl]oxy}-5-[5-(4,4,5,5-tetramethyl-1,3,2-dioxaborolan-2-yl)-1*H-*benzimidazol-1-yl]-2-thiophenecarboxamide (215 mg, 0.41 mmol) in *N,N-*dimethylacetamide (3 mL) was treated with bis(1,1-dimethylethyl) (6-iodo-3-pyridazinyl)imidodicarbonate (260 mg, 0.62 mmol), aqueous 1 N sodium carbonate (0.82 mL, 0.82 mmol), and 1,1'-bisdiphenylphosphinoferrocene dichloropalladium(II) dichloromethane adduct (33 mg, 0.04 mmol) and heated to 80 °C for 3 h. After cooling to room temperature the reaction was partitioned between ethyl acetate and water. The layers were separated and the organic layer was dried over sodium sulfate. The solution was filtered and concentrated. The crude compound was purified by silica gel chromatography (hexanes:ethyl acetate) to yield 104 mg (37%) of the title compound as a brown solid. MS (ESI): 691.29 [M+H]⁺

### Step D -5-[5-(6-amino-3-pyridazinyl)-1H-benzimidazol-1-yl]-3-{([(1R)-1-(2-chlorophenyl)ethyl]oxy}-2-thiophenecarboxamide (Title Compound)

Bis(1,1-dimethylethyl) {6-[1-(5-(aminocarbonyl)-4-{[(1*R*)-1-(2-chlorophenyl)ethyl]oxy}-2-thienyl)-1*H*-benzimidazol-5-yl]-3-pyridazinyl}imidodicarbonate (74 mg, 0.11 mmol) was dissolved in dichloromethane (10 mL) and treated with trifluoroacetic acid (3 mL, 39 mmol). The reaction stirred for 2 h and was neutralized with saturated aqueous sodium bicarbonate. The layers were separated and the organic layer was dried over sodium sulfate, filtered, and concentrated. The residue was purified by silica gel chromatography (dichloromethane: dichloromethane/methanol/ammonium hydroxide 90:9:1) to yield 22 mg (41%) of the title compound as a pink solid. ¹H NMR (300 MHz, DMSO-*d₆*): δ 8.65 (s, 1H), 8.33 (s, 1H), 8.12 (d, 1H, *J*= 8.70 Hz), 7.97 (d, 1H, *J*= 9.41 Hz), 7.86 (s, 1H), 7.73 (d, 1H *J*= 7.44 Hz), 7.63 (d, 1H, *J*= 8.70 Hz), 7.56 (d, 1H, *J*= 7.72 Hz), 7.52-7.37 (m, 2H), 7.26 (s, 1H), 7.12 (s, 1H), 6.90 (d, 1H, *J*= 9.41 Hz), 6.50 (s, 2H), 6.04 (q, 1H, *J*= 6.27 Hz), 1.78 (d, 3H, *J*= 6.32 Hz); HRMS calc'd for C₂₄H₂₀ClN₆O₂S [M+H]⁺ 491.1050, found 491.1048.

### Example 105: 3-{[(1R)-1-(2-chlorophenyl)ethyl]oxy}-5-[5-(3,5-dimethyl-4-isoxazolyl)-1H-benzimidazol-1-yl]-2-thiophenecarboxamide

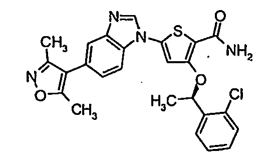

### Step A - methyl 3-{[(1R)-1-(2-chlorophenyl)ethyl]oxy}-5-[5(3,5-dimethyl-4-isoxazolyl)-1H-benzimidazol-1-yl]-2-thiophenecarboxylate

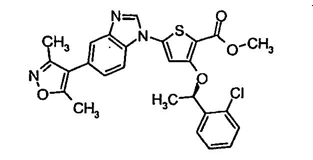

Methyl 5-(5-bromo-1*H*-benzimidazol-1-yl)-3-{[(1*R*)-1-(2-chlorophenyl)ethyl]-oxy}-2-thiophenecarboxylate (250 mg, 0.51 mmol) and (3,5-dimethyl-4-isoxazolyl)boronic acid (141 mg, 1.0 mmol) were coupled according to the procedure of **Example 98**, Step A to give 80 mg (31 %) of the title compound. HRMS calc'd for C₂₆H₂₃ClN₃O₄S [M+H]⁺ 508.10923, found 508.10965. *Step B -3-{[(1*R*)-1-(2-chlorophenyl)ethyl]oxy}-5-[5(3,5-dimethyl-4-isoxazolyl)-1*H*-benzimidazol-1-yl]-2-thiophenecarboxamide (Title Compound)* Methyl 3-{[(1*R*)-1-(2-chlorophenyl)ethyl]oxy}-5-[5-(3,5-dimethyl-4-isoxazolyl)-1*H*-benzimidazol-1-yl]-2-thiophenecarboxylate (75 mg, 0.15 mmol) was subjected to aminolysis according to the procedure of **Example 98**, Step B to give 47 mg (64%) of the title compound (64%) after purification by flash column chromatography. ¹H NMR (400 MHz, DMSO-*d₆*): δ 8.61 (s, 1H), 7.81 (br s, 1H), 7.76 (s, 1H), 7.67 (dd, 1H, *J*= 1.4, 7.8), 7.59 (d, 1H, *J*= 8.4 Hz), 7.48 (d, 1H, *J*= 7.6 Hz), 7.41 (m, 1H), 7.37-7.33 (m, 2H), 7.22 (s, 1H), 7.10 (br s, 1H), 5.97 (m, 1H), 2.38 (s, 3H), 2.21 (s, 3H), 1.71 (d, 3H, *J*= 6.4 Hz); HRMS calc'd. for C₂₅H₂₂ClN₄O₃S [M+H]⁺ 493.10957, found 493.10892.

### Example 106: 5-(6-chloro-5-hydroxy-1H-benzimidazol-1-yl)-3-{[(1R)-1-(2-chlorophenyl)ethyl]oxy}-2-thiophenecarboxamide

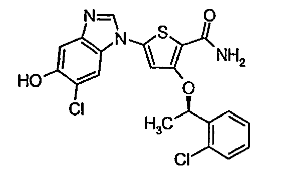

### Step A - {2-[(2-chloro-4-nitrophenyl)oxy]ethyl}(trimethyl)silane

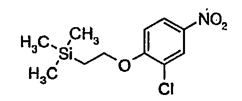

3-Chloro-4-nitrobenzene (7.50 g, 42.7 mmol) and 2-(trimethylsily))ethanol (12.2 mL, 85.1 mmol) were dissolved in dichloromethane (160 mL) with stirring. Tetrabutylammonium hydrogensulfate (1.45 g, 4.27 mmol) was added followed by aqueous 1 N sodium hydroxide solution (160 mL, 160 mmol). The mixture was stirred for 2.5 days. The pH was adjusted to acidic with concentrated sulfuric acid. The layers were separated, and the aqueous layer was washed with ethyl acetate. The combined organic layers were dried over magnesium sulfate, filtered, and concentrated *in vacuo.* Purification by flash chromatography afforded 9.31 g (80%) of the title compound. ¹H NMR (300 MHz, DMSO-*d₆*): δ 8.33 (d, 1H, *J*= 2.8 Hz), 8.24 (dd, 1H, *J*= 2.8, 9.2 Hz), 7.42 (d, 1H, *J*= 9.2 Hz), 4.37 (m, 2H), 1.19 (m, 2H), 0.12 (s, 9H).

### Step B - (3-chloro-4-{[2-(trimethylsilyl)ethyl]oxy}phenyl)amine

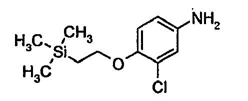

(2-[(2-Chloro-4-nitrophenyl)oxy]ethyl}(trimethyl)silane (9.31 g, 34.0 mmol) was dissolved in ethyl acetate (150 mL) with stirring. Sulfided platinum on carbon (2.65 g, 5 wt%, 0.679 mmol) was added in a single portion. The reaction was placed under 1 atm of hydrogen and stirred for 24 h. The mixture was filtered through Celite and washed with ethyl acetate. The filtrate was concentrated *in vacuo.* Purification by flash chromatography gave 6.76 g (82%) of the title compound. ¹H NMR (400 MHz, DMSO-*d₆*): δ 6.81 (d, 1H, *J*= 8.7 Hz), 6.60 (d, 1H, *J*= 2.7 Hz), 6.44 (dd, 1 H, *J*= 2.7, 8.7 Hz), 4.85 (br s, 2H), 3.94 (m, 2H), 1.01 (m, 2H), 0.03 (s, 9H).

### Step C - N-(5-chloro-2-nitro-4-{[2-(trimethylsilyl)ethyl]oxy}phenyl)-2,2,2 trifluoroacetamide

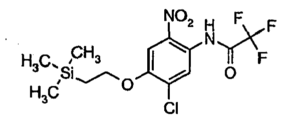

(3-Chloro-4-{[2-(trimethylsilyl)ethyl]oxy}phenyl)amine (6.76 g, 27.7 mmol) was dissolved in chloroform (150 mL) with stirring. Ammonium nitrate (5.32 g, 66.5 mmol) was added in a single portion. Trifluoroacetic anhydride (28.2 mL, 200 mmol) was added dropwise via addition funnel. The reaction was stirred for 3 h and quenched by the slow addition of aqueous sodium bicarbonate solution (20 g in 200 mL water). Saturated sodium bicarbonate was then used to adjust the pH to basic. The layers were separated, and the aqueous layer was washed with dichloromethane (1x) and ethyl acetate (1x). The combined organic layers where dried over magnesium sulfate, filtered, and concentrated *in vacuo* to afford 9.41 g (88%) of the title compound. ¹H NMR (400 MHz, DMSO-*d₆*): δ 11.55 (br s, 1H), 7.79 (s, 1H), 7.75 (s, 1H), 4.29 (m, 2H), 1.13 (m, 2H), 0.07 (s, 9H).

### Step D - (5-chloro-2-nitro-4-{[2-(trimethylsilyl)ethyl]oxy}phenyl)amine

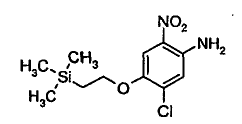

*N-*(5-Chloro-2-nitro-4-{[2-(trimethylsilyl)ethyl]oxy}phenyl)-2,2,2-trifluoroacetamide (9.41 g, 24.5 mmol) was dissolved in 1,4-dioxane (80 mL) with stirring. Aqueous 1 N sodium hydroxide solution (60 mL, 60 mmol) was added, and the reaction was heated to 50 °C for 2 h. The reaction was cooled to room temperature and poured into ethyl acetate and saturated aqueous sodium chloride. The layers were separated, and the organic layer washed with saturated aqueous sodium chloride. The combined aqueous layers were extracted with ethyl acetate. The combined organic layers were dried over magnesium sulfate, filtered, and concentrated *in vacuo.* Purification by flash chromatography gave 5.41 g (76%) of the title compound. ¹H NMR (400 MHz, DMSO-*d₆*): δ 7.51 (s, 1H), 7.27 (br s, 1H), 7.17 (s, 1H), 4.08 (m, 2H), 1.07 (m, 2H), 0.05 (s, 9H).

### Step E-{2-[(4-bromo-2-chloro-5-nitrophenyl)oxy]ethyl}(trimethyl)silane

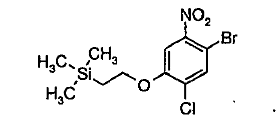

Copper(II) bromide (6.27 g, 28.1 mmol) was suspended in acetonitrile (40 mL) with stirring. *Tert-*butyl nitrite (4.90 mL, 41.2 mmol) was added and the mixture was heated to 60°C.(5-Chloro-2-nitro-4-{[2-(trimethylsilyl)ethyl]-oxy}phenyl)-amine (5.41 g, 18.7 mmol) in acetonitrile (60 mL) was added dropwise *via* addition funnel. The residue was further rinsed with acetonitrile (20 mL). The reaction was stirred an additional 10 min and cooled to room temperature. The reaction was poured into 2N aqueous hydrochloric acid (400 mL). The mixture was extracted with 1:1 ethyl acetate/hexanes, and the organic layer was washed with saturated aqueous sodium chloride. The combined aqueous layers were extracted with ethyl acetate. The combined organic layers were dried over magnesium sulfate, filtered, and concentrated *in vacuo.* Purification by flash chromatography gave 5.04 g (76%) of the title compound. ¹H NMR (400 MHz, DMSO-*d₆*): δ 8.03 (s, 1H), 7.86 (s, 1H), 4.22 (m, 2H), 1.12 (m, 2H), 0.06 (s, 9H).

### Step F - Methyl 5-({5-chloro-2-nitro-4-[2-(trimethylsilyl)ethyl]pheny/}amino)-3-{[(1R)-1-(2-chlorophenyl)ethyl]oxy}-2-thiophenecarboxylate

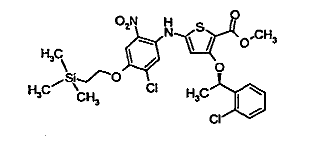

{2-[(4-Bromo-2-chloro-5-nitrophenyl)oxy]ethyl}(trimethyl)silane (1.50 g, 4.25 mmol) and methyl 5-amino-3-{[(1*R*)-1-(2-chlorophenyl)ethyl]oxy}-2-thiophenecarboxylate (1.33 g, 4.27 mmol) were dissolved in 40 mL of dioxane with stirring and degassed with nitrogen for 30 min. Cesium carbonate (6.92 g, 21.2 mmol), 4,5-bis(diphenylphosphino)-9,9 dimethylxanthene (0.108 g, 0.187 mmol), and tris(dibenzylideneacetone) dipalladium (0) (0.0782 g, 0.0854 mmol) were added. The mixture was heated at 60°C for 20 hours and cooled to room temperature. The mixture was adsorbed onto silica gel. Purification by flash chromatography afforded 1.10 g (44%) of the title compound. ¹H NMR (400 MHz, DMSO-*d₆*): δ 9.56 (s, 1H), 7.71 (s, 1H), 7.61-7.19 (m, 5H), 6.36 (s, 1H), 5.68 (q, 1H, *J*= 6.2 Hz), 4.20 (m, 2H), 3.69 (s, 3H), 1.52 (d, 3H, *J*= 6.2 Hz), 1.10 (m, 2H), 0.05 (s, 9H).

### Step G-methyl 3{[(1R)-1-(2-chlorophenyl)ethyl]oxy}-5-(6-chloro-5-{[2-(trimethy/sily/)ethy/]oxy}-1H-benzimidazo/-1-y/)-2-thiophenecarboxylate

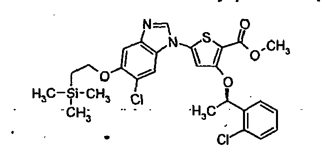

Methyl 5-({5-chloro-2-nitro-4-[2-(trimethylsilyl)ethyl]phenyl}amino)-3-{[(1*R*)*-1-*(2-chlorophenyl)ethyl]oxy)-2-thiophenecarboxylate (1.10 g, 1.88 mmol) was dissolved in trimethyl orthoformate (40 mL). Pyridinium p-toluenesulfonate (0.0236 g, 0.0939 mmol) and sulfided platinum on carbon (0.147 g, 5 wt%, 0.0377 mmol) was added in a single portion. The reaction was placed under 1 atm of hydrogen and stirred for 24 hours. An additional amount of sulfided platinum on carbon (0.147 g, 5 wt%, 0.0377 mmol) was added and stirring was continued for 24 hours. The mixture was filtered through Celite and washed with ethyl acetate. The filtrate was concentrated *in vacuo.* Purification by flash chromatography gave 0.491 g (46%) of the title compound. ¹H NMR (400 MHz, DMSO-*d₆*): δ 8.60 (s, 1H), 7.71 (dd, 1H, *J*= 1.7, 7.9 Hz), 7.62 (s, 1H), 7.52 (s, 1H), 7.49-7.39 (m, 2H), 7.36 (s, 1H), 7.34 (m, 1H), 5.99 (q, 1H, *J*= 6.4 Hz), 4.20 (m, 2H), 3.81 (s, 3H), 1.62 (d, 3H, *J*= 6.4 Hz), 1.13 (m, 2H), 0.07 (s, 9H).

### Step H -methyl 5-(6-chloro-5-hydroxy-1H-benzimidazol-1-y/}-3-{[(1R)-1-(2-ch/oropheny/)ethy]oxy}-2-thiophenecarboxylate

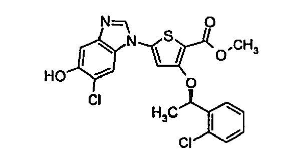

Methyl 3-{[(1*R*)-1-(2-chlorophenyl)ethyl]oxy}-5-(6-chloro-5-{[2-(trimethylsilyl)ethyl]oxy}-1*H*-benzimidazol-1-yl)-2-thiophenecarboxylate (0.490 g, 0.869 mmol) was dissolved in tetrahydrofuran (20 mL) with stirring. Tetrabutylammonium fluoride (1.0 M in tetrahydrofuran, 1.0 mL, 1.0 mmol) was added *via* syringe. After 1 h, an additional amount of tetrabutylammonium fluoride (1.0M in tetrahydrofuran, 0.50 mL, 0.50 mmol) was added. After an additional 30 minutes, the reaction was poured into 0.5 N aqueous sodium bisulfate and extracted with ethyl acetate. The organic layer was washed with saturated aqueous sodium chloride, and the combined aqueous layers were extracted with ethyl acetate. The combined organic layers were dried over magnesium sulfate, filtered, and concentrated *in vacuo.* Purification by flash chromatography gave 0.300 g (74%) of the title compound. ¹H NMR (400 MHz, DMSO-*d₆*): δ 10.15 (s, 1H), 8.54 (s, 1H), 7.69 (dd, 1H, *J*= 1.7, 7.7 Hz), 7.53 (s, 1H), 7.48-7.37 (m, 2H), 7.36-7.30 (m, 2H), 7.25 (s, 1H), 5.97 (q, 1H, *J*= 6.2 Hz), 3.79 (s, 3H), 1.60 (d, 3H, *J*= 6.4 Hz).

### Step l-5-(6-chloro-5-hydroxy-1H-benzimidazol-1-yl)-3-{[(1R)-1-(2-chlorophenyl)ethyl]oxy}-2-thiophenecarboxamide (Title Compound)

Methyl 5-(6-chloro-5-hydroxy-1*H*-benzimidazol-1-yl)-3-{[(1*R)-1-(2-*chlorophenyl)ethyl]oxy}-2-thiophenecarboxylate (0.299 g, 0.645 mmol) was dissolved in 7.0 N ammonia in methanol (12.0 mL, 84 mmol) in a pressure vessel with a stir bar. The vessel was sealed and heated to 80°C for 2.5 days. The reaction was cooled to room temperature and concentrated. Purification by flash chromatography followed by an additional purification using reverse phase chromatography afforded 0.0744 g (26%) of the title compound. ¹H NMR (400 MHz, DMSO-*d₆*): δ 10.28 (br s, 1H), 8.44 (s, 1H), 7.77 (br s, 1H), 7.65 (dd, 1H, *J*= 1.7, 7.7 Hz), 7.48 (dd, 1H, *J*= 1.3, 7.9 Hz), 7.43-7.32 (m, 3H), 7.22 (s, 1H), 7.09 (s, 1H), 7.09 (br s, 1H), 5.98 (q, 1H, *J*= 6.2 Hz), 1.70 (d, 3H, *J*= 6.2 Hz); MS (ESI): 448 [M+H]⁺.

### Example 107: 5-[5-(6-Amino-2-methyl-3-pyridinyl)-1H-benzimidazol-1-yl]-3-{[(1R)-1-(2-chlorophenyl)ethyl]oxy}-2-thiophenecarboxamide

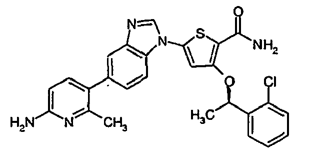

The title compound was prepared from 3-{[(1*R*)-1-(2-chlorophenyl)ethyl]oxy}-5-[5-(4,4,5,5-tetramethyl-1,3,2-dioxaborolan-2-yl)-1*H*-benzimidazol-1-yl]-2-thiophenecarboxamide and 6-amino-3-bromo-2-methylpyridine by a procedure analogous to Example 59, Step A. Purification by column chromatography (10 to 40% 1/9/90 ammonium hydroxide/methanol/dichloromethane:dichloromethane) provided 0.080 g (40%) of the title compound as a beige solid. ¹H NMR (400 MHz, CDCl₃): δ 7.98 (s, 1H), 7.70 (s, 1H), 7.48-7.40 (m, 3H), 7.36-7.22 (m, 4H), 7.19 (br s, 1H), 6.64 (s, 1H), 6.42 (d, 1H, *J*= 8.2 Hz), 5.87 (q, 1H, *J* = 6.4 Hz), 5.82 (br s, 1H), 4.51 (br s, 2H), 2.35 (s, 3H), 1.77 (d, 3H, *J*= 6.2 Hz); MS (ESI): 504.2 [M+H]⁺.

### Example 108: 3-{[(1R)-1-(2-Chlorophenyl)ethyl]oxy}-5-[5-(1-methyl-1H-imidazol-5-yl)-1H-benzimidazol-1-yl]-2-thiophenecarboxamide

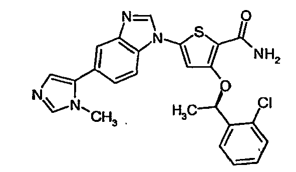

The title compound was prepared from 3-{[(1*R*)-1-(2-chlorophenyl)ethyl]oxy}-5-[5-(4,4,5,5-tetramethyl-1,3,2-dioxaborolan-2-yl)-1*H*-benzimidazol-1-yl]-2-thiophenecarboxamide and 5-bromo-1-methyl-1*H*-imidazole by a procedure analogous to Example 59, Step A. Purification by column chromatography (10 to 90% 1/9/90 ammonium hydroxide/methanol/dichloromethane: dichloromethane) provided 0.050 g (30%) of the title compound as a tan solid. ¹H NMR (400 MHz, CDCl₃): δ 8.00 (s, 1H), 7.81 (s, 1H), 7.55 (s, 1H), 7.47-7.40 (m, 3H), 7.36-7.25 (m, 3H), 7.19 (br s, 1H), 7.12 (s,1H), 6.64 (s, 1H), 5.94 (br s, 1H),5.87 (q, 1H, *J*= 6.3 Hz), 3.67 (s, 3H), 1.77 (d, 3H, *J*= 6.4 Hz); MS (APCI): 478.1 [M+H]⁺.

### Example 109: 5-[5-(2-Amino-5-pyrimidinil)-1H-benzimidazol-1-yl]-3-({(1R)-1-[2-(trifluoromethyl)phenyl]ethyl}oxy)-2-thiophenecarboxamide

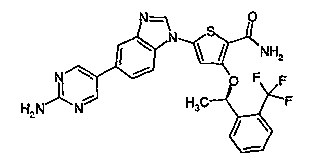

The title compound was prepared from 5-[5-(4,4,5,5-tetramethyl-1,3,2-dioxaborolan-2-yl)-1*H*-benzimidazol-1-yl]-3-({(1*R*)-1-[2-(trifluoromethyl)phenyl]ethyl}oxy)-2-thiophenecarboxamide and 5-iodo-2-pyrimidinamine by a procedure analogous to Example 59, Step A. Purification by column chromatography (10 to 80% 1/9/90 ammonium hydroxide/methanol/dichloromethane:dichloromethane) provided 0.018 g (37%) of the title compound as a tan solid.¹H NMR (400 MHz, CDCl₃): δ 8.58 (s, 1H), 7.97 (s, 1H), 7.90 (s, 1H), 7.75-7.69 (m, 2H), 7.67-7.61 (m, 1H), 7.53-7.47 (m, 2H), 7.46-7.42 (m, 1H), 7.21 (br s, 1H), 6.68 (s, 1H), 5.87 (m, 2H), 5.27 (br s, 2H), 1.82 (d, 3H, *J*= 6.2 Hz); MS (ESI): 525.2 [M+H]⁺.

### Example 110: 5-{5-[2-(Dimethylamino)-4-pyridinyl]-1H-benzimidazol-1-yl}-3-({(1R)-1-[2-(trifluoromethyl)phenyl]ethyl}oxy)-2-thiophenecarboxamide

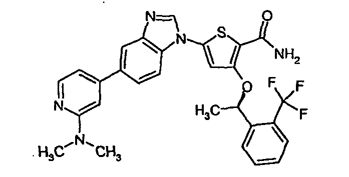

A solution of 5-[5-(2-fluoro-4-pyridinyl)-1*H*-benzimidazol-1-yl]-3-({(1*R*)-1-[2-(trifluoromethyl)phenyl]ethyl}oxy)-2-thiophenecarboxamide (0.050 g, 0.09 mmol) and dimethylamine (1.0 mL, 40% wt. in water) and ethanol (95%, 1.0 mL) was heated in a sealed tube at 115 °C for 4 h after which time the mixture was diluted with dichloromethane and concentrated onto silica gel. Purification by column chromatography (5 to 70% 1/9/90 ammonium hydroxide/methanol/dichloromethane:dichloromethane) provided 0.025 g (48%) of the title compound as a light yellow solid. ¹H NMR (400 MHz, CDCl₃): δ 8.22 (d, 1H, *J*= 5.5 Hz), 8.05 (s, 1H), 7.96 (s, 1H), 7.74-7.67 (m, 2H), 7.63 (t, 1H, *J*= 7.4 Hz), 7.58 (dd, 1H, *J*= 8.6, 1.5 Hz), 7.51-7.44 (m, 2H), 7.19 (br s, 1H), 6.82 (br s, 1H), 6.73 (s, 1H), 6.66 (s, 1H), 5.86 (q, 1H, *J*= 6.2 Hz), 5.75 (br s, 1H), 3.17 (s, 6H), 1.80 (d, 3H, *J*= 6.2 Hz); MS (APCI): 552.0 [M+H]⁺.

### Example 111: 5-{5-[2-(Methylamino)-4-pyridinyl]-1H-benzimidazol-1-yl}-3-({(1R-1-[2-(trifluoromethyl)phenyl]ethyl}oxy)-2-thiophenecarboxamide

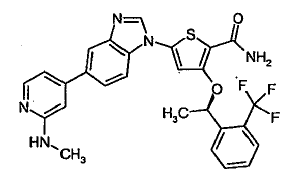

The title compound was prepared from 5-[5-(2-fluoro-4-pyridinyl)-1*H-*benzimidazol-1-yl]-3-({(1*R*)-1-[2-(trifluoromethyl)phenyl]ethyl}oxy)-2-thiophenecarboxamide and methylamine (33 wt%) by a procedure analogous to **Example 110.** Purification by column chromatography (5-70% 1/9/90 ammonium hydroxide/methanol/dichloromethane:dichloromethane) provided 0.030 g (49%) of the title compound as a yellow solid. ¹H NMR (400 MHz, CDCl₃): δ 8.15 (d, 1H, *J*= 5.5 Hz), 8.05 (s, 1H), 7.97 (s, 1H), 7.74-7.68 (m, 2H); 7.64 (t, 1H, *J*= 7.6 Hz), 7.58 (dd, 1H, *J*= 8.6, 1.5 Hz), 7.52-7.45 (m, 2H), 7.21 (br s, 1H), 6.86 (dd, 1H, *J*= 5.3, 1.3 Hz), 6.68 (s, 1H), 6.62 (s, 1H), 6.01 (br s, 1H), 5.87 (q, 1H, *J*= 6.2 Hz), 4.77 (d, 1H, *J*= 4.0 Hz), 3.00 (s, 3H), 1.81 (d, 3H, *J*= 6.2 Hz); MS (ESI): 538.2 [M+H]⁺.

### Example 112:5-[5-(2-Methyl-4-pyridinyl)-1H-benzimidazol-1-yl]-3-({(1R)-1=[2-(trifluoromethyl)phenyl]ethyl}oxy)-2-thiophenecarboxamide

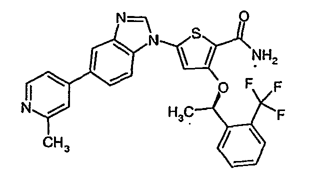

The title compound was prepared from 5-(5-bromo-1*H*-benzimidazol-1-yl)-3-({1-[2-(trifluoromethyl)phenyl]ethyl}oxy)-2-thiophenecarboxamide and 2-picoline-4-boronic acid by a procedure analogous to **Example 59**, Step A. Purification by column chromatography (10-80% 1/9/90 ammonium hydroxide/methanol/dichloromethane:dichloromethane) provided 0.084 g (82%) of the title compound as a light yellow solid. 1H NMR (400 MHz, CDCl3): δ 8.56 (d, 1H, *J*= 5.1 Hz), 8.07 (s, 1H), 7.99 (s, 1H), 7.74-7.69 (m, 2H), 7.67-7.58 (m, 2H), 7.54-7.43 (m, 3H), 7.39 (d, 1H, *J*= 5.3 Hz), 7.23 (br s, 1H), 6.69 (s, 1H), 6.51 (br s, 1H), 5.87 (q, 1H, *J*= 6.2 Hz), 2.65 (s, 3H), 1.81 (d, 3H, *J*= 6.2 Hz); MS (ESI): 523.1 [M+H]+.

### Example 113: 3-{[(1R)-1-(2-Chlorophenyl)ethyl]oxy}-5-(6-{[4-dimethylamino)-1-piperidinyl]methyl}-1H-benzimidazol-1-yl)-2-thiophenecarboxamide

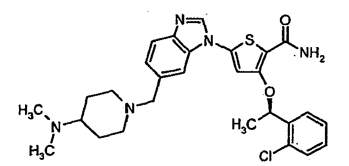

### Step A - methy/5-{6-[(4-amino-1-piperidinyl)methyl]-1H-benzimidazo/-1-yl}-3-{[(1R)-1-(2-chlorophenyl)ethyl]oxy}-2-thiophenecarboxylate

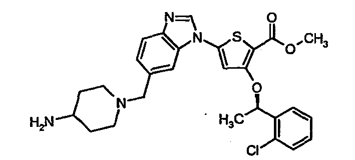

To a stirred solution of methyl 3-{[(1*R*)-1-(2-chlorophenyl)ethyl]oxy}-5-(6-formyl-1*H*-benzimidazol-1-yl)-2-thiophenecarboxylate (2.0 g, 4.54 mmol), 1,1-dimethylethyl 4-piperidinylcarbamate (2.5 g, 41.2 mmol) and acetic acid (0.33 g, 15.0 mmol) in dichloroethane (50 mL) was added sodium triacetoxyborohydride (2.63 g, 12.47 mmol). The reaction was stirred for 3.0 h, then trifluoroacetic acid (17 mL) was added with ice bath cooling. The ice bath was removed and the mixture was allowed to stir at room temperature for 30 min. Solid potassium carbonate was added to neutralize the trifluoroacetic acid. The solids were removed by filtration. Silica gel (50 g) was added to the filtrate and the volatiles were evaporated under reduced pressure, and the residue was purified by flash column chromatography (0 to 100% 80/20/1 dichloromethane/methanol/ammonium hydroxide:dichloromethane) to afford 2.25 g (94%) of the title compound as solid. MS (ESI): 525 [M+H]⁺.

### Step B - methyl 3-{[(1R)- 1-(2-chlorophenyl)ethyl]oxy}-5-(6-{[4-(dimethylamino)-1-piperidinyl]methyl}-1H-benzimidazol-1-yl)-2-thiophenecarboxylate

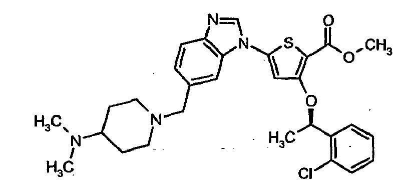

To a solution of methyl 5-{6-[(4-amino-1-piperidinyl)methyl]-1*H*-benzimidazol-1-y}-3-{[(1*R*)-1-(2-chlorophenyl)ethy)]oxy}-2-thiophenecarboxylate (2.25 g, 4.29 mmol) in 3:2 dichloromethane:methanol (50 mL) was added formaldehyde (0.386 g, 12.85 mmol), followed by sodium triacetoxyborohydride (4.72 g, 22.3 mmol) and the mixture stirred at room temperature for 2 h. Silica gel (10 g) was added, the volatiles were evaporated under reduced pressure and the residue was purified by flash column chromatography (0 to 100% 80/20/1 dichloromethane/methanol/ammonium hydroxide:dichloromethane) to afford 2.16 g (91 %) of the title compound as a light yellow solid. MS (ESI): 553 [M+H]⁺.

### Step C - 3-{[(1R)- 1-(2-chlorophenyl)ethyl]oxy}-5-(6-{[4-(dimethylamino)-1-piperidinyl]methyl}-1H-benzimidazol-1-yl)-2-thiophenecarboxamide (Title Compound)

A mixture of methyl 3-{[(1*R*)-1-(2-chlorophenyl)ethyl]oxy}-5-(6-{[4-(dimethylamino)-1-piperidinyl]methyl}-1*H*-benzimidazol-1-yl)-2-thiophenecarboxylate (2.16 g, 3.91 mmol) and 7 N ammonia in methanol (100 mL) was added to a high-pressure glass reaction flask. The flask was sealed, then heated to 80 °C for ∼40 h. The flask was cooled to room temperature, opened, and the reaction mixture concentrated under vacuum, then purified by silica gel chromatography (0 to 100% 80/20/1 dichloromethane/methanol/ammonium hydroxide:dichloromethane) to afford 1.76 g (84%) of the title compound as a light yellow solid. ¹H NMR (400 MHz, CD₃OD): δ 8.34 (s, 1H), 7.67 (d, 1H, *J*= 8.24 Hz), 7.60 (d, 1H, *J*= 7.51 Hz), 7.46 (d, 2H, *J*= 9.34 Hz), 7.36 (m, 3H), 7.01 (s, 1H), 6.04 (q, 1H, *J*= 6.35 Hz), 3.63 (s, 2H), 2.97 (d, 2H, *J*= 11.35 Hz), 2.55 (m, 2H), 2.47 (s, 3H), 2.07 (t, 2H, *J*= 11.99 Hz), 1.91 (d, 2H, *J*= 12.09 Hz),1.78 (d, 3H, *J*= 6.23 Hz), 1.58 (q, 2H, *J*= 12.03 Hz); MS (ESI): 538 [M+H]⁺.

### Example 114: 5-{6-[(Aminosulfonyl)methyl]-1H-benzimidazol-1-yl}-3-{[(1R)-1-(2-chlorophenyl)ethyl]oxy}-2-thiophenecarboxamide

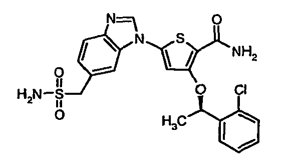

### Step A - methyl 3-{[(1R)-1-(2-chloropheny/)ethyl]oxy}-5-[6-({[3-(methyloxy)-3-oxopropyl]thio}methy/}-1H -benzimidazol-1-yl]-2-thiophenecarboxylate

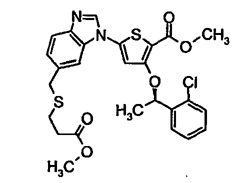

A mixture of methyl 5-[6-(chloromethyl)-1*H*-benzimidazol-1-yl]-3-{[(1*R*)-1-(2-chlorophenyl)ethyl]oxy}-2-thiophenecarboxylate (1.00 g, 2.17 mmol), methyl 3-mercaptopropionate (0.28 mL, 2.60 mmol) and potassium carbonate (0.509 g, 3.68 mmol) in *N,N*-dimethylformamide (10 mL) was stirred at 55 °C for 24 h, then at room temperature for 48 h. The reaction mixture was quenched with water (20 mL) and the whole was extracted with ethyl acetate (3 x 75 mL). The combined organic layers were washed with water (5 x 40 mL), dried over sodium sulfate and filtered. The filtrate was concentrated *in vacuo* to give 1.20 g (100%) of the title compound as a colorless foam. MS (ESI): 545 [M+H]⁺.

### Step B - methyl 3-{[(1R)-1-(2-chloropheny/)ethy/]oxy}-5-[6-({[3-(methyloxy)-3-oxopropy/]su/fony/}methyl)-1H-benzimidazol-1-yl]-2-thiophenecarboxylate

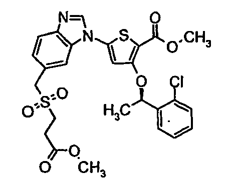

To a stirred solution of methyl 3-{[(1*R*)-1-(2-chlorophenyl)ethyl]oxy}-5-[6-({[3-(methyloxy)-3-oxopropyl]thio}methyl)-1H-benzimidazol-1-yl]-2-thiophenecarboxylate (1.18 g, 2.16 mmol) in dichloromethane (20 mL) at 0 °C was added 3-chloroperbenzoic acid (0.77 g, 4.44 mmol) in portions. The resultant mixture was allowed to stir at 0 °C for 2 h and then the reaction mixture was quenched with a saturated solution of sodium bicarbonate. The whole was extracted with dichloromethane (3 x 75 mL) and the combined organic extracts were dried over sodium sulfate, filtered and concentrated *in vacuo.* Purification of the residue by flash chromatography afforded 0.75 g (60%) of the title compound as a colorless foam. MS (ESI): 577 [M+H)⁺.

### Step C - methyl 5-{6-[(aminosulfonyl)methyl]-1H-benzimidazol-1-yl}-3-{[(1R)-1-(2-chlorophenyl)ethyl]oxy}-2-thiophenecarboxylate

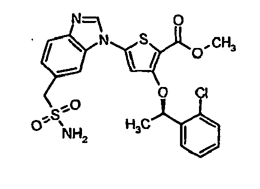

A solution of methyl 3-{[(1*R*)-1-(2-chlorophenyl)ethyl]oxy}-5-[6-({[3-(methyloxy)-3-oxopropyl]sulfonyl}methyl)-1*H*-benzimidazol-1-yl]-2-thiophenecarboxylate (0.600 g, 1.04 mmol) in dimethyl sulfoxide (2 mL) was treated with the dropwise addition of sodium methoxide (260 µL of a 25 wt% solution in MeOH, 1.04 mmol) at room temperature. After stirring for 20 min, a solution of hydroxylamine-O-sulfonic acid (0.588 g, 5.2 mmol) and sodium acetate (0.320 g, 2.35 mmol) in water (5 mL) was added and the resultant mixture was allowed to stir for 18 h. The reaction was diluted with ethyl acetate (20 mL) and water (20 mL) and the layers were separated. The aqueous layer was extracted with ethyl acetate (3 x 25 mL) and the combined organic extracts were washed with a saturated sodium chloride solution, dried over sodium sulfate, filtered and concentrated *in vacuo.* Purification of the residue by flash chromatography afforded 0.35 g (67%) of the title compound as a colorless foam. MS (ESI): 506 [M+H]⁺.

### Step D -5-{6-[(aminosulfonyl)methyl]-1H-benzimidazol-1-yl}-3-{[(1R)-1-(2-chlorophenyl)ethyl]oxy}-2-thiophenecarboxamide

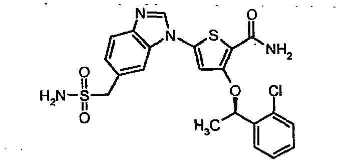

The title compound was prepared from methyl 5-{6-[(aminosulfonyl)methyl]-1 H-benzimidazol-1-yl}-3-{[(1*R*)-1-(2-chlorophenyl)ethyl]oxy}-2-thiophenecarboxylate by a procedure analogous to **Example 5**, Step D. ¹H NMR (400 MHz, DMSO-*d₆*): δ 8.58 (s, 1H), 7.83 (br s, 1H), 7.76-7.72 (m, 1H), 7.69-7.63 (m, 2H), 7.50-7.46 (m, 1H), 7.44-7.39 (m, 1H), 7.38-7.31 (m, 2H), 7.25 (s, 1H), 7.11 (br s, 1H), 6.79 (br s, 2H), 5.94 (q, 1H, J = 6.3 Hz), 4.43-4.36 (m, 2H), 1.72 (d, 3H, *J*= 6.3Hz); MS (ESI): 491 [M+H]⁺.

### Example 115: 5-{5-[3-Amino-4-methyloxy)phenyl]-1H-benzimidazol-1-yl}-3-{[(1R)-1-(2-chlorophenyl)ethyl]oxy}-2-thiophenecarboxamide

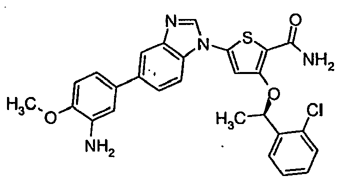

The title compound was prepared from 5-(5-bromo-1*H*-benzimidazol-1-yl)-3-{[(1*R*)-1-(2-chlorophenyl)ethyl]oxy)thiophene-2-carboxamide by a procedure analogous to **Example 7**. ¹H NMR (300 MHz, CD₃OD): δ 8.41 (s, 1H), 7.89 (s, 1H), 7.68-7.53 (m, 3H), 7.48-7.37 (m, 3H), 7.13 (d, 1H, *J*= 2.0 Hz), 7.05-6.95 (m, 3 H), 6.10 (q, 1H, *J*= 6.4 Hz), 3.94 (s, 3H), 1.84 (d, 2H, *J*= 5.9 Hz), 1.20 (d, 3H, *J*= 5.7 Hz); MS (ESI): 519 [M+H]⁺.

### Example 116: 3-{3-[1-(5-(Aminocarbonyl)-4-{[(1R)-1-(2-chlorophenyl)ethyl]-oxy}-2-thienyl)-1H-benzimidazol-5-yl]phenyl}propanoic acid

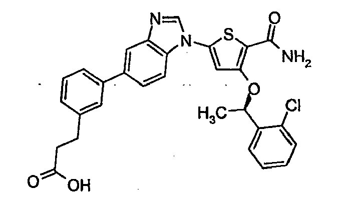

The title compound was prepared from 5-(5-bromo-1*H*-benzimidazol-1-yl)-3-{[(1*R*)-1-(2-chlorophenyl)ethyl]oxy}thiophene-2-carboxamide by a procedure analogous to **Example 7**. ¹H NMR (300 MHz, DMSO-*d₆*): δ 12.16 (s, 1H), 8.64 (s, 1H), 8.04 (s, 1H), 7.77-7.31 (m, 12H), 7.27 (s, 1H), 6.08-6.00 (m, 1H), 3.00-2.61 (m, 4H), 1.78 (d, 3H, *J*= 6.3 Hz); MS (ESI): 546 [M+H]⁺.

### Example 117: 3-{[(1R)-1-(2-Chlorophenyl)ethyl]oxy}-5-{5-[6-(methylamino)-3-pyridinyl]-1H-benzimidazol-1-yl}-2-thiophenecarboxamide

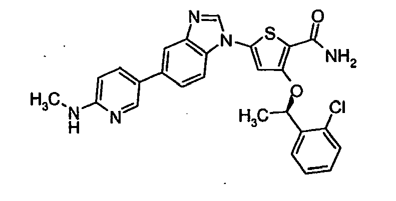

The title compound was prepared from 3-{[(1*R*)-1-(2-chlorophenyl)ethyl]oxy}-5-[5-(6-fluoropyridin-3-yl)-1*H*-benzimidazol-1-yl]thiophene-2-carboxamide by a procedure analogous to **Example 57**. ¹H NMR (400 MHz, CD₃OD): δ 8.38 (s, 1H), 8.23 (d, 1H, *J*= 2.2 Hz), 7.83 (s, 1H), 7.77 (dd, 1H, *J*= 2.5, 8.8 Hz), 7.60 (dd, 1H, *J*= 1.8, 7.6 Hz), 7.55 (dd, 1H, *J*=1.4, 8.7 Hz), 7.50-7.44 (m, 2H), 7.41-7.32 (m, 2H), 7.00 (s, 1H), 6.61 (d, 1H, *J*= 8.8 Hz), 6.05 (q, 1H, *J*= 6.3 Hz), 2.90 (s, 3H), 1.79 (d, 3H, *J*= 6.5 Hz); MS (ESI): 504 [M+H]⁺.

### Example 118: 5-(5-[6-(Methyloxv)-3-pyridinyl]-1H-benzimidazol-1-yl}-3-({(1R)-1-[2-(trifluoromethyl)phenyl]ethyl}oxy)-2-thiophenecarboxamide

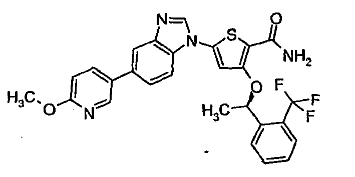

The title compound was prepared from methyl 5-(5-bromo-1H-benzimidazol-1-yl)-3-({(1*R*)-1-[2-(trifluoromethyl)pheny)]ethyl}oxy)-2-thiophenecarboxylate by a procedure analogous to **Example 98**; Step A and B. ¹H NMR (400 MHz, DMSO-*d₆*): δ 8.56 (s, 1H), 8.52 (d, 1H, *J*= 2.6 Hz), 8.07 (dd, 1H, *J*= 2.5, 8.6 Hz), 8.03 (s, 1H), 7.93 (d, 1H, *J*= 7.6 Hz), 7.83 (s, 1H), 7.78-7.74 (m, 2H), 7.64 (d, 1H, *J*= 8.2 Hz), 7.57-7.53 (m, 2H), 7.13 (m, 2H), 6.89 (d, 1H, *J*= 8.7 Hz), 5.97-5.93 (m, 1H), 3.88 (s, 3H), 1.74 (d, 3H, *J*= 6.3 Hz); MS (ESI): 539 [M+H]+.

### Example 119: [5-(6-amino-3-pyridinyl)-1H-benzimidazol-1-yl]-3-{[(1R)-1-(2-chlorophenyl)ethyl]oxy}-2-thiophenecarboxamide

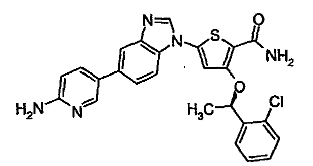

The title compound was prepared from 3-{[(1*R*)-1-(2-chlorophenyl)ethyl]oxy}-5-[5-(6-fluoropyridin-3-yl)-1 H-behzimidazol-1-yl]thiophene-2-carboxamide by a procedure analogous to **Example 57**. ¹H NMR (400 MHz, DMSO-*d₆*): δ 8.55 (s, 1H), 8.27 (d, 1H, *J*= 2.2 Hz), 7.89 (s, 1H), 7.79 (s, 1H), 7.74 (dd, 1H, *J*= 2.6, 8.2 Hz), 7.67 (d, 1H, *J*= 7.3 Hz), 7.58-7.49 (m, 3H), 7.45-7.31 (m, 2H), 7.19 (s, 1H), 7.09 (s, 1H), 6.51 (d, 1H, *J*= 9.1 Hz), 6.00-5.96 (m, 3H), 1.71 (d, 3H, *J*= 5.9 Hz); MS (ESI): 490 [M+H]⁺.

### Example 120: 3-{[(1R)-1-(2-Chlorophenyl)ethyl]oxy}-5-{5-[6-(4-methyl-1 piperazinyl)-3-pyridinyl]-1H-benzimidazol-1-yl}-2-thiophenecarboxamide

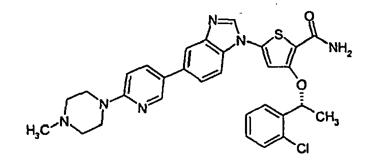

The title compound was prepared from 3-{[(1*R*)-1-(2-chlorophenyl)ethyl]oxy}-5-[5-(6-fluoropyridin-3-yl)-1*H*-benzimidazol-1-yl]thiophene-2-carboxamide by a procedure analogous to **Example 57**. ¹H NMR (400 MHz, DMSO-*d₆*): δ 8.58 (s, 1H), 8.49 (d, 1H, *J*= 2.6 Hz), 7.98 (s, 1H), 7.91 (dd, 1H, *J*= 2.6, 8.8 Hz), 7.82 (s, 1H), 7.68 (dd, 1H, *J*= 1.7, 7.6), 7.63 (dd, 1H, *J*= 1.4,8.7 Hz), 7.58-7.50 (m, 2H), 7.43 (t, 1H, *J*= 7.5 Hz), 7.37 (t, 1H, *J*= 7.6 Hz), 7.21 (s, 1H), 7.11 (s, 1H), 6.91 (d, 1H, *J*= 8.8 Hz), 5.99 (q, 1H, *J*= 6.6 Hz), 3.54-3.47 (m, 4H), 2.41-2.36 (m, 4H), 2.21 (s, 3H), 1.72 (d, 3H, *J*= 5.9 Hz); MS (ESI): 573.31 [M+H]⁺.

### Example 121: 5-{5-[6-(4-Amino-1-piperidinyl)-3-pyridinyl]-1H-benzimidazol-1-yl}-3-{[(1R)-1-(2-chlorophenl)ethyl]oxy}-2-thiophenecarboxamide

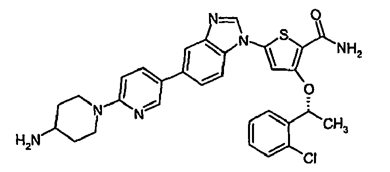

The title compound was prepared from 3-{[(1*R*)-1-(2-chlorophenyl)ethyl]oxy}-5-[5-(6-fluoropyridin-3-yl)-1*H*-benzimidazol-1-yl]thiophene-2-carboxamide by a procedure analogous to **Example 57**. ¹H NMR (400 MHz, CD₃OD): δ 8.39-8.37 (m, 2H), 7.87-7.84 (m, 2H), 7.61-7.55 (m, 2H), 7.49-7.45 (m, 2H), 7.41-7.32 (m, 2H), 7.00 (s, 1H), 6.93 (d, 1H, *J*= 9.4 Hz), 6.05 (m, 1H), 4.32 (d, 2H, *J*= 13.9 Hz), 4.02 (d, 2H, *J*= 14.5 Hz), 2.99-2.92 (m, 3H), 2.83-2.72 (m, 2H), 1.93 (d, 2H, *J*= 13.1 Hz), 1.79 (d, 3H, *J*= 5.5 Hz); MS (ESI): 573.22 [M+H]⁺.

### Example 122: 3-{[(1R)-1-(2-Chlorophenyl)ethyl]oxy}-5-(5-{6-[methyl(1-methyl-4-piperidinyl)amino]-3-pyridinyl}-1H-benzimidazol-1-yl)-2-thiophene-carboxamide

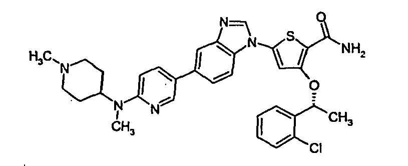

The title compound was prepared from 3-{[(1*R*)-1-(2-chlorophenyl)ethyl]oxy}-5-[5-(6-fluoropyridin-3-yl)-1*H*-benzimidazol-1-yl]thiophene-2-carboxamide by a procedure analogous to **Example 57**. ¹H NMR (400 MHz, CDCl₃): δ 8.44 (d, 1H, *J*= 2.5 Hz), 7.96 (s, 1H), 7.91 (s, 1H), 7.72 (dd, 1H, *J*= 2.5,8.7 Hz), 7.50-7.40 (m, 3H), 7.36-7.26 (m, 3H), 6.62 (s,1H), 6.58 (d, 1H, *J*= 9.1 Hz), 5.87 (m, 1H), 5.67 (s, 1H), 4.63-4.54 (m, 2H), 3.03-2.95 (m, 2H), 2.92 (s, 3H), 2.34 (s, 3H), 2.28-2.16 (m, 2H), 1.98-1.86 (m, 2H), 1.77 (d, 3H, *J*= 6.1 Hz), 1.74-1.70 (m, 2H); MS (ESI): 601.31 [M+H]+.

### Example 123: 3-[(1R)-(2-Chlorophenyl)ethoxy]-5-(6-{(1R)-1-[(3R)-piperidin-3-yl]ethoxy}-1H-benzimidazol-1-yl)thiophene-2-carboxamide

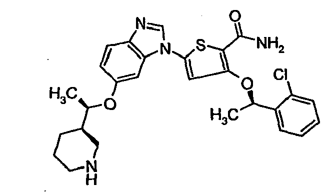

### Step A -tert-Butyl(3R)-3-(hydroxymethyl)piperidine-1-carboxylate

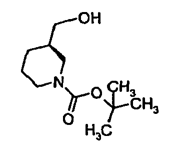

1*-tert-*Butyl 3-ethyl (3*R*)-piperidine-1,3-dicarboxylate (2.40 g, 9.33 mmol) was dissolved in tetrahydrofuran (50.0 mL) and the solution cooled to -78 °C. Next, diisobutylaluminum hydride (28.0 mL, 28.0 mmol) was added dropwise over 15 min. After 1h, the mixture was warmed to -40 °C, and saturated aqueous Rochelle's salt (50.0 mL) was added. The mixture was allowed to warm to room temperature and stirred for 2 h. Ethyl acetate (300 mL) was added and the organic layer separated. The organic layer was dried over magnesium sulfate and adsorbed onto silica gel. Purification *via* silica gel chromatography afforded 1.23 g (61 %) of the title compound. ¹H NMR (400 MHz, DMSO-*d₆*): δ 4.50 (t, 1H, *J*= 5.3 Hz), 3.86 - 4.04 (m, 1H), 3.84-3.71 (m, 1H), 3.32-3.23 (m, 1H), 3.23-3.11 (m, 1H), 2.80-2.63 (m, 1H), 1.62-1.72 (m, 1H), 1.62-1.52 (m, 1H), 1.52-1.41 (m, 2H), 1.38 (s, 9H), 1.35-1.20 (m, 1H), 1.14-1.01 (m,1H).

### Step B - tert-butyl (3R)-3-formylpiperidine-1-carboxylate

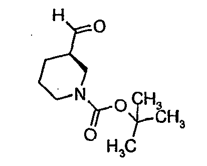

*tert-Butyl* (3*R*)-3-(hydroxymethyl)piperidine-1-carboxylate (1.23 g, 5.71 mmol) was dissolved in dichloromethane (20.0 mL) and cooled to 0 °C. Next, Dess-Martin periodinane (3.60 g, 8.57 mmol), sodium bicarbonate (960 mg, 11.4 mmol), and water (0.100 mL, 5.71 mmol) was added and the reaction warmed to room temperature. The suspension was stirred for 3 h at room temperature and quenched with saturated aqueous sodium sulfite (100 mL) at 0 °C. Dichloromethane (100 mL) was added and the organic layer separated and dried over magnesium sulfate. Purification *via* silica gel chromatography afforded 1.08 g (88%) of the title compound. ¹H NMR (300 MHz, CDCl₃): δ 9.71 (d, 1H, *J*= 0.6 Hz), 4.01-3.84 (m, 1H), 3.72-3.56 (m, 1H), 3.33 (dd, 1H,*J* = 8.4,13.5 Hz), 3.16-2.98 (m, 1H), 2.49-2.33 (m, 1H), 2.03-1.88 (m, 1H), 1.77-1.60 (m, 2H), 1.57-1.48 (m, 1H), 1.49-1.44 (m, 9H).

### Step C - 1, 1-dimethylethyl (3R)-3-[(1S)-1-hydroxyethy/]-1-piperidinecarboxylate

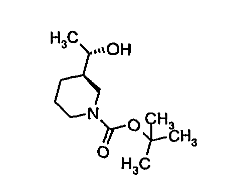

*tert*-Butyl (3*R*)-3-formylpiperidine-1-carboxylate (1.00 g, 4.69 mmol) was dissolved into tetrahydrofuran (10.0 mL) and cooled to -78°C. Next, Methylmagnesiumbromide (0.5 M in tetrahydrofuran, 14.1 mL, 7.03 mmol) was added over 15 min. The reaction was stirred for 45 minutes at -78 °C and quenched with water (20.0 mL) at this temperature. The mixture was warmed to 0 °C and ethyl acetate added. The organic phase was separated and dried over magnesium sulfate. The crude material was adsorbed onto silica gel and purified by column chromatography to give 300 mg (56%) of the title compound. ¹H NMR (400 MHz, CDCl₃): δ 3.77-3.67 (m, 1H), 3.66-3.51 (m, 2H), 3.21-2.99 (m, 2H), 2.01-1.83 (m, 1H), 1.76-1.66 (m, 1H), 1.66-1.53 (m, 1H), 1.45 (s, 9H), 1.43-1.33 (m, 3H), 1.20 (d, 3H, *J*= 6.4 Hz).

### Step D - tert-butyl (3R)-3-[(1R)-1-({1-[4-[(1R)-1-(2-chlorophenyl)ethoxy]-5-(methoxycarbonyl)-2-thienyl]-1H -benzimidazol-6-yl}oxy)ethyl]piperidine-1-carboxylate

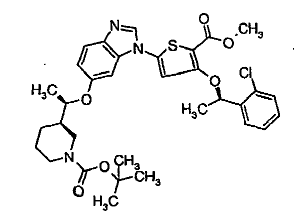

The title compound was prepared from *tert*-butyl (3*R*)-3-[(1*S*)-1-hydroxyethyl] piperidine-1-carboxylate and methyl 3-[(1*R*)-1-(2-chlorophenyl)ethoxy]-5-(6-hydroxy-1*H*-benzimidazol-1-yl)thiophene-2-carboxylate by a procedure analogous to **Example 20**, Step A. MS (ESI): 640 [M+H]⁺.

### Step E - tert-butyl (3R)-3-{(1R)-1-[(1-{5-(aminocarbonyl)-4-[(1R)-1-(2-chlorophenyl)ethoxy]-2-thienyl}-1H -benzimidazo/-6-y/)oxy]ethy}piperidine-1-carboxylate

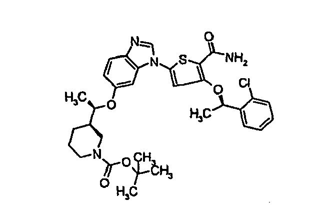

The title compound was prepared from tert-butyl (3*R*)-3-[(1*R*)-1-({1-[4-[(1*R*)-1-(2-chlorophenyl)ethoxy]-5-(methoxycarbonyl)-2-thienyl]-1*H*-benzimidazol-6-yl}oxy)ethyl]piperidine-1-carboxylate by a procedure analogous to **Example 20,** Step C. MS (APCI): 625 [M+H]⁺.

### Step F - 3-[(1R)-1-(2-chlorophenyl)ethoxy]-5-(6-{(1R)- 1-[(3R)-piperidin-3-yl]ethoxy}- 1H-benzimidazol- 1-yl)thiophene-2-carboxamide (Title Compound)

The title compound was prepared from *tert-*butyl (3*R*)-3-{(1*R*)-1-[(1-{5-(aminocarbonyl)-4-[(1*R*)-1-(2-chlorophenyl)ethoxy]-2-thienyl}-1*H-*benzimidazol-6-yl)oxy]ethyl}piperidine-1-carboxylate by a procedure analogous to **Example 20**, Step B. ¹H NMR (400 MHz, DMSO-*d₆*): δ 8.36 (s, 1H), 7.77 (br s, 1H), 7.64 (d, 1H, *J*= 9.0 Hz), 7.59 (d, 1H, *J*= 8.6 Hz), 7.44 (d, 1H, *J*= 7.9 Hz), 7.39 (t, 1H, *J*= 7.5 Hz), 7.36-7.30 (m, 1H), 7.09 (s, 2H), 6.93 (s, 1H), 6.90 (br s, 1H), 5.97 (q, 1H, *J*=6.2 Hz), 4.44-4.13 (m, 1H), 2.95-2.87 (m, 1H), 2.85-2.76 (m, 1H), 2.40-2.28 (m, 2H), 1.92-1.84 (m, 1H), 1.69 (d, 3H, *J*= 6.4 Hz), 1.59-1.51 (m, 2H), 1.35-1.28 (m, 1H), 1.23-1.19 (m,2H),1.15 (d, 3H, *J*= 6.4 Hz); MS (ESI): 525 [M+H]⁺.

### Example 124: 5-{5-[2-(4-Methyl-1-piperazinyl)-4-pyridinyl]-1H-benzimidazol-1-yl}-3-({(1R)-1-[2-(trifluoromethyl)phenyl]ethyl}oxy)-2-thiophenecarboxamide

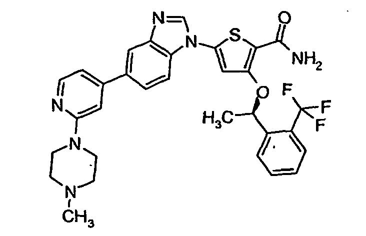

A solution of 5-[5-(2-fluoro-4-pyridinyl)- 1H-benzimidazol-1-yl]-3-({(1*R*)-1-[2-(trifluoromethyl)phenyl]ethyl}oxy)-2-thiophenecarboxamide (0.243 g, 0.46 mmol), 1-methyl piperazine (0.4 mL, 3.6 mmol) and ethanol (95%, 0.4 mL) was heated in a Personal Chemistry microwave at 180 °C for 36 min, after which time the mixture was diluted with dichloromethane, washed with water, dried over magnesium sulfate and concentrated onto silica gel. Purification by column chromatography (10 to 100% 90/9/1 dichloromethane/methanol/ammonium hydroxide:dichloromethane) provided 0.200 g (71%) of the title compound as a tan solid. ¹H NMR (400 MHz, CDCl₃): δ 8.26 (d, 1H, *J*= 5.1 Hz), 8.04 (s, 1H), 7.98 (s, 1H), 7.74 (d, 1H, *J*= 7.9 Hz), 7.71 (d, 1H, *J*= 7.9 Hz), 7.64 (t, 1H, *J*= 7.6 Hz), 7.57 (dd, 1H, *J*= 8.5, 1.6 Hz), 7.50 (m, 2H), 7.21 (br s, 1H), 6.92 (d, 1H, *J*= 5.1 Hz), 6.88 (s, 1H), 6.68 (s, 1H), 5.87 (q, 1H, *J*= 6.3 Hz), 5.81 (br s, 1H), 3.74 (m, 4H), 2.67 (m, 4H), 2.39 (s, 3H), 1.82 (d, 3H, *J*= 6.4 Hz); MS (ESI): 607.3 [M+H]⁺.

### Example 125: 5-[5-(2-Amino-4-pyridinyl)-1H-benzimidazol-1-yl]-3-({(1R)-1-[2-(trifluoromethyl)phenyl]ethyl}oxy)-2-thiophenecarboxamide

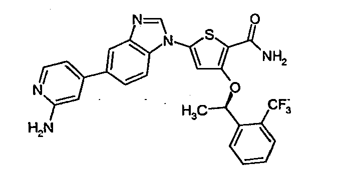

### Step A - methyl 5-(5-{2-[(diphenylmethylidene)amino]-4-pyridinyl}-1H-benzimidazo/-1-yl}-3-({(1R)-1-[2-(trifluoromethyl)phenyl]ethyl}oxy)-2-thiophenecarboxylate

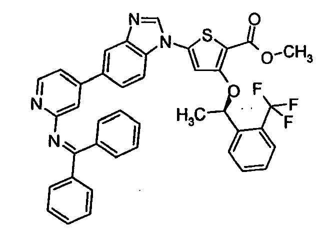

To a degassed solution of methyl 5-[5-(2-chloro-4-pyridinyl)-1*H*-benzimidazol-1-yl]-3-({(1*R*)-1-[2-(trifluoromethyl)phenyl]ethyl}oxy)-2-thiophenecarboxylate (0.250 g, 0.45 mmol) and benzophenone imine (0.09 mL, 0.54 mmol) in 1,4-dioxane (2 mL), cesium carbonate (367 mg, 1.13 mmol) was added followed by 4,5-bis(diphenylphosphino)-9,9-dimethyl-xanthene (10.0 mg, 0.018 mmol) and tris(dibenzylidineacetone)dipalladium (0) (8.0 mg, 0.009 mmol). The reaction mixture was heated to 90°C for 24 h. The reaction mixture was then diluted with ethyl acetate, concentrated onto silica gel and purified by column chromatography (0 to 60% ethyl acetate:hexanes) to afford 0.255 g (83%) of the title compound as an off white solid. MS (ESI): 703.3 [M+H]+.

### Step B -methyl 5-[5-(2-amino-4-pyridinyl)-1H-benzimidazol-1-yl]-3-({(1R)- 1-[2-(trifluoromethyl)phenyl]ethyl}oxy)-2-thiophenecarboxylate

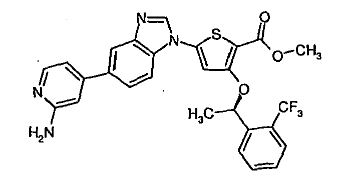

To a solution of methyl 5-(5-{2-[(diphenylmethylidene)amino]-4-pyridinyl}-1*H-*benzimidazol-1-yl)-3-({(1*R*)-1-[2-(trifluoromethyl)phenyl]ethyl}oxy)-2-thiophenecarboxylate (0.160 mg, 0.228 mmol) in tetrahydrofuran (6 mL), 2 N aqueous hydrochloric acid (3.0 mL, 6.0 mmol) was added and the solution was stirred at room temperature overnight. The reaction mixture was then diluted with dichloromethane, washed with saturated aqueous sodium bicarbonate and dried over magnesium sulfate. The filtrate was evaporated onto silica gel. Purification by column chromatography (10 to 100% 1/9/90 ammonium hydroxide/methanol/dichloromethane: dichloromethane) provided 0.107 g (88%) of the title compound as an off-white solid. MS (ESI): 539.2 [M+H]+.

### Step C - 5-[5-(2-amino-4-pyridinyl)-1H-benzimidazol-1-yl]-3-({(1R)- 1-[2-(trifluoromethyl)phenyl]ethyl}oxy)-2-thiophenecarboxamide (Title Compound)

The title compound was prepared from methyl 5-[5-(2-amino-4-pyridinyl)-1*H-*benzimidazol-1-yl]-3-({(1*R*)-1-[2-(trifluoromethyl)phenyl]ethyl}oxy)-2-thiophenecarboxylate (0.107 g, 0.199 mmol) and 8 mL 7 N ammonia in methanol heating in a sealed vessel at 85 °C for 10 h. The mixture was evaporated onto silica gel and purified by column chromatography (10 to 90% 1/9/90 ammonium hydroxide/methanol/dichloromethane: dichloromethane) to give the 0.085 g (82%) of the desired product as a white solid. ¹H NMR (400 MHz, DMSO-*d₆*): δ 8.58 (s, 1H), 7.98 (s, 1H), 7.92 (m, 2H), 7.83 (br s, 1H), 7.76 (m, 2H), 7.57 (m, 3H), 7.14 (s, 1H), 7.12 (br s, 1H), 6.84 (d, 1H, *J*= 5.3 Hz), 6.75 (s, 1H), 5.95 (m, 3H), 1.73 (d, 3H, *J*= 6.0 Hz); HRMS calc'd for C₂₆H₂₁N₅O₂F₃S [M+H]⁺ 524.1368, found 524.1367.

### Example 126: 5-[5-(1H-pyrazol-4-yl)-1H-benzimidazol-1-yl]-3({(1R)-1-[2-(triftuoromethyl)phenyl]ethyl}oxy)-2-thiophenecarboxamide

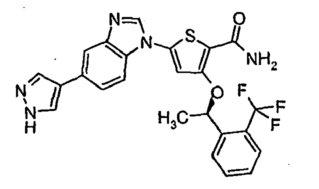

### Step A - methy/ 5-[5-(1H-pyrazo/-4-yl)-1H-benzimidazo/-1-yl]-3-({(1R)-1-[2-(trifluoromethyl)phenyl]ethyl}oxy)-2-thiophenecarboxy/ate

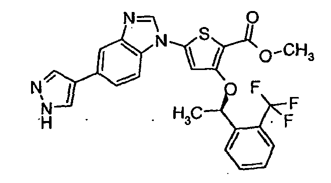

4-(4,4,5,5-Tetramethyl-1,3,2-dioxaborolan-2-yl)-1*H*-pyrazole (148 mg, 0.76 mmol) in *N,N-*dimethylacetamide (3 mL) was treated with aqueous 1 N sodium carbonate (1.5 mL, 1.5 mmol), methyl 5-(5-bromo-1*H*-benzimidazol-1-yl)-3-({(1*R*)-1-[2-(trifluoromethyl)-phenyl]ethyl}oxy)thiophene-2-carboxylate (200 mg, 0.38 mmol) and 1,1'-bis diphenylphosphinoferrocene dichloropalladium(II) dichloromethane adduct (70 mg, 0.07 mmol) and heated to 80°C for 2 h. After cooling to room temperature the reaction mixture was partitioned between 5:1 chloroform:methanol and saturated aqueous sodium bicarbonate. The organic layer was washed with brine (2x). The layers were separated and the organic layer was dried over sodium sulfate. The solution was filtered and concentrated and placed on a vacuum pump overnight to remove remaining *N*,*N*-dimethylacetamide. The crude compound was purified by column chromatography (50 to 100% ethyl acetate in hexanes, with 0.5% triethylamine) to yield 132 mg (68%) of the title compound as a foam. MS (APCl): 512.9 [M+H]⁺.

### Step B-5-[5-(1H-pyrazol-4-yl)-1H-benzimidazol-1-yl]-3-({(1R)-1-[2-(trifluoromethy/)phenyl]ethyl}oxy)-2-thiophenecarboxamide (Title Compound)

The title compound was prepared from methyl 5-[5-(1*H*-pyrazol-4-yl)-1*H-*benzimidazol-1-yl]-3-({(1*R*)-1-[2-(trifluoromethyl)phenyl]ethyl}oxy)-2-thiophenecarboxylate by a procedure analogous to **Example 59**, Step B. ¹H NMR (400 MHz, CDCl₃): δ 7.91 (m, 4H), 7.72 (d, 1H, *J*= 7.9 Hz), 7.69 (d, 1H, *J*= 8.0 Hz), 7.45 (m, 3H), 7.20 (br s, 1H), 5.91 (br s, 1H), 5.85 (q, 1H, *J*= 6.2 Hz), 1.80 (d, 3H, *J*= 6.0 Hz); HRMS calc'd. for C₂₄H₁₉N₅O₂F₃S [M+H]⁺ 498.1212, found 498.1204.

### Example 127: 3-{[(1R)-1-(2-Chlorophenyl)ethyl]oxy}-5-{5-[6-(1-piperazinylyl)-3-pyridinyl]-1H-benzimidazol-1-yl}-2-thiophenecarboxamide

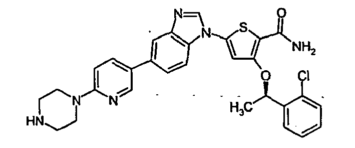

### Step A - 1, 1-dimethy/ethy/4-[5-(1-{4-{[(1R)-1-(2-chlorophenyl)ethyl]oxy}-5-[(methyloxy)carbonyl]-2-thienyl}-1H-benzimidazol-5-yl)-2-pyridinyl]-1-piperazinecarboxylate

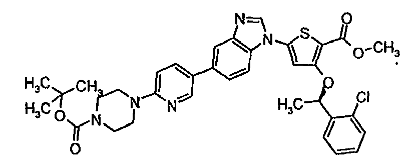

The title compound was prepared from methyl 5-(5-bromo-1*H*-benzimidazol-1-yl)-3-{[(1*R*)-1-(2-chlorophenyl)ethy)]oxy}thiophene-2-carboxylate and 1,1-dimethylethyl 4-[5-(4,4,5,5-tetramethyl-1,3,2-dioxaborolan-2-yl)-2-pyridinyl]-1-piperazinecarboxylate by a procedure analogous to **Example 59**, Step A. MS (APCl): 674.1 [M+H]⁺.

### Step B-1,1-dimethylethyl 4-{5-[1-(5-(aminocarbonyl)-4-{[(1R)-1-(2-chlorophenyl)ethyl]oxy}-2-thienyl)-1H -benzimidazo/-5-yl]-2-pyridinyl}-1-piperazinecarboxylate

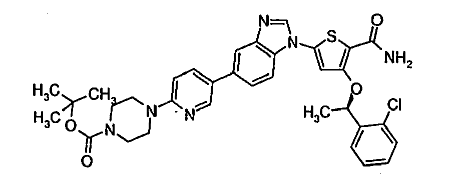

The title compound was prepared from 1,1-dimethylethyl 4-[5-(1-{4-{[(1*R*)-1-(2-chlorophenyl)ethyl]oxy)-5-[(methyloxy)carbonyl]-2-thienyl)-1*H-*benzimidazol-5-yl)-2-pyridinyl]-1-piperazinecarboxylate by a procedure analogous to **Example 59**, Step B. MS (APCl): 659.1 [M+H]⁺.

### Step C-3-{[(1R)-1-(2-chlorophenyl)ethyl]oxy}-5-{5-[6-(1-piperazinyl)-3-pyridinyl]-1H-benzimidazol-1-yl}-2-thiophenecarboxamide (Title Compound)

The title compound was prepared from 1,1-dimethylethyl 4-{5-[1-(5-(aminocarbonyl)-4-([(1*R*)-1-(2-chlorophenyl)ethyl]oxyl-2-thienyl)-1*H-*benzimidazol-5-yl]-2-pyridinyl}-1-piperazinecarboxylate by a procedure analogous to **Example 59**, Step C. ¹H NMR (400 MHz, CDC₃): δ 8.63 (s, 1H), 8.54 (d, 1H, *J*= 2.5 Hz), 8.02 (s, 1H), 7.96 (dd, 1H, *J*= 8.8, 2.7 Hz), 7.85 (br s, 1H), 7.73 (dd, 1H, *J*= 7.6, 1.7 Hz), 7.69-7.55 (m, 3H), 7.51-7.37 (m, 2H), 7.26 (s, 1H), 7.16 (br s, 1H), 6.93 (d, 1H, *J*= 8.8 Hz), 6.04 (q, 1H, *J*= 6.3 Hz), 3.53-3.47 (m, 4H), 2.87-2.81 (m, 4H), 1.78 (d, 3H, *J*= 6.3 Hz); HRMS calc'd. for C₂₉H₂₇ClN₆O₂S [M+H]⁺ 559.1680, found 559.1676.

### Example 128: 5-[6-(4-Piperidinyloxy-1H-benzimidazo)-1-yl)-3-({(1R)-1-[2-(triftuoromethyl)pheny]ethyl}oxy)-2-thiophenecarboxamide

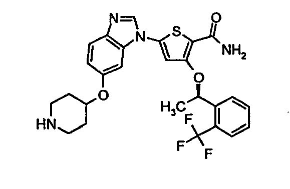

### Step A-1,1-Dimethylethyl 4-({1-[5-[methyloxy)carbonyl]-4-({(1R)-1-[2-(trifluoromethy/)phenyl]ethyl}oxy)-2-thieny/]-1H-benzimidazo/-6-y/}oxy)-1-piperidinecarboxylate

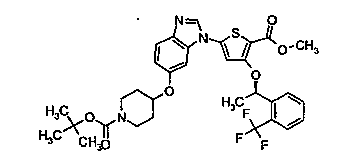

Methyl 5-(6-hydroxy-1*H*-benzimidazol-1-yl)-3-({(1*R*)-1-[2-(trifluoromethyl)phenyl]-ethyl)oxy)-2-thiophenecarboxylate (0.478 g, 1.03 mmol), cesium carbonate (0.470 g, 1.44 mmol), and 4-(toluene-4-sulfonyloxy)-piperidine-1-carboxylic acid *tert*-butyl ester (0.439 g, 1.24 mmol) were combined in 10 mL of *N,N*-dimethylformamide and heated to 60°C with stirring. The reaction was heated for 36 h and cooled to room temperature. The mixture was poured into ethyl acetate and water, and the layers were separated. The organic layer was washed with saturated aqueous sodium chloride, and the combined aqueous layers were extracted with ethyl acetate. The combined organic layers were dried over magnesium sulfate, filtered, and concentrated *in vacuo.* Purification by flash chromatography afforded 0.482 g (72%) of the title compound. ¹H NMR (400 MHz, DMSO-*d₆*): δ 8.43 (s, 1H), 7.95 (dd, 1H, *J*= 7.7 Hz), 7.78-7.66 (m, 2H), 7.63 (d, 1H, *J*= 8.8 Hz), 7.50 (m, 1H), 7.29 (s, 1H), 7.09 (d, 1H, *J*= 2.2 Hz), 7.01 (dd, 1H, J= 2.2, 8.8 Hz), 5.97 (q, 1H, *J*= 6.2 Hz), 4.59 (m, 1H), 3.81 (s, 3H), 3.65-3.56 (m, 2H), 3.25-3.15 (m, 2H), 1.91-1.82 (m, 2H), 1.63 (d, 3H, *J*= 6.2 Hz), 1.59-1.49 (m, 2H), 1.38 (s, 9H). MS (ESI): 646 [M+H]⁺.

### Step B - Methyl 5-[6-(4-piperidinyloxy)-1H-benzimidazol-1-yl]-3-({(1R)-1 -[2-(trifluoromethyl)phenyl]ethyl}oxy)-2-thiophenecarboxylate

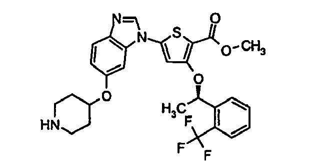

1,1-Dimethylethyl 4-({1-[5-[(methyloxy)carbonyl]-4-({(1*R*)-1-[2-(trifluoromethyl)phenyl]ethyl}oxy)-2-thienyl]-1*H*-benzimidazol-6-yl}oxy)-1-piperidinecarboxylate (1.84 g, 2.85 mmol) was dissolved in dichloromethane (30 mL) with stirring and cooled to 0 °C. Trifluoroacetic acid (10.0 mL, 130 mmol) was added dropwise *via* addition funnel. The reaction was stirred for 1 h, and 2 N aqueous sodium hydroxide solution (60 mL) was added dropwise *via* addition funnel. Saturated aqueous sodium bicarbonate solution was used to adjust the pH to basic. The mixture was poured into a separatory funnel, and the layers were separated. The aqueous layer was washed once with dichloromethane and once with diethyl ether. The combined organic layers were dried over magnesium sulfate, filtered, and concentrated *in vacuo.* Purification by flash chromatography provided 1.37 g (88%) of the title compound. ¹H NMR (400 MHz, DMSO-*d₆*): δ 8.42 (s, 1H), 7.95 (d, 1H, *J*= 7.9 Hz), 7.78-7.67 (m, 2H), 7.61 (d, 1H, *J*= 8.6 Hz), 7.61 (m, 1H), 7.30 (s, 1H), 7.05 (d, 1H, *J*= 2.2 Hz), 6.97 (dd, 1H, *J*= 2.2, 8.8 Hz), 5.96 (q, 1H, *J*= 6.1 Hz), 4.41 (m, 1H), 3.80 (s, 3H), 2.97-2.88 (m, 2H), 2.59-2.49 (m, 2H), 1.92-1.83 (m, 2H), 1.63 (d, 3H, *J*= 6.1 Hz), 1.51-1.38 (m, 2H); MS (ESI): 546 [M+H]⁺.

### Step C - 5-[6-(4-Piperidinyloxy)-1H-benzimidazol-1-y/]-3-({(1R)-1-[2-(trifluoromethyl)phenyl]ethyl}oxy)-2-thiophenecarboxamide (Title Compound)

Methyl 5-[6-(4-piperidinyloxy)-1*H*-benzimidazol-1-yl]-3-({(1*R*)-1-[2-(trifluoromethyl)phenyl]ethyl}oxy)-2-thiophenecarboxylate (0.154 g, 0.282 mmol) was dissolved in 7 N ammonia in methanol (12.0 mL, 84.0 mmol) in a sealed tube and heated to 80 °C for 2 days. The reaction was cooled to room temperature and concentrated *in vacuo.* Purification by flash chromatography afforded 0.129 g (86%) of the title compound. ¹H NMR (400 MHz, DMSO-*d₆*): δ 8.34 (s, 1H), 7.91 (d, 1H, *J*= 8.0 Hz), 7.81 (br s, 1H), 7.78-7.70 (m, 2H), 7.61 (d, 1H, *J*= 8.4 Hz), 7.53 (m, 1H), 7.12 (br s, 1H), 7.03 (s, 1H), 7.00-6.93 (m, 2H), 5.94 (q, 1H, *J*= 6.2 Hz), 4.44 (m, 1H), 3.03-2.94 (m, 2H), 2.70-2.61 (m, 2H), 1.96-1.86 (m, 2H), 1.72 (d, 3H, *J*= 6.2 Hz), 1.59-1.46 (m, 2H); MS (ESI): 531 [M+H]⁺.

### Example 129: 5-{6-[(1-Methyl-4-piperidinylyoxy]-1H-benzimidazol-1-yl}-3-({(1R)-1-[2-(trifluoromethyl)phenyl]ethyl}oxy)-2-thiophenecarboxamide

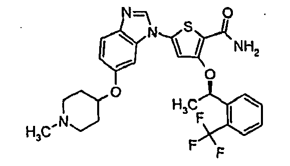

### Step A - methyl 5-{6-[(1-methyl-4-piperidinyl)oxy]-1H -benzimidazol-1-yl}-3-({(1R)-1-[2-(trifluoromethyl)pheny/]ethyl}oxy)-2-thiophenecarboxylate

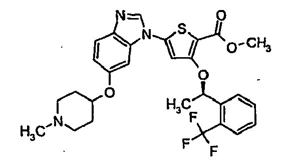

Methyl 5-[6-(4-piperidinyloxy)-1*H*-benzimidazol-1-yl]-3-({(1*R*)-1-[2-(trifluoromethyl)phenyl]ethyl}oxy)-2-thiophenecarboxylate (0.200 g, 0.367 mmol) was dissolved in dichloromethane (4 mL) and methanol (2 mL). Acetic acid (0.025 mL, 0.44 mmol) and formaldeldehyde (0.055 mL, 37% in water, 0.74 mmol) were added *via* syringe. Sodium triacetoxyborohydride (0.117 g, 0.552 mmol) was added in a single portion. The reaction was stirred for 1 h and quenched with saturated aqueous sodium bicarbonate solution. The mixture was poured into dichloromethane and half-saturated aqueous sodium bicarbonate. The layers were separated, and the aqueous layer was washed with dichloromethane (3x) and ethyl acetate (1x). The combined organic layers were dried over magnesium sulfate, filtered, and concentrated *in vacuo.* Purification by flash chromatography afforded 0.150 g (73%) of the title compound. ¹H NMR (400 MHz, DMSO-*d₆*): δ 8.43 (s, 1H), 7.96 (d, 1H,*J* = 7.7 Hz), 7.78-7.68 (m, 2H), 7.62 (d, 1H, *J*= 9.0 Hz), 7.51 (m, 1H), 7.32 (s, 1H), 7.06 (br s, 1H), 6.98 (m, 1H), 5.97 (q, 1H, *J*= 6.0 Hz), 4.41 (m, 1H), 3.80 (s, 3H), 3.26 (s, 3H), 2.52-2.43 (m, 2H), 2.27-2.11 (m, 2H), 1.98-1.85 (m, 2H), 1.73-1.60 (m, 2H), 1.62 (d, 3H, *J*= 6.0 Hz); MS (ESI): 560 [M+H]⁺.

### Step B - 5{6-[(1-methyl-4-piperidinyl)oxy]-1H -benzimidazol-1-yl}-3({(1R)-1-[2-(trifluoromethyl)phenyl]ethyl}oxy)-2-thiophenecarboxamide

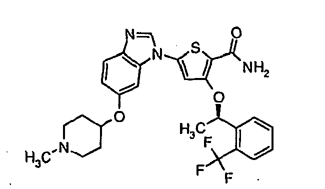

Methyl 5-{6-[(1-methyl-4-piperidinyl)oxy]-1*H*-benzimidazol-1-yl}-3-({(1*R*)-1-[2-(trifluoromethyl)phenyl]ethyl)oxy)-2-thiophenecarboxylate (0.148 g, 0.264 mmol) was dissolved in 7 N ammonia in methanol (12.0 mL, 84.0 mmol) in a sealed tube and heated to 80 °C for 24 h. The reaction was cooled to room temperature and concentrated *in vacuo.* Purification by flash chromatography afforded 0.138 g (96%) of the title compound. ¹H NMR (400 MHz, DMSO-*d₆*): δ 8.35 (s, 1H), 7.92 (d, 1H, *J*= 7.7 Hz), 7.82 (br s, 1H), 7.80-7.71 (m, 2H), 7.64-7.51 (m, 2H), 7.12 (br s, 1H), 7.06 (s, 1H), 6.99-6.94 (m, 2H), 5.95 (q, 1H, *J*= 6.2 Hz), 4.36 (m, 1H), 2.64-2.53 (m, 2H), 2.23-2.11 (m, 2H), 2.17 (s, 3H), 1.94-1.84 (m, 2H), 1.73 (d, 3H, *J*= 6.2 Hz),1.71-1.57 (m, 2H); MS (ESI): 545 [M+H]⁺.

### Example 130: 5-6-trans-4-Aminocyclohexyl)-1H-benzimidazol-1-yl]-3-{[(1R)-1 -(2-chlorophenyl)ethyl]oxy}-2-thiophenecarboxamide

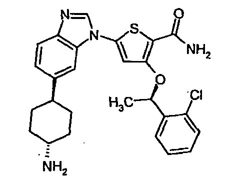

### Step A - 2(cis-4-iodocyclohexyl)-1H-isoindole-1,3(2H)-dione

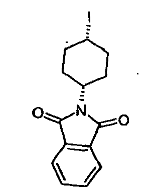

To a cooled (0 °C) solution of 2-(*trans*-4-hydroxycyclohexyl)-1*H*-isoindole-1,3(2*H*)-dione (10.0 g, 40.8 mmol, prepared by a procedure analogous to that in EP0186087A1) in dichloromethane (140 mL) and carbon tetrachloride (270 mL) was added triphenylphosphine (12.8 g, 48.9 mmol), imidazole (3.33 g, 48.9 mmol), and iodine (12.6 g, 49.8 mmol). The reaction was warmed to room temperature and stirred a total of 17 h, then quenched by addition of 10% w/v aqueous sodium thiosulfate (300 mL). The mixture was extracted with dichloromethane (2 x 200 mL). The combined organic fractions were washed with 10% w/v aqueous sodium thiosulfate (1 x 200 mL), saturated aqueous sodium chloride (1 x 200 mL), dried over sodium sulfate, filtered, and concentrated onto silica gel. Purification by flash column chromatography (15 to 70% ethyl acetate:hexanes) afforded 8.52 g (59%) of the title compound. ¹H NMR (300 MHZ, CDCl₃): δ 7.84 (m, 2H), 7.72 (m, 2H), 4.84 (m, 1H), 4.22-4.11 (m, 1H), 2.91-2.77 (m, 2H), 2.21 (m, 2H), 1.76-1.62 (m, 4H).

### Step B - [4-(1,3-dioxo-1,3-dihydro-2H-isoindol-2-yl)cyclohexyl](iodo)zinc

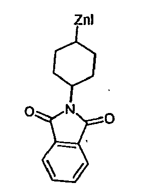

The title compound was prepared from 2-(*cis*-4-iodocyclohexyl)-1*H*-isoindole-1,3(2*H*)-dione by a procedure analogous to **Example 94**, Step A.

### Step C -methyl 3-{[(1R)-1-(2chlorophenyl)ethyl]oxy}-5-{6-[4-(1,3-dioxo-1,3-dihydro-2H-isoindol-2-yl)cyclohexyl]-1H-benzimidazol-1-yl}-2-thiophenecarboxylate

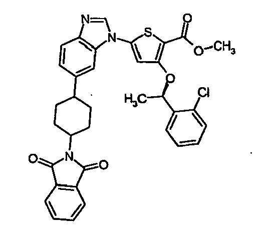

The title compound was prepared from triethyl 3-{[(1*R)-*1-(2-chlorophenyl)ethyl]oxy}-5-(6-{[(trifluoromethyl)sulfonyl]oxy}-1*H*-benzimidazol-1-yl)-2-thiophenecarboxylate and 4-(1,3-dioxo-1,3-dihydro-2*H*-isoindol-2-yl)cyclohexyl](iodo)zinc by a procedure analogous to **Example 94**, Step B. MS (APCI): 640.25 [M+H]⁺.

### Step D - 5-[6-trans-4-aminocyclohexyl)-1H-benzimidazo/-1-yl]-3-{[(1R)-1-(2-chlorophenyl)ethyl]oxy}-2-thiophenecarboxamide (Title Compound)

The title compound was prepared from methyl 3-{[(1*R)-1-(2-*chlorophenyl)ethyl]oxy}-5-{6-(4-(1,3-dioxo-1,3-dihydro-2*H*-isoindol-2-yl)cyclohexyl]-1*H*-benzimidazol-1-yl}-2-thiophenecarboxylate by a procedure analogous to **Example 49**, Step C. Purification by flash column chromatography (0 to 10% methanol:chloroform w/1% aqueous ammonium hydroxide) afforded the title compound as a mixture of *trans: cis* isomers along with a portion of *N*-{4-[1-(5-(aminocarbonyl)-4-{[(1*R*)*-1*-(2-chlorophenyl)ethyl]oxy}-2-thienyl)-1*H*-benzimidazol-6-yl]cyclohexyl)-1,2-benzenedicarboxamide. This latter material was resubjected to the reaction conditions as described above to yield an additional amount of the title compound as a mixture of *trans*:*cis* isomers. These two batches of title compound were combined and further purified by preparative HPLC (10 to 50% acetonitrile:water w/ 0.1 % trifluoroacetic acid) and then free-based by stirring with MP-Carbonate resin in methanol for 2 h to afford the pure *trans* title compound.¹H NMR (300 MHz, CDCl₃): δ 7.91 (s, 1H), 7.72 (d, 1H, *J*= 8.3 Hz), 7.48 (dd, 1H, *J*= 2.0, 7.4 Hz), 7.47-7.32 (m, 4H), 7.23 (br s, 1H), 7.19 (dd, 1H, J= 1.5, 8.4 Hz), 6.60 (s, 1H), 6.07 (br s, 1H), 5.87 (q,1H, *J*= 6.3 Hz), 2.92 (m, 2H), 2.59 (m, 2H), 2.10 (m, 2H), 1.94 (m, 2H), 1.77 (d, 3H, *J*= 6.5 Hz), 1.62-1.34 (m, 4H); MS (ESI): 495.23 [M+H]⁺.

### Example 131: 5-[6-(cis-4-Aminocyclohexyl)-1H-benzimidazol-1-yl]-3-{[(1R)-1-(2-chlorophenyl)ethyl]oxy}-2-thiophenecarboxamide

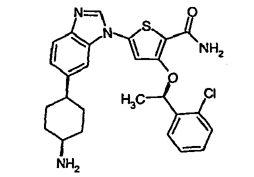

The pure *cis* title compound was isolated during the purification procedure described in **Example 130**, Step D. ¹H NMR (300 MHz, CDCl₃): δ 7.91 (s, 1H), 7.73 (d, 1H), 7.49 (dd, 1H, *J*= 1.9, 7.5 Hz), 7.44-7.28 (m, 4H), 7.25 (m, 2H), 6.62 (s, 1H), 6.27 (br s, 1H), 5.88 (q, 1H, *J*= 6.5 Hz), 3.29 (t, 1H, *J*= 3.0 Hz), 2.70-2.60 (m, 2H), 1.94-1.65 (m, 12H); MS (ESI): 495.22 [M+H]⁺.

### Example 132: 3-{[1R)-1-(2-Chlorophenyl)ethyl]oxy}-5-{6-[trans-4-(dimethylamino)cyclohexyl]-1H-benzimidazol-1-yl}-2-thiophenecarboxamide

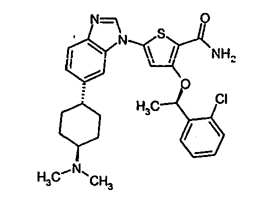

The title compound was prepared by a procedure analogous to **Example 52.** ¹H NMR (300 MHz, CDCl₃): δ 7.91 (s, 1H), 7.72 (d, 1H, *J*= 8.1 Hz), 7.49 (dd, 1H, *J*= 1.9, 7.5 Hz), 7.47-7.27 (m, 4H), 7.20 (m, 2H), 6.61 (s, 1H), 5.87 (q, 1H, *J*= 6.5 Hz), 5.77 (br s, 1H), 2.57 (m, 1H), 2.37 (m, 7H), 2.11-1.96 (m, 4H), 1.78 (d, 3H, *J*= 6.3 Hz), 1.59-1.36 (m, 4H); MS (APCI): 523.22 [M+H]⁺.

### Example 133: 3-{[1R)-1-(2-Chlorophenyl)ethyl]oxyl-5-[6-(trans-4-1[2-(methylsulfonyl)ethyl]amino}cycloheyl)-1H-benzimidazol-1-yl]-2-thiophenecarboxamide

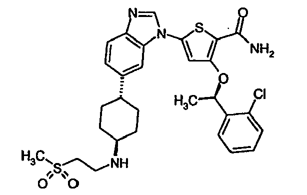

The title compound was prepared by a procedure analogous to **Example 95**, with the reaction conducted at 70 °C for 21.5 h. ¹H NMR (400 MHz, CDCl₃): δ 7.92 (s, 1H), 7.72 (d, 1H, *J*= 8.4 Hz), 7.49 (dd, 1H, *J*= 1.9, 7.6 Hz), 7.42 (dd, 1H, *J*= 1.5, 7.7 Hz), 7.37-7.28 (m, 3H), 7.23 (br s, 1H), 7.20 (dd, 1H, *J*= 1.5, 8.4 Hz), 6.60 (s, 1H), 5.88 (q, 1H, *J*= 6.4 Hz), 5.81 (br s, 1H), 3.23 (m, 2H), 3.04 (s, 3H), 3.00-2.89 (m, 2H), 2.61 (m, 2H), 2.11 (m, 2H), 1.94 (m, 2H), 1.78 (d, 3H, *J*= 6.4 Hz), 1.68 (br s, 1H), 1.56 (m, 2H), 1.27 (m, 2H); MS (APCI): 601.59 [M+H]⁺.

### Example 134: 3-{[(1R)-1-(2-Chlorophenyl)ethyl]oxy}-5-{6-[(3S)-3-piperidinylmethyl]-1H-benzimidazol-1-yl}-thiophenecarboxamide bis(trifluoroacetate) salt

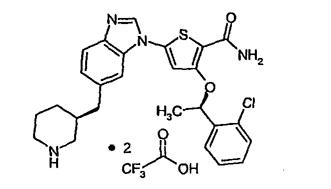

### Step A - 1,1-dimethylethyl (3R)-3-(iodomethyl)-1-piperidinecarboxylate

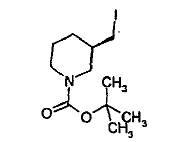

To a cooled (0 °C) solution of triphenylphosphine (7.38 g, 28.1 mmol), imidazole (1.91 g, 28.1 mmol), and 1,1-dimethylethyl (3*R*)-3-(hydroxymethyl)-1-piperidinecarboxylate (5.05 g, 23.5 mmol) in tetrahydrofuran (12.4 mL) at 0 °C was added *via* an addition funnel iodine (7.13 g, 28.1 mmol) in tetrahydrofuran (12.2 mL) over 15 min. The reaction was allowed to warm to room temperature and stirred for 17.5 h, then was quenched by addition of 10% w/v aqueous sodium thiosulfate (20 mL). The mixture was washed with hexanes (2 x 50 mL). The combined hexane washings were washed with water (1 x 50 mL), dried over sodium sulfate, filtered, and concentrated. Purification by flash column chromatography (10 to 30% ethyl acetate:hexanes) afforded 5.94 g (65%) of the title compound. ¹H NMR (300 MHz, CDCl₃): δ 4.05 (m, 1H), 3.84 (m, 1H), 3.08 (d, 2H, *J*= 6.6 Hz), 2.81 (m, 1H), 2.64 (m, 1H), 1.92 (m, 1H), 1.65 (m, 2H), 1.47 (m, 10H), 1.24 (m, 1H).

### Step B - [((3R)-1-{[(1,1-dimethylethyl)oxy]carbonyl}-3-piperidinyl)methyl](iodo)zinc

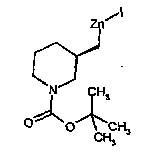

The title compound was prepared from 1,1-dimethylethyl (3*R*)-3-(iodomethyl)-1-piperidinecarboxylate by a procedure analogous to **Example 94,** Step A.

### Step C - 1,1-dimethylethyl (3S)-3-[(1-{4-{[(1R)-1-(2-chlorophenyl)ethyl]oxy}-5-(methyloxy)carbonyl]-2-thienyl}-1H-benzimidazol-6-yl)methyl]-1-piperidinecarboxylate

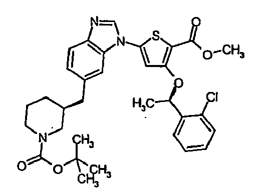

The title compound was prepared from methyl 3-{[(1*R*)-1-(2-chlorophenyl)ethyl]oxyl}-5-(6-{[(trifluoromethyl)sulfonyl]oxy}-1*H*-benzimidazol-1-yl)-2-thiophenecarboxylate and [((3*R*-1-{[(1,1-dimethylethyl)oxy]carbonyl}-3-piperidinyl)methyl](iodo)zinc by a procedure analogous to **Example 94**, Step B. ¹H NMR (300 MHz, CDCl₃): δ 7.95 (s, 1H), 7.69 (m, 2H), 7.42-7.29 (m, 3H), 7.16 (m, 2H), 6.70 (s, 1H), 5.84 (q, 1H, *J*= 6.1 Hz), 3.93 (s, 3H), 3.53 (m, 1H), 2.78 (m, 1H), 2.56 (m, 2H), 1.75 (m, 4H), 1.62 (m, 3H), 1.44 (m, 11H), 1.12 (m, 1H); MS (APCI): 610.30 [M+H]⁺.

### Step D - 1,1-dimethylethyl (3S)-3-{[1-(5-(aminocarbonyl)-4-{[(1R)-1-(2 chlorophenyl)ethyl]oxy-2-thienyl)-1H-benzimidazol-6-yl]methyl}-1-piperidinecarboxylate

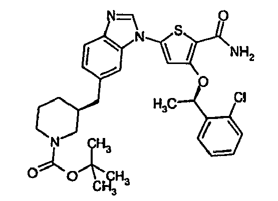

The title compound was prepared from 1,1-dimethylethyl (3*S*)-3-[(1-{4-{[(1*R*)-1-(2-chlorophenyl)ethyl]oxy}-5-[(methyloxy)carbonyl]-2-thienyl}-1*H-*benzimidazol-6-yl)methyl]-1-piperidinecarboxylate by a procedure analogous to **Example 49**, Step C. ¹H NMR (400 MHz, CDCl₃): δ 7.93 (s, 1H), 7.71 (d, 1H, *J*= 8.2 Hz), 7.47 (m, 2H), 7.34 (m, 2H), 7.20 (m, 2H), 7.13 (dd, 1H, *J*= 1.1, 8.2 Hz), 6.62 (s, 1H), 5.89 (m, 2H), 3.93 (m, 2H), 2.75 (m, 2H), 2.52 (m, 2H), 1.78 (d, 3H, *J*= 6.4 Hz), 1.66 (m, 3H), 1.41 (m, 11H); MS (APCI): 595.34 [M+H]⁺.

### Step E - 3-{[(1R)-1-(2-chlorophenyl)ethyl]oxy}-5-{6-[(3S)-3-piperidinylmethyl] 1H-benzimidazol-1-yl}-2-thiophenecarboxamide bis(trifluoroacetate) salt (Title Compound)

The title compound was prepared from 1,1-dimethylethyl (3*S*)-3-{[1-(5-(aminocarbonyl)-4-{[(1*R*)-1-(2-chlorophenyl)ethyl]oxy}-2-thienyl)-1*H-*benzimidazol-6-yl]methyl}-1-piperidinecarboxylate by a procedure analogous to Example 49, Step D. ¹H NMR (400 MHz, CDCl₃): δ 9.26 (m, 1H), 8.79 (m, 1H), 8.48 (s, 1H), 7.98 (br s, 2H), 7.81 (d, 1H, *J*= 8.1 Hz), 7.47-7.28 (m, 5H), 6.91 (br s, 1H), 6.77 (s, 1H), 5.89 (q, 1H, *J*= 6.3 Hz), 3.35 (m, 1H), 3.24 (m, 1H), 2.82 (m, 2H), 2.71 (m, 2H), 2.58 (m, 1H), 2.18 (m, 1H), 1.88 (m, 2H), 1.78 (m, 4H), 1.19 (m, 1H); MS (APCI): 495.30 [M+H]⁺.

### Example 135: 3-{[(1R)-1-(2-Chlorophenyl)ethyl]oxy}-5-(6-{[(3S)-1-methyl-3-piperidinyl]methyl}1H-benzimidazol-1-yl)-2-thiophenecarboxamide

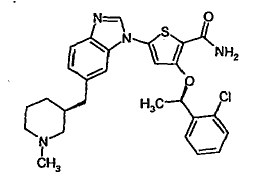

The title compound was prepared from 3-{[(1*R*)-1-(2-chlorophenyl)ethyl]oxy}-5-(6-[(3*S*)-3-piperidinylmethyl]-1*H*-benzimidazol-1-yl}-2-thiophenecarboxamide by a procedure analogous to **Example 52**. ¹H NMR (400 MHz, DMSO-*d₆*): δ 8.49 (s, 1H), 7.83 (br s, 1H), 7.66 (m, 2H), 7.52-7.37 (m, 3H), 7.22 (br s, 1H), 7.14 (m, 2H), 7.09 (s, 1H), 6.00 (q, 1H, *J*= 6.3 Hz), 2.66-2.53 (m, 4H), 2.08 (s, 3H), 1.80 (m, 2H), 1.72 (d, 3H, *J*= 6.4 Hz), 1.57 (m, 3H), 1.40 (m, 1H), 0.91 (m, 1H); MS (ESI): 509.27 [M+H]⁺.

### Example 136: 3-{[(1R)-1-(2-Chlorophenyl)ethyl]oxy}-5-[5-(1-methyl-1H-pyrazol-4-yl)-1H-benzimidazol-1-yl]-2-thiophenecarboxamide

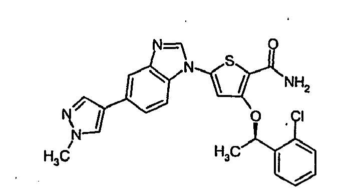

1-Methyl-4(4,4,5,5-tetramethyl-1,3,2-dioxaborolan-2-yl)1H-pyrazole (1.6 g, 7.6 mmol) was suspended in *N,N*-dimethylacetamide (22 mL) and treated with aqueous 1 N sodium carbonate (11.7 mL, 11.7 mmol), 5-(5-bromo-1*H-*benzimidazol-1-yl)-3-{[(1*R*)-1-(2-chlorophenyl)ethyl]oxy}-2-thiophenecarboxamide (2.8 g, 5.9 mmol) and 1,1'-bis diphenylphosphinoferracene dichloropalladium(II) dichloromethane adduct (479 mg, 0.59 mmol) and heated to 80 °C for 30 minutes. The reaction was allowed to cool to room temperature and partitioned between 5:1 dichloromethane: methanol and water. The layers were separated and the organic layer was dried over sodium sulfate. The mixture was filtered, concentrated and placed on vacuum pump at 50 °C overnight to remove residual *N,N*-dimethylacetamide. The oil was dissolved in dichloromethane. Silica gel was added and the mixutre was concentrated. The compound was purified by column chromatography (90/9/1 dichloromethane/methanol/ammonium hydroxide: dichloromethane). The product was triturated in diethyl ether and filtered to yield 2.85g of the title compound as a white solid. Due to an impurity the solid was dissolved in dichloromethane and repurified by column chromatography (ethyl acetate:methanol) to yield the product as a white solid (2.76g) ¹H NMR (400 MHz, DMSO-*d₆*): δ 8.54 (s, 1H), 8.19 (s, 1H), 7.95 (s, 1H), 7.93 (s, 1H), 7.81 (br s, 1H), 7.68 (dd, 1H, J = 7.7, 1.5 Hz), 7.58 (d, 1H, *J*= 8.5 Hz), 7.52-7.47 (m, 2H), 7.45-7.34 (m, 2H), 7.18 (s, 1H), 7.11 (br s, 1H), 5.99 (q, 1H, *J*= 6.3 Hz), 3.85 (s, 3H), 1.72 (d, 3H, *J*= 6.2 Hz); HRMS C₂₄H₂₀ClN₅O₂S: [M⁺H]⁺ calc'd. 478.1100, found 478.1102.

### Example 137: 5-(1H-Benzimidazol-1-yl)-3-{[2-trifluoromethyl)benzyl]oxy}-thiophene-2-carboxamide

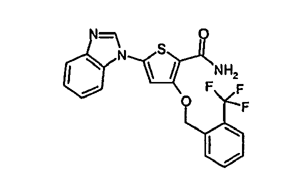

### Step A - methyl 3-[(triisopropylsilyl)oxy]thiophene-2-carboxylate

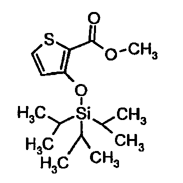

Under an inert atmposhepre, methyl 3-hydroxythiophene-2-carboxylate (15 g, 94.8 mmol) and imidazole (16.1 g, 237 mmol) were dissolved in dichloromethane (120 mL) followed by the dropwise addition of triisopropyl silyl chloride (21.3 mL, 99.6 mmol). The mixture was stirred at room temperature for 3 hours. Poured mixture into separatory funnel containing dichloromethane (200mL). Washed organic solution with 0.1 N HCl aqueous solution (3x200mL), distilled water (1x100mL), followed by brine (2x200mL). Dried organic solution (MgSO₄), filtered and removed solvent to give 28.8g of a gray oil. Dissolved residue in dichloromethane (200 mL), and added silica gel (75 g). Following evaporation of the volatiles under reduced pressure, the pre-adsorbed solids were loaded into two separate solid loading cartridge and subjected to a gradient elution with hexanes:ethyl acetate (100:0) to hexanes:ethyl acetate (70:30) using two separate RediSep silica gel cartridge (330 g; ISCO). The appropriate fractions were combined and concentrated under reduced pressure to give methyl 3-[(triisopropylsilyl)oxy]thiophene-2-carboxylate (25.68 g) as a gold oil. ¹H NMR (300 MHz, CDCl₃): δ 7.36 (d, *J*= 5.3 Hz, 1H), 6.70 (d, *J*= 5.5 Hz, 1H), 3.87 (s, 3H), 1.40-1.28 (m, 3H), 1.16 (d, *J*= 7.0 Hz, 18H). MS (ES-, m/z) 313 (M-1).

### Step B - methyl 5-iodo-3-[(triisopropylsilyl)oxy]thiophene-2-carboxylate

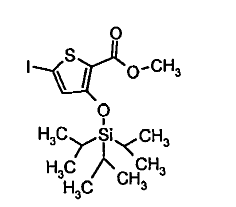

Method A: Under nitrogen to an oven dried 2L 3-necked round bottomed flask equipped with a 100 mL addition funnel, thermometer, and rubber septum was charged with anhydrous THF (500 mL, Sure Seal) through a cannula. To this was added methyl 3-[(triisopropylsilyl)oxy]thiophene-2-carboxylate (25.16g, 79.9 mmol) and cooled to -78°C with a dry ice/acetone bath. A 1.8 M solution of LDA (48.8 mL, 87.9 mmol) was then added dropwise keeping the temperature of the reaction below -70°C. Stirred for 2 hours at -78°C followed by the addition of solid iodine (22.3g, 87.9 mmol) causing an exotherm and turning the mixture a deep purple color. After stirring at -78°C for 30 minutes, allowed reaction to warm to room temperature. Poured mixture into separatory funnel containing dichloromethane (500 mL). Washed with 1 N HCl aqueous solution (2x250 mL), a 10% aqueous sodium meta bisulfite solution (1x250 mL) and brine (2x200 mL). Dried organic solution (MgSO₄), filtered and removed solvent to give a dark orange oil. Diluted in dichloromethane (300 mL) and added silica gel (75 g). Following evaporation of the volatiles under reduced pressure, the pre-adsorbed solids were loaded into two separate solid loading cartridge and subjected to a gradient elution with hexanes:ethyl acetate (100:0) to hexanes:ethyl acetate (70:30) using three separate RediSep silica gel cartridge (330 g; ISCO). The appropriate fractions were combined and concentrated under reduced pressure to give an impure gold oil (31.6g). The oil was then dissolved in hexanes and added to a flash column (1 kg SiO₂) eluting with hexanes:toluene (70:30). The appropriate fractions were combined and concentrated under reduced pressure to methyl 5-iodo-3-[(triisopropylsilyl)oxy]thiopherie-2-carboxylate (20 g, 85%) as a gold oil. ¹H NMR (400 MHz, CDCl₃): δ 6.79 (s, 1H), 3.78 (s, 3H), 1.33-1.22 (m, 3H), 1.10 (d, *J*= 7.1 Hz, 18H). MS (ES+, m/z) 441 (M+1).

Method B: Under nitrogen to a 100 mL round bottomed flask was added dry THF (25 mL), and N,N-diisopropylamine (0.89 mL, 6.4 mmol) followed by the dropwise addition of a 2.0M solution of propylmagnesium chloride in diethyl ether (3.0 mL, 6.0 mmol) and stirred at room temperature for 20 hours. To this mixture was added a solution of methyl 3-[(triisopropylsilyl)oxy]thiophene-2-carboxylate (1g, 3.18 mmol) in anhydrous THF (4 mL) and allowed to react for 10 minutes. A solution of iodine (1.94 g, 7.64 mmol) in anhydrous THF (4 mL) was added dropwise until dark brown color persisted. Quenched reaction with a saturated aqueous ammonium chloride solution, and poured reaction into separatory funnel containing diethyl ether (100 mL). Washed organic layer with a 10% sodium thiosulfate aqueous solution, and brine. Dried organic layer (MgSO₄), filtered and removed solvent to give oil. Purified using flash chromatography eluting with hexanes:toluene (80:20) to hexanes:toluene (65:35). The appropriate fractions were combined and concentrated under reduced pressure to methyl 5-iodo-3-[(triisopropylsilyl)oxy]thiophene-2-carboxylate (1.12 g) as a gold oil. ¹H NMR (400 MHz, CDCl₃): δ 6.80 (s, 1H), 3.80 (s, 3H), 1.34-1.22 (m, 3H), 1.11 (d, *J*= 7.1 Hz, 18H). MS (ES+, m/z) 441 (M+1).

### Step C- methyl 5-[(2-nitrophenyl)aminol-3-{[tris(1-methylethyl)silyl]oxy}-2-thiophenecarboxylate

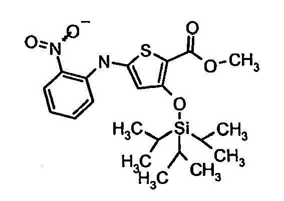

Under nitrogen to a 50 mL round bottomed flask was added methyl 5-iodo-3-[(triisopropylsilyl)oxy]thiophene-2-carboxylate (0.250 g, 0.57 mmol) followed by the addition of dry toluene (5 mL), tris(dibenzylideneacetone)-dipalladium (0) (0.026 g, 0.028 mmol), 2-(di-t-butylphosphino)biphenyl (0.017 g, 0.056 mmol), cesium carbonate (0.277 g, 0.85 mmol) and finally 2-nitroaniline (0.102 g, 0.74 mmol). The mixture was stirred at 50°C for 24 hours. Poured reaction mixture into a separatory funnel containing dichloromethane (50 mL) and washed with distilled water (2x50 mL) followed by brine (2x50 mL). Dried organic layer (MgSO₄), filtered and removed solvent to give brown oil. Diluted in dichloromethane (25 mL) and added silica gel (1 g). Following evaporation of the volatiles under reduced pressure, the pre-adsorbed solids were loaded into a solid loading cartridge and subjected to a gradient elution with hexanes:ethyl acetate (100:0) to hexanes:ethyl acetate (70:30) using a RediSep silica gel cartridge (4 g; ISCO). The appropriate fractions were combined and concentrated under reduced pressure to give methyl 5-[(2-nitrophenyl)amino]-3-{[tris(1-methy)ethyl)silyl]oxy}-2-thiophenecarboxytate (0.138 g) as a bright orange solid. ¹H NMR (400 MHz, CDCl₃): δ 9.63 (s, 1H), 8.24-8.21 (m, 1H), 7.52-7.46 (m, 2H), 6.95-6.90 (m, 1H), 6.42 (s, 1H), 3.82 (s, 3H), 1.35-1.24 (m, 3H), 1.14 (d, *J*= 7.3 Hz, 18H). MS (ES+, m/z) 451 (M+1).

### Step D- methyl 5-[(2-aminophenyl)amino]-3-hydroxy-2-thiophenecarboxylate

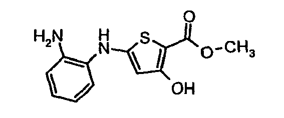

To a 150 mL Fischer Porter flask was added methyl 5-[(2-nitrophenyl)amino]-3-{[tris(1-methylethyl)silyl]oxy}-2-thiophenecarboxylate (0.138 g) and dissolved in ethanol (10 mL). To this mixture was added 5% Palladium on Carbon (wet) (0.050 mg) and the flask was connected to a Fischer Porter hydrogenator. The flask was evacutred and purged with nitrogen (3x's) and then evacuted before adding Hydrogen gas (55 psi). Stirred at room temperature for 4 hours after which the flask was evacuated and purged with nitrogen (3x's). Removed the flask and vacuum filtered over celite washing with ethanol (3x10 mL), methanol (1x10 mL) and ethyl acetate (1x10 mL). Removed solvent under reduced pressure to give a gold oil. Diluted in dichloromethane (25 mL) and added silica gel (0.200 g). Following evaporation of the volatiles under reduced pressure, the pre-adsorbed solids were loaded into a solid loading cartridge and subjected to a gradient elution with dichloromethane:methanol (100:0) to dichloromethane:methanol (90:10) using a RediSep silica gel cartridge (4 g; ISCO). The appropriate fractions were combined and concentrated under reduced pressure to give methyl 5-[(2-aminophenyl)amino]-3-hydroxy-2-thiophenecarboxylate (0.045 g) as a gold oil. ¹H NMR (400 MHz, CDCl₃) accounted for all non-exchangeable protons: δ 7.21 (dd, *J*= 7.9, 7.9 Hz, 1H), 7.1-7.06( m, 1H), 6.82-6.77 (m, 2H), 5.84 (s, 1H), 3.79 (s, 3H). MS (ES+, m/z) 265 (M+1).

### Step E- methyl 5-(1H-benzimidazol-1-yl)-3-hydroxy-2-thiophenecarboxylate

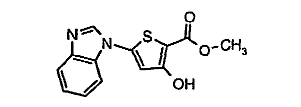

In a sealed tube, methyl 5-[(2-aminophenyl)amino]-3-hydroxy-2-thiophenecarboxylate (0.045 g, 0.17 mmol) was dissolved in anhydrous triethyl orthoformate (2 mL) and heated to 90°C for 1 hour. The reaction was cooled to room temperature and the solvent removed under reduced pressure. The residue was diluted into dichloromethane (25 mL), washed with 0.1 N HCl aqueous solution (2x25 mL) and then with brine (1x25 mL). Dried organic layer (MgSO₄), filtered and removed solvent under reduced pressure to give a yellow oil. Dissolved in dichloromethane (10 mL) and added silica gel (0.2 g). Following evaporation of the volatiles under reduced pressure, the pre-adsorbed solids were loaded into a solid loading cartridge and subjected to a gradient elution with hexanes:ethyl acetate (100:0) to hexanes:ethyl acetate (60:40) using a RediSep silica gel cartridge (4 g; ISCO). The appropriate fractions were combined and concentrated under reduced pressure to give methyl 5-(1*H*-benzimidazol-1-yl)-3-hydroxy-2-thiophenecarboxylate (0.039 g) as a off-white solid. ¹H NMR (400 MHz, CDCl₃): δ 9.81 (bs, 1H), 8.12 (s, 1H), 7.88-7.86 (m, 1H), 7.73-7.70 (m, 1H), 7.44-7.36 (m, 2H), 6.87 (s, 1H), 3.94 (s, 3H). MS (ES+, m/z) 275 (M+1).

### Step F- 5-(1H-Benzimidazol-1-yl)-3-{[2-(trifluoromethyl)benzyl]oxy}-thiophene-2-carboxamide

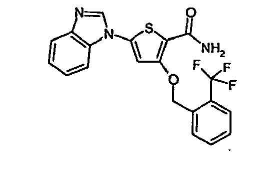

To a solution of methyl 5-(1*H*-benzimidazol-1-yl)-3-hydroxy-2-thiophenecarboxylate (75 mg, 0.27 mmol) and K₂CO₃ (76 mg, 0.55 mmol) in DMF (2 mL) was added 1-(bromomethyl)-2-(trifluoromethyl)benzene (78 mg, 0.33 mmol). Reaction mixture was stirred at rt for overnight. 2 mL of H₂O was added to the reaction mixture and precipitate was collected via filtration and washed with water followed by Hexane. Methyl 5-(1*H*-benzimidazol-1-yl)-3-({[2-(trifluoromethyl)phenyl]methyl}oxy)-2-thiophenecarboxylate was isolated as a yellow solid (100 mg).

Methyl 5-(1*H*-benzimidazol-1-yl)-3-({[2-(trifluoromethyl)phenyl]methyl}oxy)-2-thiophenecarboxylate (96 mg, 0.22 mmol) and 7M ammonium in MeOH (~10 mL) were combined in a sealed tube. The reaction mixture was heated to 80 °C and stirred for overnight. Reaction mixture was cooled to rt. Reaction mixture was evaporated to a small volume and cooled to rt. Solid was collected via filtration to give 5-(1*H*-Benzimidazol-1-yl)-3-{[2-(trifluoromethyl)benzyl]oxylthiophene-2-carboxamide as a yellow solid (50 mg). ¹H NMR (400 MHz, DMSO-d₆) δ 8.67 (s, 1H), 7.87-7.85 (m, 2H), 7.82-7.77 (m, 3H), 7.72-7.64 (m, 3H), 7.45-7.36 (m, 2H), 6.79 (br s, 1H), 5.56 (s, 2H). MS (ES+, m/z) 418 (M+1).

## Claims

1. A process for preparing a compound of formula (I): 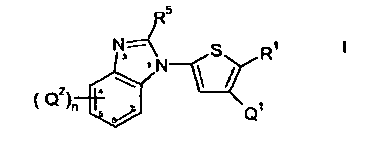
R¹ is selected from the group consisting of H, alkyl, alkenyl, alkynyl, -C(O)R⁷, -CO₂R⁷, -C(O)NR⁷R⁸, -C(O)N(R⁷)OR⁸, -C(O)N(R⁷)-R²-OR⁸, -C(O)N(R⁷)-Ph, -C(O)N(R⁷)-R²-Ph, -C(O)N(R⁷)C(O)R⁸, -C(O)N(R⁷)CO₂R⁸, -C(O)N(R⁷)C(O)NR⁷R⁸, -C(O)N(R⁷)S(O)₂R⁸, -R₂-OR⁷, -R²-O-C(O)R⁷, -C(S)R⁷, -C(S)NR⁷R⁸, -C(S)N(R⁷)-Ph, -C(S)N(R⁷)-R²-Ph, -R²-SR⁷, -C(=NR⁷)NR⁷R⁸, -C(=NR⁷)N(R⁸)-Ph, -C(=NR⁷)N(R⁸)R²-Ph, -R²-NR⁷R⁸, -CN, -OR⁷, -NR⁷R⁸, N(R⁷)-Ph, -N(R⁷)-R²-Ph, -N(R⁷)-SO₂R⁸ and Het;
Ph is phenyl optionally substituted from 1 to 3 times with a substituent selected from the group consisting of halo, alkyl, -OH, -R²-OH, -O-alkyl, -R²-O-alkyl, -NH₂, -N(H)alkyl, -N(alkyl)₂, -CN and -N₃;
Het is a 5-7 membered heterocycle having 1, 2, 3 or 4 heteroatoms selected from N, O and S, or a 5-6 membered heteroaryl having 1, 2, 3 or 4 heteroatoms selected from N, O and S, each optionally substituted from 1 to 2 times with a substituent selected from the group consisting of halo, alkyl, oxo, -OH, -R²-OH, -O-alkyl, -R²-O-alkyl, -NH₂. -N(H)alkyl, -N(alkyl)₂, -CN and -N₃;
Q¹ is a group of formula: -(R²)ₐ-(Y¹)_{b}-(R²)_{c}-R³
a, b and c are the same or different and are each independently 0 or 1 and at least one of a or b is 1;
n is 0, 1, 2 or 3;
each Q² is the same or different and is a group of formula:
-(R²)ₐₐ₋(Y²)_{bb}-(R²)_{cc}-R⁴
or two adjacent Q² groups are selected from the group consisting of alkyl, alkenyl, -OR⁷, -S(O)_{f}R⁷ and -NR⁷R⁸ and together with the carbon atoms to which they are bound, they form a C₅₋₆cycloalkyl, C₅₋₆cycloalkenyl, phenyl, 5-7 membered heterocycle having 1 or 2 heteroatoms selected from N, O and S, or 5-6 membered heteroaryl having 1 or 2 heteroatoms selected from N, O and S;
aa, bb and cc are the same or different and are each independently 0 or 1;
each Y¹ and Y² is the same or different and is independently selected from the group consisting of -O-, -S(O),-, -N(R⁷)-, -C(O)-, -OC(O)-, -CO₂-, -C(O)N(R⁷)-, -C(O)N(R⁷)S(O)₂-, -OC(O)N(R⁷)-, -OS(O)₂-, -S(O)₂N(R⁷)-, -S(O)₂N(R⁷)C(O), -N(R⁷)S(O)₂-, -N(R⁷)C(O)-, -N(R⁷)CO₂- and -N(R⁷)C(O)N(R⁷)-;
each R² is the same or different and is independently selected from the group consisting of alkylene, alkenylene and alkynylene;
each R³ and R⁴ is the same or different and is each independently selected from the group consisting of H, halo, alkyl, alkenyl, alkynyl, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃ and a group of formula (ii): 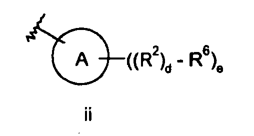 wherein:
Ring A is selected from the group consisting of C₅₋₁₀cycloalkyl, C₅₋₁₀cycyloalkenyl, aryl, 5-10 membered heterocycle having 1, 2 or 3 heteroatoms selected from N, O and S and 5-10 membered heteroaryl having 1, 2 or 3 heteroatoms selected from N, O and S;
each d is 0 or 1;
each e is 0, 1, 2, 3 or 4;
each R⁶ is the same or different and is independently selected from the group consisting of H, halo, alkyl, alkenyl, alkynyl, cycloalkyl, cycloalkenyl, Ph, Het, -CH(OH)-R²-OH, -C(O)R⁷ -CO₂R⁷, -CO₂-R₂-Ph, -CO₂-R²-Het, -C(O)NR⁷R⁸, -C(O)N(R⁷)C(O)R⁷, -C(O)N(R⁷)CO₂R⁷, -C(O)N(R⁷)C(O)NR⁷R⁸, -C(O)N(R⁷)S(O)₂R⁷, -C(S)R⁷, -C(S)NR⁷R⁸, -C(=NR⁷)R⁸, -C(=NR⁷)NR⁷R⁸, -CR⁷=N-OR⁸, =O, -OR⁷, -OC(O)R⁷, -OC(O)Ph, -OC(O)Het, -OC(O)NR⁷R⁸, -O-R²-S(O)R⁷, -S(O)_{f}R⁷, -S(O)₂NR⁷R⁸, -S(O)₂Ph, -S(O)₂Het, -NR⁷R⁸, -N(R⁷)C(O)R⁸, -N(R⁷)CO₂R⁸, -N(R⁷)-R²-CO₂R⁸, -N(R⁷)C(O)NR⁷R⁸, -N(R⁷)-R²-C(O)NR⁷R⁸, -N(R⁷)C(O)Ph, -N(R⁷)C(O)Het, -N(R⁷)Ph, -N(R⁷)Het, -N(R⁷)C(O)NR⁷-R²-NR⁷R⁸, -N(R⁷)C(O)N(R⁷)Ph, -N(R⁷)C(O)N(R⁷)-R²-Het, -N(R⁷)S(O)₂R⁸, -N(R⁷)-R²-S(O)₂R⁸, -NO₂, -CN and -N₃;
wherein when Q¹ is defined where b is 1 and c is 0, R³ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃;
wherein when Q² is defined where bb is 1 and cc is O, R⁴ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃;
R⁵ is H.
f is 0, 1 or 2; and
each R⁷ and each R⁸ are the same or different and are each independently selected from the group consisting of H, alkyl, alkenyl, alkynyl, cycloalkyl and cycloalkenyl; or a pharmaceutically acceptable salt thereof;
said process comprising cyclizing a compound of formula (VIII): 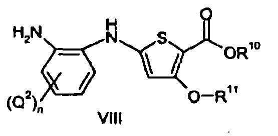
R¹⁰ is selected from alkyl, alkenyl, alkynyl, cycloalkyl, cycloalkenyl and suitable carboxylic acid protecting groups;
R¹¹ is H or a triisopropylsilyl protecting group;
and optionally removing the triisopropylsilyl protecting group to prepare a compound of formula (I-A): 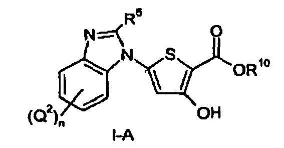 wherein
Alkyl (and alkylene) refer to straight or branched hydrocarbon chains containing from 1 to 8 carbon atoms optionally substituted one or more time with a halogen.
wherein when Q² is defined where bb is 1 and cc is 0, R⁴ is not halo, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)NR⁷R⁸, -NR⁷R⁸, -N(R⁷)C(O)R⁸, -N(R⁷)S(O)₂R⁸, -NO₂, -CN or -N₃;
R⁵ is selected H.
f is 0, 1 or 2; and
each R⁷ and each R⁸ are the same or different and are each independently selected from the group consisting of H, alkyl, alkenyl, alkynyl, cycloalkyl and cycloalkenyl;
or a pharmaceutically acceptable salt or solvate thereof.
According to the first aspect the present invention provides a process for preparing compounds of formula (I) above or a pharmaceutically acceptable salt or solvate thereof, comprising the the steps of:
a) cyclizing a compound of formula (VIII): 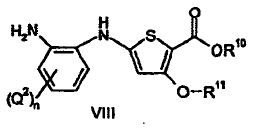 wherein
R¹⁰ is selected from alkyl, alkenyl, alkynyl, cycloalkyl, cycloalkenyl and suitable carboxylic acid protecting groups; and
R¹¹ is H or a triisopropylsilyl protecting group;
and optionally removing the triisopropylsilyl protecting group to prepare a compound of formula (I-A): 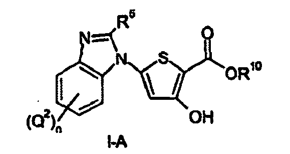
b) optionally converting the compound of formula (I-A) to a pharmaceutically acceptable salt or solvate thereof; and members and containing 1, 2, 3 or 4 heteroatoms selected from N, O and S (unless a different number of heteroatoms is specified).

2. A process for preparing a compound of formula (VIII): 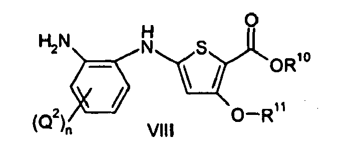 wherein:
n, Q² R¹¹ and R¹⁰ are as defined in claim 1.
or a pharmaceutically acceptable salt thereof;
comprising reducing a compound of formula (VII): 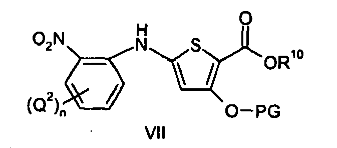 wherein PG is a triisopropylsilyl protecting group.

3. A process for preparing a compound of formula (VII): 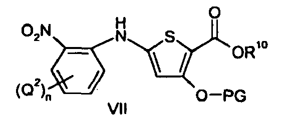 wherein:
n, Q² , and R¹⁰ are as defined in claim 1 and
PG is a triisopropylsilyl protecting group;
or a pharmaceutically acceptable salt thereof;
comprising reacting a compound of formula (V) with a compound of formula (VI)

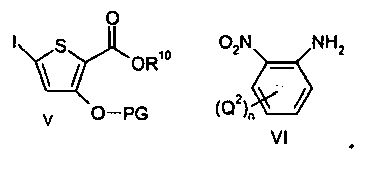

4. A process according to claim 1 for preparing a compound of formula (1): 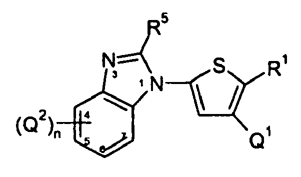 wherein:
R¹, Q¹, R⁵, are as defined in claim 1 and
n is 1, 2 or 3;
each Q² is the same or different and is independently a group of formula: -(R²)ₐₐ-(Y²)_{bb}-(R²)_{cc}-R⁴
wherein R2, Y², R² and R⁴ are as defined in claim1;
or a pharmaceutically acceptable salt thereof;
said process comprising cyclizing a compound of formula (XXIII): 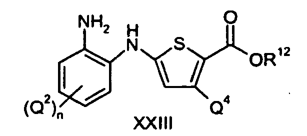 wherein:
Q⁴ is a group of formula -O-(R²)_{c}-R^{3a}, -O- Si(alkyl)₃, or -O-(R²)_{c}-Si(alkyl)₃;
wherein c is 1; and
R^{3a} is a group of formula (iii): 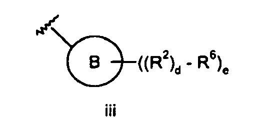 wherein Ring B is phenyl or 5-6 membered heteroaryl containing 1, 2 or 3 heteroatoms selected from N, O and S; and
R¹² is methyl;
to prepare a compound of formula (I-G):

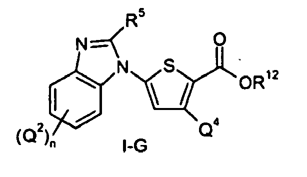

5. A compound of formula (XXIII): 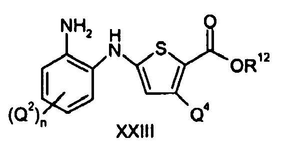 wherein
n and Q² and Q⁴ and R12 are as defined in claim 4.
or a pharmaceutically acceptable salt thereof.

6. A process for preparing a compound according to claim 5, comprising reducing a compound of formula (XXII):

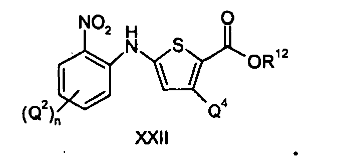

7. A compound of formula (XXII): 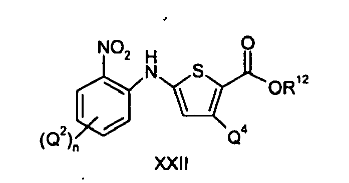 wherein
n, Q², Q⁴, R¹² are as defined in claim 4.

8. A process for preparing a compound according to claim 7, comprising reacting a compound of formula (XX) with a compound of formula (XXI): 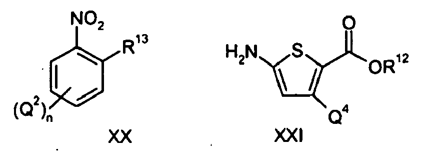 wherein R¹³ is halide or is O-triflate.

9. The process according to claim 4, wherein said step of cyclizing a compound of formula (XXIII) comprises cyclizing a compound of formula (XXIII-A): 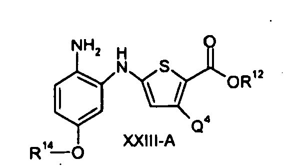 wherein R¹⁴ is a suitable phenol protecting group.

10. The process according to claim 4, wherein said step, of cyclizing a compound of formula (XXIII) comprises cyclizing a compound of formula (XXIII-B): 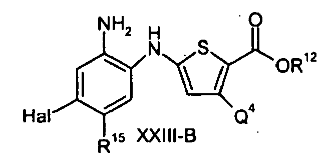 wherein Hal is Cl, Br or I; and
R¹⁵ is H or -O-R¹⁴.

11. The process according to claim 4, wherein said step of cyclizing a compound of formula (XXIII) comprises cyclizing a compound of formula (XXXII):

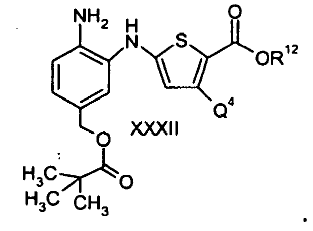

## Patentansprüche

1. Verfahren zur Herstellung einer Verbindung der Formel (I): 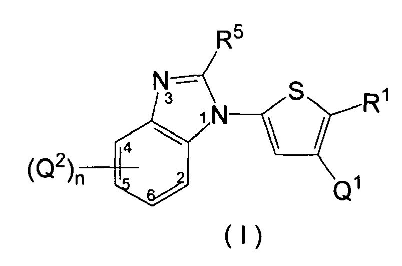 R¹ ist ausgewählt aus der Gruppe, bestehend aus H, Alkyl, Alkenyl, Alkinyl, -C(O)R⁷, -CO₂R⁷, -C(O)NR⁷R⁸, C(O)N(R⁷)OR⁸, -C(O)N(R⁷)-R²-OR⁸, -C(O)N(R⁷)-Ph, -C(O)N(R⁷)-R²-Ph, -C(O)N(R⁷)C(O)R⁸, -C(O)N(R⁷)CO₂R⁸, -C(O)N(R⁷)C(O)NR⁷R⁸, -C(O)N(R⁷)S(O)₂R⁸, -R²-OR⁷, -R²-O-C(O)R⁷, -C(S)R⁷, -C(S)NR⁷R⁸, -C(S)N(R⁷)-Ph, -C(S)N(R⁷)-R²-Ph, -R²-SR⁷, -C(=NR⁷)NR⁷R⁸, -C(=NR⁷)N(R⁸)-Ph, -C(=NR⁷)N(R⁸)-R²Ph, -R²-NR⁷R⁸, -CN, -OR⁷, -NR⁷R⁸, N(R⁷)-Ph, -N(R⁷)-R²-Ph, -N(R⁷)-SO₂R⁸ und Het;
Ph ist Phenyl, das gegebenenfalls 1 bis 3 mal mit einem Substituenten, ausgewählt aus der Gruppe bestehend aus Halogen, Alkyl, -OH, -R²-OH, -O-Alkyl, -R²-O-Alkyl, -NH₂, -N(H)-Alkyl, -N(Alkyl)₂, -CN und -N₃, substituiert ist;
Het ist ein 5- bis 7-gliedriger Heterocyclus mit 1, 2, 3 oder 4 Heteroatomen, ausgewählt aus N, O und S, oder 5- bis 6-gliedriges Heteroaryl mit 1, 2, 3 oder 4 Heteroatomen, ausgewählt aus N, O und S, wobei jedes gegebenenfalls 1 bis 2 mal mit einem Substituenten, ausgewählt aus der Gruppe bestehend aus Halogen, Alkyl, Oxo, -OH, -R²-OH, -O-Alkyl, -R²-O-Alkyl, -NH₂, -N(H)-Alkyl, -N(Alkyl)₂, -CN und -N₃, substituiert ist;
Q¹ ist eine Gruppe der Formel -(R²)ₐ-(Y¹)_{b}-(R²)_{c}-R³,
a, b und c sind gleich oder verschieden und sind jeweils unabhängig voneinander 0 oder 1 und wenigstens eines von a oder b ist 1;
n ist 0, 1, 2 oder 3;
jedes Q² ist gleich oder verschieden und ist eine Gruppe der Formel:
-(R²)ₐₐ-(Y²)_{bb}-(R²)_{cc}-R⁴,
oder zwei benachbarte Q²-Gruppen sind ausgewählt aus der Gruppe, bestehend aus Alkyl, Alkenyl, -OR⁷, -S(O)_{f}R⁷ und -NR⁷R⁸ und bilden zusammen mit dem Kohlenstoffatom, an das sie gebunden sind, C₅-₆-Cycloalkyl, C₅₋₆-Cycloalkenyl, Phenyl, einen 5- bis 7-gliedrigen Heterocyclus mit 1 oder 2 Heteroatomen, ausgewählt aus N, 0 und S, oder 5- bis 6-gliedriges Heteroaryl mit 1 oder 2 Heteroatomen, ausgewählt aus N, O und S;
aa, bb und cc sind gleich oder verschieden und sind unabhängig voneinander 0 oder 1;
jedes Y¹ und Y² ist gleich oder verschieden und ist unabhängig voneinander ausgewählt aus der Gruppe, bestehend aus -O-, -S(O)_{f}-, -N(R⁷)-, -C(O)-, -OC(O)-, -CO₂-, -C(O)N(R⁷)-, -C(O)N(R⁷)S(O)₂-, -OC(O)N(R⁷)-_{,} -OS(O)₂-,-S(O)₂N(R⁷), -S(O)₂N(R⁷)C(O)-, -N(R⁷)S(O)₂-, -N(R⁷)C(O)-, -N(R⁷)CO₂- und -N(R⁷)C(O)N(R⁷);
jedes R² ist gleich oder verschieden und ist unabhängig voneinander ausgewählt aus der Gruppe, bestehend aus Alkylen, Alkenylen und Alkinylen;
jedes R³ und R⁴ ist gleich oder verschieden und ist unabhängig voneinander ausgewählt aus der Gruppe, bestehend aus H, Halogen, Alkyl, Alkenyl, Alkinyl, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷)R⁸, -CR7=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN, -N₃, und einer Gruppe der Formel (ii): 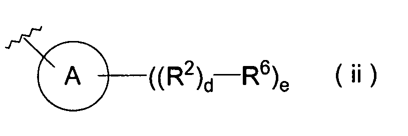 wobei:
Ring A ausgewählt ist aus der Gruppe, bestehend aus C₅₋₁₀-Cycloalkyl, C₅₋₁₀-Cycloalkenyl, Aryl, einem 5-bis 10-gliedrigen Heterocyclus mit 1, 2 oder 3 Heteroatomen, ausgewählt aus N, O und S, und 5- bis 10-gliedrigem Heteroaryl mit 1, 2 oder 3 Heteroatomen, ausgewählt aus N, O und S;
jedes d 0 oder 1 ist;
jedes e 0, 1, 2, 3 oder 4 ist;
jedes R⁶ gleich oder verschieden ist und unabhängig voneinander ausgewählt ist aus der Gruppe, bestehend aus H, Halogen, Alkyl, Alkenyl, Alkinyl, Cycloalkyl, Cycloalkenyl, Ph, Het, -CH(OH)-R²-OH, -C(O)R⁷, -CO₂R⁷, -CO₂-R₂-Ph, -CO₂-R²-Het, -C(O)NR⁷R⁸, -C(O)N(R⁷)C(O)R⁷, -C(O)N(R⁷)CO₂R⁷, -C(O)N(R⁷)C(O)NR⁷R⁸, -C(O)N(R⁷)S(O)₂R⁷, -C(S)R⁷, -C(S)NR⁷R⁸, -C(=NR⁷)R⁸, -C(=NR⁷)NR⁷R⁸, -CR⁷=N-OR⁸, =O, -OR⁷, -OC(O)R⁷, -OC(O)Ph, -OC(O)Het, -OC(O)NR⁷R⁸, -O-R²(O)₂R⁷, -S(O)_{f}R⁷, -S(O)₂NR⁷R⁸, -S(O)₂Ph, -S(O)₂Het, -NR⁷R⁸, -N(R⁷)C(O)R⁸, -N(R⁷)CO₂R⁸, -N(R⁷)-R²-CO₂R⁸, -N(R⁷)C(O)Ph, -N(R⁷)C(O)Het, -N(R⁷)Ph, -N(R⁷)Het, -N(R⁷)C(O)NR⁷-R²-NR⁷R⁸, -N(R⁷)C(O)N(R⁷)Ph, -N(R⁷)C(O)N(R⁷)Het, -N(R⁷)C(O)N(R⁷)-R²-Het, -N(R⁷)S(O)₂R⁸, -N(R⁷)-R²-S(O)₂R⁸, -NO₂, -CN und -N₃;
wobei, wenn Q¹ so definiert ist, dass b 1 und c 0 ist, R³ nicht Halogen, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN oder -N₃ ist;
wobei, wenn Q² so definiert ist, dass bb 1 und cc 0 ist, R⁴ nicht Halogen, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN oder -N₃ ist;
R⁵ ist H;
f ist 0, 1 oder 2; und
jedes R⁷ und jedes R⁸ sind gleich oder verschieden und sind unabhängig voneinander ausgewählt aus der Gruppe, bestehend aus H, Alkyl, Alkenyl, Alkinyl, Cycloalkyl und Cycloalkenyl;
oder eines pharmazeutisch annehmbaren Salzes davon;
wobei das Verfahren umfasst: Cyclisierung einer Verbindung der Formel (VIII) 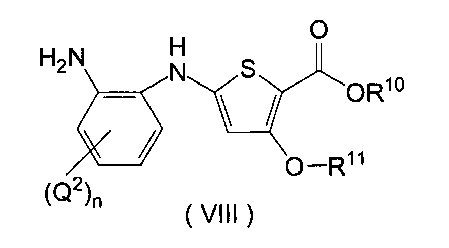 wobei
R¹⁰ ausgewählt ist aus Alkyl, Alkenyl, Alkinyl, Cycloalkyl, Cycloalkenyl und geeigneten Carbonsäure-Schutzgruppen;
R¹¹ H oder eine Triisopropylsilyl-Schutzgruppe ist;
und gegebenenfalls Entfernen der Triisopropylsilyl-Schutzgruppe, um eine Verbindung der Formel (I-A) herzustellen: 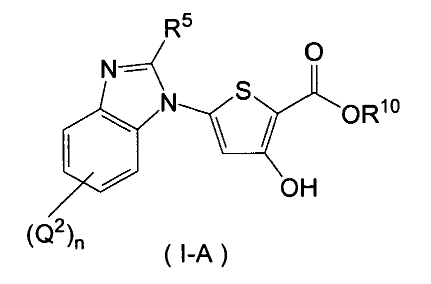 wobei
Alkyl (und Alkylen) eine geradkettige oder verzweigte Kohlenwasserstoffkette, enthaltend 1 bis 8 Kohlenstoffatome, die gegebenenfalls einfach oder mehrmals mit Halogen substituiert sind, bedeutet.

2. Verfahren zur Herstellung einer Verbindung der Formel (VIII): 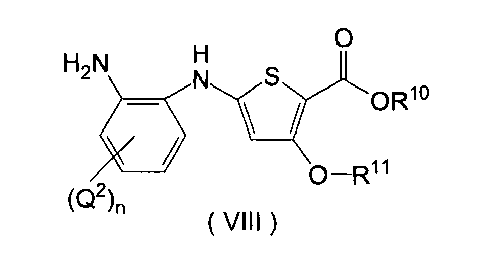 wobei n, Q², R¹¹ und R¹⁰ wie in Anspruch 1 definiert sind,
oder eines pharmazeutisch annehmbaren Salzes davon,
umfassend das Reduzieren einer Verbindung der Formel (VII): 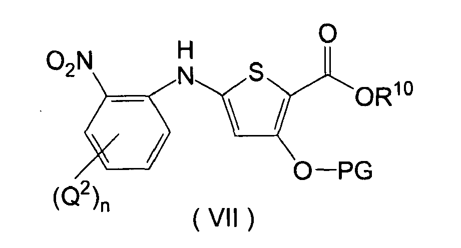 wobei PG eine Triisopropylsilyl-Schutzgruppe ist.

3. Verfahren zur Herstellung einer Verbindung der Formel (VII): 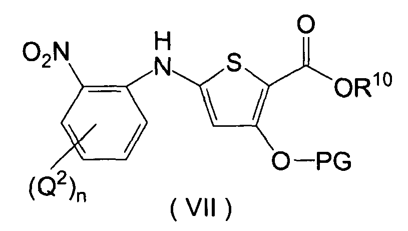 wobei n, Q² und R¹⁰ wie in Anspruch 1 definiert sind und PG eine Triisopropylsilyl-Schutzgruppe ist;
oder eines pharmazeutisch annehmbaren Salzes davon;
umfassend das Umsetzen einer Verbindung der Formel (V) mit einer Verbindung der Formel (VI):

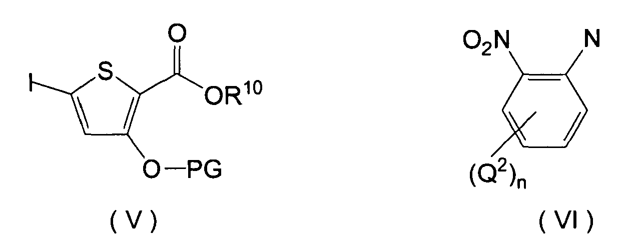

4. Verfahren gemäss Anspruch 1 zur Herstellung einer Verbindung der Formel (I): 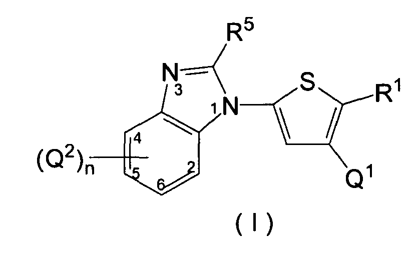 wobei R¹, Q¹ und R⁵ wie in Anspruch 1 definiert sind und
n 1, 2 oder 3 ist;
jedes Q² gleich oder verschieden ist und unabhängig voneinander eine Gruppe der Formel
-(R²)ₐₐ-(Y² )_{bb}-(R²)_{cc}-R⁴
ist, wobei R², Y², R² und R⁴ wie in Anspruch 1 definiert sind;
oder eines pharmazeutisch annehmbaren Salzes davon;
wobei das Verfahren umfasst: Cyclisieren einer Verbindung der Formel (XXIII): 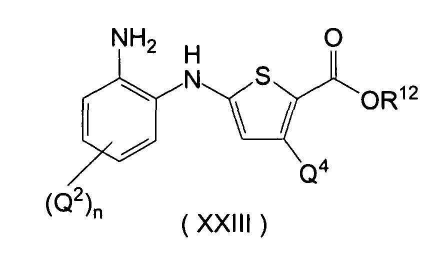 wobei Q⁴ eine Gruppe der Formel -O-(R²)_{c}-R^{3a}-O-Si(Alkyl)₃ oder -O-(R²)_{c}-Si(Alkyl)₃ ist; wobei c 1 ist; und
R³ ist eine Gruppe der Formel (iii): 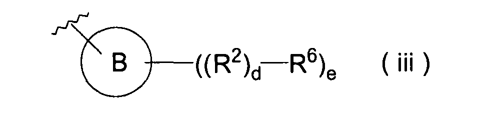 wobei Ring B Phenyl oder 5- bis 6-gliedriges Heteroaryl mit 1, 2 oder 3 Heteroatomen, ausgewählt aus N, O und S, ist; und
R¹² ist Methyl;
zur Herstellung einer Verbindung der Formel (I-G):

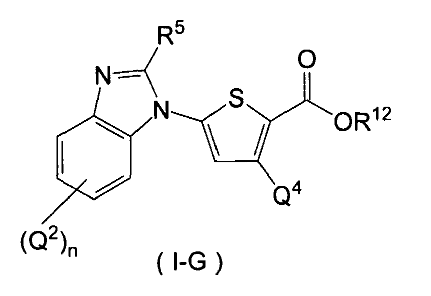

5. Verbindung der Formel (XXIII): 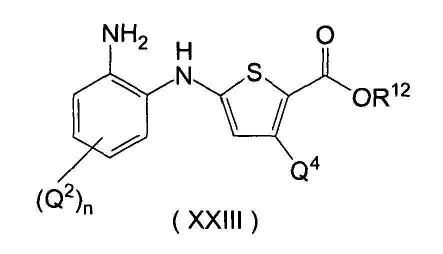 wobei n, Q², Q⁴ und R¹² wie in Anspruch 4 definiert sind, oder ein pharmazeutisch annehmbares Salz davon.

6. Verfahren zur Herstellung einer Verbindung gemäss Anspruch 5, umfassend das Reduzieren einer Verbindung der Formel (XXII):

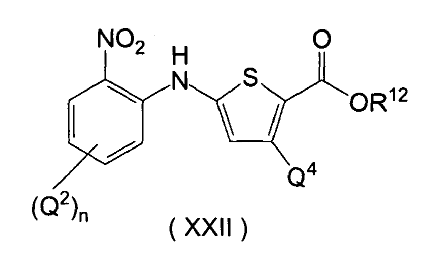

7. Verbindung der Formel (XXII): 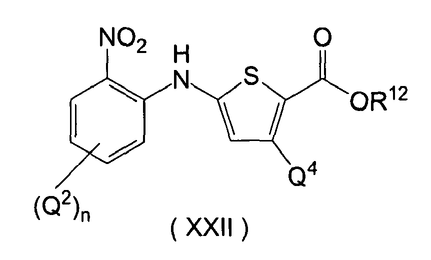 wobei n, Q², Q⁴ und R¹² wie in Anspruch 4 definiert sind.

8. Verfahren zur Herstellung einer Verbindung gemäss Anspruch 7, umfassend das Umsetzen einer Verbindung der Formel (XX) mit einer Verbindung der Formel (XXI) : 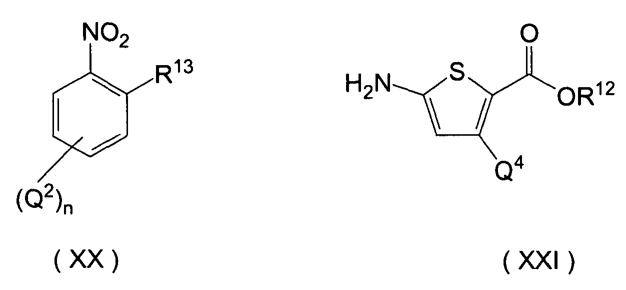 wobei R¹³ Halogenid oder O-Triflat ist.

9. Verfahren gemäss Anspruch 4, wobei der Schritt der Cyclisierung einer Verbindung der Formel (XXIII) das Cyclisieren einer Verbindung der Formel (XXIII-A) umfasst: 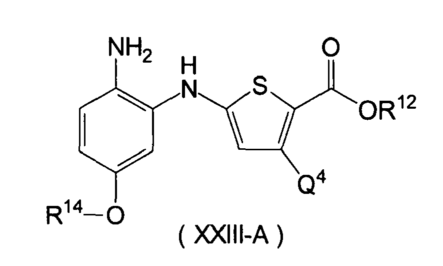 wobei R¹⁴ eine geeignete Phenol-Schutzgruppe ist.

10. Verfahren gemäss Anspruch 4, wobei der Schritt der Cyclisierung einer Verbindung der Formel (XXIII) das Cyclisieren einer Verbindung der Formel (XXIII-B) umfasst: 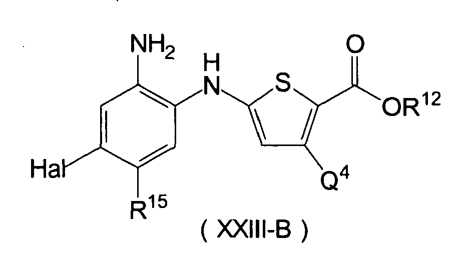 wobei Hal Cl, Br oder I ist; und R¹⁵ H oder -O-R¹⁴ ist.

11. Verfahren gemäss Anspruch 4, wobei der Schritt der Cyclisierung einer Verbindung der Formel (XXIII) das Cyclisieren einer Verbindung der Formel (XXXII) umfasst:

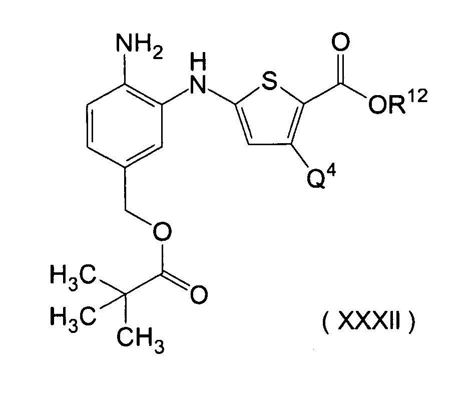

## Revendications

1. Procédé de préparation d'un composé de formule (I) : 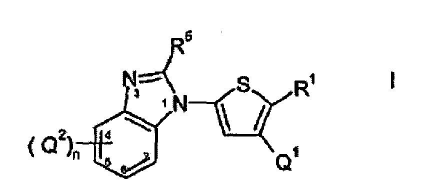 R¹ et choisi dans le groupe constitué par H, un alkyle, un alcényle, un alcynyle, -C(O)R⁷, -CO₂R⁷, -C(O)NR⁷R⁸, -C(O)N(R⁷)OR⁸, -C(O)N(R⁷)-R²-OR⁸, -C(O)N(R⁷)-Ph, -C(O)N(R⁷)-R²-Ph, -C(O)N(R⁷)C(O)R⁸, -C(O)N(R⁷)CO₂R⁸, -C(O)N(R⁷)C(O)NR⁷R^{B}, -C(O)N(R⁷)S(O)₂R⁸, -R²-OR⁷, -R²-OC(O)R⁷ -C(S)R⁷, -C(S)NR⁷R⁸, -C(S)N(R⁷)-Ph, -C(S)N(R⁷)-R²-Ph, -R²-SR⁷, -C (=NR⁷)NR⁷R⁸, -C (=NR⁷)N(R⁸)-Ph, -C(=NR⁷)N(R⁸)-R²-Ph, -R²-NR⁷R⁸, -CN, -OR⁷, -NR⁷R⁸, N(R⁷) -Ph, -N (R⁷) -R²-Ph, -N(R⁷) -SO₂R⁸ et Hét ;
Ph est un phényle facultativement substitué 1 à 3 fois avec un substituant choisi dans le groupe constitué par un halogène, un alkyle, -OH, -R²-OH, -O-alkyle, -R²-O-alkyle, -NH₂, -N(H)alkyle, -N(alkyle)₂, -CN et -N₃ ;
Hét est un hétérocycle à 5 à 7 chaînons contenant 1, 2, 3 ou 4 hétéroatomes choisis parmi N, O et S, ou un hétéroaryle à 5 à 6 chaînons contenant 1, 2, 3 ou 4 hétéroatomes choisis parmi N, O et S, chacun étant facultativement substitué 1 à 2 fois avec un substituant choisi dans le groupe constitué par un halogène, un alkyle, un oxo, -OH, -R²-OH, -O-alkyle, -R²-O-alkyle, -NH₂, -N(H)alkyle, -N(alkyle)₂, -CN et -N₃ ;
Q¹ est un groupe de formule: -(R²)ₐ-(Y¹)_{b}-(R²)_{c}-R³
a, b et c sont identiques ou différents et sont chacun indépendamment 0 ou 1 et au moins un parmi a ou b est 1 ;
n est 0, 1, 2 ou 3 ;
chaque Q² est identique ou différent et est un groupe de formule: -(R²)ₐₐ-(Y²)_{bb}-(R²)_{cc}-R⁴
ou deux groupes Q² adjacents sont choisis dans le groupe constitué par un alkyle, un alcényle, -OR⁷, -S(O)_{f}R⁷ et -NR⁷R⁸ et ensemble avec les atomes de carbone auxquels ils sont liés, ils forment un cycloalkyle en C₅ à C₆, un cycloalcényle en C₅ à C₆, un phényle, un hétérocycle à 5 à 7 chaînons contenant 1 ou 2 hétéroatomes choisis parmi N, O et S, ou un hétéroaryle à 5 à 6 chaînons contenant 1 ou 2 hétéroatomes choisis parmi N, O et S ;
aa, bb et cc sont identiques ou différents et sont chacun indépendamment 0 ou 1 ;
Y¹ et Y² sont chacun identiques ou différents et sont indépendamment choisis dans le groupe constitué par -O-, -S(O)ᵣ, -N(R⁷)-, -C(O)-, -OC(O)-, -CO₂-, -C(O)N(R⁷) -, -C(O)N(R⁷)S(O)₂-, -OC(O)N(R⁷)-, -OS(O)₂-, -S(O)₂N(R⁷)-, -S(O)₂N(R⁷)C(O)-, -N(R⁷)S(O)₂-, -N(R⁷)C(O)-, -N(R⁷)CO₂- et -N(R⁷)C(O)N(R⁷)- ;
chaque R² est identique ou différent et est indépendamment choisi dans le groupe constitué par un alkylène, un alcénylène et un alcynylène ;
R³ et R⁴ sont chacun identiques ou différents et sont chacun indépendamment choisis dans le groupe constitué par H, un halogène, un alkyle, un alcényle, un alcynyle, -C(O)R⁷ -C(O)NR⁷R⁸, -CO₂R⁷, -C(S)R⁷, -C(S)NR⁷R⁸, - C(=NR⁷)R⁸ -C(=NR⁷)NR⁷R⁸, -CR⁷=N-OR⁷, -OR⁷, -S(O)_{f}R⁷-S(O)₂NR⁷R⁸, -NR⁷R^{B}, -N(R⁷)C(O)R⁸, -N(R⁷)S(O)₂R⁸, -NO₂, -CN, -N₃ et un groupe de formule (ii) : 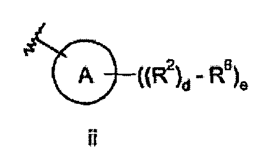 dans laquelle :
le cycle A est choisi dans le groupe constitué par un cycloalkyle en C₅ à C₁₀, un cycloalcényle en C₅ à C₁₀, un aryle, un hétérocycle à 5 à 10 chaînons contenant 1, 2 ou 3 hétéroatomes choisis parmi N, O et S, et un hétéroaryle à 5 à 10 chaînons contenant 1, 2 ou 3 hétéroatomes choisis parmi N, O et S ;
chaque d est 0 ou 1 ;
chaque e est 0, 1, 2, 3 ou 4 ;
chaque R⁶ est identique ou différent et est indépendamment choisi dans le groupe constitué par H, un halogène, un alkyle, un alcényle, un alcynyle, un cycloalkyle, un cycloalcényle, Ph, Hét, -CH(OH)-R²-OH, -C(O)R⁷, -CO₂R⁷, -CO₂-R₂-Ph, -CO₂-R₂-Hét, -C(O)NR⁷R⁸, -C(O)N(R⁷)C(O)R⁷, -C(O)N(R⁷)CO₂R⁷, -C(O)N(R⁷)C(O)NR⁷R⁸, -C(O)N(R⁷)S(O)₂R⁷, -C(S)R⁷, -C(S)NR⁷R⁸, -C(=NR⁷)R⁸, -C (=NR⁷)NR⁷R⁸, -CR⁷=N-OR⁸, =O, -OR⁷, -OC(O)R⁷, -OC(O)Ph, -OC (O) Hét, -OC(O)NR⁷R⁸, -O-R²-S(O)₂R⁷, -S(O)_{f}R⁷, -S(O)₂NR⁷R⁸, -S(O)₂Ph, -S(O)₂Hét, -NR⁷R⁸, -N(R⁷)C(O)R⁸, -N(R⁷)CO₂R⁸, -N(R⁷)-R²-CO₂R⁸, -N(R⁷)C(O)NR⁷R⁸, -N(R⁷)-R²-C(O)NR⁷R⁸, -N(R⁷)C(O)Ph, -N(R⁷)C(O)Hét, -N(R⁷)Ph, -N(R⁷)Hét, -N(R⁷)C(O)NR⁷-R²-NR⁷R⁸, -N(R⁷)C(O)N(R⁷)Ph, -N(R⁷)C(O)N(R⁷)Hét, -N(R⁷)C(O)N(R⁷)-R²-Hét, -N(R⁷)S(O)₂R⁸, -N(R⁷)-R²-S(O)₂R⁸, -NO₂, -CN et -N₃ ;
dans laquelle quand Q¹ est défini avec b = 1 et c = 0, R³ n'est pas un halogène, -C(O)R⁷, -C(O)NR⁷R⁸, -CO₂R⁷, -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN ou -N₃ ;
dans laquelle quand Q² est défini avec bb = 1 et cc = 0, R⁴ n'est pas un halogène, -C(O)R⁷, -C(O)NR⁷R⁸, -CO₂R⁷ -C(S)R⁷, -C(S)NR⁷R⁸, -C(=NR⁷)R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN ou -N₃ ;
R⁵ est H ;
f est 0, 1 ou 2 ; et
chaque R⁷ et chaque R⁸ sont identiques ou différents et sont chacun indépendamment choisis dans le groupe constitué par H, un alkyle, un alcényle, un alcynyle, un cycloalkyle et un cycloalcényle ;
ou de l'un de ses sels pharmaceutiquement acceptables ;
ledit procédé comprenant la cyclisation d'un composé de formule (VIII) : 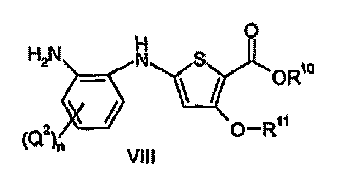 dans laquelle
R¹⁰ est choisi parmi un alkyle, un alcényle, un alcynyle, un cycloalkyle, un cycloalcényle et des groupes protecteurs d'acide carboxylique appropriés ;
R¹¹ est H ou un groupe protecteur triisopropylsilyle ;
et facultativement l'élimination du groupe protecteur triisopropylsilyle pour préparer un composé de formule (I-A) : 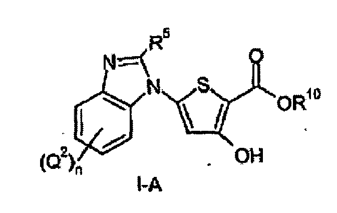 où
alkyle (et alkylène) font référence à des chaînes hydrocarbonées linéaires ou ramifiées contenant 1 à 8 atomes de carbone facultativement substitués une ou plusieurs fois avec un halogène,
dans laquelle quand Q² est défini avec bb = 1 et cc = 0, R⁴ n'est pas un halogène, -C(O)R⁷, -C(O)NR⁷R⁸, -CO₂R⁷, -C(S)R⁷, -C(S)NR⁷R⁸, -C(=NR⁷) R⁸, -C(=NR⁷)NR⁷R⁸, -CR⁷=N-OR⁷, -OR⁷, -S(O)_{f}R⁷, -S(O)₂NR⁷R⁸, -NR⁷R⁸, -N(R⁷)C(O)R⁸, -N(R⁷)S(O)₂R⁸, -NO₂, -CN ou -N₃ ;
R⁵ est H ;
f est 0, 1 ou 2 ; et
chaque R⁷ et chaque R⁸ sont identiques ou différents et sont chacun indépendamment choisis dans le groupe constitué par H, un alkyle, un alcényle, un alcynyle, un cycloalkyle et un cycloalcényle ;
ou l'un de ses sels ou solvates pharmaceutiquement acceptables.
Selon le premier aspect, la présente invention propose un procédé de préparation de composés de formule (I) ci-dessus ou de l'un de ses sels ou solvates pharmaceutiquement acceptables, comprenant les étapes suivantes :
a) la cyclisation d'un composé de formule (VIII) : 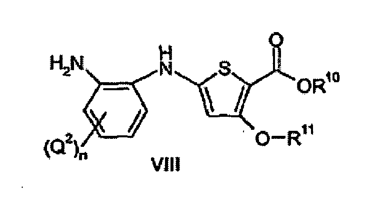 dans laquelle
R¹⁰ est choisi parmi un alkyle, un alcényle, un alcynyle, un cycloalkyle, un cycloalcényle et des groupes protecteurs d'acide carboxylique appropriés ; et
R¹¹ est H ou un groupe protecteur triisopropylsilyle ;
et facultativement l'élimination du groupe protecteur triisopropylsilyle pour préparer un composé de formule (I-A) : 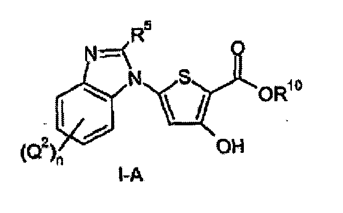
b) facultativement la conversion du composé de formule (I-A) en l'un de ses sels ou solvates pharmaceutiquement acceptables ; et
les éléments et contenant 1, 2, 3 ou 4 hétéroatomes choisis parmi N, O et S (sauf si un nombre différent d'hétéroatomes est spécifié).

2. Procédé de préparation d'un composé de formule (VIII) : 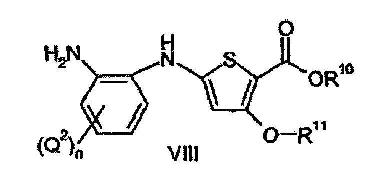 dans laquelle :
n, Q², R¹¹ et R¹⁰ sont tels que définis dans la revendication 1,
ou de l'un de ses sels pharmaceutiquement acceptable :
comprenant la réduction d'un composé de formule (VII) : 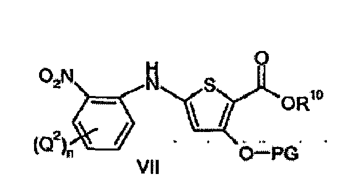 dans laquelle PG est un groupe protecteur triisopropylsilyle.

3. Procédé de préparation d'un composé de formule (VII) : 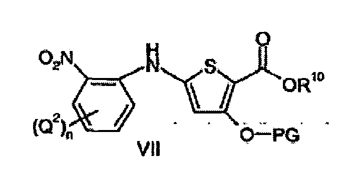 dans laquelle :
n, Q² et R¹⁰ sont tels que définis dans la revendication 1, et
PG est un groupe protecteur triisopropylsilyle ;
ou de l'un de ses sels pharmaceutiquement acceptables ;
comprenant la réaction d'un composé de formule (V) avec un composé de formule (VI)

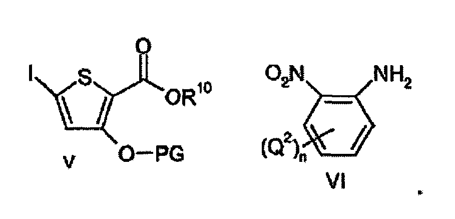

4. Procédé selon la revendication 1, pour la préparation d'un composé de formule (I) : 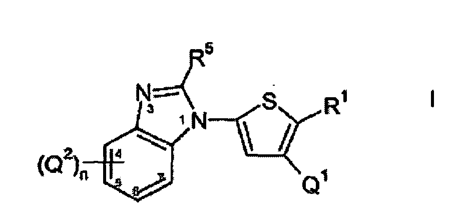 dans laquelle :
R¹, Q¹, R⁵ sont tels que définis dans la revendication 1, et
n est 1, 2 ou 3;
chaque Q² est identique ou différent et est indépendamment un groupe de formule: -(R²)ₐₐ-(Y²)_{bb}-(R²)_{cc}-R⁴
dans laquelle R², Y², R² et R⁴ sont tels que définis dans la revendication 1 ;
ou de l'un de ses sels pharmaceutiquement acceptables ;
ledit procédé comprenant la cyclisation d'un composé de formule (XXIII) : 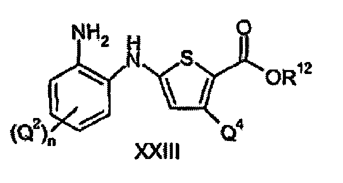 dans laquelle :
Q⁴ est un groupe de formule -O-(R²)_{c}-R^{3a}, -O-Si(alkyle)₃ ou -O-(R²)_{c}-Si(alkyle)₃ ;
dans lesquelles c est 1 ; et
R^{3a} est un groupe de formule (iii) : 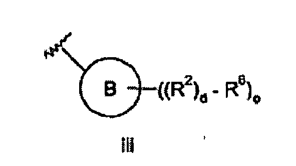 dans laquelle le cycle B est un phényle ou un hétéroaryle à 5 à 6 chaînons contenant 1, 2 ou 3 hétéroatomes choisis parmi N, O et S ; et
R¹² est un méthyle ;
pour préparer un composé de formule (I-G) :

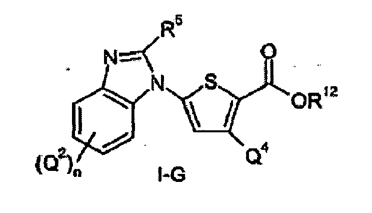

5. Composé de formule (XXIII) : 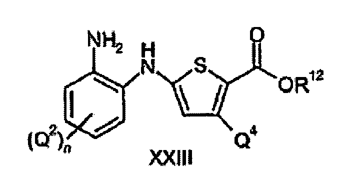 dans laquelle
n et Q² et Q⁴ et R¹² sont tels que définis dans la revendication 4,
ou l'un de ses sels pharmaceutiquement acceptables.

6. Procédé de préparation d'un composé selon la revendication 5, comprenant la réduction d'un composé de formule (XXII) :

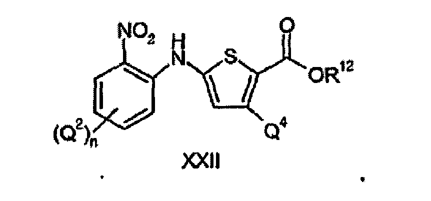

7. Composé de formule (XXII) : 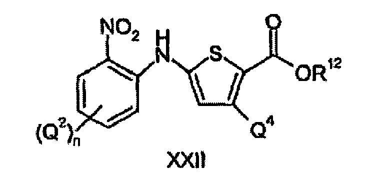 dans laquelle
n, Q², Q⁴, R¹² sont tels que définis dans la revendication 4.

8. Procédé de préparation d'un composé selon la revendication 7, comprenant la réaction d'un composé de formule (XX) avec un composé de formule (XXI) : 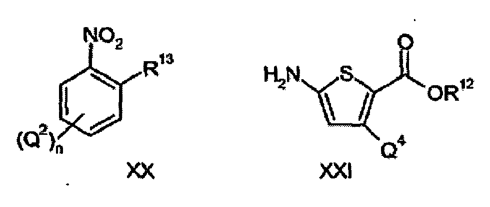 où R¹³ est un halogénure ou un O-triflate.

9. Procédé selon la revendication 4, dans lequel ladite étape de cyclisation d'un composé de formule (XXIII) comprend la cyclisation d'un composé de formule (XXIII-A) : 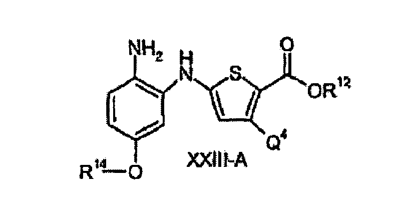 dans laquelle R¹⁴ est un groupe protecteur de phénol approprié.

10. Procédé selon la revendication 4, dans lequel ladite étape de cyclisation d'un composé de formule (XXIII) comprend la cyclisation d'un composé de formule (XXIII-B) : 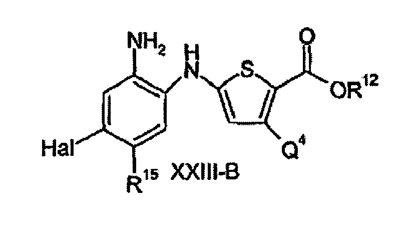 dans laquelle Hal est Cl, Br ou I ; et
R¹⁵ est H ou -O-R¹⁴.

11. Procédé selon la revendication 4, dans lequel ladite étape de cyclisation d'un composé de formule (XXIII) comprend la cyclisation d'un composé de formule (XXXII) :
